(19) Europäisches Patentamt
European Patent Office
Office européen des brevets

(11) EP 4 043 041 A1

(12) EUROPEAN PATENT APPLICATION

(43) Date of publication:
**17.08.2022 Bulletin 2022/33**

(21) Application number: **21157225.0**

(22) Date of filing: **15.02.2021**

(51) International Patent Classification (IPC):
***A61K 51/08*** (2006.01) ***A61P 35/00*** (2006.01)
***A61K 101/02*** (2006.01) ***A61K 103/00*** (2006.01)
***A61K 103/30*** (2006.01)

(52) Cooperative Patent Classification (CPC):
**A61K 51/08; A61K 51/088; A61P 35/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Technische Universität München**
**80333 München (DE)**

(72) Inventors:
- **Konrad, Matthias**
  **80939 München (DE)**
- **Schottelius, Margret**
  **1005 Lausanne (CH)**
- **Wester, Hans-Jürgen**
  **85301 Schweitenkirchen (DE)**

(74) Representative: **Vossius & Partner**
**Patentanwälte Rechtsanwälte mbB**
**Siebertstrasse 3**
**81675 München (DE)**

# (54) CXCR4-LIGANDS FOR DIAGNOSTIC AND THERAPEUTIC USE AND PRECURSORS THEREOF

(57) The invention provides a CXCR4 receptor ligand compound of formula (I) or a salt thereof:

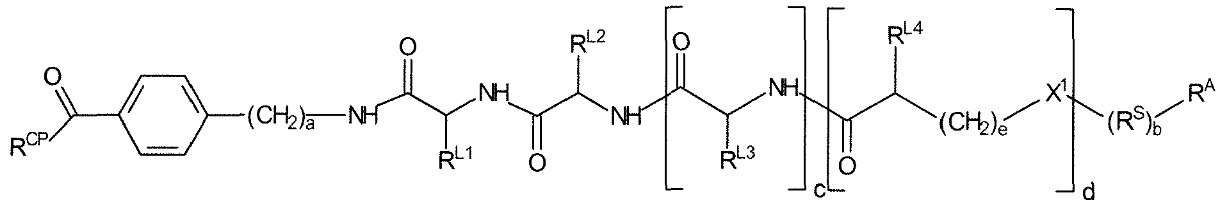

(I)

wherein:
a is 0 or 1; b is 0 or 1; c is 0 or 1, and d is 0 or 1, with the proviso that at least one of c and d is 1; e is an integer of 1 to 4;
$R^{CP}$ is a binding motif which allows the compound to bind to the CXCR4 receptor;
$R^{L1}$ is H or alkyl;
$R^{L2}$ is substituted alkyl, which substituted alkyl is substituted with at least one group selected from $-NH_2$ and $-NH-C(=X)-NH_2$ with X being selected from NH and O;
$R^{L3}$ is $-CH_2-NH_2$ or $-CH_2$-(1H-imidazol-4-yl);
$R^{L4}$ is $-NH_2$;
$X^1$ is a coupling group;
$R^S$ is a divalent spacer group; and
$R^A$ is a functional group comprising a moiety with diagnostic or therapeutic utility.
The compounds of the invention are suitable for use in the treatment, prevention, and/or diagnosis of a disease or disorder which can be treated or prevented by blocking the CXCR4 receptor, or which is associated with an increased or aberrant expression of the CXCR4 receptor.

EP 4 043 041 A1

## Description

The present invention relates to compounds that are capable of binding to the seven transmembrane G-protein coupled chemokine receptor subtype (CXCR4) with high affinity and are thus considered CXCR4 ligands. They preferably act as agonists, or may also act as antagonists, inverse or partial agonists. The compounds are suitable for use in diagnostic and therapeutic applications.

The binding of stromal cell-derived factor 1 (SDF-1) (now referred to as C-X-C motif chemokine 12 (CXCL12)) to CXCR4 *(1)* activates the downstream protein kinase B (AKT)/mitogen-activated protein kinases (MAPK) signaling pathway, which leads to the alteration of gene expression, actin polymerization, cell skeleton rearrangement and cell migration. The physiological functions of the CXCL12/CXCR4 axis include embryogenesis, immune response, hematopoiesis, brain development and neo-angiogenesis *(2-6)*. Besides its fundamental involvement in physiological processes, elevated CXCR4 expression is associated with diverse malignancies. It mediates HIV-1 entry into T-cells as a co-receptor where it was first identified *(3)*. CXCR4 is involved in B-cell trafficking and tissue localization in chronic leukemia patients *(7)* as well as the regulation of organ specific metastasis in different breast cancer models *(8)*. Hence, CXCR4 overexpression is known in more than 20 human tumor types, including hematopoietic malignancies, brain neoplasm, gastrointestinal cancer and other cancer types *(2, 8-10)*. Increasing evidence indicates that the CXCL12/CXCR4 axis functions as a critical communication bridge between tumor cells and stromal cells to create a permissive microenvironment *(11)*. Cytokine CXCL12 and its receptor CXCR4 therefore represent a promising actionable target for therapeutic strategies, since the aberrant expression of CXCR4 strongly promotes proliferation, migration and invasion of different cancer types *(12)*.

Several peptidic and non-peptidic CXCR4 antagonists that target the CXCL12/CXCR4 axis have been developed, due to their potential use for medicinal applications. The most established example is the bicyclam AMD3100 (Plerixafor®, Tetrazacyclotetradec-1-ylmethyl)phenyl]methyl}-1,4,8,11-tetrazacyclo-tetradecan) that has been approved by the FDA for the treatment of non-Hodgkin's lymphoma and multiple myeloma. Further peptidic CXCR4 antagonists have been developed, e.g. T140 and its derivatives which are side-chain cyclized peptides that contain one or two cyclization sites *(13-15)*. A less cytotoxic and biologically stable derivative of T140 is TN14003 *(16)*. Introduction of a 4-fluorobenzoyl group constituted a novel pharmacophore for T140-based CXCR4 antagonists, TF14016, with subnanomolar binding affinity *(16)*. This peptide CXCR4 antagonist was further employed in $^{18}$F-or $^{68}$Ga based positron emission tomography (PET) imaging of CXCR4 expression *in vivo (17-19)*. T140-based CXCR4 antagonists are already used for the prevention and/or therapy of cancers and chronic rheumatoid arthritis (US 8410059 B2, US 8765683 B2).

LY2510924 (cyclo[Phe-Tyr-Lys(iPr)-D-Arg-2-Nal-Gly-D-Glu]-Lys(iPr)-$NH_2$), a potent CXCR4 antagonist was demonstrated to exhibit good antitumor activities in solid tumor and breast cancer metastatic models and is currently in phase II clinical studies (NCT01391130 and NCT1439568) *(20)*.

Moreover, three cyclic pentapeptides (peptide R, I and S) that are based on the *N*-terminal sequence of CXCL12 significantly inhibit subcutaneous growth of renal cancer cells. They also effect lung metastases and primary tumor growth *(21)*. In addition, lactam-cyclized heptapeptides were reported to be potent CXCR4 antagonists useful in the treatment of cancers, rheumatoid arthritis, pulmonary fibrosis, and HIV infection (WO 2008/150689 A1).

T140-derived, cyclic pentapeptides based on *cyclo*(D-Tyr$^1$-Arg$^2$-Arg$^3$-Nal$^4$-Gly$^5$) (from now on referred to as Fc-131) are used for therapy of cancer and anti-inflammation (22). SAR studies including alanine scanning, *N*-methyl amino acid scanning, optimization of amino acid residues and design of retro-inverso sequence peptides all failed to improve the binding affinity or anti-HIV activity compared to Fc-131 *(23-25)* and demonstrated the highly optimized binding scaffold of Fc-131. However, the introduction of amidine type dipeptide equivalents resulted in new lead structures of cyclic pentapeptides addressing CXCR4 (WO 2012/118124 A1). In addition, *N*-methylation of the peptide bonds of Fc-131 significantly affected its activity, resulting in the cyclic pentapeptide-based CXCR4 antagonist Fc-122 that shows a significant enhancement of CXCR4 antagonistic activity *(26-28)*.

Within the scope of the development of molecular imaging probes for CXCR4, the *N*-methylation approach was employed to enhance binding affinity, while all side chains of Fc-131 were tested for their feasibility of exchange. The substitution of Arg$^2$ with D-Ornithine and subsequent methylation of the *N*-terminus yielded CPCR4 (*cyclo*(D-Tyr$^1$-D-[NMe]Orn$^2$-Arg$^3$-Nal$^4$-Gly$^5$)), which exhibits good binding affinity towards CXCR4. This lead structure served as an anchor point for further modifications *(29)*, (WO 2007/096662). Dimeric derivatives of CPCR4 have been described, although the application for in vivo diagnostics was prevented by elevated accumulation of the CXCR4 ligands in the liver (WO 2009/027706) *(29, 30)*.

Recently, a minimalistic approach was employed in which the Technetium chelator hydrazino-nicotinic acid was attached directly at the D-Orn$^2$ side chain, resulting in a CXCR4 SPECT imaging agent *(31)*. This compound is currently under examination in a first proof of concept study in men *(32)*.

Nevertheless, further structural modifications, e.g. the introduction of an aromatic spacer attached to the side chain of [*N*Me]Orn$^2$ of *cyclo*(D-Tyr$^1$-D-[NMe]Orn$^2$-Arg$^3$-Nal$^4$-Gly$^5$) facilitated the introduction of 1,4,7,10-tetraazacyclododecane-1,4,7,10-tetraacetic acid (DOTA) as a labeling moiety (WO 2011/131735) for medicinal applications (33-35).

In the majority of the cases, additional structural changes on the detectable label *(36)* resulted in severe losses of binding affinity. An additional modification (introduction of (3-iodo)tyrosine) in the cyclic pentapeptide scaffold of *cyclo*(D-Tyr[1]-D-[*N*Me](DOTA)-Orn[2]Arg[3]-Nal[4]-Gly[5]) (pentixafor) was described, which significantly increased the binding affinity towards hCXCR4. Consequently, the resulting $^{177}$Lu- and $^{90}$Y-pentixather was utilized in therapeutic approaches of CXCR4 associated malignancies (WO 2015/185162) *(37).*

Building on the success of these clinically applied peptides, further optimization was undertaken to enhance affinity and versatility of the peptide scope. Replacement of the short aromatic spacer with a tailormade peptidic linker unit ultimately resulted in a CXCR4 binding motif with enhanced affinity and significantly increased internalization rates due to newly induced semi-agonistic properties of the peptide scaffold *(38).* These modifications allowed the introduction of a variety of functional modalities such as $mas_3$-based technetium chelators or $AmBF_3$ as a $^{18}$F-labeling unit without greater impairment of the affinity of the resulting peptides (WO 2020/053255).

The diagnostic and therapeutic potential of CXCR4 ligands has been shown in many different cases e.g. for the treatment of HIV infection and cancer, or for the visualization of CXCR4 expression in patients. Especially, the cyclic pentapeptides are optimized for the perfect interaction of the compounds in the binding cavity of CXCR4 and small modifications, e.g. the introduction of labeling moieties or residues to influence the pharmacokinetics of the CXCR4 ligands, result in considerably high affinity losses *(36)* and therefore have to be structurally optimized for each desired application. Consequently, the design and development of novel CXCR4 targeting compounds requires elaborated structure-activity-relationship studies (SAR studies).

For this reason, there is a need for an universally applicable ligand design which ensures high affinity towards the human CXCR4 receptor while allowing the linkage of a broad variety of functional groups with diagnostic or therapeutic utility. The present invention provides such novel ligand compounds and their uses in medical and scientific applications. The compounds of the invention are capable of binding the human CXCR4 receptor with high affinity and the murine receptor with moderate affinity and, hence, are suitable as CXCR4 ligands. These ligands may function as agonists, inverse agonists, partial agonists or antagonists. The structure of the linker in the ligand compounds provided by the invention surprisingly results in high flexibility of the compounds towards the attachment of various functional moieties while preserving or even enhancing CXCR4 affinity. Moreover, based on favorable *in vitro* characteristics like higher affinity and boosted internalization, higher and more consistent tumor uptake is often reached. The compounds of the invention are thus particularly suitable for medical applications such as preclinical and clinical imaging, and therapeutic applications, such as endoradiotherapy.

Before the background described above, the present invention provides a compound of formula (I) or a salt thereof, which is a C-X-C chemokine receptor type 4 ligand compound, or briefly a CXCR4 receptor ligand compound:

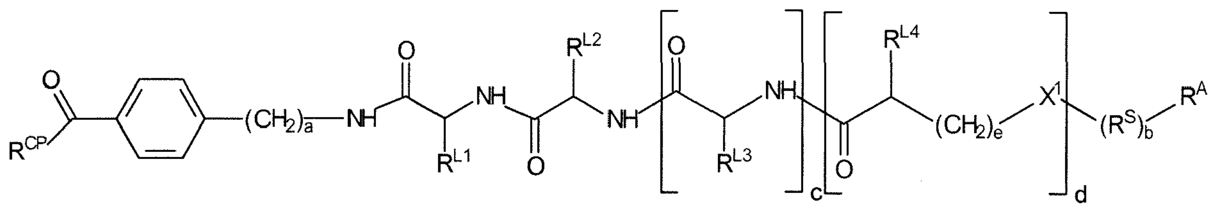

(I)

wherein:

| | |
|---|---|
| a | is 0 or 1, preferably 0; |
| b | is 0 or 1; |
| c | is 0 or 1, and |
| d | is 0 or 1, with the proviso that at least one of c and d is 1; |
| e | is an integer of 1 to 4, preferably 2 to 4; |
| $R^{CP}$ | is a cyclopeptide group of formula (II): |

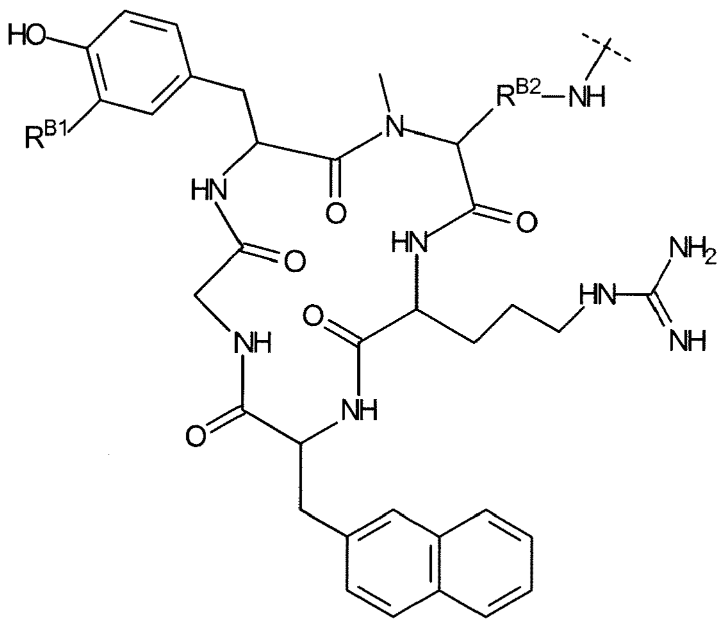

(II)

wherein, in formula (II)
$R^{B1}$ is H or I, preferably H;
$R^{B2}$ is an alkanediyl chain;
and wherein the dashed line marks a bond which attaches the group $R^{CP}$ to the remainder of the compound of formula (I);

$R^{L1}$ is H or alkyl;
$R^{L2}$ is substituted alkyl, which substituted alkyl is substituted with at least one group selected from $-NH_2$ and $-NH-C(=X)-NH_2$ with X being selected from NH and O;
$R^{L3}$ is $-CH_2-NH_2$ or $-CH_2-$(1*H*-imidazol-4-yl);
$R^{L4}$ is $-NH_2$;
$X^1$ is a coupling group;
$R^S$ is a divalent spacer group; and
$R^A$ is a functional group comprising a moiety with diagnostic or therapeutic utility.

In the CXCR4 receptor ligand compounds of the present invention, the binding motif $R^{CP}$ and the functional group $R^A$ are linked by a linker of the following structure.

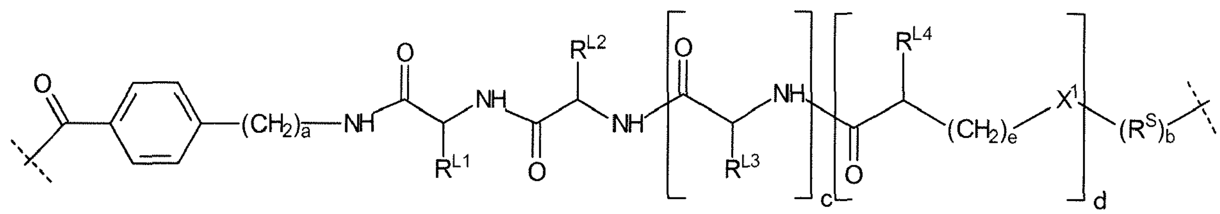

This linker forming part of the compounds of formula (I) is characterized by the presence of a substituent with a small size and a functional group that typically carries a positive charge under physiological conditions, provided as $R^{L3}$, $R^{L4}$ or as $R^{L3}$ and $R^{L4}$. In the context of the invention, it has been found that this linker structure allows, amongst other beneficial properties, a high CXCR4 affinity to be retained for the compounds of the present invention with no or only little detrimental influence of the functional group $R^4$. Thus, relying on the combination of the binding motif and the linker contained in the compounds in accordance with the invention, CXCR4 receptor ligands with a broad variety of functional groups can be provided without concerns about a significant loss of the affinity provided by the binding motif.

In accordance with further related aspects, the invention provides a therapeutic or a diagnostic composition comprising a CXCR4 receptor ligand compound of formula (I) or a salt thereof.

As noted above, salts, typically pharmaceutically acceptable salts, of the compounds of formula (I) are encompassed by the present invention. Thus, unless indicated to the contrary, any reference to a compound in accordance with the invention herein encompasses both the compound of formula (I) and the preferred embodiments of this formula disclosed herein, and the salts thereof. Moreover, any racemates, enantiomers, or diastereomers of the compounds of formula (I) are encompassed, unless a specific stereochemistry of the compound under consideration is indicated in a specific context.

As illustrated by the reference to the compounds of formula (I) as CXCR4 receptor ligand compounds, the compounds are capable of acting as ligand compounds which bind to the CXCR4 receptor. To that extent, the compounds of formula (I) and their salts comprise a cyclopeptide group $R^{CP}$, which may also be referred to herein as the "binding motif" of the CXCR4 receptor ligand compounds of formula (I), since it ensures a binding interaction between the compounds in accordance with the invention or their salts and the CXCR4 receptor and thus serves as an affinity anchor for the compounds towards the CXCR4 receptor.

A binding motif of a CXCR4 receptor ligand compound is preferably capable of specifically binding to the CXCR4 receptor. In this connection, specifically binding preferably means that the binding motif of the CXCR4 receptor ligand compound does not, or essentially does not, bind to other proteins than the CXCR4 receptor, in particular does not bind, or essentially does not bind, to other members of the CXC chemokine receptor family. The term "essentially does not bind" preferably means that the binding affinity of the binding motif, as determined e.g. by an $IC_{50}$ value, to CXCR4 is at least 100, preferably at least 1000 and most preferably at least 10000 times stronger than the binding affinity to other proteins, in particular other members of the CXC chemokine receptor family.

The cyclopeptide group $R^{CP}$ as a binding motif in formula (I) is a group of the formula (II):

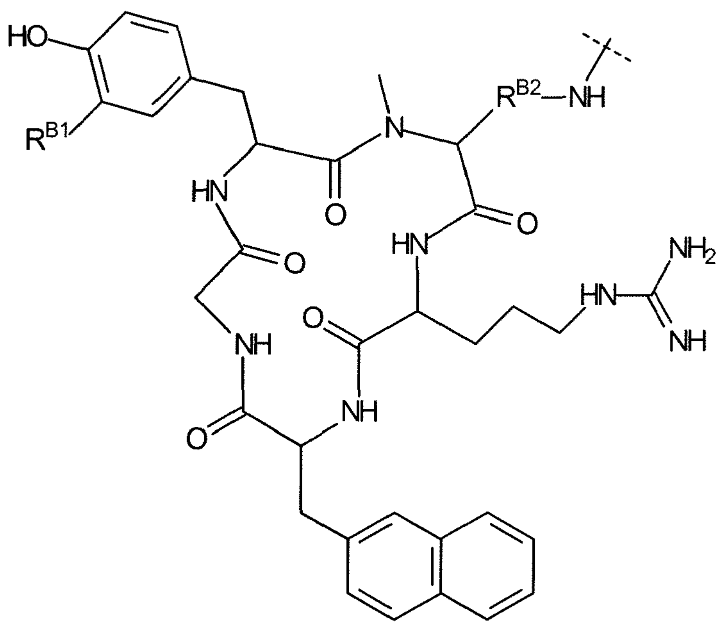

(II)

wherein

$R^{B1}$ is H or I, preferably H; and
$R^{B2}$ is an alkanediyl chain.

As will be understood by the skilled reader, the bond marked by the dashed line in formula (II) does not carry a methyl group at its end opposite to the nitrogen atom, but represents a bond which attaches the group $R^{CP}$ to the remainder of the compound of formula (I), i.e. in this case to the point of attachment of $R^{CP}$ in formula (I). In other words, the bond marked by the dashed line in formula (II) represents a covalent bond which is present in the compounds in accordance with the invention between the nitrogen atom of the -NH- group indicated in formula (II) and the carbon atom of the carbonyl group shown at the left side of formula (I) to which $R^{CP}$ is attached. Thus, an amide bond is provided using the -NH- group of $R^{CP}$ shown in formula (II), and the carbonyl group shown in formula (I).

In the group of formula (II), $-R^{B2}-$ is an alkanediyl chain, preferably a C2-C6 alkanediyl chain, more preferably a C2-C3 alkanediyl chain, and most preferably $-CH_2-CH_2-CH_2-$. Thus, $R^{CP}$ is preferably a group of formula (IIa):

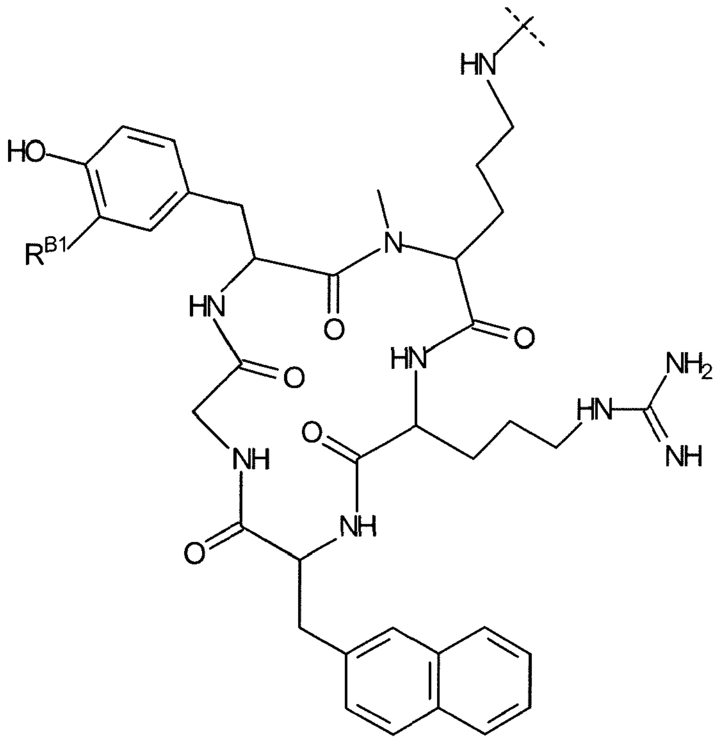

(IIa),

wherein $R^{B1}$ is defined as for formula (II), including any preferred embodiments of this variable as further defined herein, and wherein the dashed line marks a bond which attaches the group $R^{CP}$ to the remainder of the compound of formula (I).

It is still further preferred that $R^{CP}$ is a group of formula (IIb):

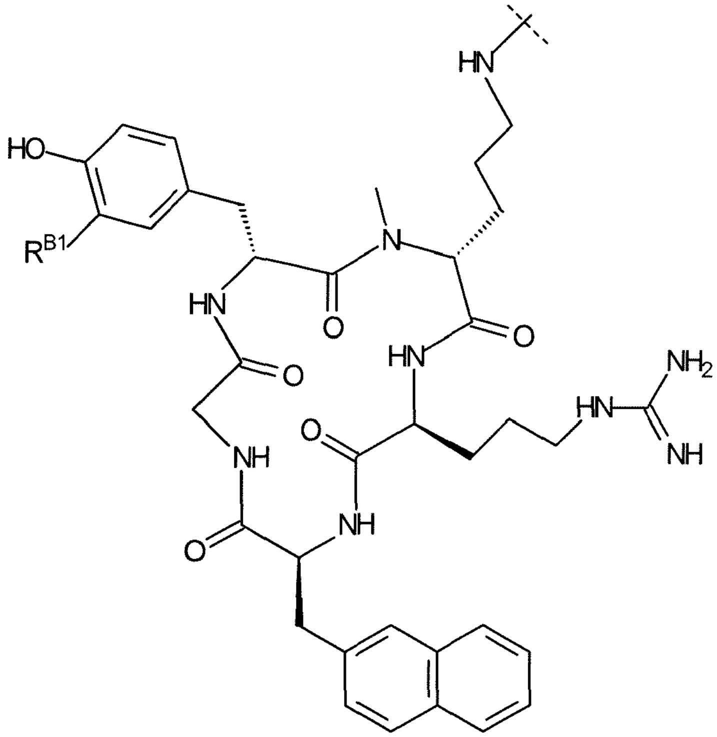

(IIb),

wherein $R^{B1}$ is defined as for formula (II), including any preferred embodiments of this variable as further defined herein, and wherein the dashed line marks a bond which attaches the group $R^{CP}$ to the remainder of the compound of formula (I).

The variable a in formula (I) is either 0 or 1, such that the group $-CH_2-$ attached to the phenylene ring in the linker structure of formula (I) is an optional group which can be present or absent. As will be understood, if it is absent, i.e. if a is 0, the group $-CH_2-$ is replaced by a direct bond between the atoms adjacent to the group in formula (I).

The group $R^{L1}$ in formula (I) is H or C1-C6 alkyl, more preferably H or C1-3 alkyl, still more preferably H or methyl, and most preferably methyl.

$R^{L2}$ in formula (I) is substituted alkyl, which substituted alkyl is substituted with at least one group selected from $-NH_2$ and $-NH-C(=X)-NH_2$. X is selected from NH and O. The alkyl moiety of the substituted alkyl is preferably C1-C6 alkyl, more preferably C1-C4 alkyl, and still more preferably C2-C4 alkyl. The alkyl moiety is preferably a linear alkyl moiety. The alkyl moiety carries at least one, preferably exactly one, substituent which is selected from $-NH_2$ and the group $-NH-C(=X)-NH_2$. X is preferably NH, in which case the group $-NH-C(=X)-NH_2$ is a guanidino group. In line with the above, $R^{L2}$ is preferably a group selected from $-(CH_2)_A-NH_2$ and $-(CH_2)_A-NH-C(=NH)-NH_2$, wherein A is an integer of 1 to 6, preferably 1 to 4, and more preferably 2 to 4. Most preferably, $R^{L2}$ is $-(CH_2)_3-NH-C(=NH)-NH_2$.

$R^{L3}$ in formula (I) and its preferred embodiments as defined herein is $-CH_2-NH_2$ or $-CH_2-$(1*H*-imidazol-4-yl) and is preferably $-CH_2-NH_2$. The group $-CH_2-$(1*H*-imidazol-4-yl) can be illustrated by the following formula, wherein the dashed line marks a bond which attaches the group to the remainder of the compound of formula (I):

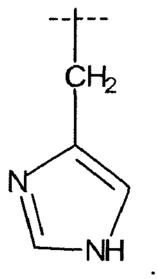

$R^{L4}$ in formula (I) and its preferred embodiments as defined herein is $-NH_2$.

The variable e in formula (I) and its preferred embodiments as defined herein is an integer of 1 to 4, preferably 2 to 4, and is more preferably 2.

$X^1$ in formula (I) is a coupling group. As will be understood by the skilled reader, the coupling group $X^1$ is a functional group which allows $R^S$ or $R^A$ to be coupled to the remainder of the compound of formula (I) via a covalent bond which is formed between the group $X^1$ and $R^S$ or between the group $X^1$ and $R^A$. The coupling group may consist of one or more atoms. A preferred coupling group $X^1$ is selected from -NH-, -C(O)-, -O-, and -S-. Typically, the coupling group $X^1$ is covalently linked to a further, complementary coupling group comprised in $R^S$ or $R^A$, so that the two coupling groups combine to form a binding unit, such as an amide bond -C(O)-NH-, an ester bond -C(O)-O-, or a thiosuccinimidyl group which can be illustrated by the following formula, wherein each dashed line marks a bond which attaches the group to an adjacent atom or group within the compound of formula (I).

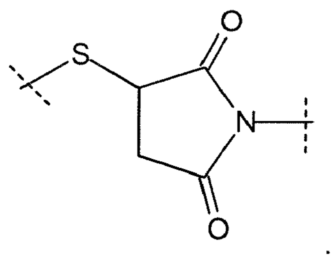

For example, a coupling group $X^1$ = -NH- may form an amide bond -NH-C(O)- with a complementary group -C(O)- comprised in $R^S$ or $R^A$; a coupling group $X^1$ = -C(O)- may form an amide bond -C(O)-NH- with a complementary group -NH- comprised in $R^S$ or $R^A$, or may form an ester bond -C(O)-O- with a complementary group -O- comprised in $R^S$ or $R^A$; a coupling group $X^1$ = -O- may form an ester bond -O-C(O)- with a complementary group - C(O)- comprised in $R^S$ or $R^A$, or may form an ether bond -O- with a carbon atom comprised in $R^S$ or $R^A$; a coupling group $X^1$ = -S- may form a thioester bond -S-C(O)- with a complementary group -C(O)- comprised in $R^S$ or $R^A$, or may form a thioether bond -S- with a carbon atom comprised in $R^S$ or $R^A$. In accordance with a preferred embodiment, $X^1$ is the sulfur atom -S- and forms a covalent bond with a complementary succinimidyl group comprised in $R^S$ or $R^A$. It will be understood that the latter combination can be conveniently achieved by allowing a compound with a thiol group to react with a compound containing a maleimidyl group.

The variable c in formula (I) is either 0 or 1, such that the group carrying the substituent $R^{L3}$ contained within the brackets [...] carrying the index c can be present or absent. As will be understood, if it is absent, i.e. if c is 0, the group is replaced by a direct bond. Preferably, c is 1. Likewise, the variable d in formula (I) is either 0 or 1, such that the group carrying the substituent $R^{L4}$ contained within the brackets [...] carrying the index d can be present or absent. As will be understood, if it is absent, i.e. if d is 0, the group is replaced by a direct bond. However, in the compounds in accordance with the invention, at least one of c and d must be 1.

Thus, it is preferred that the compound of formula (I) has the formula (Ia), (Ib) or (Ic):

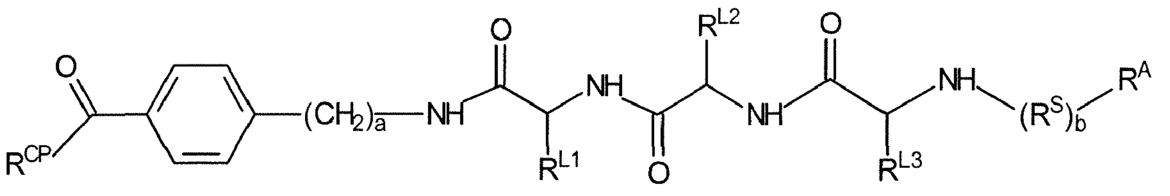

(Ia)

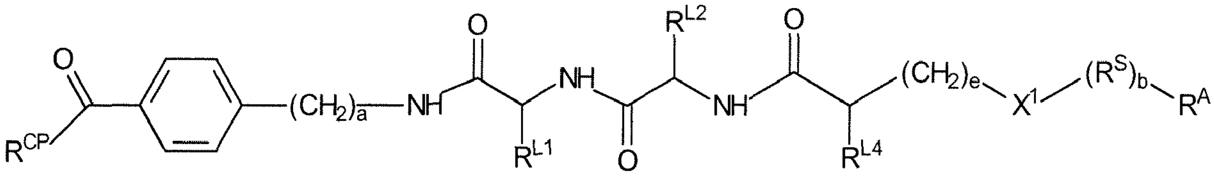

(Ib)

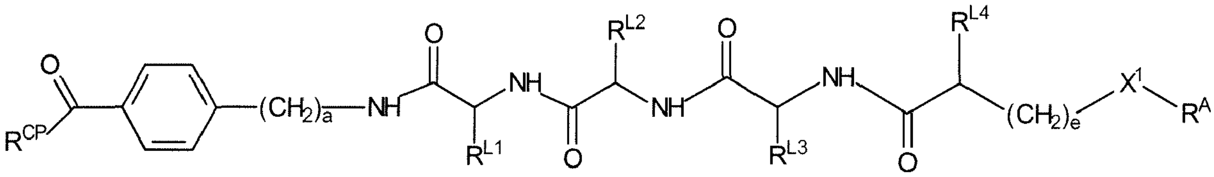

(Ic),

wherein the variables $R^{CP}$, a, $R^{L1}$, $R^{L2}$, $R^{L3}$, $R^S$, b, $R^{L4}$, e, $X^1$ and $R^A$ are defined as for formula (I), including any preferred embodiments of these variables as further defined herein.

Among these preferred formulae for the compound of formula (I), further preference is given to formula (Ia):

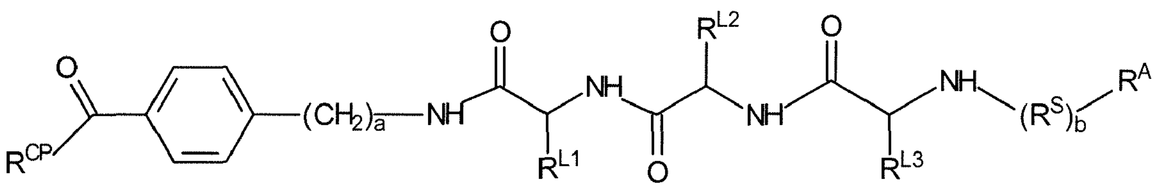

(Ia).

It is particularly preferred that the compound of formula (I) is a compound of formula (Iaa):

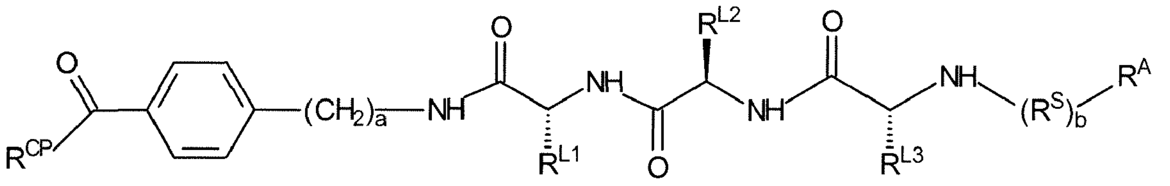

(Iaa),

wherein the variables $R^{CP}$, a, $R^{L1}$, $R^{L2}$, $R^{L3}$, $R^S$, b and $R^A$ are defined as for formula (I), including any preferred embodiments of these variables as further defined herein.

$R^S$ in formula (I) and its preferred embodiments as defined herein is a divalent spacer group. The variable b is 0 or 1, such that the spacer group $R^S$ is an optional group which can be present or absent. As will be understood, if it is absent, i.e. if e is 0, the group $R^S$ is replaced by a direct bond between the atoms adjacent to $R^S$ in formula (I). The group $R^S$ preferably comprises a linear chain of 3 to 10, preferably of 4 to 6 carbon atoms. This linear chain of carbon atoms extends between the group NH and the group $R^A$ (if b is 1 and d is 0) or between the group $X^1$ and the group $R^A$ (if b is 1 and d is 1). In addition to the linear chain of 3 to 10, preferably of 4 to 6 carbon atoms, $R^S$ may comprise one or two, preferably two, coupling groups which allow the chain of carbon atoms to be attached to the group NH and the group $R^A$ or the group $X^1$ and the group $R^A$, respectively. Typically, the spacer group $R^S$ is unbranched and comprises no charged group.

More preferably, the group $R^S$ comprises a linear alkanediyl chain having 3 to 10, preferably having 4 to 6 carbon atoms. This alkanediyl chain extends between the group NH and the group $R^A$ (if b is 1 and d is 0) or the group $X^1$ and the group $R^A$ (if b is 1 and d is 1). In addition to the linear alkanediyl chain having 3 to 10, preferably having 4 to 6 carbon atoms, $R^{6L}$ may comprise one or two, preferably two, coupling groups which allow the alkanediyl chain to be attached to the group NH and the group $R^A$ or the group $X^1$ and the group $R^A$, respectively.

Thus, it is preferred that the group $R^S$ is a group of the formula

$$-X^2-(CH_2)_B-X^3-$$

wherein

$X^2$ is a coupling group attached to the group NH in formula (I) if d is 0 or to the group $X^1$ in formula (I) if d is 1;
B is an integer of 3 to 10, preferably 4 to 6; and
$X^3$ is a coupling group attached to $R^A$.

For the coupling groups $X^2$ and $X^3$, similar considerations apply as for the coupling group $X^1$ discussed above. Thus, the coupling group $X^2$ is a functional group which allows $R^S$ to be coupled either to the NH group via a covalent bond which is formed between the group $X^2$ and NH, or to the group $X^1$ via a covalent bond which is formed between the group $X^2$ and $X^1$. The coupling group $X^2$ may consist of one or more atoms. Preferred coupling groups are selected from -NH-, -C(O)-, -O-, and -S-. If $X^2$ is linked to NH, it is preferably -C(O)-, such that $X^2$ and $X^1$ form an amide bond -C(O)-NH-. If $X^2$ is linked to $X^1$, the coupling group $X^2$ and $X^1$ are typically complementary coupling groups which combine to form a binding unit, such as an amide bond -C(O)-NH-, an ester bond -C(O)-O-, or a thiosuccinimidyl group

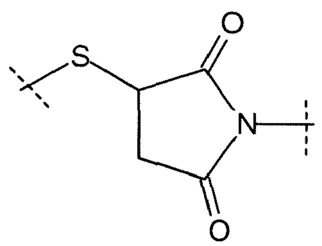

.

A particularly preferred group $R^S$ is a group of the formula $-C(O)-(CH_2)_B-NH-$, wherein B is as defined above, and wherein the bond at the N-terminus is attached to $R^A$.

In line with the above, it will be understood that a preferred combination of variables for the compound of formula (I) is the one wherein $R^{B2}$ in $R^{CP}$ is $-CH_2-CH_2-CH_2-$, $R^{L1}$ is methyl, $R^{L2}$ is $-(CH_2)_3-NH-C(=NH)-NH_2$, c is 1, and the remaining variables are defined as for formula (I) and (II), including any preferred embodiments of these variables as

further defined herein. A still more preferred combination of variables for the compound of formula (I) is the one wherein $R^{B2}$ in $R^{CP}$ is $-CH_2-CH_2-CH_2-$, $R^{L1}$ is methyl, $R^{L2}$ is $-(CH_2)_3-NH-C(=NH)-NH_2$, c is 1, d is 0, b is 0 or 1, $R^S$, if present, is $-C(O)-(CH_2)_B-NH-$, wherein B is as defined above and wherein the bond at the N-terminus is attached to $R^A$, and the remaining variables are defined as for formula (I) and (II), including any preferred embodiments of these variables as further defined herein.

Thus, a particularly preferred compound of formula (I) has the following formula (Iab) or the following formula (Iac)

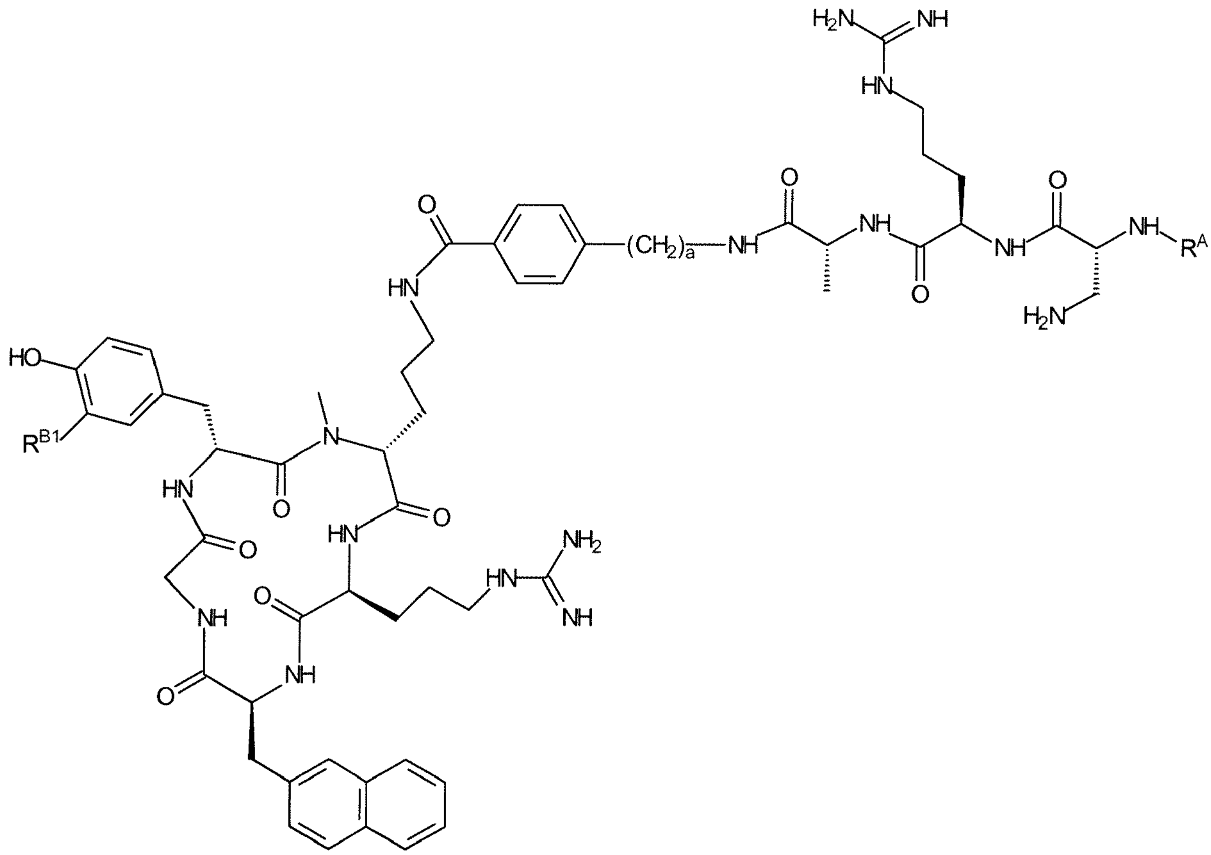

(Iab)

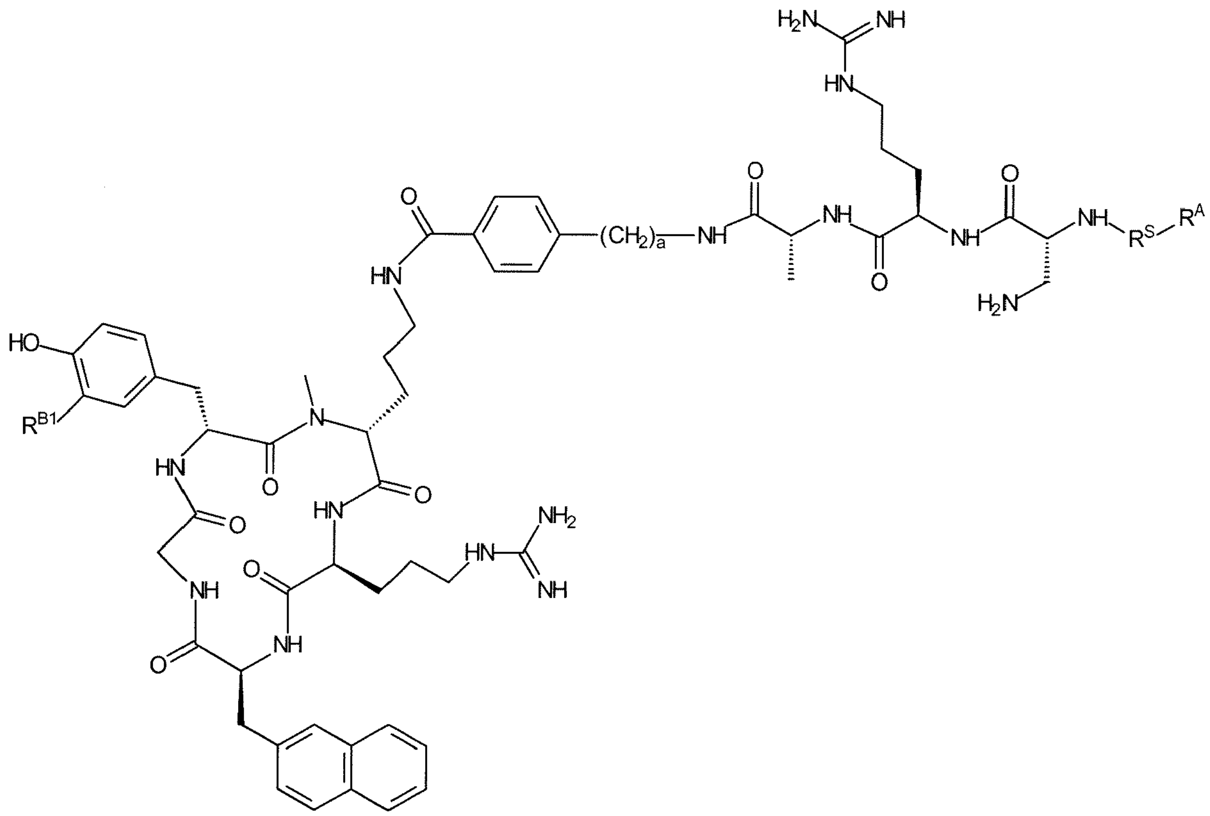

(Iac)

wherein $R^{B1}$, a, $R^S$ and $R^A$ are defined as for formulae (I) and (II), respectively, including any preferred embodiments of these variables as further defined herein.

$R^A$ is a functional group comprising a moiety with diagnostic or therapeutic utility, e.g. a labeling group. As will be understood by the skilled person, a moiety with diagnostic utility is a group which facilitates the detection of the ligand compound in accordance with the invention after administration to a patient or after it has been brought into contact *in vitro* or *ex vivo* with a physiological sample, or a precursor of such a group. An example of such a precursor is a group which can carry a radioactive element that can be detected, but wherein such an element is not yet contained, e.g. a SiFA moiety or a chelating moiety. Preferably, a moiety with diagnostic utility is a group or a precursor thereof which allows the compound in accordance with the invention to be detected and located in the body of a patient after its administration to the patient. Due to its affinity to CXCR4, the compound of the invention comprising a moiety with diagnostic utility may function in particular as a tracer for CXCR4. A moiety with therapeutic utility is a group which allows the compound of the invention to treat or prevent a disease or disorder after its administration to a patient, in particular a disease or disorder which can be treated or prevented by blocking of the CXCR4 receptor or which is associated with an increased or aberrant expression of CXCR4, or a precursor of such a group. An example of such a precursor is a group which can carry a radioactive element that has a therapeutic effect, but wherein such an element is not yet contained, e.g. a chelating moiety.

The group $R^A$ comprises a moiety with diagnostic or therapeutic utility. Preferably, it comprises one or two of these moieties. A combination of two moieties can be useful, e.g., to provide a compound of the invention which combines diagnostic and therapeutic utility.

Preferably, $R^A$ in the compound formula (I) and its preferred embodiments defined herein comprises, or consist of, at least one of the following moieties with diagnostic or therapeutic utility:

(i) a chelating moiety;

(ii) a chelate formed by a chelating moiety (i) with a chelated radioactive or non-radioactive cation or anion, preferably a chelated radioactive or non-radioactive cation;

(iii) a silicon-fluoride acceptor (SiFA) moiety which comprises a silicon atom and a fluorine atom, wherein the fluorine atom is linked via a covalent bond directly to the silicon atom, and which SiFA moiety can be labeled with $^{18}F$ by isotopic exchange of $^{19}F$ by $^{18}F$ or which is labeled by $^{18}F$;

(iv) a cytotoxic moiety; and
(v) a fluorescent moiety.

More preferably, $R^A$ comprises, or consists of, one of the moieties (i) to (v), or a combination of one moiety selected from the chelating moiety (i) and the chelate (ii), and one SiFA moiety (iii).

The chelating moiety of (i) and (ii) is suitable to form a chelate with a radioactive or non-radioactive cation or anion, preferably a radioactive cation. Suitable chelating agents providing chelating moieties for diverse cations and anions are well known in the art and can be used in the context of the present invention. Metal- or cation-chelating agents, e.g. macrocyclic or acyclic compounds, which are suitable to provide a chelating moiety, are available from a number of manufacturers. It will be understood that numerous chelating agents can be used in an off-the-shelf manner by a skilled person without further ado. It will further be understood that the suitability of the chelating moiety to form a chelate with a given anion or cation requires the chelating moiety to be able to provide a chelate ligand in a chelate complex comprising the anion or cation under consideration, but does not require the chelating moiety to form the only ligand of the anion or cation in the chelate complex. Thus, a chelate in accordance with option (ii) above may comprise a chelated cation or anion, the chelating moiety (i) as a chelating ligand, and an additional ligand coordinated with the chelated cation or anion.

For example, a chelating moiety may comprise at least one of

a macrocyclic ring structure with 8 to 20 ring atoms of which 2 or more, preferably 3 or more, are selected from oxygen atoms, sulfur atoms and nitrogen atoms; and

an acyclic, open chain chelating structure with 8 to 20 main chain atoms of which 2 or more, preferably 3 or more are heteroatoms selected from oxygen atoms, sulfur atoms and nitrogen atoms.

Preferably, the chelating moiety referred to in (i) or (ii) above is a chelating moiety which is suitable as a chelate ligand for a cation selected from the cations of $^{43}Sc$, $^{44}Sc$, $^{47}Sc$, $^{51}Cr$, $^{52m}Mn$, $^{58}Co$, $^{52}Fe$, $^{56}Ni$, $^{57}Ni$, $^{nat}Cu$, $^{62}Cu$, $^{64}Cu$, $^{67}Cu$, $^{66}Ga$, $^{nat}Ga$, $^{68}Ga$, $^{67}Ga$, $^{89}Zr$, $^{90}Y$, $^{86}Y$, $^{94m}Tc$, $^{99m}Tc$, $^{97}Ru$, $^{105}Rh$, $^{109}Pd$, $^{111}Ag$,$^{110m}In$, $^{111}In$, $^{113m}In$, $^{114m}In$, $^{117m}Sn$, $^{121}Sn$, $^{127}Te$, $^{142}Pr$, $^{143}Pr$, $^{147}Nd$, $^{149}Gd$, $^{149}Pm$, $^{151}Pm$, $^{149}Tb$, $^{152}Tb$, $^{155}Tb$, $^{153}Sm$, $^{156}Eu$, $^{157}Gd$, $^{161}Tb$, $^{164}Tb$, $^{161}Ho$, $^{166}Ho$, $^{157}Dy$, $^{165}Dy$, $^{166}Dy$, $^{160}Er$, $^{165}Er$, $^{169}Er$, $^{171}Er$, $^{166}Yb$, $^{169}Yb$, $^{175}Yb$, $^{167}Tm$, $^{172}Tm$, $^{nat}Lu$, $^{177}Lu$, $^{186}Re$, $^{188}Re$, $^{188}W$, $^{191}Pt$, $^{195m}Pt$, $^{194}Ir$, $^{197}Hg$, $^{198}Au$, $^{199}Au$, $^{nat}Pb$, $^{212}Pb$, $^{203}Pb$, $^{211}At$, $^{212}Bi$, $^{213}Bi$, $^{223}Ra$, $^{224}Ra$, $^{225}Ac$, and $^{227}Th$, and from a cationic molecule comprising $^{18}F$, such as $^{18}F$-$[AlF]^{2+}$.

Thus, preferred chelating agents which can be used to provide a chelating moiety of (i) or (ii) above are selected from bis(carboxymethyl)-1,4,8,11-tetraazabicyclo[6.6.2]hexadecane (CBTE2a), cyclohexyl-1,2-diaminetetraacetic acid (CDTA), 4-(1,4,8,11-tetraazacyclotetradec-1-yl)-methylbenzoic acid (CPTA), N'-[5-[acetyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxymethyl)-1 ,4,8,1 1-tetraazabicycle[6.6.2]hexadecan (DO2A), 1,4,7,10-tetraazacyclododecan-N,N',N",N'''-tetraacetic acid (DOTA), 2-[1,4,7,10-tetraazacyclododecane-4,7,10-triacetic acid]-pentanedioic acid (DOTAGA or DOTA-GA), N,N'-dipyridoxylethylendiamine-N,N'-diacetate-5,5'-bis(phosphat) (DPDP), diethylenetriaminepentaacetic acid (DTPA), ethylenediamine-N,N'-tetraacetic acid (EDTA), ethyleneglykol-O,O-bis(2-aminoethyl)-N,N,N',N'-tetraacetic acid (EGTA), N,N-bis(hydroxybenzyl)-ethylenediamine-N,N'-diacetic acid (HBED), hydroxyethyldiaminetriacetic acid (HEDTA), 1-(p-nitrobenzyl)-1,4,7,10-tetraazacyclodecan-4,7,10-triacetate (HP-DOA3), 1,4,7-triazacyclononan-1-succinic acid-4,7-diacetic acid (NODASA), 1-(1-carboxy-3-carboxypropyl)-4,7-(carboxy)-1,4,7-triazacyclononane (NODAGA), 1,4,7-triazacyclononanetriacetic acid (NOTA), 4,11-bis(carboxymethyl)-1,4,8,11-tetraaza-bicyclo[6.6.2]hexadecane (TE2A), 1,4,8,11-tetraazacyclododecane-1,4,8,11-tetraacetic acid (TETA), terpyridine-bis(methyleneamine) tetraacetic acid (TMT), 1,4,7,10-tetraazacyclotridecan-N,N',N",N"'-tetraacetic acid (TRITA), and triethylenetetra-aminehexaacetic acid (TTHA), *N,N'*-bis[(6-carboxy-2-pyridyl)methyl]-4,13-diaza-18-crown-6 ($H_2$macropa), 4-amino-4-{2-[(3-hydroxy-1,6-dimethyl-4-oxo-1,4-dihydro-pyridin-2-ylmethyl)-carbamoyl]-ethyl} heptanedioic acid bis-[(3-hydroxy-1,6-dimethyl-4-oxo-1,4-dihydro-pyridin-2-ylmethyl)-amide] (THP), 1,4,7-triazacyclononane-1,4,7-tris[methylene(2-carboxyethyl)phosphinic acid (TRAP), 2-(4,7,10-tris(2-amino-2-oxoethyl)-1,4,7,10-tetraazacyclododecan-1-yl)acetic acid (DO3AM), and 1,4,7,10-tetraazacyclododecane-1,4,7, 10-tetrakis[methylene(2-carboxyethylphosphinic acid)] (DOTPI), S-2-(4-isothiocyanatobenzyl)-1,4,7,10-tetraazacyclododecane tetraacetic acid, mercaptoacetyl-triserine (mas3), hydrazinonicotinic acid (HYNIC) and 3-(2-aminoethylamino)-2-[(2-aminoethylamino)methyl]propanoic acid (N4 chelator, 6-carboxy-1,4,8,11-tetraazaundecane). As a further preferred example, reference can be made to a modified mercaptoacetylserine chelating agent, wherein one or more of the serine residues are replaced by another amino acid containing a hydrophilic side chain.

As will be understood by the skilled reader, the preferred chelating agents listed above can conveniently provide a chelating moiety in a compound in accordance with the invention by using a functional group contained in the chelating agent to provide a binding unit which attaches the chelating moiety to the remainder of the compound. As examples of such a binding unit, reference can be made to an amide bond (-C(O)-NH-) or an ester bond (-C(O)-O-) which can be provided e.g. using a carboxyl group or an amino group which may be contained as a functional group in the chelating agent.

In line with the above, the chelated cation referred to in (ii) above is preferably selected from the cations of

$^{43}Sc$, $^{44}Sc$, $^{47}Sc$, $^{51}Cr$, $^{52m}Mn$, $^{58}Co$, $^{52}Fe$, $^{56}Ni$, $^{57}Ni$, $^{nat}Cu$, $^{62}Cu$, $^{64}Cu$, $^{67}Cu$, $^{66}Ga$, $^{nat}Ga$, $^{68}Ga$, $^{67}Ga$, $^{89}Zr$, $^{90}Y$, $^{86}Y$, $^{94m}Tc$, $^{99m}Tc$, $^{97}Ru$, $^{105}Rh$, $^{109}Pd$, $^{111}Ag$, $^{110m}In$, $^{111}In$, $^{113m}In$, $^{114m}In$, $^{117m}Sn$, $^{121}Sn$, $^{127}Te$, $^{142}Pr$, $^{143}Pr$, $^{147}Nd$, $^{149}Gd$, $^{149}Pm$, $^{151}Pm$, $^{149}Tb$, $^{152}Tb$, $^{155}Tb$, $^{153}Sm$, $^{156}Eu$, $^{157}Gd$, $^{161}Tb$, $^{164}Tb$, $^{161}Ho$, $^{166}Ho$, $^{157}Dy$, $^{165}Dy$, $^{166}Dy$, $^{160}Er$, $^{165}Er$, $^{169}Er$, $^{171}Er$, $^{166}Yb$, $^{169}Yb$, $^{175}Yb$, $^{167}Tm$, $^{172}Tm$, $^{nat}Lu$, $^{177}Lu$, $^{186}Re$, $^{188}Re$, $^{188}W$, $^{191}Pt$, $^{195m}Pt$, $^{194}Ir$, $^{197}Hg$, $^{198}Au$, $^{199}Au$, $^{nat}Pb$, $^{212}Pb$, $^{203}Pb$, $^{211}At$, $^{212}Bi$, $^{213}Bi$, $^{223}Ra$, $^{224}Ra$, $^{225}Ac$, and $^{227}Th$, and from a cationic molecule comprising $^{18}F$, such as $^{18}F$-$[AlF]^{2+}$. A chelate formed with such a cation may involve, in addition to the chelating moiety provided by the compound of formula (I) or its salt, one or more additional ligands which are coordinated to the chelated cation and which are not part of the compound of formula (I) or its salt, such as an oxo-ligand in a chelate including a $^{99m}Tc(V)$-oxo core.

Particularly preferred as a group $R^A$ is a group comprising a chelating moiety which can form a chelate with a cation selected from a cation of $^{99m}Tc$, $^{177}Lu$, $^{67}Ga$ and $^{68}Ga$. Likewise, preferred as a group $R^A$ is a group comprising a chelate with a chelated cation selected from a cation of $^{99m}Tc$, $^{177}Lu$, $^{67}Ga$ and $^{68}Ga$.

As noted above, the structure of the linker forming part of the compound of the invention allows a broad variety of functional groups $R^A$ to be used in these compounds while retaining a high CXCR4 affinity. In the case of a functional group comprising a chelating moiety as referred to in options (i) and (ii) above, the positive influence of the linker is particularly pronounced for compounds of formula (I) and their salts wherein $R^A$ is a group other than a group consisting of a DOTA or DOTAGA residue, or other than a group consisting of a residue of a chelator for $M^{3+}$ (i.e. for three-valent metal cations) in general.

A silicon-fluoride acceptor (SiFA) moiety in line with option (iii) above preferably comprises a group of formula (S-1):

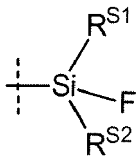

(S-1)

wherein

$R^{S1}$ and $R^{S2}$ are independently a linear or branched C3 to C10 alkyl group, preferably $R^{S1}$ and $R^{S2}$ are independently selected from isopropyl and *tert*-butyl, and more preferably $R^{S1}$ and $R^{S2}$ are both *tert*-butyl, and wherein the bond marked with the dashed line attaches the group to the remainder of the compound of formula (I). Preferably, the group of formula (S-1) is attached as a substituent to a phenyl ring.

More preferably, the SiFA moiety is a group selected from a group of formula (S-2) and a group of formula (S-3).

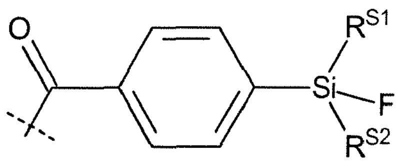

(S-2)

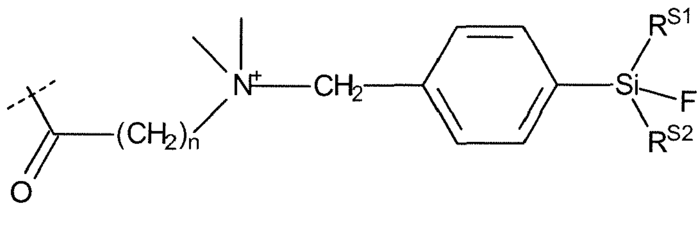

(S-3)

wherein

n is 1, 2, or 3 and is preferably 1, $R^{S1}$ and $R^{S2}$ are independently a linear or branched C3 to C10 alkyl group, preferably $R^{S1}$ and $R^{S2}$ are independently selected from isopropyl and *tert*-butyl, and more preferably $R^{S1}$ and $R^{S2}$ are both *tert-butyl,* and wherein the bond marked by the dashed line attaches the group to the remainder of the compound of formula (I). Suitable counterions for the positively charged quaternary nitrogen atom indicated in formula (S-3), which carries two methyl substituents, include anions as they are discussed herein with regard to salts formed of the compound of formula (I), and may include, e.g., trifluoro acetate or acetate anions.

As will be understood by the skilled reader, the bond at the carbonyl group marked by the dashed line in formulae (S-2) and (S-3) does not carry a methyl group at its end opposite to the carbonyl group, but represents a bond which attaches the SiFA moiety to the remainder of the compound of formula (I). In other words, the bond marked by the dashed line represents a covalent bond which is present between the carbon atom of the carbonyl group shown in formulae (S-2) and (S-3) and an atom or group adjacent to the group (S-2) or (S-3) in the compounds in accordance with the invention. For example, an amide bond (-C(O)-NH-) or an ester bond (-C(O)-O-), preferably an amide bond, is provided using the carbonyl group shown in formulae (S-2) and (S-3) and a group -NH- or -O- adjacent to these groups. For example, such a group -NH- or -O- may be comprised by a linker forming part of $R^A$.

As will be further understood, the fluorine atom contained in formulae (S-1) to (S-3) may be a $^{18}F$ atom or a $^{19}F$ atom which can be exchanged to provide $^{18}F$ by isotopic exchange of $^{19}F$ by $^{18}F$.

A cytotoxic moiety as option (iv) discussed above may be provided, for example, by a residue of a cytotoxic compound, e.g. an auristatin analogue, such as monomethyl auristatin E (MMAE), monomethyl auristatin F (MMAF), or PF-06380101. The residue may be provided using a functional group contained in the cytotoxic compound to form a binding unit which attaches the cytotoxic moiety to the remainder of the compound in accordance with the invention. Optionally, a metabolically cleavable linker may further be provided as a linker attaching the cytotoxic moiety to the remainder of the compound in accordance with the invention.

A fluorescent moiety as option (v) discussed above may be provided, for example, by a residue of a fluorescent dye. Such fluorescent dyes are known in the art, and include, e.g., Cy5- and Cy7-based cyanine dyes. The residue may be provided using a functional group contained in the fluorescent dye to provide a binding unit which attaches the cytotoxic moiety to the remainder of the compound in accordance with the invention.

In addition to a moiety with diagnostic or therapeutic utility as discussed above, $R^A$ may comprise one or more further moieties intended for a different purpose. For example, $R^A$ may comprise a divalent or higher valent linker which allows one or more moieties with diagnostic or therapeutic utility to be attached the remainder of the compound. As another example, reference may be made to a moiety which is comprised by $R^A$ in order to adjust the hydrophilic/hydrophobic characteristics of the compound in accordance with the invention, e.g. a moiety which carries one or more polar groups.

As will be understood by the skilled person, $R^A$ comprises a coupling group which allows $R^A$ to be covalently attached to a group -NH- (if, in the compound of formula (I), d is 0 and b is 0), to a group $-X^1-$ (if, in the compound of formula (I), d is 1 and b is 0), or to the terminus of - $R^S$- (if, in the compound of formula (I), b is 1). Suitable coupling groups can be selected and provided relying on well-established principles of synthetic chemistry. For example, a coupling group can be contained in a linker optionally comprised by $R^A$, or can be a part of the moiety with diagnostic or therapeutic utility comprised by $R^A$. For example, if, in the compound of formula (I), d is 0 and b is 0, it is preferred that $R^A$ comprises a coupling group - C(O)- for attachment to -NH- to provide an amide bond. Such a coupling group can be conveniently derived e.g. from a carboxyl group. Likewise, if, in the compound of formula (I), b is 1, and $R^S$ is a group of formula $-C(O)-(CH_2)_B-NH-$, it is preferred that $R^A$ comprises a coupling group -C(O)- for attachment to -NH- to provide an amide bond. As another example, if, in the compound of formula (I), d is 1, b is 0, and $X^1$ is -S-, $R^A$ may comprise a succinimidyl group as a coupling group for attachment to -S- to yield a thiosuccinimidyl group. Such a coupling group can be conveniently derived e.g. from a maleimidyl group.

In line with the above, particularly preferred embodiments of $R^A$ can be exemplified as follows:

- $R^A$ is a chelating moiety provided by a mercaptoacetyl triserine ($mas_3$) chelating agent attached via an amide bond to the remainder of the compound of formula (I);
- $R^A$ is a chelate comprising a chelating moiety provided by a mercaptoacetyl triserine ($mas_3$) chelating agent attached via an amide bond to the remainder of the compound of formula (I) and a chelated cation, such as a $^{99m}Tc$ cation;
- $R^A$ is a chelating moiety provided by a modified mercaptoacetyl triserine ($mas_3$) chelating agent attached via an amide bond to the remainder of the compound of formula (I), wherein one or more of the serine residues are replaced by another amino acid residue carrying a hydrophilic side chain, such as citrulline, or by an amino acid residue with a glycosylated side chain;
- $R^A$ is a chelate comprising a chelating moiety provided by a modified mercaptoacetyl triserine (mass) chelating agent attached via an amide bond to the remainder of the compound of formula (I), wherein one or more of the serine residues are replaced by another amino acid residue carrying a hydrophilic side chain, such as citrulline, or by an amino acid residue with a glycosylated side chain, and a chelated cation, such as a $^{99m}Tc$ cation;
- $R^A$ is a chelating moiety provided by a hydrazinonicotinic acid (HYNIC) chelating agent attached via an amide bond to the remainder of the compound of formula (I);
- $R^A$ is a chelate comprising a chelating moiety provided by a hydrazinonicotinic acid (HYNIC) chelating agent attached via an amide bond to the remainder of the compound of formula (I) and a chelated cation, such as a $^{99m}Tc$ cation.
- $R^A$ is a chelating moiety provided by a 3-(2-aminoethylamino)-2-[(2-aminoethylamino)methyl]propanoic acid (N4) chelating agent attached via an amide bond to the remainder of the compound of formula (I) or $R^A$ comprises a

chelating moiety provided by an N4 chelating agent attached via an amide bond to the remainder of the compound of formula (I);

- $R^A$ is a chelate comprising a chelating moiety provided by a 3-(2-aminoethylamino)-2-[(2-aminoethylamino)methyl]propanoic acid (N4) chelating agent attached via an amide bond to the remainder of the compound of formula (I) and a chelated cation, such as a $^{99m}Tc$ cation, or $R^A$ comprises a chelate comprising a chelating moiety provided by an N4 chelating agent attached via an amide bond to the remainder of the compound of formula (I) and a chelated cation, such as a $^{99m}Tc$ cation;
- $R^A$ comprises a SiFA moiety and a chelating moiety, such as a chelating moiety provided by a DOTA or a DOTAGA chelating agent attached via an amide bond to the remainder of the compound of formula (I);
- $R^A$ comprises a SiFA moiety and a chelate which comprises a chelating moiety, such as a chelating moiety provided by a DOTA or a DOTAGA chelating agent, and a chelated cation, such as a $^{177}Lu$ cation a $^{68}Ga$ cation or a $^{69}Ga$ cation;
- $R^A$ comprises a cytotoxic moiety provided by MMAE; or
- $R^A$ comprises a fluorescent moiety provided by the fluorescent dye Cy5.5.

The presence of $R^S$ (i.e. the selection of b as 1 in formula (I)) may lead to an additional benefit with a view to a high CXCR4 affinity of the compounds of the present invention in particular if the group $R^A$ has a high molecular weight, if it contains a bulky group in the proximity to its point of attachment to the remainder of the compound of formula (I), or if the group $R^A$ comprises negatively charged functional groups (without taking into account functional groups wherein a negative charge is neutralized by the formation of a chelate complex). Thus, as an orientation, it is expected that the presence of the optional spacer group $R^S$ additionally improves the affinity for compounds containing a group $R^A$ which fulfills two of the following three requirements:

i) the molecular weight of $R^A$ is more than 300 g/mol;
ii) $R^A$ has a charge of < -1 if the compound of formula (I) is kept in a solution at neutral pH (not taking into account charged groups in chelating agents which are neutralized by the formation of a chelate complex);
iii) $R^A$ comprises a phenyl ring or a larger aromatic group in the proximity to the point of attachment of $R^A$ to the remainder of the compound of formula (I), e.g. less than 7 covalent bonds (C-C bonds, C-O-bonds, or C-N bonds) away from the atom to which $R^A$ is attached.

As noted above, the ligand compounds in accordance with the invention encompass the compounds of formula (I) and their salts, preferably pharmaceutically acceptable salts. Such salts may be formed, e.g., by protonation of an atom carrying an electron lone pair which is susceptible to protonation, such as a nitrogen atom, with an inorganic or organic acid, or by separating a proton from an acidic group, such as a carboxylic acid group, e.g. by neutralization with a base. Other charged groups which may be present in the compounds in accordance with the invention include groups which are continuously charged, such as quaternary ammonium cations substituted by four organyl groups, or charged chelate complexes.

As exemplary anions which may be present in salt forms of the compounds of the invention if the salt form comprises a positively charged form of the compound of formula (I), mention may be made, for example, of an anion selected from chloride, bromide, iodide, sulfate, nitrate, phosphate (such as, e.g., phosphate, hydrogenphosphate, or dihydrogenphosphate salts), carbonate, hydrogencarbonate or perchlorate; acetate, trifluoroacetate, propionate, butyrate, pentanoate, hexanoate, heptanoate, octanoate, cyclopentanepropionate, undecanoate, lactate, maleate, oxalate, fumarate, tartrate, malate, citrate, nicotinate, benzoate, salicylate or ascorbate; sulfonates such as methanesulfonate, ethanesulfonate, 2-hydroxyethanesulfonate, benzenesulfonate, p-toluenesulfonate (tosylate), 2-naphthalenesulfonate, 3-phenylsulfonate, or camphorsulfonate. Since trifluoroacetic acid is frequently used during the synthesis of peptides, trifluoroacetate salts are typical salts which are provided if a compound comprising a peptide structure is formed. Such trifluoroacetate salts may be converted to acetate salts during their workup. As exemplary cations which may be present in salt forms of the compounds of the invention if the salt form comprises a negatively charged form of the compound of formula (I), mention may be made, for example, of a cation selected from alkali metal cations, such as lithium, sodium or potassium, alkaline earth metal cations, such as calcium or magnesium; and ammonium (including ammonium ions substituted by organic groups).

The ligand compound in accordance with the invention is preferably capable of binding to human CXCR4 with an affinity reflected by an $IC_{50}$ value of 100 nM or less, more preferably 10 nM or less, and still more preferably 5 nM or less.

As exemplary compounds in accordance with the invention, the following are mentioned.

A compound of the following formula, a compound wherein the $mas_3$ chelating moiety shown in the formula forms a chelate with a chelated radioactive or non-radioactive cation, or a salt of any of these:

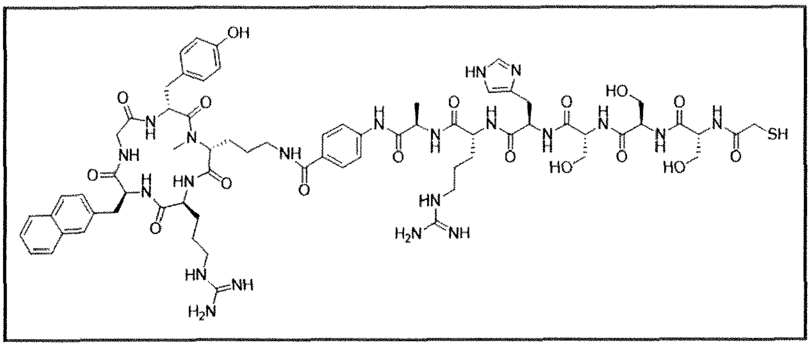

;

a compound of the following formula, a compound wherein the $mas_3$ chelating moiety shown in the formula forms a chelate with a chelated radioactive or non-radioactive cation, or a salt of any of these:

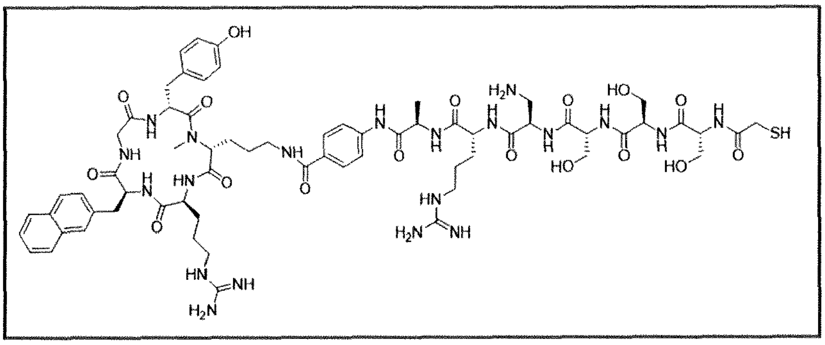

;

a compound of the following formula, a compound wherein the $mas_3$ chelating moiety shown in the formula forms a chelate with a chelated radioactive or non-radioactive cation, or a salt of any of these:

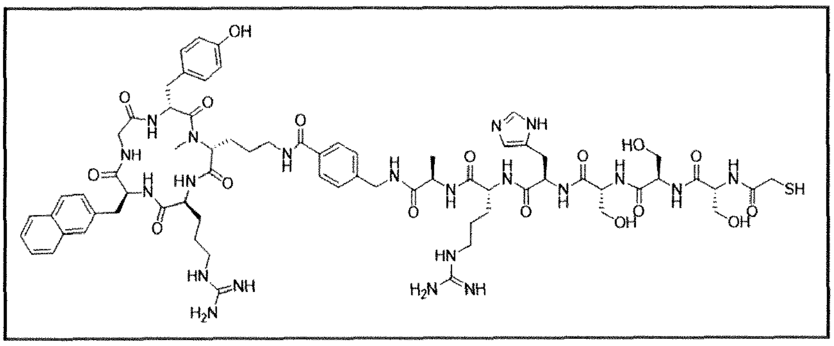

;

a compound of the following formula, a compound wherein the $mas_3$ chelating moiety shown in the formula forms a chelate with a chelated radioactive or non-radioactive cation, or a salt of any of these:

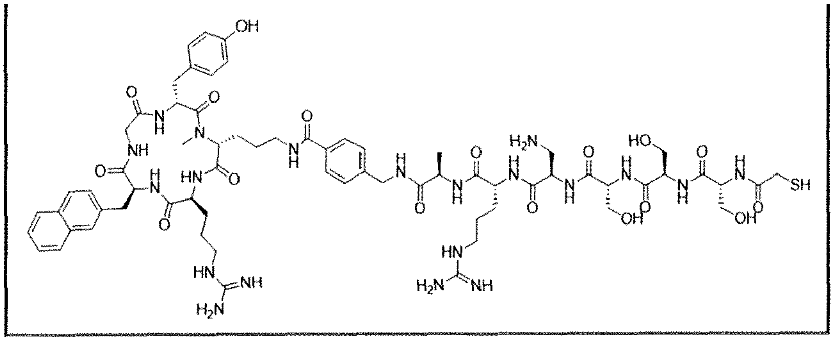

;

a compound of the following formula, a compound wherein the modified $mas_3$ chelating moiety shown in the formula forms a chelate with a chelated radioactive or non-radioactive cation, or a salt of any of these:

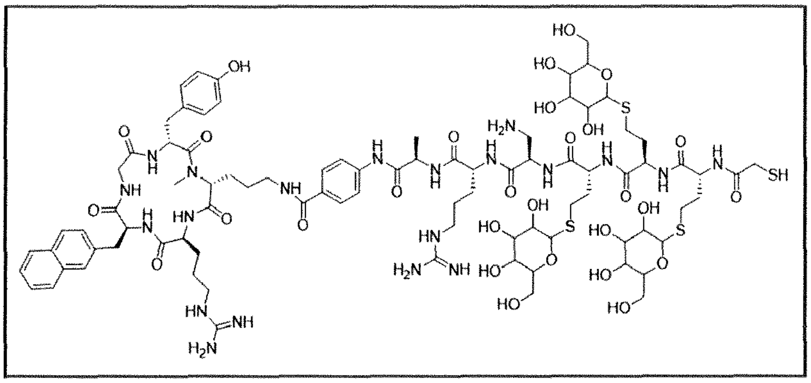

;

a compound of the following formula, a compound wherein the modified $mas_3$ chelating moiety shown in the formula forms a chelate with a chelated radioactive or non-radioactive cation, or a salt of any of these:

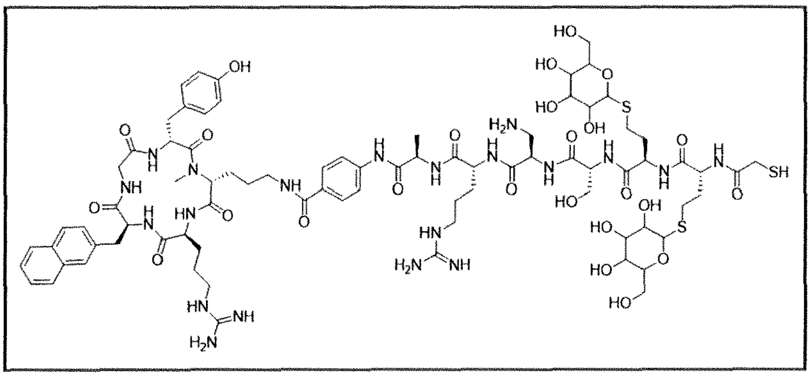

;

a compound of the following formula, a compound wherein the modified $mas_3$ chelating moiety shown in the formula forms a chelate with a chelated radioactive or non-radioactive cation, or a salt of any of these:

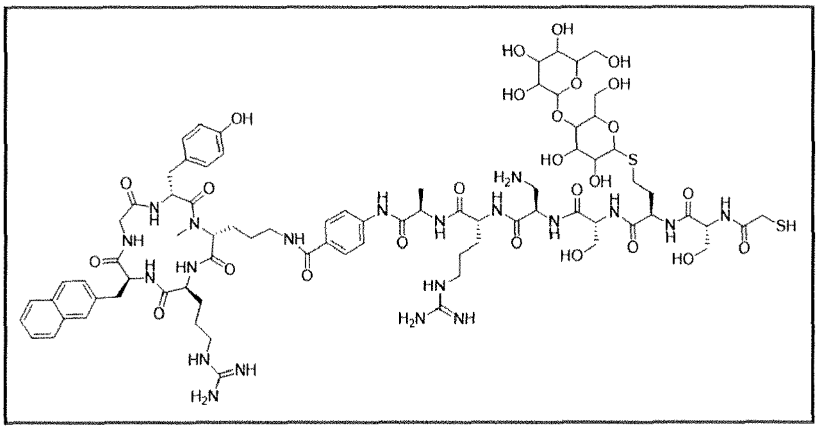;

a compound of the following formula, a compound wherein the modified $mas_3$ chelating moiety shown in the formula forms a chelate with a chelated radioactive or non-radioactive cation, or a salt of any of these:

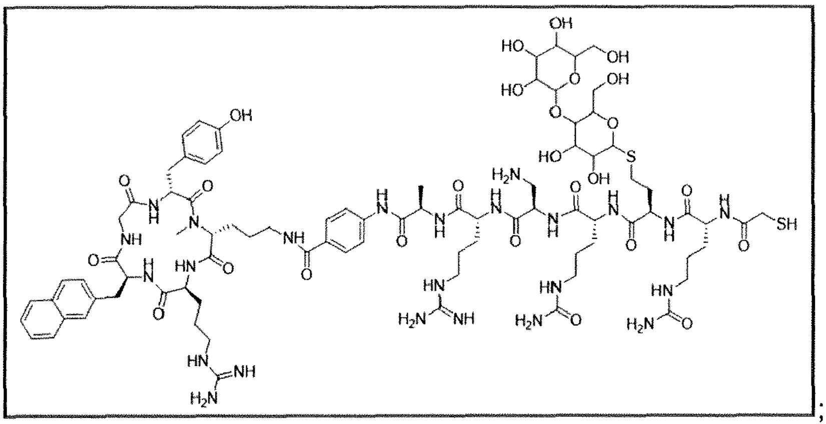;

a compound of the following formula, a compound wherein the HYNIC chelating moiety shown in the formula forms a chelate with a chelated radioactive or non-radioactive cation, or a salt of any of these:

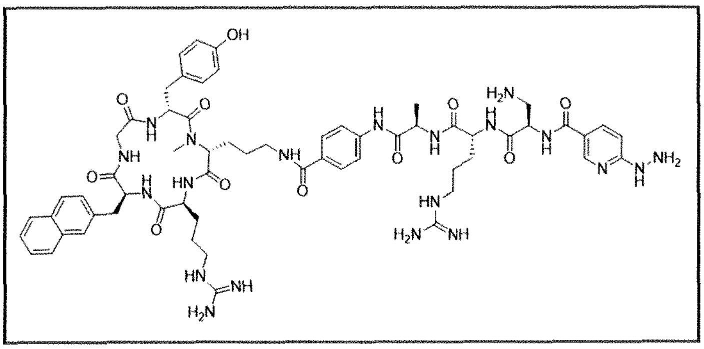;

a compound of the following formula, a compound wherein the N4 chelating moiety shown in the formula forms a chelate with a chelated radioactive or non-radioactive cation, or a salt of any of these:

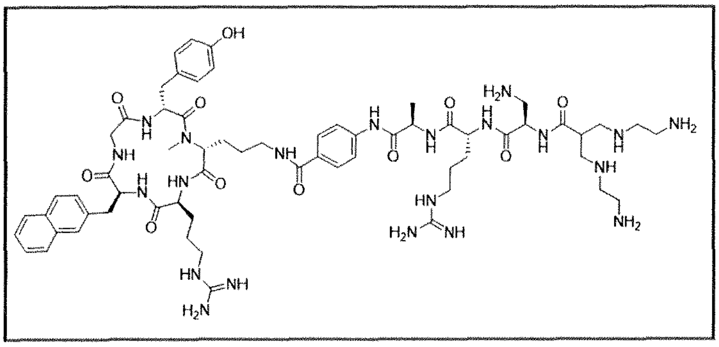

;

a compound of the following formula, a compound wherein the N4 chelating moiety shown in the formula forms a chelate with a chelated radioactive or non-radioactive cation, or a salt of any of these:

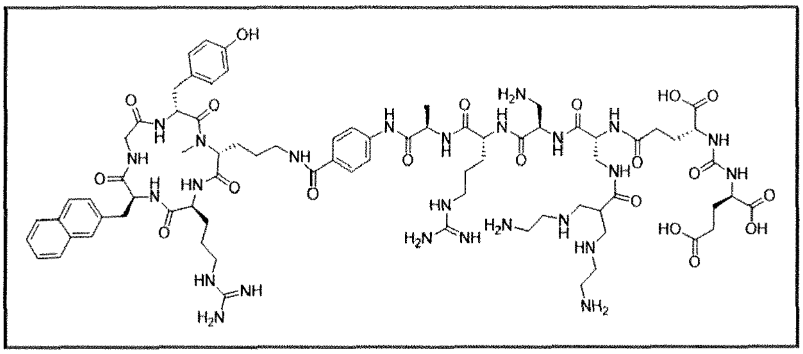

;

a compound of the following formula, a compound wherein the N4 chelating moiety shown in the formula forms a chelate with a chelated radioactive or non-radioactive cation, or a salt of any of these:

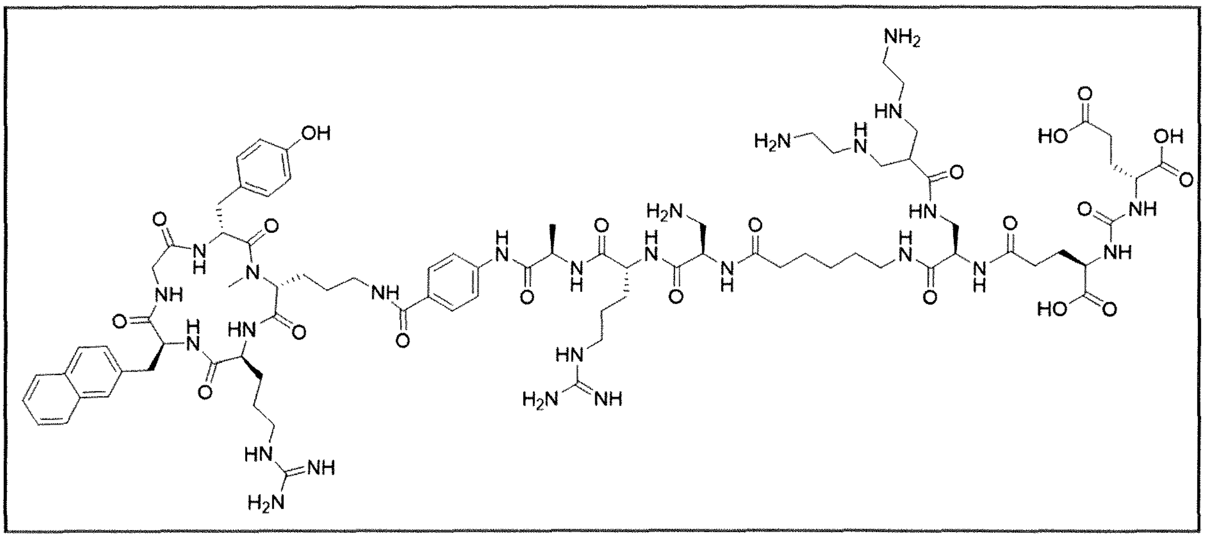

;

a compound of the following formula, a compound wherein the DOTA chelating moiety shown in the formula forms a chelate with a chelated radioactive or non-radioactive cation, or a salt of any of these:

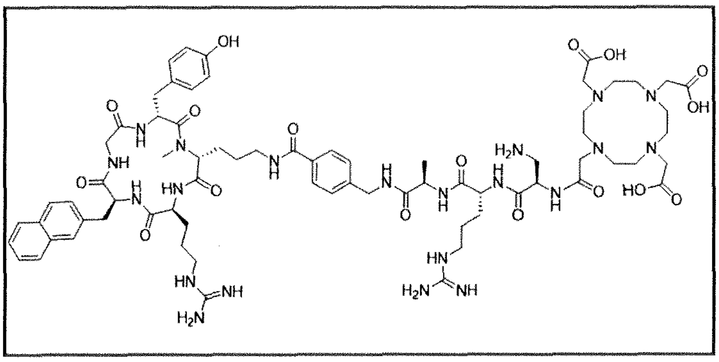

;

a compound of the following formula, a compound wherein the DOTA chelating moiety shown in the formula forms a chelate with a chelated radioactive or non-radioactive cation, or a salt of any of these:

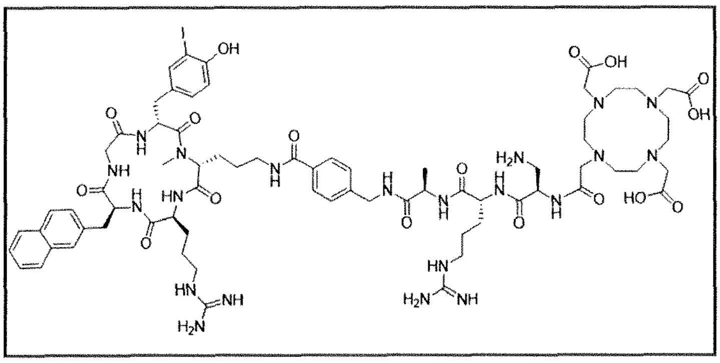

;

a compound of the following formula, a compound wherein the DOTA chelating moiety shown in the formula forms a chelate with a chelated radioactive or non-radioactive cation, or a salt of any of these:

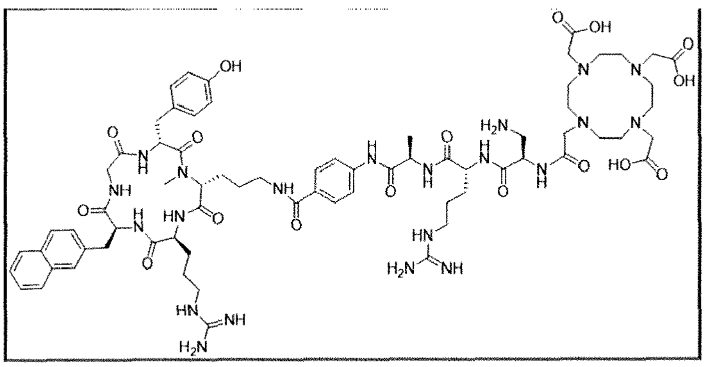

a compound of the following formula, a compound wherein the DOTA chelating moiety shown in the formula forms a chelate with a chelated radioactive or non-radioactive cation, or a salt of any of these:

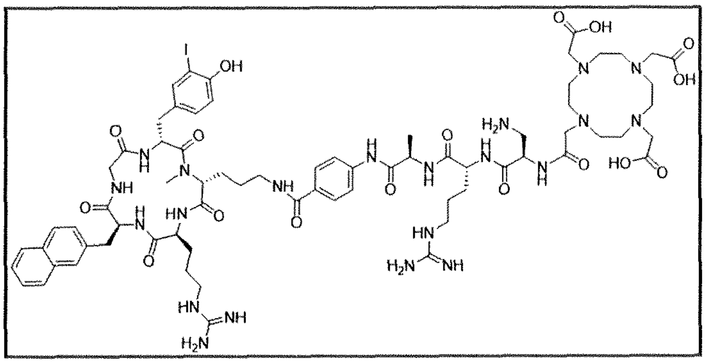

;

a compound of the following formula, a compound wherein the DOTA chelating moiety shown in the formula forms a chelate with a chelated radioactive or non-radioactive cation, or a salt of any of these:

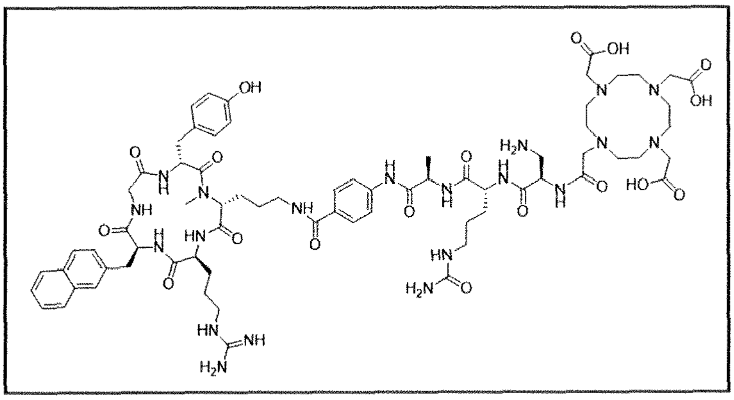

;

a compound of the following formula, a compound wherein the DOTAGA chelating moiety shown in the formula forms a chelate with a chelated radioactive or non-radioactive cation, or a salt of any of these:

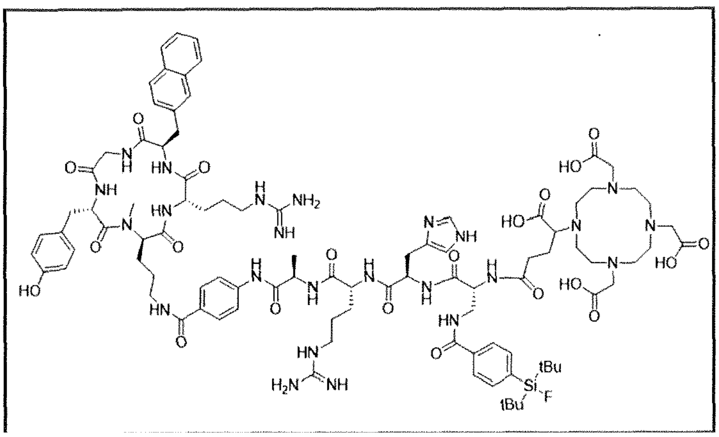

.

a compound of the following formula, a compound wherein any of the DOTAGA chelating moieties shown in the formula forms a chelate with a chelated radioactive or non-radioactive cation, or a salt of any of these:

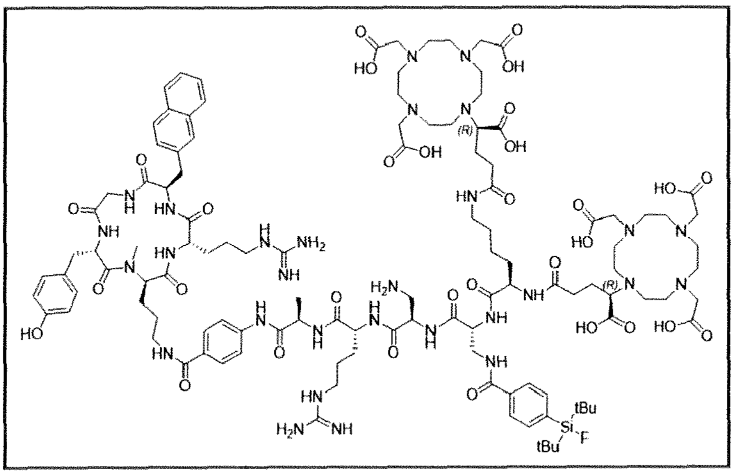

;

a compound of the following formula, a compound wherein the DOTAGA chelating moiety shown in the formula forms a chelate with a chelated radioactive or non-radioactive cation, or a salt of any of these:

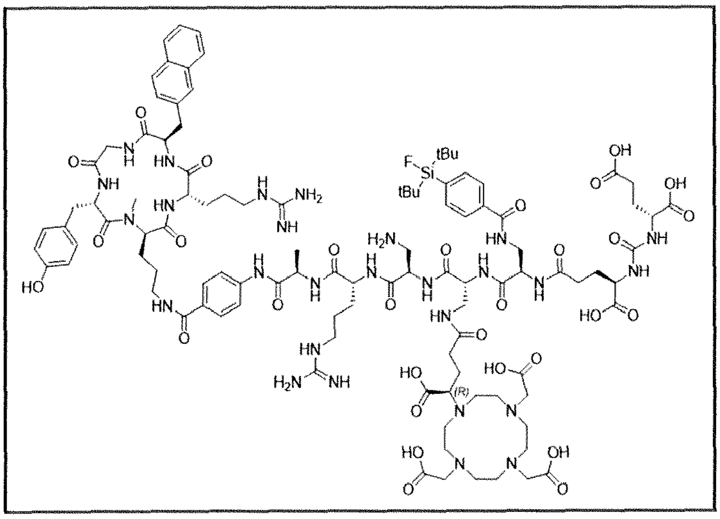

a compound of the following formula, a compound wherein the DOTAGA chelating moiety shown in the formula forms a chelate with a chelated radioactive or non-radioactive cation, or a salt of any of these:

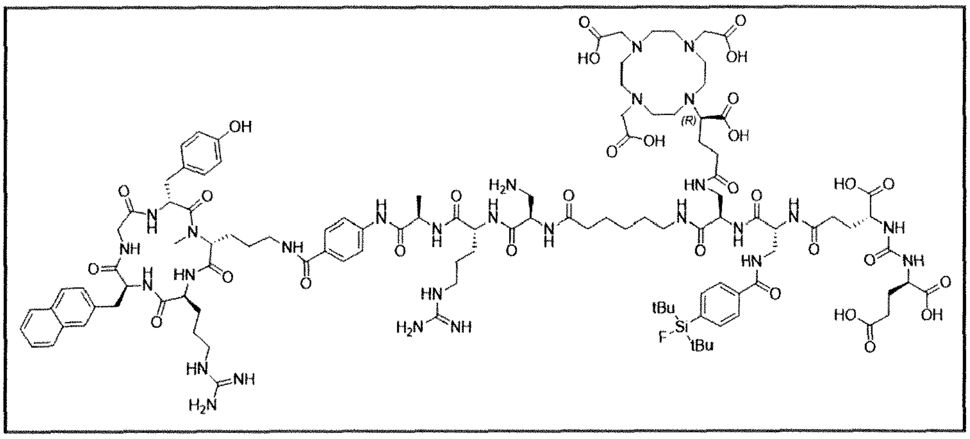

;

a compound of the following formula, a compound wherein the DOTAGA chelating moiety shown in the formula forms

a chelate with a chelated radioactive or non-radioactive cation, or a salt of any of these:

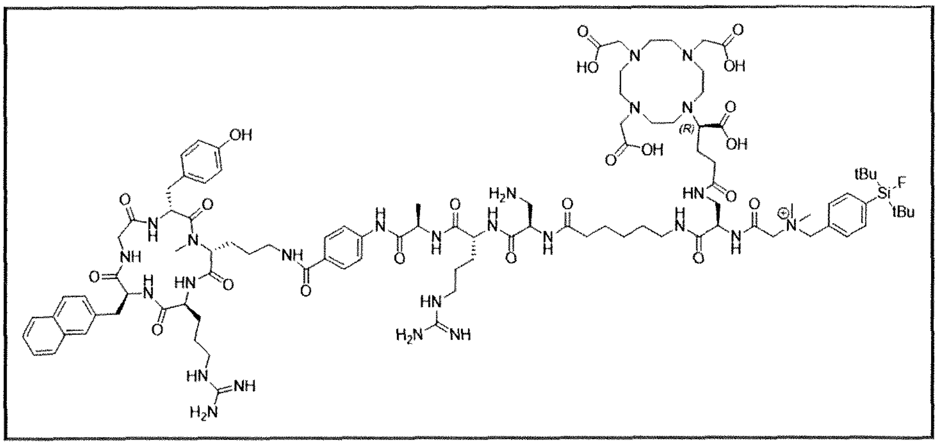

;

a compound of the following formula, a compound wherein the DOTA chelating moiety shown in the formula forms a chelate with a chelated radioactive or non-radioactive cation, or a salt of any of these:

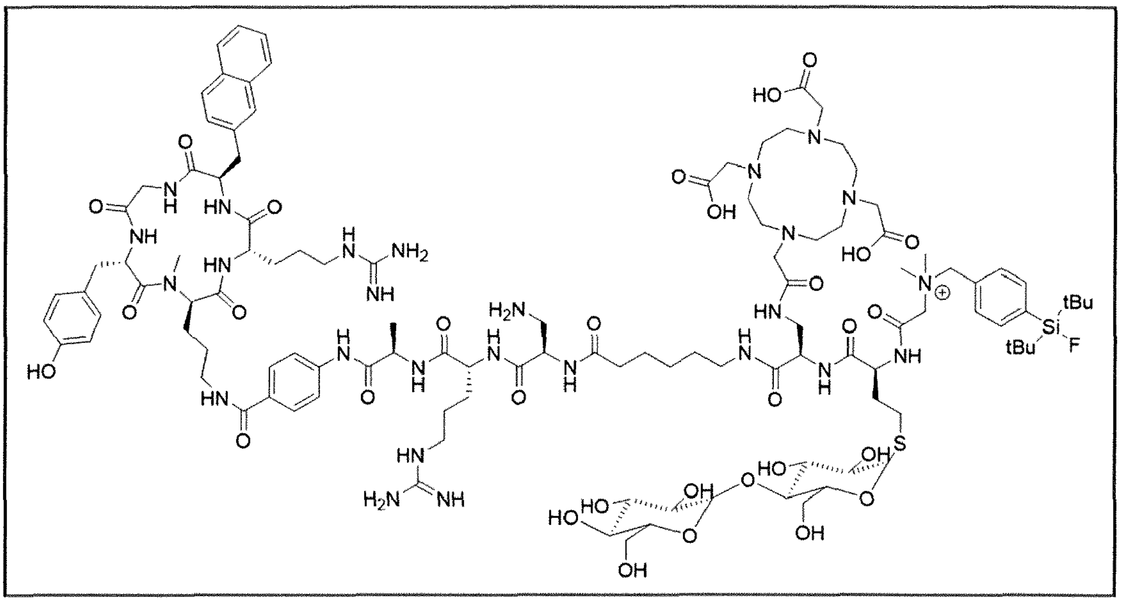

;

a compound of the following formula, or a salt thereof:

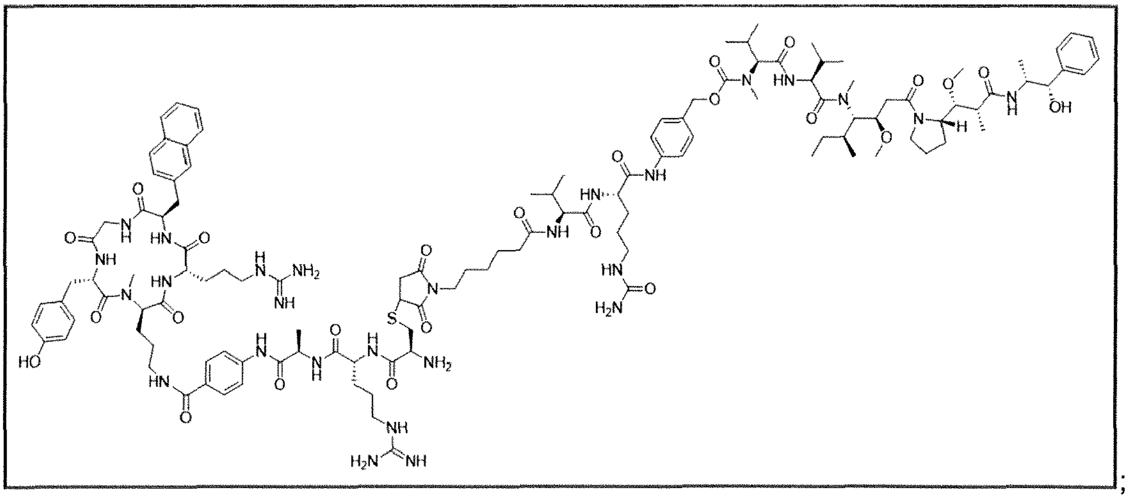

;

a compound of the following formula, or a salt thereof:

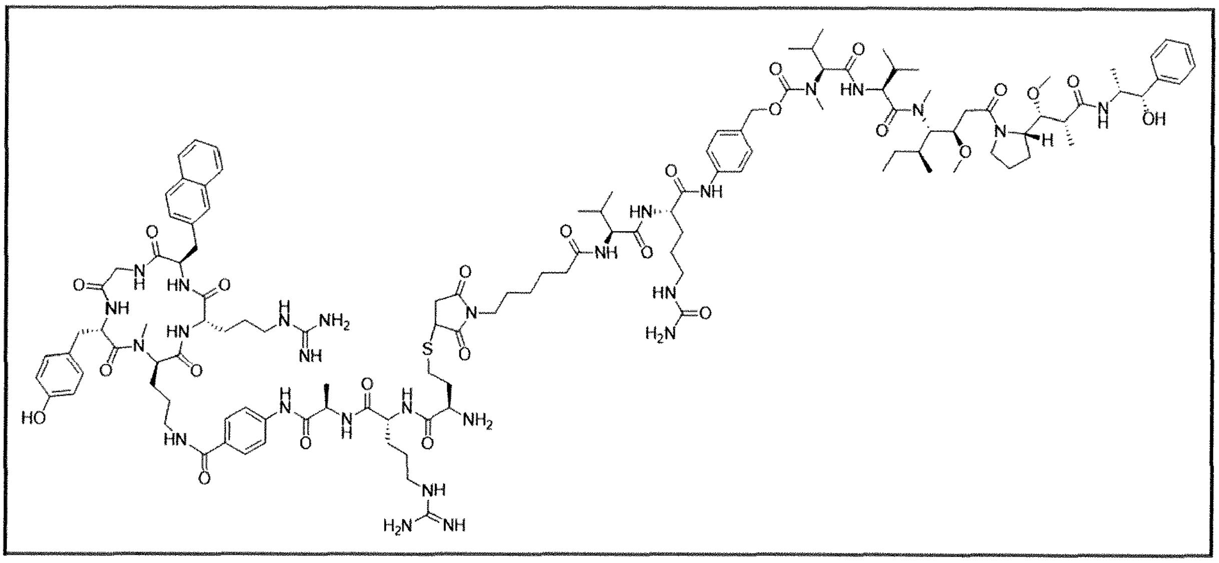

;

a compound of the following formula, or a salt thereof:

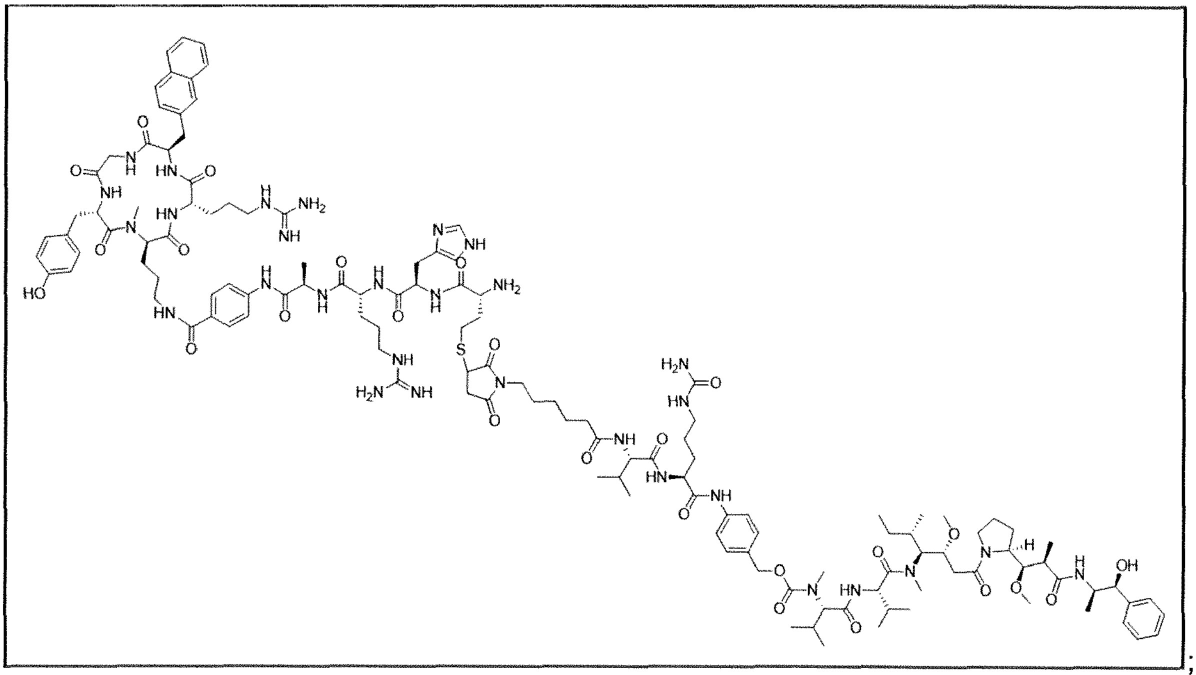

;

a compound of the following formula, a compound wherein the DOTAGA chelating moiety shown in the formula forms a chelate with a chelated radioactive or non-radioactive cation, or a salt of any of these:

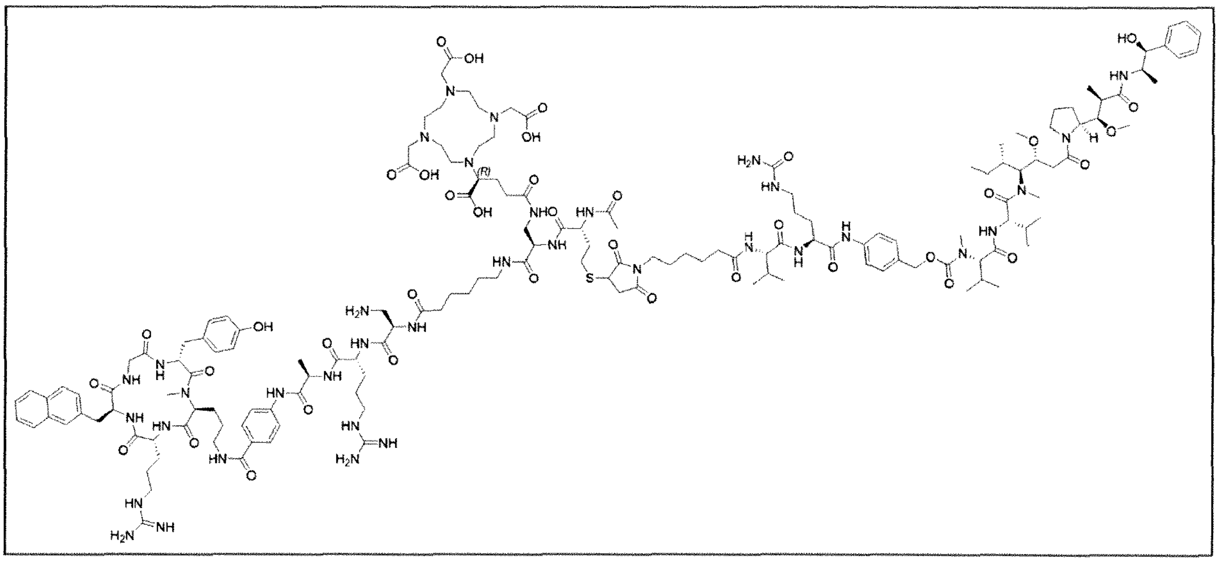

a compound of the following formula, or a salt thereof:

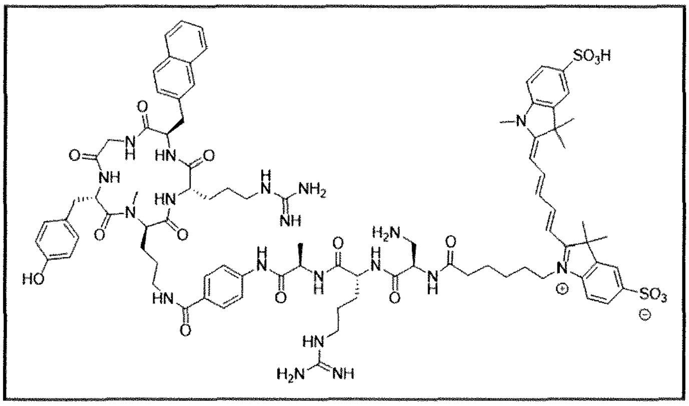

;

a compound of the following formula, or a salt thereof:

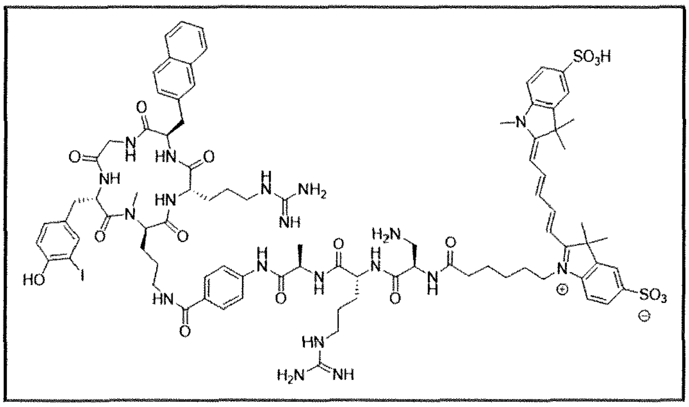

;

and

a compound of the following formula, a compound wherein the DOTA chelating moiety shown in the formula forms a chelate with a chelated radioactive or non-radioactive cation, or a salt of any of these:

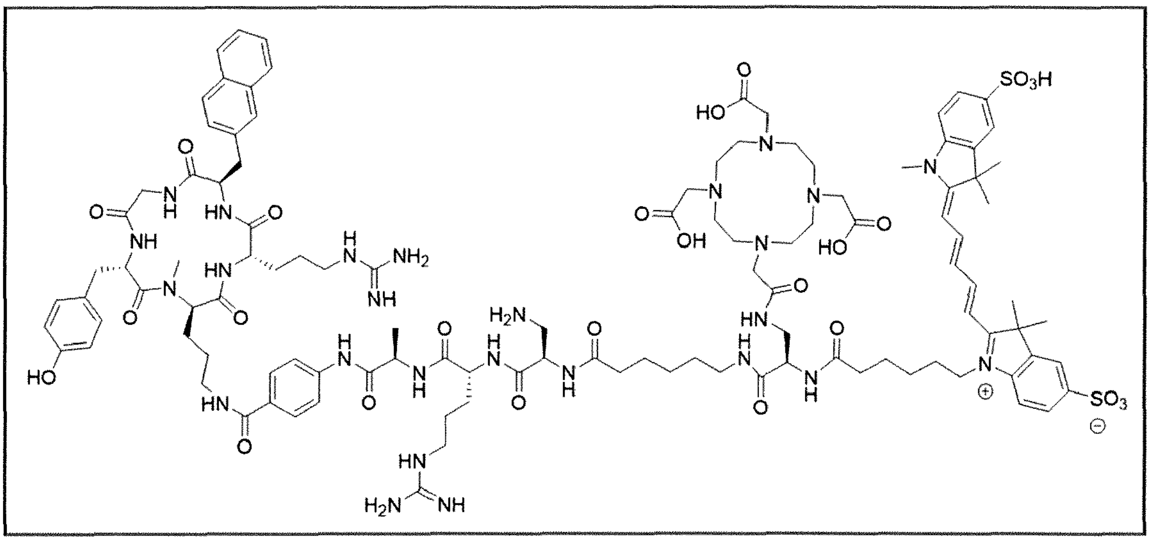

In a further aspect, the present invention provides a pharmaceutical composition (also referred to as a therapeutic composition) comprising or consisting of one or more types, preferably one type, of the ligand compound in accordance with the invention, i.e. a compound of formula (I) including any preferred embodiments thereof as discussed herein, or

a salt thereof. In a related aspect, the ligand compound in accordance with the invention is provided for use in therapy or for use as a medicament. Thus, the ligand compound of the invention can be used in a therapeutic method, which method may comprise administering the ligand compound to a subject. The subject may be a human or an animal and is preferably a human. It is to be understood that in accordance with these medical aspects of the invention the functional group $R^A$ generally comprises a moiety with therapeutic utility, e.g. a group which carries a radioactive element that has a therapeutic effect, or a cytotoxic moiety.

The therapy or therapeutic method referred to above aims at the treatment or prevention of a disease or disorder of the human or animal body, generally a disease or disorder that can be treated or prevented by blocking the CXCR4 receptor or which is associated with increased or aberrant expression of the CXCR4 receptor, such as cancer, a cardiovascular disorder or an inflammatory disorder. Thus, in terms of a therapeutic application, the compound of the invention is preferably provided for use in the treatment or prevention of cancer, an inflammatory disorder or a cardiovascular disorder, such as atherosclerosis, myocardial infarction, or stroke.

Due to the versatility provided by the invention in terms of the choice of the group $R^A$ in formula (I), the compounds of the invention can be conveniently adapted to various therapeutic approaches. For example, a compound in accordance with the invention comprising a chelating moiety capable of forming a chelate with a radioactive component, e.g. a radioactive metal cation, or a compound comprising such a chelate, can be used for radiotherapy, in particular targeted radioligand therapy (RLT). For radiotherapy, the chelated radioactive cation is preferably a gamma or beta emitter since they may emit a radiation dose in the target area that weakens or destroys particular targeted cells. Examples of gamma or beta emitters are $^{177}Lu$, $^{89}Zr$ and $^{186}Re$. As another example, a compound in accordance with the invention comprising a cytotoxic moiety may be provided for use in a therapy involving the chemotherapeutic destruction of cells, e.g. cancer cells.

In another aspect, the present invention provides a diagnostic composition comprising or consisting of one or more types, preferably one type, of the ligand compound in accordance with the invention, i.e. a compound of formula (I) including any preferred embodiments thereof as discussed herein, or a salt thereof. In a related aspect, the ligand compound in accordance with the invention is provided for use in a method of diagnosis *in vivo* of a disease or disorder. Thus, the ligand compound in accordance with the invention can be used in a method of diagnosis, which method may comprise administering the ligand compound to a subject and detecting the ligand compound in the subject, or monitoring the distribution of the ligand compound in the subject thereby detecting or monitoring the disease to be diagnosed. The subject may be a human or an animal and is preferably human. Alternatively, a method of diagnosis may also comprise adding the ligand compound to a sample, e.g. a physiological sample obtained from a subject *in vitro* or *ex vivo,* and detecting the ligand compound in the sample. It is to be understood that in accordance with these diagnostic aspects of the invention the functional group $R^A$ generally comprises a moiety with diagnostic utility, such as a group carrying a detectable radioactive element, or a fluorescent moiety.

The method of diagnosis referred to above aims at the identification of a disease or disorder of the human or animal body, generally a disease or disorder that can be treated or prevented by blocking the CXCR4 receptor or which is associated with increased expression of the CXCR4 receptor, such as cancer, a cardiovascular disorder or an inflammatory disorder. Thus, in terms of a diagnostic application, the compounds of the invention are preferably provided for use in a method of diagnosis *in vivo* of cancer, a cardiovascular disorder or an inflammatory disorder, e.g. a disorder such as atherosclerosis, myocardial infarction, or stroke.

Due to the versatility provided by the invention in terms of the choice of the group $R^A$ in formula (I), the compounds of the invention can be conveniently adapted to various diagnostic approaches. For example, a compound in accordance with the invention comprising a chelating moiety capable of forming a chelate with a radioactive component, e.g. a radioactive metal cation, a compound comprising such a chelate, or a compound comprising an $^{18}F$ atom, can be used for nuclear diagnostic imaging. For instance, if the compound in accordance with the invention comprises a positron emitter, such as a chelated $^{64}Cu$ cation, a chelated $^{68}Ga$ cation, or a $^{18}F$ atom bound in a SiFA moiety, the compound can be used for diagnosis via positron emission tomography (PET) imaging. Other compounds in accordance with the invention may be used for diagnosis via single photon emission computerised tomography (SPECT) imaging, e.g. a compound comprising a chelated $^{99m}Tc$ cation. As further examples, a compound in accordance with the present invention comprising a SiFA moiety can be used for diagnosis via $^{19}F$ MRI, or a compound in accordance with the invention comprising a fluorescent moiety can be used in a diagnostic method involving optical imaging.

It will be understood that suitability for a therapeutic and a diagnostic application is not mutually exclusive. i.e. a compound in accordance with the invention may be suitable for both applications and, thus, a functional group $R^A$ may comprise a moiety with both diagnostic and therapeutic utility, or a moiety with diagnostic utility and a moiety with therapeutic utility as separate moieties. For example, the compounds in accordance with the invention may comprise a radioactive species active in radiotherapy and diagnostic imaging. Moreover, the compounds of the invention encompass radiohybrid compounds which comprise both a SiFA moiety and a chelating moiety suitable to form a chelate with a therapeutically active radioactive cation. Such a radiohybrid compound may be used for diagnostic purposes if the SiFA moiety carries a $^{18}F$ atom, and the chelating moiety forms a chelate with a cold (non-radioactive) cation, and it may be

used for therapeutic purposes if the SiFA moiety carries a $^{19}F$ atom and the chelating moiety forms a chelate with a corresponding hot (radioactive) cation. Advantageously, if the radioactive and the non-radioactive cation are different isotopes of the same chemical species, the pharmacokinetic properties of the diagnostic and the therapeutic variant of the radiohybrid compound remain the same. Exemplary radiohybrid ligand compounds for use in diagnosis and therapy are a compound combining an $^{18}F$ atom/a $^{nat}Ga$ cation and compound combining a $^{19}F$ atom/a $^{68}Ga$ cation, a compound combining an $^{18}F$ atom/a $^{nat}Y$ cation and compound combining a $^{19}F$ atom/a $^{90}Y$ cation, or a compound combining an $^{18}F$ atom/a $^{nat}Lu$ cation and compound combining a $^{19}F$ atom/a $^{177}Lu$ cation.

The pharmaceutical or diagnostic composition may further comprise one or more pharmaceutically acceptable carriers, excipients and/or diluents. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well-known conventional methods. These compositions can be administered to the subject at a suitable dose. Administration of the suitable compositions may be accomplished in different ways, e.g., by intravenous, intraperitoneal, subcutaneous, intramuscular, topical, intradermal, intranasal or intrabronchial administration. It is particularly preferred that said administration is carried out by injection and/or delivery. The compositions may be administered directly to the target site. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Pharmaceutically active matter may be present e.g. in amounts between 0,1 ng and 10 mg/kg body weight per dose; however, doses below or above this exemplary range are envisioned, especially considering the aforementioned factors.

The following items summarize aspects of the invention. It will be understood that these items are closely related to the above parts of the description, and that the information provided in these items may supplement the above parts of the description and *vice versa.*

1. A compound of formula (I) or a salt thereof:

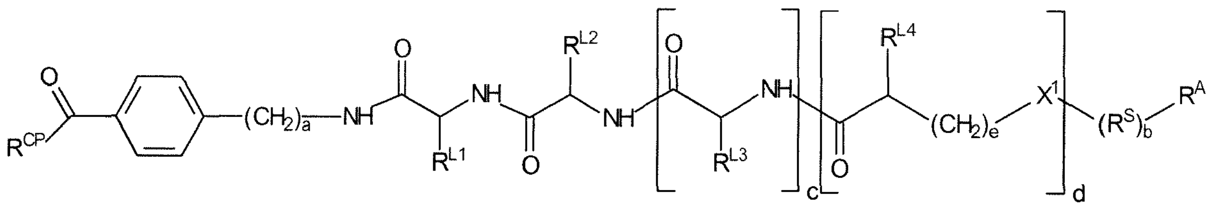

(I)

wherein:

| | |
|---|---|
| a | is 0 or 1, preferably 0; |
| b | is 0 or 1; |
| c | is 0 or 1, and |
| d | is 0 or 1, with the proviso that at least one of c and d is 1; |
| e | is an integer of 1 to 4, preferably 2 to 4; |
| $R^{CP}$ | is a cyclopeptide group of formula (II): |

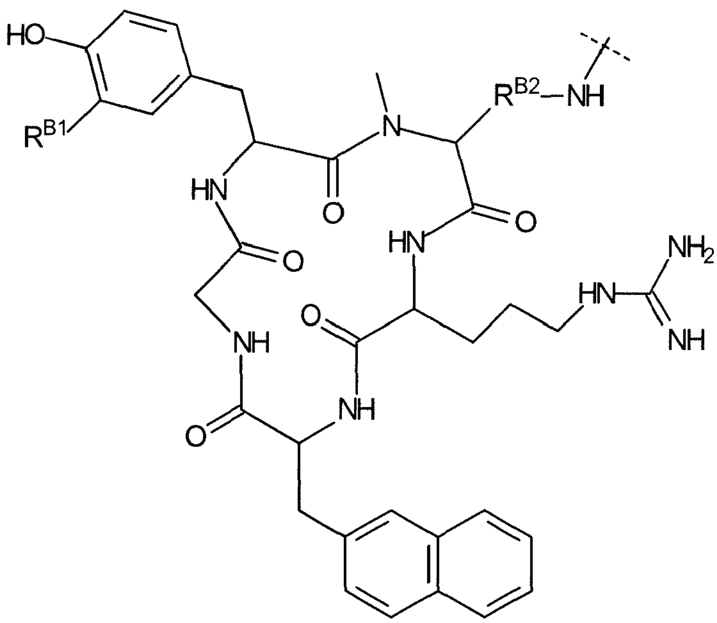

(II)

wherein, in formula (II)

$R^{B1}$ is H or I, preferably H;
$R^{B2}$ is an alkanediyl chain;
and wherein the dashed line marks a bond which attaches the group $R^{CP}$ to the remainder of the compound of formula (I);

$R^{L1}$ is H or alkyl;
$R^{L2}$ is substituted alkyl, which substituted alkyl is substituted with at least one group selected from $-NH_2$ and $-NH-C(=X)-NH_2$ with X being selected from NH and O;
$R^{L3}$ is $-CH_2-NH_2$ or $-CH_2-$(1*H*-imidazol-4-yl);
$R^{L4}$ is $-NH_2$;
$X^1$ is a coupling group;
$R^S$ is a divalent spacer group; and
$R^A$ is a functional group comprising a moiety with diagnostic or therapeutic utility.

2. The compound of item 1, wherein $R^{CP}$ in formula (I) is a group of formula (IIa):

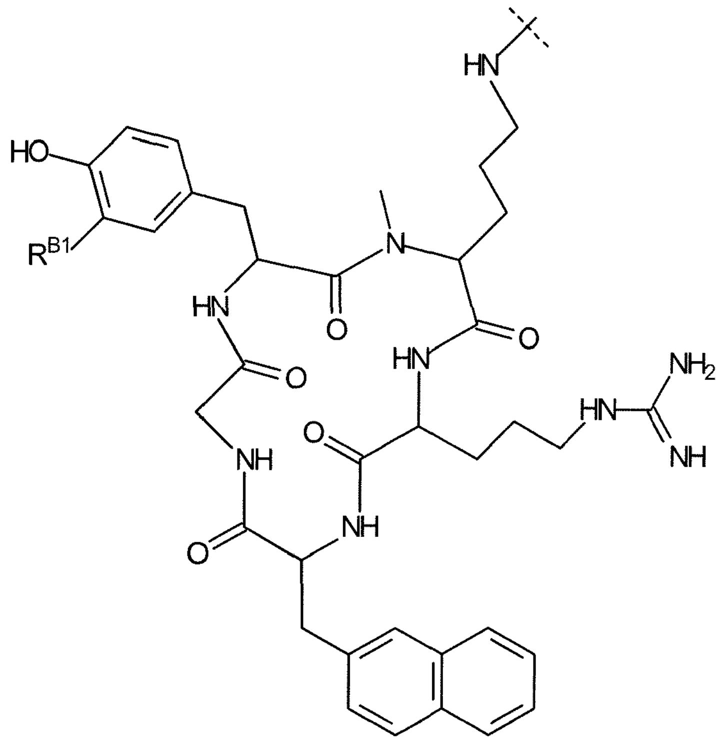

(IIa),

wherein $R^{B1}$ is defined as in item 1, and wherein the dashed line marks a bond which attaches the group $R^{CP}$ to the remainder of the compound of formula (I).

3. The compound or salt of item 1 or 2, wherein the group $R^{L1}$ in formula (I) is H or C1-C6 alkyl, more preferably H or C1-3 alkyl.

4. The compound or salt of item 3, wherein the group $R^{L1}$ in formula (I) is methyl.

5. The compound or salt of any of items 1 to 4, wherein $R^{L2}$ in formula (I) is C1-C6 alkyl, more preferably C1-C4 alkyl, and still more preferably C2-C4 alkyl, carrying one substituent which is selected from $-NH_2$ and the group -NH-C(=X)-$NH_2$, wherein X is NH or O.

6. The compound or salt of item 5, wherein $R^{L2}$ in formula (I) is a group selected from $-(CH_2)_A-NH_2$ and $-(CH_2)_A$-NH-C(=NH)-$NH_2$, wherein A is an integer of 1 to 6, preferably 1 to 4, and more preferably 2 to 4.

7. The compound or salt of item 6, wherein $R^{L2}$ in formula (I) is $-(CH_2)_3$-NH-C(=NH)-$NH_2$.

8. The compound or salt of any of items 1 to 7, wherein $R^{L3}$ in formula (I) is $-CH_2-NH_2$.

9. The compound or salt of any of items 1 to 8, wherein e in formula (I) is 2.

10. The compound or salt of any of items 1 to 9, wherein $X^1$ in formula (I) is -S-.

11. The compound or salt of any of items 1 to 10, wherein c in formula (I) is 1 and d in formula (I) is 0, or wherein c in formula (I) is 0 and d in formula (I) is 1.

12. The compound or salt of any of items 1 to 10, wherein c in formula (I) is 1 and d in formula (I) is 0.

13. The compound or salt of any of items 1 to 12 wherein b in formula (I) is 0.

14. The compound or salt of any of items 1 to 12, wherein $R^S$ in formula (I) is $-C(O)-(CH_2)_B$-NH-, wherein B is an integer of 3 to 10, preferably 4 to 6, and wherein the bond at the N-terminus is attached to $R^A$.

15. The compound or salt of any of items 1 to 14, wherein $R^A$ in formula (I) comprises one or two moieties with diagnostic or therapeutic utility.

16. The compound of or salt any of items 1 to 15, wherein the moiety with diagnostic or therapeutic utility comprised by $R^A$ in formula (I) is selected from:

(i) a chelating moiety;
(ii) a chelate formed by a chelating moiety (i) with a chelated radioactive or non-radioactive cation or anion, preferably a chelated radioactive or non-radioactive cation;
(iii) a silicon-fluoride acceptor (SiFA) moiety which comprises a silicon atom and a fluorine atom, wherein the fluorine atom is linked via a covalent bond directly to the silicon atom, and which SiFA moiety can be labeled with $^{18}F$ by isotopic exchange of $^{19}F$ by $^{18}F$ or which is labeled by $^{18}F$;
(iv) a cytotoxic moiety; and
(v) a fluorescent moiety.

17. The compound or salt of item 16, wherein $R^A$ in formula (I) comprises one of the moieties (i) to (v), or comprises a combination of one moiety selected from the chelating moiety (i) and the chelate (ii), and one SiFA moiety (iii).

18. The compound or salt of item 16 or 17, wherein the chelating moiety referred to in (i) and (ii) is a chelating moiety which is suitable as a chelate ligand for a cation selected from the cations of $^{43}Sc$, $^{44}Sc$, $^{47}Sc$, $^{51}Cr$, $^{52m}Mn$, $^{58}Co$, $^{52}Fe$, $^{56}Ni$, $^{57}Ni$, $^{nat}Cu$, $^{62}Cu$, $^{64}Cu$, $^{67}Cu$, $^{66}Ga$, $^{nat}Ga$, $^{68}Ga$, $^{67}Ga$, $^{89}Zr$, $^{90}Y$, $^{86}Y$, $^{94m}Tc$, $^{99m}Tc$, $^{97}Ru$, $^{105}Rh$, $^{109}Pd$, $^{111}Ag$, $^{110m}In$, $^{111}In$, $^{113m}In$, $^{114m}In$, $^{117m}Sn$, $^{121}Sn$, $^{127}Te$, $^{142}Pr$, $^{143}Pr$, $^{147}Nd$, $^{149}Gd$, $^{149}Pm$, $^{151}Pm$, $^{149}Tb$, $^{152}Tb$, $^{155}Tb$, $^{153}Sm$, $^{156}Eu$, $^{157}Gd$, $^{161}Tb$, $^{164}Tb$, $^{161}Ho$, $^{166}Ho$, $^{157}Dy$, $^{165}Dy$, $^{166}Dy$, $^{160}Er$, $^{165}Er$, $^{169}Er$, $^{171}Er$, $^{166}Yb$, $^{169}Yb$, $^{175}Yb$, $^{167}Tm$, $^{172}Tm$, $^{nat}Lu$, $^{177}Lu$, $^{186}Re$, $^{188}Re$, $^{188}W$, $^{191}Pt$, $^{195m}Pt$, $^{194}Ir$, $^{197}Hg$, $^{198}Au$, $^{199}Au$, $^{nat}Pb$, $^{212}Pb$, $^{203}Pb$, $^{211}At$, $^{212}Bi$, $^{213}Bi$, $^{223}Ra$, $^{224}Ra$, $^{225}Ac$, and $^{227}Th$, and from a cationic molecule comprising $^{18}F$, such as $^{18}F$-$[AlF]^{2+}$.

19. The compound or salt of any of items 16 to 18, wherein the chelating moiety referred to in (i) and (ii) is provided by a chelating agent selected from bis(carboxymethyl)-1,4,8,11-tetraazabicyclo[6.6.2]hexadecane (CBTE2a), cyclohexyl-1,2-diaminetetraacetic acid (CDTA), 4-(1,4,8,11-tetraazacyclotetradec-1-yl)-methylbenzoic acid (CPTA), N'-[5-[acetyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxymethyl)-1,4,8,11-tetraazabicycle[6.6.2]hexadecan (DO2A), 1,4,7,10-tetraazacyclododecan-N,N',N",N"'-tetraacetic acid (DOTA), 2-[1,4,7,10-tetraazacyclododecane-4,7,10-triacetic acid]-pentanedioic acid (DOTAGA or DOTA-GA), N,N'-dipyridoxylethylendiamine-N,N'-diacetate-5,5'-bis(phosphat) (DPDP), diethylenetriaminepentaacetic acid (DTPA), ethylenediamine-N,N'-tetraacetic acid (EDTA), ethyleneglykol-O,O-bis(2-aminoethyl)-N,N,N',N'-tetraacetic acid (EGTA), N,N-bis(hydroxybenzyl)-ethylenediamine-N,N'-diacetic acid (HBED), hydroxyethyldiaminetriacetic acid (HEDTA), 1-(p-nitrobenzyl)-1,4,7,10-tetraazacyclodecan-4,7,10-triacetate (HP-DOA3), 1,4,7-triazacyclononan-1-succinic acid-4,7-diacetic acid (NODASA), 1-(1-carboxy-3-carboxypropyl)-4,7-(carboxy)-1,4,7-triazacyclononane (NODAGA), 1,4,7-triazacyclononanetriacetic acid (NOTA), 4,11-bis(carboxymethyl)-1,4,8,11-tetraazabicyclo[6.6.2]hexadecane (TE2A), 1,4,8,11-tetraazacyclododecane-1,4,8,11-tetraacetic acid (TETA), terpyridine-bis(methyleneamine) tetraacetic acid (TMT), 1,4,7,10-tetraazacyclotridecan-N,N',N",N"'-tetraacetic acid (TRITA), and triethylenetetra-aminehexaacetic acid (TTHA), *N,N'*-bis[(6-carboxy-2-pyridyl)methyl]-4,13-diaza-18-crown-6 ($H_2$macropa), 4-amino-4-{2-[(3-hydroxy-1,6-dimethyl-4-oxo-1,4-dihydro-pyridin-2-ylmethyl)-carbamoyl]-ethyl} heptanedioic acid bis-[(3-hydroxy-1,6-dimethyl-4-oxo-1,4-dihydro-pyridin-2-ylmethyl)-amide] (THP), 1,4,7-triazacyclononane-1,4,7-tris[methylene(2-carboxyethyl)phosphinic acid (TRAP), 2-(4,7,10-tris(2-amino-2-oxoethyl)-1,4,7,10-tetraazacyclododecan-1-yl)acetic acid (DO3AM), and 1,4,7,10-tetraazacyclododecane-1,4,7, 10-tetrakis[methylene(2-carboxyethylphosphinic acid)] (DOTPI), S-2-(4-isothiocyanatobenzyl)-1,4,7,10-tetraazacyclododecane tetraacetic acid, mercaptoacetyl-triserine ($mas_3$), hydrazinonicotinic acid (HYNIC) and 3-(2-aminoethylamino)-2-[(2-aminoethylamino)methyl]propanoic acid (N4 chelator, 6-carboxy-1,4,8,11-tetraazaundecane), or by a modified mercaptoacetylserine chelating agent, wherein one or more of the serine residues are replaced by another amino acid containing a hydrophilic side chain.

20. The compound or salt of any of items 16 to 19, wherein $R^A$ comprises a chelating moiety which can form a chelate with a cation selected from a cation of $^{99m}Tc$, $^{177}Lu$ and $^{68}Ga$, or wherein $R^A$ comprises a chelate with a chelated cation selected from a cation of $^{99m}Tc$, $^{177}Lu$ and $^{68}Ga$.

21. The compound or salt of any of items 16 to 20, wherein the SiFA moiety (iii) comprises a group of formula (S-1):

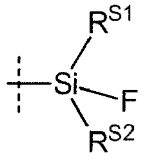

(S-1)

wherein

$R^{S1}$ and $R^{S2}$ are independently a linear or branched C3 to C10 alkyl group, preferably $R^{S1}$ and $R^{S2}$ are independently selected from isopropyl and *tert*-butyl, and more preferably $R^{S1}$ and $R^{S2}$ are both *tert*-butyl, and wherein the dashed bond attaches the group to the remainder of the compound of formula (I).

22. The compound or salt of item 21, wherein the SiFA moiety (iii) is selected from a group of formula (S-2) and a group of formula (S-3).

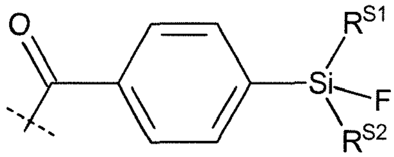

(S-2)

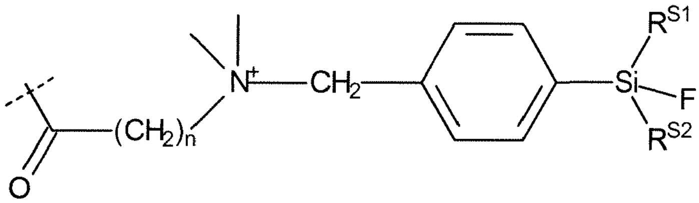

(S-3)

wherein

n is 1, 2, or 3 and is preferably 1, $R^{S1}$ and $R^{S2}$ are independently a linear or branched C3 to C10 alkyl group, preferably $R^{S1}$ and $R^{S2}$ are independently selected from isopropyl and *tert*-butyl, and more preferably $R^{S1}$ and $R^{S2}$ are both *tert*-butyl, and wherein the bond marked by the dashed line attaches the group to the remainder of the compound of formula (I).

23. The compound or salt of any of items 16 to 22, wherein the cytotoxic moiety (iv) is provided by a residue of an auristatin analogue, preferably selected from monomethyl auristatin E (MMAE), and monomethyl auristatin F (MMAF), or by a residue of PF-06380101.

24. The compound or salt of any of items 16 to 23, wherein the fluorescent moiety (v) is provided by a residue of a fluorescent dye, preferably a Cy5- or Cy7-based cyanine dye.

25. A pharmaceutical composition comprising or consisting of a compound or salt of any of items 1 to 24.

26. The compound or salt of any of items 1 to 24 for use as a medicament.

27. The compound or salt of any of items 1 to 24 for use in the treatment or prevention of a disease or disorder that can be treated or prevented by blocking the CXCR4 receptor, or of a disease or disorder that is associated with an increased or aberrant expression of the CXCR4 receptor.

28. The compound or salt of any of items 1 to 24 and 27 for use in the treatment or prevention of cancer, a cardiovascular disorder or an inflammatory disorder.

29. A diagnostic composition comprising or consisting of a compound or salt of any of items 1 to 24.

30. The compound or salt of any of items 1 to 24 for use in a method of diagnosis *in vivo* of a disease or disorder.

31. The compound or salt of any of items 1 to 24 for use in a method of diagnosis *in vivo* of a disease or disorder that can be treated or prevented by blocking the CXCR4 receptor or of a disease or disorder that is associated with an increased or aberrant expression of the CXCR4 receptor.

32. The compound or salt of any of items 1 to 24 and 31 for use in a method of diagnosis *in vivo* of cancer, a cardiovascular disorder or an inflammatory disorder.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

**References**

1. Zlotnik A, Yoshie O. Chemokines. Immunity. 2000;12:121-127. doi:10.1016/s1074-7613(00)80165-x.
2. Domanska UM, Kruizinga RC, Nagengast WB, et al. A review on CXCR4/CXCL12 axis in oncology: No place to hide. Eur J Cancer. 2013;49:219-230. doi:10.1016/j.ejca.2012.05.005.
3. Feng Y, Broder CC, Kennedy PE, Berger EA. HIV-1 entry cofactor: Functional cDNA cloning of a seven-transmembrane, G protein-coupled receptor. Science. 1996;272:872-877. doi:10.1126/science.272.5263.872.
4. Nagasawa T, Hirota S, Tachibana K, et al. Defects of B-cell lymphopoiesis and bone-marrow myelopoiesis in mice lacking the CXC chemokine PBSF/SDF-1. Nature. 1996;382:635-638. doi:10.1038/382635a0.
5. Loetscher P, Moser B, Baggiolini M. Chemokines and Their Receptors in Lymphocyte Traffic and HIV Infection. In: Vol. 74: Elsevier; 1999:127-180. Advances in Immunology.
6. Aiuti A, Webb IJ, Bleul C, Springer T, Gutierrez-Ramos JC. The chemokine SDF-1 is a chemoattractant for human CD34+ hematopoietic progenitor cells and provides a new mechanism to explain the mobilization of CD34+ progenitors to peripheral blood. J Exp Med. 1997;185:111-120. doi:10.1084/jem.185.1.111.
7. Burger JA, Burger M, Kipps TJ. Chronic lymphocytic leukemia B cells express functional CXCR4 chemokine receptors that mediate spontaneous migration beneath bone marrow stromal cells. Blood. 1999;94:3658-3667.
8. Müller A, Homey B, Soto H, et al. Involvement of chemokine receptors in breast cancer metastasis. Nature. 2001;410:50-56. doi:10.1038/35065016.
9. Burger JA, Kipps TJ. CXCR4: A key receptor in the crosstalk between tumor cells and their microenvironment. Blood. 2006;107:1761-1767. doi:10.1182/blood-2005-08-3182.
10. Chatterjee S, Behnam Azad B, Nimmagadda S. The intricate role of CXCR4 in cancer. Adv Cancer Res. 2014;124:31-82. doi:10.1016/B978-0-12-411638-2.00002-1.
11. Guo F, Wang Y, Liu J, Mok SC, Xue F, Zhang W. CXCL12/CXCR4: A symbiotic bridge linking cancer cells and their stromal neighbors in oncogenic communication networks. Oncogene. 2016;35:816-826. doi:10.1038/onc.2015.139.
12. Orimo A, Gupta PB, Sgroi DC, et al. Stromal fibroblasts present in invasive human breast carcinomas promote tumor growth and angiogenesis through elevated SDF-1/CXCL12 secretion. Cell. 2005;121:335-348. doi:10.1016/j.cell.2005.02.034.
13.Tamamura H, Xu Y, Hattori T, et al. A low-molecular-weight inhibitor against the chemokine receptor CXCR4: A strong anti-HIV peptide T140. Biochem Biophys Res Commun. 1998;253:877-882. doi:10.1006/bbrc.1998.9871.
14. Tamamura H, Kuroda M, Masuda M, et al. A comparative study of the solution structures of tachyplesin I and a novel anti-HIV synthetic peptide, T22 ([Tyr5,12, Lys7]-polyphemusin II), determined by nuclear magnetic resonance. Biochimica et Biophysica Acta (BBA) - Protein Structure and Molecular Enzymology. 1993;1163:209-216. doi:10.1016/0167-4838(93)90183-R.
15. Tamamura H, Waki M, Imai M, et al. Downsizing of an HIV-cell fusion inhibitor, T22 ([Tyr 5, 12 , Lys 7]-Polyphemusin II), with the maintenance of anti-HIV activity and solution structure 1. Bioorganic & Medicinal Chemistry. 1998;6:473-479. doi:10.1016/s0968-0896(97)10055-4.
16. Tamamura H, Hiramatsu K, Mizumoto M, et al. Enhancement of the T140-based pharmacophores leads to the development of more potent and bio-stable CXCR4 antagonists. Org Biomol Chem. 2003;1:3663-3669. doi:10.1039/b306613b.
17. George GPC, Stevens E, Aberg O, et al. Preclinical evaluation of a CXCR4-specific (68)Ga-labelled TN14003 derivative for cancer PET imaging. Bioorganic & Medicinal Chemistry. 2014;22:796-803. doi:10.1016/j.bmc.2013.12.012.
18. Jacobson O, Weiss ID, Kiesewetter DO, Farber JM, Chen X. PET of tumor CXCR4 expression with 4-18F-T140.

J Nucl Med. 2010;51:1796-1804. doi:10.2967/jnumed.110.079418.

19. Yan X, Niu G, Wang Z, et al. Al18FNOTA-T140 Peptide for Noninvasive Visualization of CXCR4 Expression. Mol Imaging Biol. 2016;18:135-142. doi:10.1007/s11307-015-0872-2.

20. Peng S-B, Zhang X, Paul D, et al. Inhibition of CXCR4 by LY2624587, a Fully Humanized Anti-CXCR4 Antibody Induces Apoptosis of Hematologic Malignancies. PLoS ONE. 2016:11:e0150585.doi:10.1371/journal.pone.0150585.

21. Portella L, Vitale R, Luca S de, et al. Preclinical development of a novel class of CXCR4 antagonist impairing solid tumors growth and metastases. PLoS ONE. 2013;8:e74548. doi:10.1371/journal.pone.0074548.

22. Tsutsumi H, Tanaka T, Ohashi N, et al. Therapeutic potential of the chemokine receptor CXCR4 antagonists as multifunctional agents. Biopolymers. 2007;88:279-289. doi:10.1002/bip.20653.

23. Tamamura H, Araki T, Ueda S, et al. Identification of novel low molecular weight CXCR4 antagonists by structural tuning of cyclic tetrapeptide scaffolds. J Med Chem. 2005;48:3280-3289. doi:10.1021/jm050009h.

24. Tamamura H, Esaka A, Ogawa T, et al. Structure-activity relationship studies on CXCR4 antagonists having cyclic pentapeptide scaffolds. Org Biomol Chem. 2005;3:4392-4394. doi:10.1039/b513145f.

25. Tanaka T, Nomura W, Narumi T, et al. Structure-activity relationship study on artificial CXCR4 ligands possessing the cyclic pentapeptide scaffold: The exploration of amino acid residues of pentapeptides by substitutions of several aromatic amino acids. Org Biomol Chem. 2009;7:3805-3809. doi:10.1039/b908286g.

26. Narumi T, Hayashi R, Tomita K, et al. Synthesis and biological evaluation of selective CXCR4 antagonists containing alkene dipeptide isosteres. Org Biomol Chem. 2010;8:616-621. doi:10.1039/b917236j.

27. Ueda S, Oishi S, Wang Z-x, et al. Structure-activity relationships of cyclic peptide-based chemokine receptor CXCR4 antagonists: Disclosing the importance of side-chain and backbone functionalities. J Med Chem. 2007;50:192-198. doi:10.1021/jm0607350.

28. Wu B, Chien EYT, Mol CD, et al. Structures of the CXCR4 chemokine GPCR with small-molecule and cyclic peptide antagonists. Science. 2010;330:1066-1071. doi:10.1126/science.1194396.

29. Demmer O, Dijkgraaf I, Schumacher U, et al. Design, synthesis, and functionalization of dimeric peptides targeting chemokine receptor CXCR4. J Med Chem. 2011;54:7648-7662. doi:10.1021/jm2009716.

30. Tanaka T, Nomura W, Narumi T, Masuda A, Tamamura H. Bivalent ligands of CXCR4 with rigid linkers for elucidation of the dimerization state in cells. J Am Chem Soc. 2010;132:15899-15901. doi:10.1021/ja107447w.

31.Ávila-Sánchez M, Ferro-Flores G, Jiménez-Mancilla N, et al. Synthesis and preclinical evaluation of the 99mTc-/177Lu-CXCR4-L theranostic pair for in vivo chemokine-4 receptor-specific targeting. J Radioanal Nucl Chem. 2020;324:21-32. doi:10.1007/s10967-020-07043-6.

32. Vallejo-Armenta P, Santos-Cuevas C, Soto-Andonaegui J, et al. 99mTc-CXCR4-L for Imaging of the Chemokine-4 Receptor Associated with Brain Tumor Invasiveness: Biokinetics, Radiation Dosimetry, and Proof of Concept in Humans. Contrast Media Mol Imaging. 2020;2020:2525037. doi:10.1155/2020/2525037.

33. Demmer O, Dijkgraaf I, Schottelius M, Wester H-J, Kessler H. Introduction of functional groups into peptides via N-alkylation. Org Lett. 2008;10:2015-2018. doi:10.1021/ol800654n.

34. Demmer O, Gourni E, Schumacher U, Kessler H, Wester H-J. PET imaging of CXCR4 receptors in cancer by a new optimized ligand. ChemMedChem. 2011;6:1789-1791. doi:10.1002/cmdc.201100320.

35. Gourni E, Demmer O, Schottelius M, et al. PET of CXCR4 expression by a (68)Galabeled highly specific targeted contrast agent. J Nucl Med. 2011;52:1803-1810. doi:10.2967/jnumed.111.098798.

36. Poschenrieder A, Schottelius M, Schwaiger M, Kessler H, Wester H-J. The influence of different metal-chelate conjugates of pentixafor on the CXCR4 affinity. EJNMMI Res. 2016;6:36. doi:10.1186/s13550-016-0193-8.

37. Herrmann K, Schottelius M, Lapa C, et al. First-in-Human Experience of CXCR4-Directed Endoradiotherapy with 177Lu- and 90Y-Labeled Pentixather in Advanced-Stage Multiple Myeloma with Extensive Intra- and Extramedullary Disease. J Nucl Med. 2016;57:248-251. doi:10.2967/jnumed.115.167361.

38. Osl T, Schmidt A, Schwaiger M, Schottelius M, Wester H-J. A new class of PentixaFor- and PentixaTher-based theranostic agents with enhanced CXCR4-targeting efficiency. Theranostics. 2020;10:8264-8280. doi:10.7150/thno.45537.

39. Schottelius M, Konrad M, Osl T, Poschenrieder A, Wester H-J. An optimized strategy for the mild and efficient solution phase iodination of tyrosine residues in bioactive peptides. Tetrahedron Letters. 2015;56:6602-6605. doi:10.1016/j.tetlet.2015.10.032.

40. Demmer O, Frank AO, Hagn F, et al. Erhöhte CXCR4-Affinität und Anti-HIV-Aktivität eines Peptoids durch Konformationsfixierung. Angew. Chem. 2012;124:8234-8237. doi:10.1002/ange.201202090.

41. Chatterjee J, Gilon C, Hoffman A, Kessler H. N-methylation of peptides: A new perspective in medicinal chemistry. Acc Chem Res. 2008;41:1331-1342. doi:10.1021/ar8000603.

42. Weineisen M, Simecek J, Schottelius M, Schwaiger M, Wester H-J. Synthesis and preclinical evaluation of DOTAGA-conjugated PSMA ligands for functional imaging and endoradiotherapy of prostate cancer. EJNMMI Res. 2014;4:63. doi:10.1186/s13550-014-0063-1.

43. Iovkova L, Wängler B, Schirrmacher E, et al. para-Functionalized aryl-di-tert-butylfluorosilanes as potential labeling synthons for (18)F radiopharmaceuticals. Chemistry. 2009;15:2140-2147. doi:10.1002/chem.200802266.
44. Kostikov AP, Iovkova L, Chin J, et al. N-(4-(di-tert-butyl[18F]fluorosilyl)benzyl)-2-hydroxy-N,N-dimethylethylammonium bromide ([18F]SiFAN+Br-): A novel lead compound for the development of hydrophilic SiFA-based prosthetic groups for 18F-labeling. Journal of Fluorine Chemistry. 2011;132:27-34. doi:10.1016/j.jfluchem.2010.10.008.
45. Abrams MJ, Juweid M, tenKate CI, et al. Technetium-99m-human polyclonal IgG radiolabeled via the hydrazino nicotinamide derivative for imaging focal sites of infection in rats. J Nucl Med. 1990;31:2022-2028.
46. Joyard Y, Bischoff L, Levacher V, Papamicael C, Vera P, Bohn P. Synthesis and Stability Evaluation of New HYNIC Derivatives as Ligands for Technetium-99m. LOC. 2014;11:208-214. doi:10.2174/15701786113106660087.
47. Schottelius M, Schwaiger M, Wester H-J. Rapid and high-yield solution-phase synthesis of DOTA-Tyr3-octreotide and DOTA-Tyr3-octreotate using unprotected DOTA. Tetrahedron Letters. 2003;44:2393-2396. doi:10.1016/S0040-4039(03)00221-1.
48. Schottelius M, Osl T, Poschenrieder A, et al. 177Lupentixather: Comprehensive Preclinical Characterization of a First CXCR4-directed Endoradiotherapeutic Agent. Theranostics. 2017;7:2350-2362. doi:10.7150/thno.19119.
49. Robu S, Schottelius M, Eiber M, et al. Preclinical Evaluation and First Patient Application of 99mTc-PSMA-I&S for SPECT Imaging and Radioguided Surgery in Prostate Cancer. J Nucl Med. 2017;58:235-242. doi:10.2967/jnumed.116.178939.
50. Kuzmanovska S, Vaskova O, Zdraveska Kocovska M. "In-house" preparation of 99mTc-EDDA/HYNIC-TOC, a specific targeting agent for somatostatin receptor scintigraphy. Maced. Pharm. Bull. 2011;57:65-70. doi:10.33320/maced.pharm.bull.2011.57.007.
51. Wängler C, Niedermoser S, Chin J, et al. One-step (18)F-labeling of peptides for positron emission tomography imaging using the SiFA methodology. Nat Protoc. 2012;7:1946-1955. doi:10.1038/nprot.2012.109.
52. Othman MFB, Mitry NR, Lewington VJ, Blower PJ, Terry SYA. Re-assessing gallium-67 as a therapeutic radionuclide. Nucl Med Biol. 2017;46:12-18. doi: 10.1016/j.nucmedbio.2016.10.008.
53. Yamazaki K, Kanaoka M. Computational prediction of the plasma protein-binding percent of diverse pharmaceutical compounds. J Pharm Sci. 2004;93:1480-1494. doi:10.1002/jps.20059.

**List of abbreviations used**

%iD/g *percent of the injected dose per gram*
*2-CTC 2-Chlorotritylchloride*
AA *Amino acid*
Abz *para-Aminobenzoic acid*
*ACN Acetonitrile*
AKT *Protein kinase B*
Ambz *para-Aminomethylbenzoic acid*
Boc *tert-butyl Oxycarbonyl*
CDI *Carbonyldiimidazole*
CPCR4 *CYCLO(D-TYR-D-[NME]ORN-ARG-2-NAL-GLY)*
CT *Computed tomography*
CXCR4 *C-X-C chemokine receptor type 4*
dap *2,3-diamino propionic acid*
DBU *1,8-Diazabicyclo[5.4.0]undec-7-en*
DCE *Dichloroethane*
DCM *Dichloromethane*
Dde *N-(1-(4,4-dimethyl-2,6-dioxocyclohexylidene)ethyl)*
DIAD *Diisopropyl azodicarboxylate*
DIPEA *N,N-Diisopropylethyl amine*
DMAP *4-(Dimethylamino)-pyridine*
*DMEM Dulbecco's modified Eagle's medium*
DMF *N,N-Dimethylformamide*
DMG *Dimethylglycine*
DMSO *Dimethylsulfoxide*
DOTA *1,4,7,10-Tetraazacyclododecane-1,4,7,10-tetraacetic acid*
*DOTA-GA 5-(tert-butoxy)-5-oxo-4-(4, 7, 10-tris(2-(tert-butoxy)-2-oxoethyl)-1,4, 7,10-tetraazacyclododecan-1-yl)pentanoic acid*

DPPA *Diphenylphosphorylazide*
EDDA *Ethylendiaminediacetic acid*
*EDTA Ethylendiaminetetraacetic acid*
$Et_2O$ *Diethylether*
EtOAc *Ethylacetate*
EtOH *Ethanol*
EUE *(R)-5-(TERT-BUTOXY)-4-(3-((R)-1,5-DI-TERT-BUTOXY-1,5-DIOXOPENTAN-2-YL)UREIDO)-5-OXOPENTANOIC ACID*
*FBS Fetal bovine serum, fetal bovine serum*
HATU *[O-(7-Azabenzotriazol-1-yl)-N,N,N',N'-tetramethyluronium-hexafluorphosphat]*
*HBSS Hank's buffered salt solution*
HFIP *1,1,1,3,3,3-Hexafluoro-2-propanol*
HIV *Human immunodeficiency virus*
HOAt *1-Hydroxy-7-azabenzotriazol*
HOBt *1-Hydroxybenzotriazole*
HPLC *High-pressure liquid chromatography*
HSA *Human serum albumine*
HYNIC *Hydrazinonicotinic acid*
MAPK *mitogen-activated protein kinase*
$mas_3$ *mercaptoacetyl-triserine*
MMAE *Monomethylauristatine*
NEA *Non-essential amino acids*
NIS *N-Iodosuccinimide*
NMP *N-Methyl-2-pyrrolidon*
OI *Optical imaging*
*O*-NBS-Cl *2-Nitrobenzene sulfonylchloride*
p.i. *post injection*
Pbf *2,2,4,6,7-Pentamethyldihydrobenzofuran-5-suifonyl*
*PBS Phosphate Buffered Saline*
PCC *Pyridinium chlorochromate*
PenStrep *Pennicillin-Streptomycin mixture*
PET *Positrone-emission tomography*
r.t. *room temperature*
*RP-HPLC Reversed-phase high pressure liquid chromatography*
*RPMI Rosewell park memorial institute*
SAR *structure-activity relationship*
sat. *saturated*
SCID *Severe combined immunodeficiency*
SDF-1 *Stromal cell-derived factor 1*
SiFA *Silicium-fluoride acceptor*
SiFA-BA *SiFA-benzoic acid*
SiFA-Br *SiFA-bromide*
SPECT *Single-photon-emission tomography*
*SPPS solid-phase peptide synthesis*
t.b.d. *to be determined*
TBDMSCI *tert-butyldimethylsilyl chloride*
TBTU *2-(1H-Benzotriazole-1-yl)-1,1,3,3-tetramethylaminium tetrafluoroborate*
*tB*u *tert-Butyl*
TEA *Triethylamine*
*TFA Trifluoroacetic acid*
THF *Tetrahydrofurane*
TIPS *Triisopropylsilane*
TLC *Thin-layer chromatography*
$t_R$ *retention time*
Trt *Trityl*

## Examples

### I. Materials and Methods

### 1. GENERAL INFORMATION

#### *1.1 REAGENTS AND SOLVENTS*

Purchased reagents were used without further purification. The Fmoc-(9-fluorenylmethoxycarbonyl-) and all other protected amino acid analogs were purchased from Bachem (Bubendorf, Switzerland), Iris Biotech GmbH (Marktredwitz, Germany), Carbolution Chemicals GmbH (St. Ingbert, Germany) and Merck Millipore (Darmstadt, Germany). The 2-Chlorotrityl chloride (2-CTC) resin was obtained from Iris Biotech GmbH (Marktredwitz, Germany) or CEM (Matthews, USA). Reagents for peptide synthesis were purchased from Iris Biotech GmbH (Marktredwitz, Germany), Sigma-Aldrich (Munich, Germany) and Molekula GmBH (Garching, Germany). Solvents and reagents for organic synthesis were purchased from either Alfa Aesar (Karlsruhe, Germany), Sigma-Aldrich (Munich, Germany) or VWR (Darmstadt, Germany).

Chematech (Dijon, France) delivered the used DOTA-GA chelators and DOTA derivatives was provided by Macrocyclics (Plano, USA). The Cy5-carboxylic acid was obtained from Lumiprobe (Hunt Valley, USA). Cytotoxic MMAE derivatives were purchased from Creative Biolabs (Shirley, USA).

Biochemicals, such as DMEM (Ham's F-12, with stable Gin) and RPMI 1640 (w. Gln) medium, fetal bovine serum (FBS superior), phosphate-buffered saline (PBS Dulbecco, w/o $Ca^{2+}$, $Mg^{2+}$), trypsine/EDTA (0.05%/0.02% in PBS, w/o $Ca^{2+}$, $Mg^{2+}$) and Hank's buffered salt solution (HBSS, with 0.35 g/L $NaHCO_3$ and $Ca^{2+}$, $Mg^{2}$) were obtained from Biochrom GmbH (Berlin, Germany) or Sigma-Aldrich (Munich, Germany).

Water for RP-HPLC solvents was obtained from the in-house Millipore system from Thermo Fischer Scientific Inc. (Waltham MA, USA). Tracepure water for labeling experiments was received from Merck Millipore (Darmstadt, Germany).

#### 1.2 *RADIOACTIVE ISOTOPES*

Labeling with $^{125}$I was carried out with a [$^{125}$I]NaI solution in NaOH (40 mM, 74 TBq/mmol) from *Hartmann Analytik GmbH* (Braunschweig, Deutschland).

[$^{99m}$Tc]-Pertechnetate was obtained by elution of a Drytech™ Technetium Generator from GE *Healthcare* (Munich, Germany) with physiological NaCl solution (0.9%; v/v). The generator was provided by the *Klinikum Rechts der Isar* (Technical University Munich, Munich, Germany).

A solution of [$^{177}$Lu]$LuCl_3$ (HCl (0.04 M); $S_A$ > 3 TBq/mg, 740 MBq/mL) was provided *by ITM GmbH* (Garching, Germany) and used directly for labeling experiments.

[$^{18}$F]-Fluoride in target water was provided by the *Klinikum Rechts der Isar* (Technical University Munich, Munich, Germany).

[$^{68}$Ga]$GaCl_3$ for radiosynthesis was obtained by elution of a $^{68}$Ge/$^{68}$Ga-generator from *iThemba LABS* (Cape Town, South Afrika) with aqueous HCl (1.00 M). Synthesis was carried out on an automated GallElut$^+$ sytem from *Scintomics GmbH* (Fürstenfeldbruck, Germany).

[$^{67}$Ga]Ga-citrate was delivered by *Mallinckrodt Pharmaceuticals* (Dublin, Ireland) and converted to [$^{67}$]$GaCl_3$ prior to radiosynthesis.

#### 1.3 *INSTRUMENTS AND ANALYTICS*

*Solid-phase peptide synthesis (SPPS) was carried out by manual operation using an Intelli-Mixer syringe shaker from Neolab (Heidelberg, Germany).*

Eluents for all RP-HPLC operations were water (solvent A) and acetonitrile (solvent B), both containing 0.1 vol% trifluoroacetic acid. For semi-preparative RP-HPLC runs, solvent B was used with 5 vol% $H_2O$. Analytical and semi-preparative reversed-phase high pressure chromatography (RP-HPLC) runs were performed using Shimadzu gradient systems from *Shimadzu Deutschland GmbH* (*Neufahrn, Germany*), each equipped with a SPD-20A UV/Vis detector (A = 220 nm, 254 nm). A Multokrom 100 C18 (125 × 4.6 mm, 5 μm particle size) column provided by CS *GmbH* (*Langerwehe, Germany*) was used for analytical RP-HPLC runs at a flow rate of 1 mL/min. Both specific gradients and the corresponding retention times $t_R$ are cited in the text. Semi-preparative HPLC purification was performed with a Multokrom 100 RP 18 (250 × 10 mm, 5 μm particle size) column from CS *GmbH* (*Langerwehe, Germany*) at a constant flow rate of 5 mL/min.

Analytical and semi-preparative radio-RP-HPLC was performed using a Multokrom 100 C18 (5 μm, 125 × 4.0 mm) column from CS GmbH (Langerwehe, Germany). Radioactivity was detected by connection of the outlet of the UV-

photometer to a NaI(Tl) well-type scintillation counter from EG&G Ortec (Munich, Germany).

Radioactive probes such as mouse organs or cell-test vials were measured on a WIZARD2® 2480 automatic γ-Counter from *Perkin Elmer* (Waltham MA, USA).

Radio-TLC measurements were conducted on a Scan-RAM™ from *LabLogic Sstems Ltd.* (Broomhill, UK). Chromatograms thereof were analyzed using the Laura™ software from *LabLogic Sstems Ltd.* (Broomhill, UK).

RP-HPLC chromatograms were evaluated using the LabSolution software from *Shimadzu Corp.* (Kyoto, Japan)

Mass spectra for characterization of organic substances were acquired on *an expression$^L$ CMS quadrupole mass spectrometer from Advion Ltd. (Harlow, UK).* NMR spectra were recorded on a Bruker AVHD-300 or AVHD-400 spectrometers from *Bruker Corporation* (Billerica, USA) at 300 K.

pH values were measured with a SevenEasy pH-meter from *Mettler Toledo* (Gießen, Germany).

Purification via flash-chromatography was carried out on an Isolera™ Prime System from Biotage (Uppsala, Sweden), running a Biotage 09474 Rev. E Bio pump. A Biotage™ SNAP KP-$C_{18}$ cartridge (12 g, 93 Å pore diameter, 382 $m^2$/g surface) was used applying a linear gradient of solvent B (ACN, 0.1 vol% TFA, 2 vol% $H_2O$) in solvent A ($H_2O$, 0.1 vol% TFA).

Lyophilization of peptides was carried out using an Alpha 1-2 LDplus lyophilization instrument from Christ (Osterode am Harz, Germany), employing a RZ-2 vacuum pump from Vacubrand GmbH (Wertheim, Germany).

$IC_{50}$ values were calculated using GraphPad Prism 6 from GraphPad Software Inc. (San Diego, USA).

## 2. SYNTHESIS

### 2.1 SOLID-PHASE PEPTIDE SYNTHESIS FOLLOWING THE FMOC-STRATEGY

#### GP1: 2-CTC-resin loading

Loading of the 2-CTC resin with a Fmoc-protected amino acid (AA) was carried out by stirring a suspension of the 2-CTC-resin (1.6 mmol/g) and Fmoc-AA-OH (1.5 eq.) in DMF with DIPEA (3.0 eq.) at room temperature for 2-5 h. Remaining tritylchloride was capped by the addition of methanol (5 mL/g resin) and incubation for 15 min. Subsequently the resin was filtered off and washed with DMF (5 × 5 mL/g resin) and methanol (3 x 5 mL/g resin) and dried *in vacuo.* Final loading *l* of the resin with the Fmoc-AA-OH was determined by the following equation:

$$l\left[\frac{mmol}{g}\right]=\frac{(m_2-m_1)\times 1000}{(M_W-M_{HCl})\,m_2}$$

$m_2$ = mass of loaded resin [g]
$m_1$ = mass of unloaded resin [g]
$M_W$ = molecular weight of AA [g/mol]
$M_{HCl}$ = molecular weight of HCl [g/mol]

#### GP2: On-resin peptide coupling

The respective side-chain protected Fmoc-AA-OH (1.5 eq.) was dissolved in DMF (8 mL/g resin) and pre-activated by adding TBTU (1.5 eq.), HOBt (1.5 eq.) and DIPEA (3 eq.). For amino acids with low reactivity or peptide fragment condensation, HATU (1.5 eq.), HOAt (1.5 eq.) instead of TBTU and HOBt were used. After activation for 15 minutes, the solution was added to resin-bound free amine peptide 2-CTC-AA-$NH_2$ and shaken for 2h at room temperature. For dap(Boc)-OH, dap(Dde)-OH and cys(Trt)-OH as well as fragments bearing these amino acids on their C-terminus, preactivation was shortened to 2-5 min and 2,4,6-Collidine was used as base. For peptide fragments, prolonged reaction times were often needed up to 48 h at r.t.. Subsequently, the resin was washed with DMF (6 × 5 mL/g resin) and after Fmoc-deprotection, the next amino acid was coupled analogously.

#### GP3: On-resin Fmoc-deprotection

The resin-bound Fmoc-peptide was treated with 20% piperidine in DMF (v/v, 8 mL/g resin) for 5 min and subsequently for 15 min. Afterwards, the resin was washed thoroughly with DMF (8 × 5 mL/g resin).

#### GP4: On-resin Dde-deprotection:

The Dde-protected peptide (1.0 eq.) was treated with a solution of 2% hydrazine monohydrate in DMF (v/v, 5

mL/g resin) and shaken for 15 min. In the case of present Fmoc-groups, Dde-deprotection was performed by adding a solution of imidazole (0.46 g), hydroxylamine hydrochloride (0.63 g) in NMP (2.5 mL) and DMF (0.5 mL) for 3 h at r.t.. After deprotection the resin was washed with DMF (6 × 5 mL/g resin).

**GP5: *t*Bu/Boc/Pbf/Trt deprotection**

Removal of *t*Bu/Boc/Pbf-protecting groups was carried out by dissolving the crude product in TFA and stirring for 90 min at r.t.. For removal of Trt protecting groups, TIPS was added to the mixture. After removing TFA under a stream of nitrogen, the residue was dissolved in a mixture of *t*-butanol and water. After lyophilization the crude deprotected peptide was obtained.

**GP6: *N*-Acetylation**

Acetylation of an amine functionality was achieved by reacting the respective peptide with a mixture of DIPEA (5.00 eq.) and $Ac_2O$ (5.00 eq.) in DMF for 2 h.

**GP7: Iodination of CPCR4**

**Iodination** of the CPCR4 tyrosine was carried out according to the published procedure (39). In short, the fully deprotected and purified peptide was dissolved in ACN/$H_2O$ (1/1 (v/v), 1.00 mM) and 0.30 to 0.50 eq. of NIS were added. After 5 min at r.t., the reaction mixture was subjected to HPLC purification.

**GP8: Condensation of fragments in solution**

Connection of the CPCR4 binding motif with functional fragments was carried out in DMF employing small molar amounts of the synthesized fragment (1.10 - 1.30 eq.) and HOAt/HATU as coupling reagents. If the activated amino acid was dap, 2,4,6-Collidine was used as base, in every other case DIPEA.

**GP9: Peptide cleavage off the resin**

**Preservation of acid labile protecting groups**

The resin-bound peptide was treated with a mixture of DCM/HFIP (4/1 (v/v), 8 mL/g resin) and shaken for 60 min. The solution containing the fully protected peptide was filtered off and the resin was treated with another portion of the cleavage solution for 60 min. Both fractions were combined and the solvents removed under reduced pressure. After lyophilization, the crude fully protected peptide was obtained.

**Cleavage of acid-labile protecting groups**

The fully protected resin-bound peptide was treated with a mixture of TFA/TIPS/$H_2O$ (95/2.5/2.5 (v/v/v)) and shaken for 30 min. The solution was filtered off and the resin was treated in the same way for another 30 min. Both filtrates were combined and concentrated under a stream of nitrogen. After dissolving the residue in a mixture of *t*-butanol and water and subsequent lyophilization, the crude peptide was obtained.

**GP10: Sulfhydryl-Maleimido coupling**

Cysteine- or homocysteine-bearing peptides were coupled with maleimide functionalities by following procedure. The fully unprotected HS-peptide was dissolved in DMF (1.00 mg/mL) and added to the Maleimide bearing substance in DMF (1.10 mg/mL). DIPEA (0.50 μL/mg peptide) was added and the reaction mixture was allowed to stir at r.t. for 2 h. Completion of the reaction was confirmed by RP-HPLC and purification carried out by semi-preparative RP-HPLC.

**2.2 $^{NAT}$GA/$^{NAT}$Lu COMPLEXATION**

For the complexation of DOTA- and DOTA-GA-bearing peptides with $^{nat}$Ga and $^{nat}$Lu, the fully deprotected and purified peptides were used. Peptides were dissolved in DMSO, DMSO/$H_2O$ mixtures or $H_2O$, ideally to a concentration of 1 mM. The required amounts of peptide (30 - 500 nmol) were given into an Eppendorf tube and $^{nat}GaNO_3$ or $^{nat}LuCl_3$ (3.00 eq. each) in $H_2O$ were added. The vial was heated to 95°C for 30 min and the quantitative conversion

checked by RP-HPLC and ESI-MS. The reaction mixture was used without further purification, if no educt was traceable.

## 2.3 SYNTHESIS OF BUILDING BLOCKS

### 2.3.1 SYNTHESIS OF CYCLO(D-TYR-D-[NME]ORN-ARG-2-NAL-GLY) (= CPCR4)

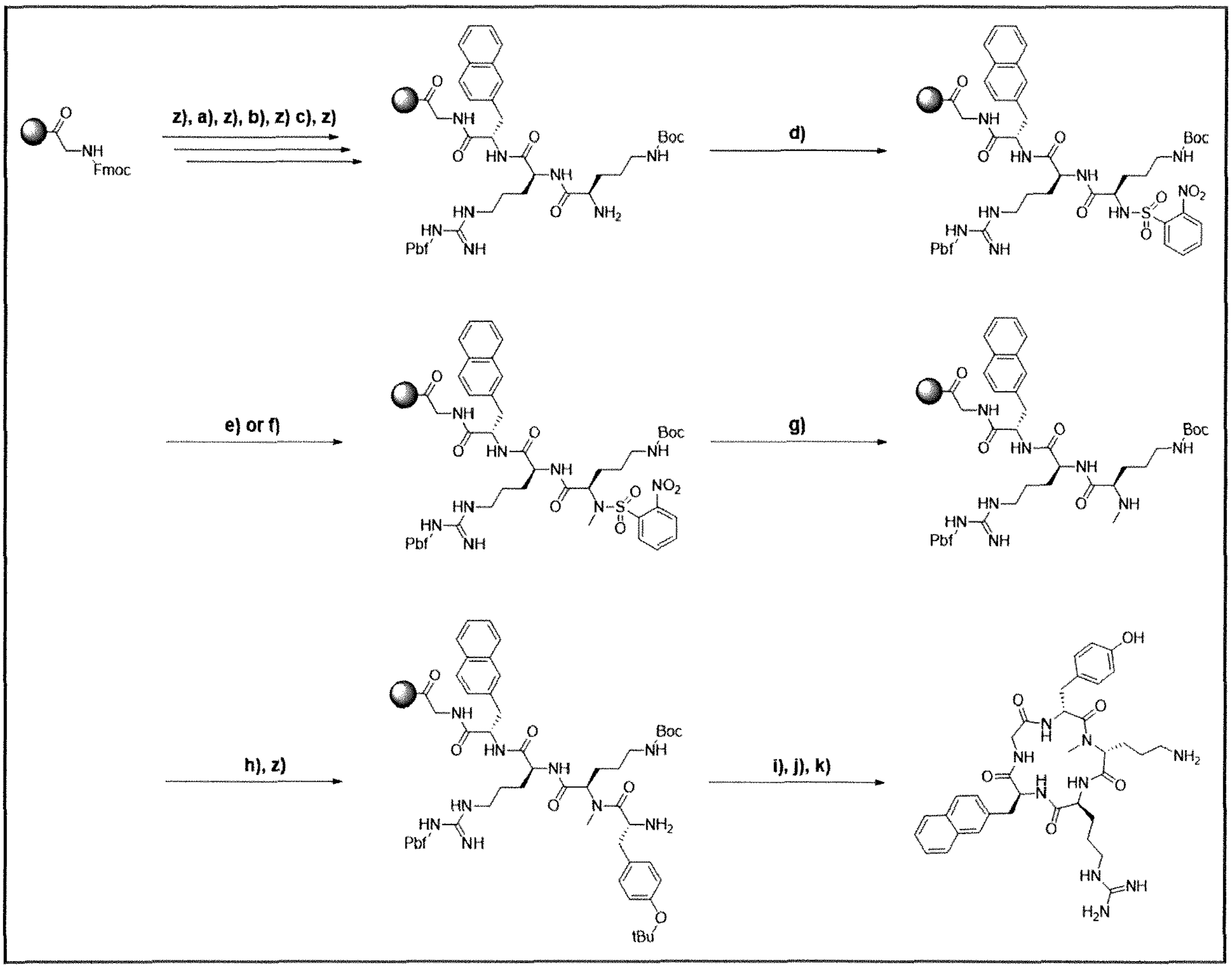


*Scheme 1: Synthesis of CPCR4: **z)** 20% Piperidine in DMF (v/v); **a)** Fmoc-L-2-Nal-OH (1.50 eq.), HOAt (1.50 eq.), TBTU (1.50 eq.), DIPEA (3.00 eq.); **b)** Fmoc-L-Arg(Pbf)-OH (1.50 eq.), HOAt (1.50 eq.), TBTU (1.50 eq.), DIPEA (3.00 eq.); **c)** Fmoc-D-Orn(Boc)-OH (1.50 eq.), HOAt (1.50 eq.), TBTU (1.50 eq.), DIPEA (3.00 eq.); **d)** Collidine (10.00 eq.), o-NBS-Cl (4.00 eq.); **e)** $Ph_3P$ (5.00 eq.), MeOH (10.00 eq.), DIAD (5.00 eq.); **f)** $Me_2SO_4$ (10.00 eq.), DBU (3.00 eq.); **g)** DBU (5.00 eq.), mercaptoethanol (10.00 eq.), **h)** Fmoc-D-Tyr(tBu)-OH (3.00 eq.), HOAt (3.00 eq.), HATU (3.00 eq.), DIPEA (5.00 eq.); **i)** 20% HFIP in DCM (v/v); **j)** DPPA (3.00 eq.), $NaHCO_3$ (5.00 eq.); **k)** TFA.*

The synthesis of the CXCR4 binding motif CPCR4 was realized in analogy to a previously described procedure (*40*). In short, Fmoc-Gly-OH was immobilized on 2-CTC resin and Fmoc-2-Nal-OH, Fmoc-Arg(Pbf)-OH and Fmoc-D-Orn(Boc)-OH were coupled according to **GP2** and **GP3**. The *N*-terminus was then Fmoc deprotected and newly protected by reaction with *O*-NBS-Cl (4.00 eq.) and 2,4,6 - Collidine (10.0 eq.) in NMP for 15 min. Methylation of the *N*-terminus was achieved by either employing *Mitsunobu* conditions ($Ph_3P$ (5.00 eq.), MeOH (10.0 eq.), DIAD (5.00 eq.) in THF, 10 min) or Dimethylsulfate ($Me_2SO_4$ (10.0 eq.), DBU (3.00 eq.) in NMP, 2x2 min) (*41*). Deprotection of the methylated terminus was achieved by incubation of the peptide with DBU (5.00 eq.) for 5 min before addition of mercaptoethanol (10.00 eq.). After 30 min, the resin was washed thoroughly. The following coupling of Fmoc-D-Tyr(*t*Bu)-OH was achieved

by using HOAt and HATU as coupling reagents. After final Fmoc deprotection, the peptide was cleaved off the resin under retention of acid-labile protecting groups (**GP9**). Cyclization was carried out using DPPA (3.00 eq.) and $NaHCO_3$ (5.00 eq.) in a 1mM solution of peptide in DMF. After completion of the reaction, monitored by RP-HPLC, the product solution was concentrated under reduced pressure. The resulting crude product was fully deprotected by treatment with TFA before precipitation in $Et_2O$. Purification of the peptide by flash-chromatography yielded an off-white solid.

CPCR4: RP-HPLC (10 - 95% B in 15 min): $t_R$ = 6.49 min. Calculated monoisotopic mass ($C_{36}H_{47}N_9O_6$): 701.36, found: 701.8 $[M+H]^+$, 351.4 $[M+2H]^{2+}$.

### 2.3.2 SYNTHESIS OF (*R*)-5-(*TERT*-BUTOXY)-4-(3-((*R*)-1,5-DI-*TERT*-BUTOXY-1,5-DIOXOPENTAN-2-YL)UREIDO)-5-OXOPENTANOIC ACID (= D-(*T*BU)E(OH)U-D-E(*T*BU)$_2$) [3]

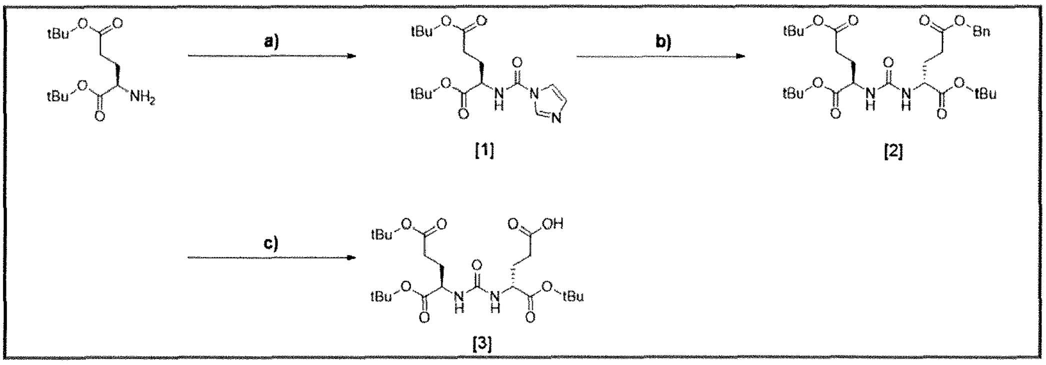


*Scheme 2: Synthesis of (tBu)e(OH)ue(tBu)$_2$: **a)** TEA (2.50 eq.), DMAP (0.04 eq.), CDI (1.10 eq.); 0°C – r.t., o.n.; **b)** TEA (2.00 eq.), D-glu(OBn)-OtBu (1.00 eq.); 0°C – r.t., 40°C, o.n.; **c)** Pd/C (10%); r.t., o.n..*

**Di-tert-butyl-(1H-imidazole-1-carbonyn-D-glutamate [1]**

Synthesis was carried out according to the published procedure (*42*) with minor variation. In short, D-glu(O*t*-Bu)-O*t*Bu (1.00 eq.) was dissolved in DCM and treated with TEA (2.50 eq.) and DMAP (0.04 eq.) on ice. CDI (1.10 eq.) was added and the mixture was stirred over night without further cooling. The reaction was stopped by addition of $NaHCO_{3,sat.}$ and the organic layer washed with $H_2O$ and brine twice each. The solvent was evaporated and the crude product, [1] was obtained as a colorless oil (91% yield). The product was used in subsequent reactions without further purification.

Di-*tert*-butyl-(1H-imidazole-1-carbonyl)-D-glutamate [1]: RP-HPLC: (10 - 90% B in 15 min): $t_R$ = 14.50 min. Calculated monoisotopic mass ($C_{17}H_{27}N_3O_5$): 353.2, found: 376.3 $[M+Na]^+$.

**5-Benzyl-1-(*tert*-butyl)-(((*R*)-1,5-di-tert-butoxy-1,5-dioxopentan-2-yl)carbamoyl)-d-alutamate [2]**

The educt [1] (1.00 eq.) was dissolved in DCE and cooled on ice before addition of TEA (2.00 eq.) and D-glu(OBn)-O*t*Bu (1.00 eq.). The mixture was heated to 40°C over night. The solvent was then concentrated *in vacuo* and the crude product underwent silica flash-chromatography employing EtOAc/n-hexane/TEA (500/500/0.8 (v/v/v)). After removal of the solvents under reduced pressure, the desired product [2] was obtained as a colorless oil (84% yield).

5-Benzyl-1-(*tert*-butyl)-(((*R*)-1,5-di-*tert*-butoxy-1,5-dioxopentan-2-yl)carbamoyl)-d-glutamate [2]: RP-HPLC: (10 - 90% B in 20 min): $t_R$ = 17.43 min. Calculated monoisotopic mass ($C_{30}H_{46}N_2O_9$): 578.3, found: 601.5 $[M+Na]^+$, 523.3 [M-*t*Bu+H]$^+$, 467.3 [M-2*t*Bu+H]$^+$, 411.3 [M-3*t*Bu+H]$^+$.

**(*t*Bu)e(OH)ue(*t*Bu)$_2$ [3]**

The benzyl-protected educt [2] (1.00 eq.) was dissolved in EtOH and palladium (10% on activated charcoal, 0.10 eq.) was added. A $H_2$-atmosphere was maintained over night at room temperature to facilitate the deprotection reaction. The catalyst was filtered off by passing the mixture through a celite pad. The solvent of the resulting clear solution was evaporated under reduced pressure to yield the desired product [3] as a colorless oil that solidifies (82% yield).

e(*t*Bu)ue(*t*Bu)$_2$ [3]: RP-HPLC (10 - 90% B in 15 min): $t_R$ = 12.00 min. Calculated monoisotopic mass ($C_{23}H_{49}N_2O_9$): 488.3, found: 489.4 $[M+H]^+$, 516.4 $[M+Na]^+$.

### 2.3.3 FMOC-D-HCY(GLYCOSYL/LACTOSYL)-OH

For the incorporation of a sugar moiety onto Fmoc-D-Hcy(Trt)-OH, the respective per-acetylated precursor (β-D-Glucose pentaacetate/β-D-Lactose octaacetate) was used. The sugar (1.00 eq.) and protected amino acid (1.20 eq.) were added in Argon stream to a round bottom flask and dissolved in $DCM_{abs}$. TIPS (1.30 eq.) was added as a scavenger and $SnCl_4$ (2.40 eq., 1.00 M in DCM) was given drop-wise to the reaction mixture. After initial yellow coloring, the mixture became colorless and a precipitate formed after stirring over night at r.t.. The mixture was diluted with DCM and acidified with HCl (1.00 M). The organic layer was extracted with HCl and $H_2O$ twice each and dried over $Na_2SO_4$. The solvent was evaporated *in vacuo* and the crude product purified via flash chromatography.

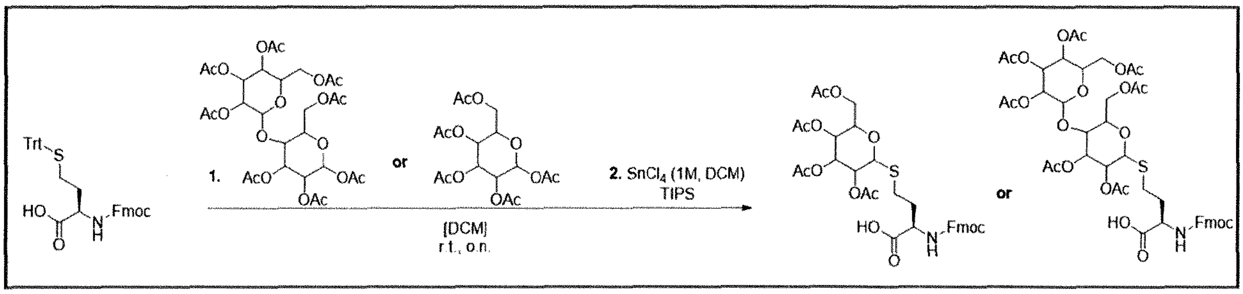


*Scheme 3: Reaction of Fmoc-D-Hcy(Trt)-OH with peracetylated sugars employing $SnCl_4$ and TIPS.*

Fmoc-D-HCy-(β-D-Gluc(OAc)$_4$)-OH: HPLC (30 - 80% B in 15 min): $t_R$ = 9.90 min. Calculated monoisotopic mass ($C_{33}H_{37}NO_{13}S$): 687.20, found: 688.2 $[M+H]^+$.

Fmoc-D-HCy-(β-L-Lac(OAc)$_7$)-OH: HPLC (30 - 80% B in 15 min): $t_R$ = 10.70 min. Calculated monoisotopic mass ($C_{45}H_{53}NO_{21}S$): 975.28, found: 976.0 $[M+H]^+$.

### 2.3.4 4-(DI-TERT-BUTYLFLUOROSILYL)BENZOIC ACID (= SIFA-BA, [8])

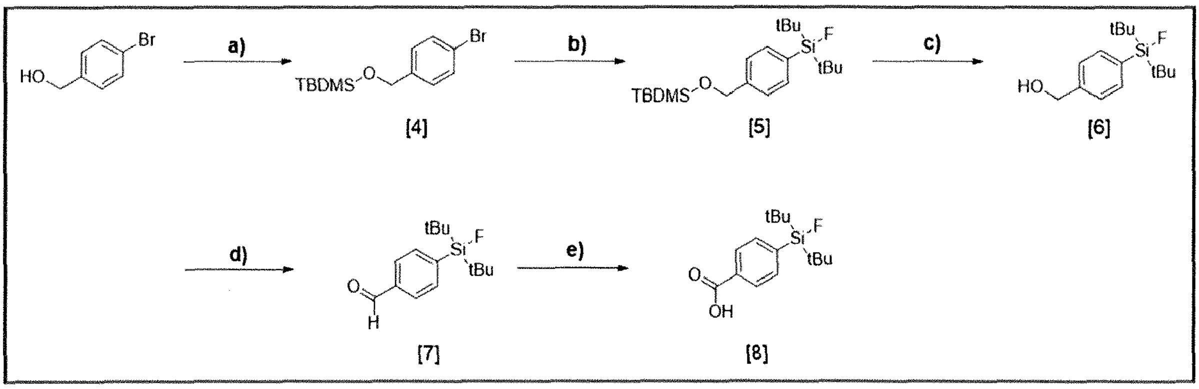


*Scheme 4: Synthesis of SiFA-BA [8]: **a)** TBDMSCl, imidazole (DMF); r.t., o.n.; **b)** tBuLi, di-tert-butyldifluorosilane (THF); −78°C – r.t., o.n.; **c)** HCl (MeOH); r.t., o.n.; **d)** pyridinium chlorochromate (DCM); 0°C, 2.5h; **e)** $KMnO_4$ (DCM, tBuOH, $NaH_2PO_4$ buffer); r.t. – 5°C.*

The synthesis of the silicon fluoride acceptor moiety [8] was achieved leaning on the published procedure (*43*) with some modifications. Reactions were carried out in dried flasks under argon atmosphere. The desired product was obtained after 5 reaction steps starting with 4-bromobenzyl alcohol.

**((4-Bromobenzvl)oxy)(*tert*-butyl)dimethylsilane [4]**

4-bromobenzyl alcohol (1.00 eq.) was dissolved in $DMF_{abs}$. (15 mL/g educt) and imidazole (1.20 eq.) and TBDMSCI (1.20 eq.) were added under vigorous stirring. The reaction mixture

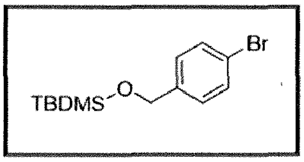

was stirred at r.t. over night, poured into cold $H_2O$ and the aqueous phase extracted 5 times with $Et_2O$. The organic phases were combined, washed twice with sat. $NaHCO_3$ and brine, dried over $MgSO_4$ and concentrated in vacuo. The crude product was purified via silica flash chromatography employing 5% EtOAc in petrol ether (v/v). After removal of the solvents under reduced pressure, the protected alcohol [4] was obtained as colorless oil (95% yield).

((4-Bromobenzyl)oxy)(*tert*-butyl)dimethylsilane [4]: RP-HPLC: (50 - 100% B in 15 min): $t_R$ = 15.0 min. $^1$H-NMR (400 MHz, $CDCl_3$, 300 K): $\delta$ = 0.10 (6H, s, $SiMe_2tBu$), 0.95 (9H, s, $SiMe_2tBu$), 4.69 (2H, s, $CH_2OSi$), 7.21 (2H, d), 7.46 (2H, d) ppm.

**Di-*tert*-butyl(4-((*tert*-butyldimethylsilvloxy)methyl)pheny)fluorosilane [5]**

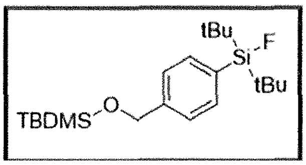

[4] (1.00 eq.) was dissolved in $THF_{abs}$ (10 mL/g educt) and cooled to -78°C before addition of *t*BuLi (2.40 eq.) in pentane (c = 1.70 mol/L). After stirring for 30 min at -78°C, the reaction mixture was added dropwise to a solution of di-tert-butyldifluorosilane (1.20 eq.) in $THF_{abs}$. (10 mL/g) at -78°C. The solution was stirred over night and allowed to warm to r.t. before addition of brine. The crude product was extracted 3 times with $Et_2O$, the combined organic phases dried over $MgSO_4$ and the solvents evaporated *in vacuo* to afford a yellowish oil (95% yield). The crude product [5] was used without further purification.

Di-*tert*-butyl(4-((*tert*-butyldimethylsilyloxy)methyl)phenyl)fluorosilane [5]: RP-HPLC: (50 - 100% B in 20 min): $t_R$ = 19.0 min.

**4-(Di-*tert*-butyfluorosilanyl)benzyl alcohol [6]**

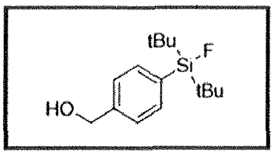

Deprotection of [5] was achieved by suspension in MeOH (25 mL/g educt) and using catalytic amounts of concentrated HCl (0.25 mL/g). After stirring the mixture over night at r.t., the solvent was removed under reduced pressure. The remainder was dissolved in $Et_2O$ (20 mL/g educt) and washed with sat. $NaHCO_3$ solution. The aqueous layer was then three times extracted with $Et_2O$, the combined organic phases dried over $MgSO_4$, and the solvent evaporated *in vacuo* to afford a yellowish oil (98% yield). The crude product [6] was used without further purification.

4-(Di-*tert*-butylfluorosilanyl)benzyl alcohol [6]: RP-HPLC: (50 - 100% B in 15 min): $t_R$ = 8.2 min.

**4-(Di-*tert*-butylfluorosilyl)benzaldehyde [7]**

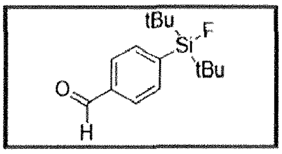

Oxidation to the aldehyde was done employing Corey-Suggs conditions. The educt [6] (1.00 eq.) was dissolved in $DCM_{abs}$. (15 mL/g educt) and added dropwise to an ice-cooled suspension of PCC (2.50 eq.) in $DCM_{abs}$. (20 mL/g PCC). After stirring for 30 min at 0°C and subsequently 2.5 h at r.t., $Et_2O$ was added and the supernatant decanted from the solid. The black remainder was washed with $Et_2O$ and the combined organic phases filtered through a pad of silica gel (10 cm/g product). The solvent was evaporated *in vacuo* and [7] obtained as a yellowish oil (96% yield).

4-(Di-*tert*-butylfluorosilyl)benzaldehyde [7]: RP-HPLC: (50 - 100% B in 15 min): $t_R$ = 10.5 min.

**4-(Di-*tert*-butylfluorosilyl)benzoic acid (= SiFA-BA) [8]**

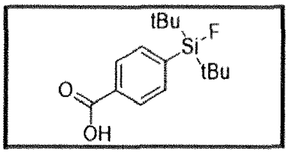

The aldehyde [7] (1.00 eq.) was dissolved in *t*BuOH (23 mL/g educt), DCM (2.5 mL/g educt) and $NaH_2PO_4xH_2O$ (1.25M, pH = 4.0-4.5, 15 mL/g educt) and $KMnO_{4aq}$. (1M, 23 mL/g educt) was added. After stirring for 25 min, the mixture was cooled to 5°C. $KMnO_4$ (1.00 eq.) was added and the reaction quenched shortly afterwards by addition of sat. $NaHCO_3$.The mixture was dried over $MgSO_4$ and the solvent evaporated under reduced pressure. The crude product [8] was purified by recrystallization from $Et_2O$/*n*-hexane (1/3, v/v) and afforded a colorless solid (60% yield).

4-(Di-*tert*-butylfluorosilyl)benzoic acid (= SiFA-BA [8]): RP-HPLC: (50 - 100% B in 15 min): $t_R$ = 8.5 min. Calculated monoisotopic mass ($C_{15}H_{23}FO_2Si$): 282.4; found: m/z = 281.1 $[M-H]^-$, 235.1 $[M-COOH]^-$.

**2.3.5 (4-Bromomethylphenyl)-di-*tert*-butyl-fluorosilane (= SiFA-Br [9])**

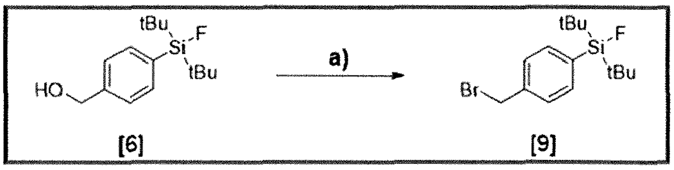


*Scheme 5: Synthesis of SiFA-Br [9]: a) $CBr_4$, $PPh_3$; 0°c – r.t..*

The Synthesis of [9] was performed according to the literature (*44*). In short, the SiFA benzylalcohol [6] was dissolved in DCM and the solution cooled to 0°C. $CBr_4$ (1.10 eq.) was added before $PPh_3$ was added over a period of 30 min in small portions. The mixture was stirred for 2 h at r.t., the solvent removed *in vacuo* and the remainder washed with n-hexane. The precipitate was filtered off and the liquid concentrated *in vacuo.* Adjacent silica flash chromatography employing n-pentane as mobile phase, afforded the desired product [9] as a colorless oil (32 - 39% yield).

(4-Bromomethylphenyl)-di-*tert*-butyl-fluorosilane (= SiFA-Br [9]): RP-HPLC: (50 - 100% B in 15 min): $t_R$ = 15.10 min. Calculated monoisotopic mass ($C_{15}H_{24}BrFSi$): 330.08; found: m/z = 331.3 $[M-H]^-$.

$^1H$ NMR (300 MHz, $CDCl_3$, 293 K) $\delta$ 7.63 - 7.54 (m, 2H, 2x-CH-), 7.40 (d, *J* = 8.0 Hz, 2H, 2x-CH-), 4.50 (s, 2H, $-CH_2-$), 1.06 (d, *J* = 1.2 Hz, 18H, 2x*t*Bu).

$^{13}C$ NMR (75 MHz, $CDCl_3$, 293 K) $\delta$ 139.50 (-CH-), 135.00 (-CH-), 134.95 (-CH-), 134.84 (-CH-), 134.66 (-CH-),

128.72 (-CH-), 33.88 (-CH2-), 27.88 (6x*t*Bu C), 20.92 (tert. C), 20.75 (tert. C).

### 2.3.6 SYNTHESIS OF LINKER STRUCTURES

#### 2.3.6.1 ABZ-AND AMBZ-BASED LINKERS

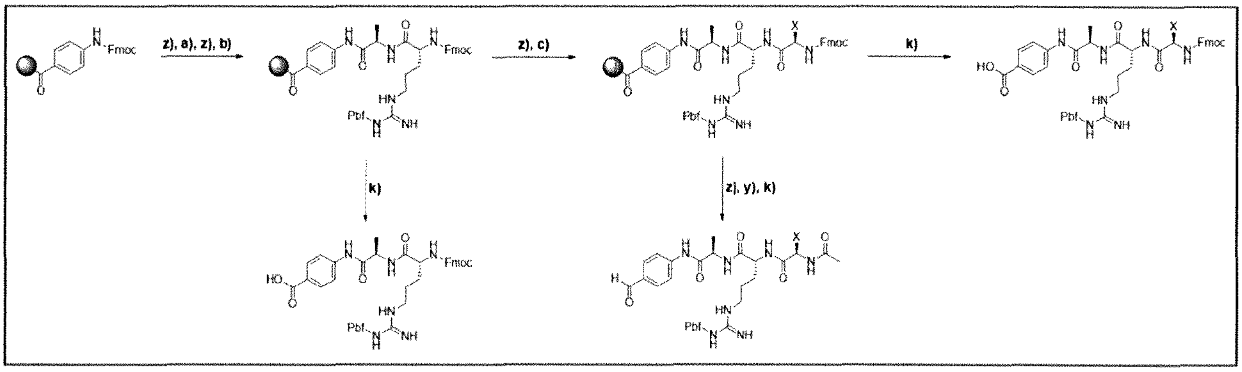


*Scheme 6: Synthesis of frequently used linker structures: z) 20% Piperidine in DMF (v/v); a) Fmoc-D-Ala-OH (2.00 eq.), HOAt (2.00 eq.), HATU (2.00 eq.), DIPEA (5.00 eq.); b) Fmoc-D-Arg(Pbf)-OH (1.50 eq.), HOAt (1.50 eq.), TBTU (1.50 eq.), DIPEA (3.00 eq.); .); c) Fmoc-AA-OH (1.50 eq.), HOAt (1.50 eq.), TBTU (1.50 eq.), DIPEA (3.00 eq.) or 2,4,6,-Collidine (3.00 eq.); y) DIPEA (5.0 eq.), $Ac_2O$ (5.00 eq.); k) 20% HFIP in DCM (v/v).*

The entirety of -Ambz- and -Abz- based linkers were synthesized alike (see **GP1,GP2,GP3**) with the difference being the coupling of Fmoc-D-Ala-OH. For -Abz- based linkers, HOAt and HATU were employed together with a prolonged reaction time of 4 h considering the lower reactivity of the amine functionality of -Abz- compared to -Ambz-. After insertion of the second or third amino acid, the respective linker was cleaved off the solid support under retention of the protecting groups (see **GP9**). For acetylated linker units, an additional Fmoc deprotection and subsequent acetylation as described (see **GP6**) was carried out on the solid support.

**HO-Abz-a-r(Pbf)-Fmoc**

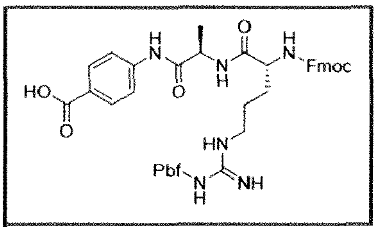

HO-Abz-a-r(Pbf)-Fmoc: RP-HPLC (10 - 90% B in 15 min): $t_R$ = 15.33 min. Calculated monoisotopic mass ($C_{44}H_{50}N_6O_9S$): 838.34, found: 839.1 $[M+H]^+$.

**HO-Abz-a-r(Pbf)-dap(Boc)-Fmoc**

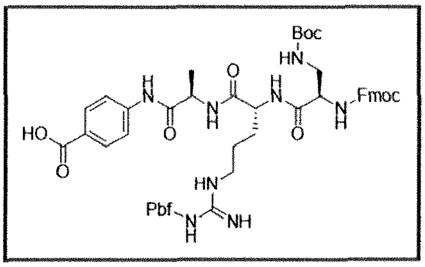

HO-Abz-a-r(Pbf)-dap(Boc)-Fmoc: RP-HPLC (50 - 95% B in 15 min): $t_R$ = 13.52 min. Calculated monoisotopic mass ($C_{52}H_{64}N_8O_{12}S$): 1024.44, found: 1025.2 $[M+H]^+$.

**HO-Abz-a-cit-dap(Boc)-Fmoc**

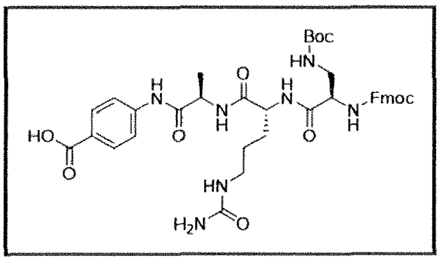

HO-Abz-a-cit-dap(Boc)-Fmoc: RP-HPLC (10 - 95% B in 15 min): $t_R$ = 10.86 min. Calculated monoisotopic mass ($C_{39}H_{47}N_7O_{19}$): 773.34, found: 774.5 $[M+H]^+$.

**HO-Abz-a-r(Pbf)-h(Trt)-Fmoc**

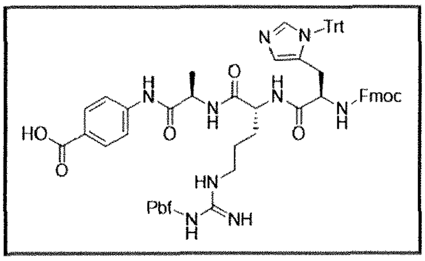

HO-Abz-a-r(Pbf)-h(Trt)-Fmoc: RP-HPLC (10 - 90% B in 15 min): $t_R$ = 17.15 min. Calculated monoisotopic mass ($C_{69}H_{71}N_9O_{10}S$): 1217.50, found: 1218.0 $[M+H]^+$, 977.3 $[M-Trt+H]^+$.

**HO-Abz-a-r(Pbt)-c(Trt)-Fmoc**

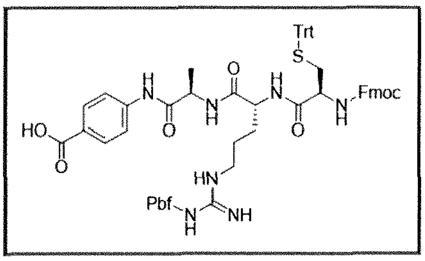

HO-Abz-a-r(Pbf)-c(Trt)-Fmoc: RP-HPLC (10 - 90% B in 15 min): $t_R$ = 17.77 min. Calculated monoisotopic mass ($C_{66}H_{69}N_7O_{10}S_2$): 1183.45, found: 1184.4 $[M+H]^+$.

## HO-Abz-a-r(Pbf)-Hcy(Trt)-Fmoc

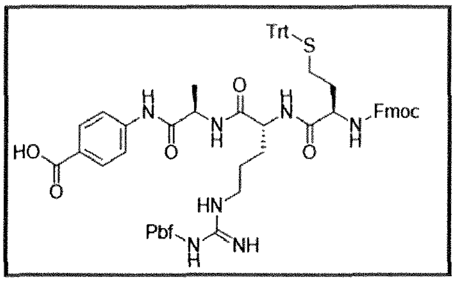

HO-Abz-a-r(Pbf)-Hcy(Trt)-Fmoc: RP-HPLC (10 - 90% B in 15 min): $t_R$ = 17.79 min. Calculated monoisotopic mass ($C_{67}H_{71}N_7O_{10}S_2$): 1197.47, found: 1198.2 $[M+H]^+$.

## HO-Abz-a-r(Pbf)-f-Fmoc

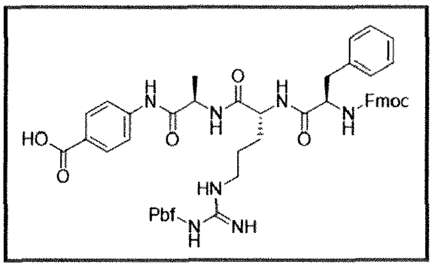

HO-Abz-a-r(Pbf)-f-Fmoc: RP-HPLC (10 - 90% B in 15 min): $t_R$ = 16.30 min. Calculated monoisotopic mass ($C_{53}H_{59}N_7O_{19}S$): 985.40, found: 986.8 $[M+H]^+$.

## HO-Abz-a-r(Pbf)-r(Pbf)-Fmoc

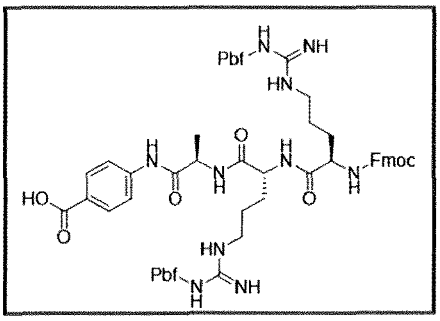

HO-Abz-a-r(Pbf)-r(Pbf)-Fmoc: RP-HPLC (10 - 90% B in 15 min): $t_R$ = 16.82 min. Calculated monoisotopic mass ($C_{63}H_{78}N_{10}O_{13}S_2$): 1246.52, found: 1248.6 $[M+H]^+$.

## HO-Ambz-a-r(Pbf)-Fmoc

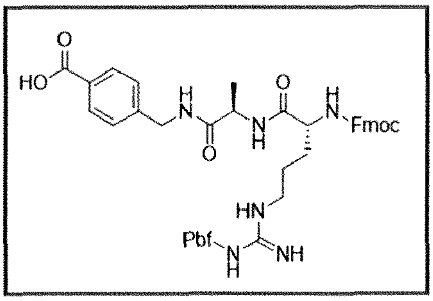

HO-Ambz-a-r(Pbf)-Fmoc: RP-HPLC (10 - 90% B in 15 min): $t_R$ = 12.51 min. Calculated monoisotopic mass ($C_{45}H_{52}N_6O_9S$): 852.35, found: 601.4 $[M-Pbf+H]^+$.

## HO-Ambz-a-r(Pbf)-dap(Boc)-Fmoc

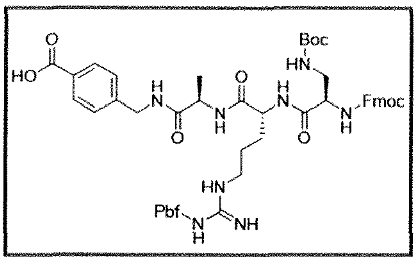

HO-Ambz-a-r(Pbf)-dap(Boc)-Fmoc: RP-HPLC (30 - 90% B in 15 min): $t_R$ = 13.70 min. Calculated monoisotopic mass ($C_{53}H_{66}N_8O_{12}S$): 1038.45, found: 1039.6 $[M+H]^+$.

## HO-Ambz-a-r(Pbfl-h(Trt)-Fmoc

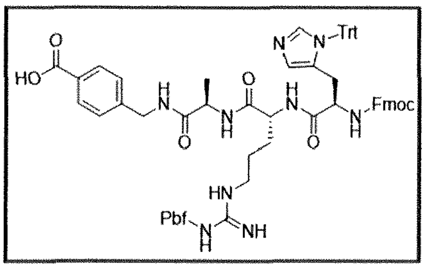

HO-Ambz-a-r(Pbf)-h(Trt)-Fmoc: RP-HPLC (10 - 90% B in 15 min): $t_R$ = 17.95 min. Calculated monoisotopic mass ($C_{70}H_{73}N_9O_{10}S$): 1231.52, found: 1233.1 $[M+H]^+$.

**HO-Abz-a-r(Pbf)-dap(Boc)-Ac**

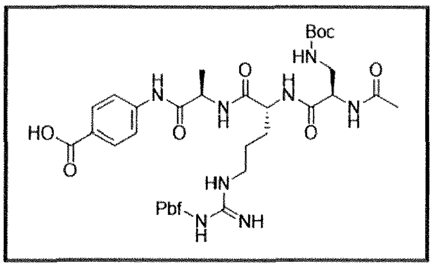

HO-Abz-a-r(Pbf)-dap(Boc)-Ac: RP-HPLC (10 - 95% B in 15 min): $t_R$ = 10.43 min. Calculated monoisotopic mass ($C_{39}H_{56}N_8O_1$,S): 844.38, found: 845.2 $[M+H]^+$.

**HO-Abz-a-r(PbfM-APipAc-Ac**

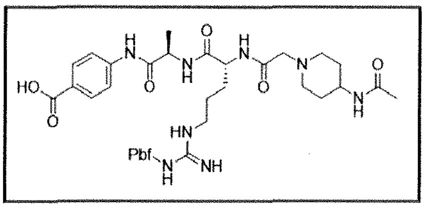

HO-Abz-a-r(Pbf)-4-APipAc-Ac: RP-HPLC (10 - 95% B in 15 min): $t_R$ = 8.13 min. Calculated monoisotopic mass ($C_{38}H_{54}N_8O_9S$): 798.37, found: 799.7 $[M+H]^+$, 400.1 $[M+2H]^{2+}$ .

**HO-Abz-a-cit-dap(Boc)-Ac**

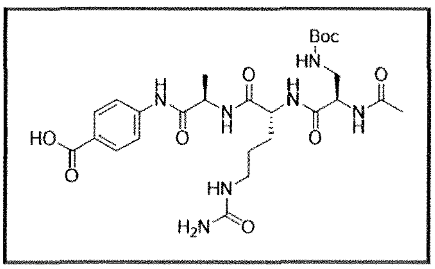

HO-Abz-a-cit-dap(Boc)-Ac: RP-HPLC (10 - 95% B in 15 min): $t_R$ = 6.61 min. Calculated monoisotopic mass ($C_{26}H_{39}N_7O_9$): 593.28, found: 594.3 $[M+H]^+$.

**HO-Abz-a-4-APipAc-dap(Boc)-Ac**

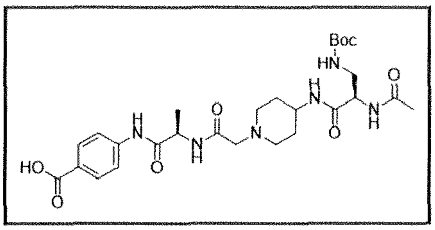

HO-Abz-a-4-APipAc-dap(Boc)-Ac: RP-HPLC (10 - 95% B in 15 min): $t_R$ = 6.62 min. Calculated monoisotopic mass ($C_{27}H_{40}N_6O_8$): 576.29, found: 577.2 $[M+H]^+$.

**AHX-BASED LINKERS**

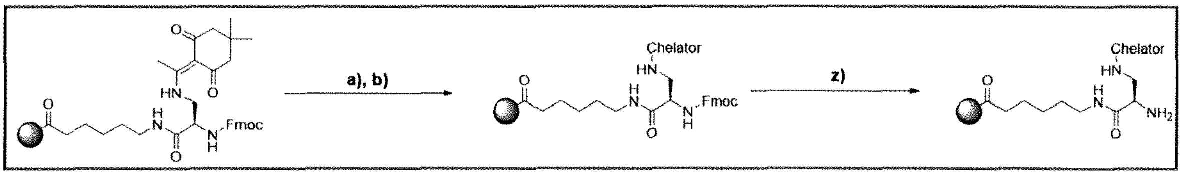


*Scheme 7: Synthesis of Ahx-based linkers with labeling modality: a) Imidazole, hydroxylamine; b) Chelator (DOTA(tBu)$_3$, DOTAGA (tBu)$_4$, N4(Boc)$_4$, 2.00 eq.), HOAt (2.00 eq.), HATU (2.00 eq.), DIPEA (5.00 eq.); z) 20% Piperidine in DMF (v/v).*

The synthesis of Ahx-based peptide linkers was achieved *via* standard SPPS according to **GP1, GP2, GP3** and **GP4.** In short, Ahx was immobilized on the 2-CTC resin, Fmoc deprotected and coupled with Fmoc-D-dap(Dde)-OH. The side chain was deprotected using imidazole and hydroxylamine before the desired chelator, namely DOTA(*t*Bu)$_3$, R-DOTAGA(*t*Bu)$_4$ or N4(Boc)$_4$ was coupled. Adjacent Fmoc-deprotection opened the possibility of further derivatization. Ahx-based linker structures were further derivatized, in detail see 2.3.8.2.

**2.3.7 CPCR4-LINKER CONJUNCTIONS**

CPCR4-linker conjunctions were synthesized by fragment condensation between CPCR4 and the respective Fmoc-protected linker unit under exertion of **GP8**. Reaction mixtures were concentrated *in vacuo* before Fmoc-deprotection by dissolution in 20 vol% piperidine in DMF. Adjacent semi-preparative RP-HPLC yielded the desired products.

**CPCR4-Abz-a-r(Pbf)-dap(Boc)-$NH_2$**

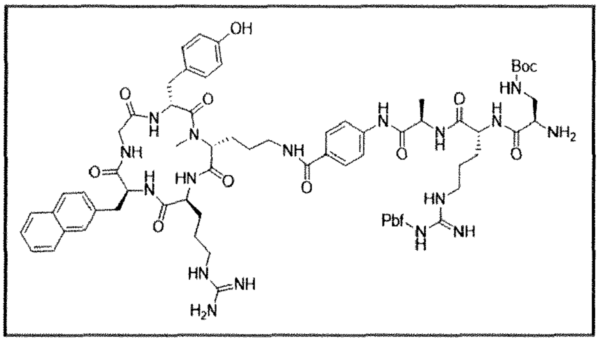

The synthesis of CPCR4-Abz-a-r(Pbf)-dap(Boc)-$NH_2$ was facilitated by fragment condensation according to **GP8.** CPCR4 (see 2.3.1) and the linker HO-Abz-a-r(Pbf)-dap(Boc)-Fmoc (see 2.3.6) were condensed and the resulting peptide Fmoc deprotected. The crude product was purified by semi-preparative RP-HPLC.

CPCR4-Abz-a-r(Pbf)-dap(Boc)-$NH_2$: RP-HPLC (10 - 95% B in 15 min): $t_R$ = 8.83 min. Calculated monoisotopic mass ($C_{73}H_{99}N_{17}O_{15}S$): 1485.72, found: 744.6$[M+2H]^{2+}$.

## CPCR4-Abz-a-cit-dap(Boc)-NH$_2$

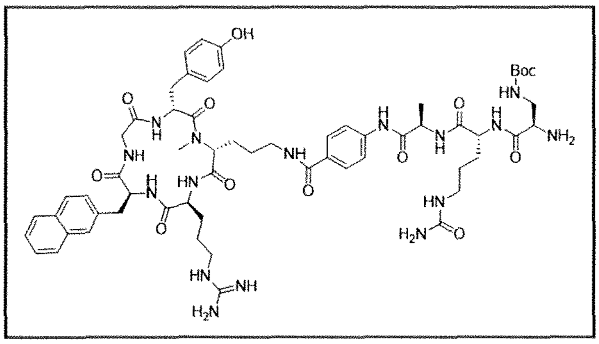

The synthesis of CPCR4-Abz-a-cit-dap(Boc)-$NH_2$ was achieved by fragment condensation according to **GP8.** CPCR4 (see 2.3.1) and the linker HO-Abz-a-cit-dap(Boc)-Fmoc (see 2.3.6) were condensed and the resulting peptide Fmoc deprotected. The crude product was purified by semi-preparative RP-HPLC.

CPCR4-Abz-a-cit-dap(Boc)-$NH_2$: RP-HPLC (10 - 95% B in 15 min): $t_R$ = 7.03 min. Calculated monoisotopic mass ($C_{60}H_{82}N_{16}O_{13}$): 1234.62, found: 618.2$[M+2H]^{2+}$.

## CPCR4-Ambz-a-r(Pbf)-dap(Boc)-NH$_2$

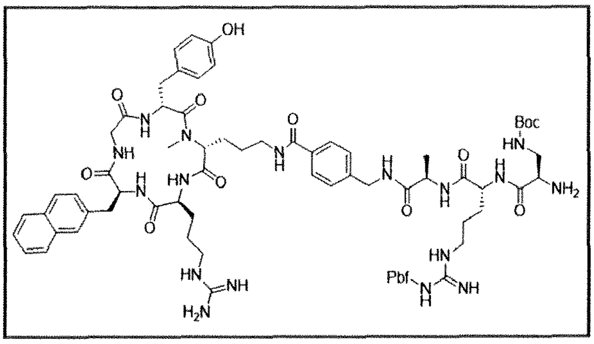

CPCR4-Ambz-a-r(Pbf)-dap(Boc)-$NH_2$ was obtained by synthesis in analogy to CPCR4-Abz-a-r(Pbf)-dap(Boc)-$NH_2$ by condensation of CPCR4 (see 2.3.1) and the linker HO-Ambz-a-r(Pbf)-dap(Boc)-Fmoc (see 2.3.6) and adjacent Fmoc deprotection and purification.

CPCR4-Ambz-a-r(Pbf)-dap(Boc)-$NH_2$: RP-HPLC (10 - 90% B in 15 min): $t_R$ = 8.97 min. Calculated monoisotopic mass ($C_{74}H_{101}N_{17}O_{15}S$): 1499.74, found: 750.2[M+2H]$^{2+}$.

**CPCR4-Ambz-a-r(Pbf)-$NH_2$**

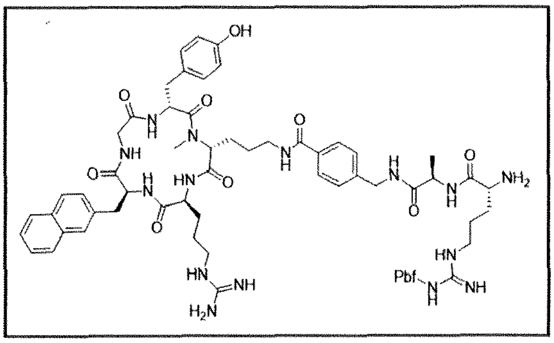

Synthesis of CPCR4-Ambz-a-r(Pbf)-$NH_2$ was achieved by condensation of CPCR4 (see 2.3.1) and the linker HO-Ambz-a-r(Pbf)-Fmoc (see 2.3.6) (**GP8**). Fmoc deprotection and purification via semi-preparative RP-HPLC afforded the desired product.

CPCR4-Ambz-a-r(Pbf)-$NH_2$: RP-HPLC (10 - 90% B in 15 min): $t_R$ = 7.94 min. Calculated monoisotopic mass ($C_{86}H_{87}N_{15}O_{12}S$): 1313.64, found: 658.2[M+2H]$^{2+}$.

**CPCR4-Ambz-a-r(Pbf)-h(Trt)-$NH_2$**

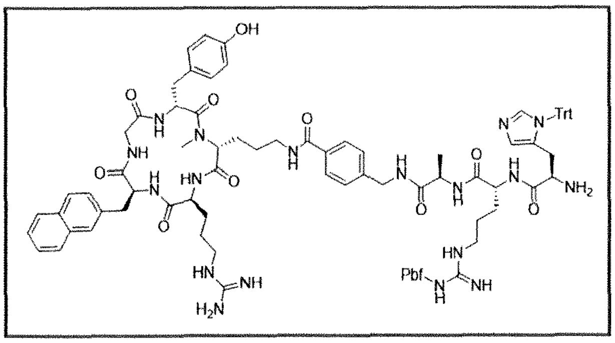

CPCR4-Ambz-a-r(Pbf)-$NH_2$ was synthesized by condensation of CPCR4 (see 2.3.1) and the linker HO-Ambz-a-r(Pbf)-h(Trt)-Fmoc (see 2.3.6) (**GP8**). Fmoc deprotection and purification via semi-preparative RP-HPLC afforded the desired product.

CPCR4-Ambz-a-r(Pbf)-h(Trt)-$NH_2$: RP-HPLC (10 - 90% B in 15 min): $t_R$ = 13.43 min. Calculated monoisotopic mass ($C_{91}H_{108}N_{18}O_{13}S$): 1692.81, found: 848.3[M+2H]$^{2+}$.

**2.3.8 SYNTHESIS OF CHELATORS**

**2.3.8.1 MODIFIED $MAS_3$-DERIVED CHELATORS**

Several $mas_3$-derived chelators were synthesized all according to standard Fmoc peptide synthesis strategy and **GP1,2,3,9** .

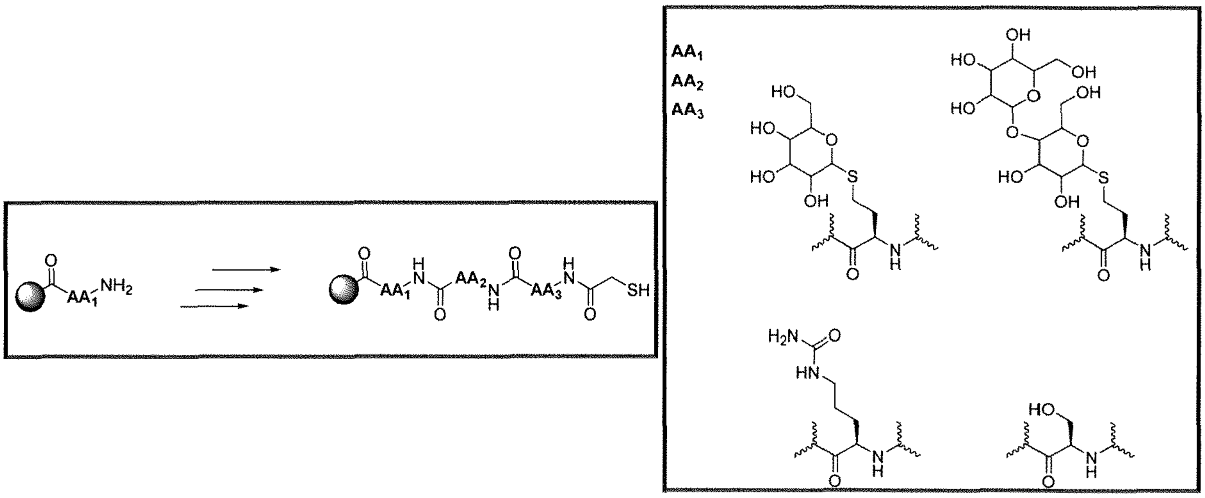

*Scheme 8: Synthesis of modified mas3-derived chelators according to Fmoc peptide synthesis strategy.*

The resulting chelators were either used in fragment condensation with the CXCR4-binding peptides or deacetylated beforehand.

**HO-(s(*t*Bu))$_3$-TMAA**

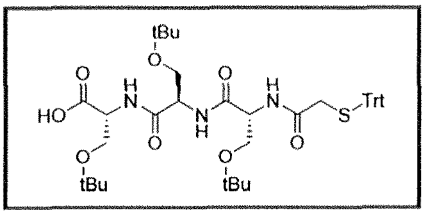

HO-(s(*t*Bu))$_3$-TMAA: HPLC (10 - 90% B in 15 min): $t_R$ = 14.85 min. Calculated monoisotopic mass ($C_{42}H_{57}N_3O_8S$): 763.39, found: 764.7[M+H]$^+$.

**HO-(Hcy(Gluc(OAc)$_4$))$_3$-TMAA**

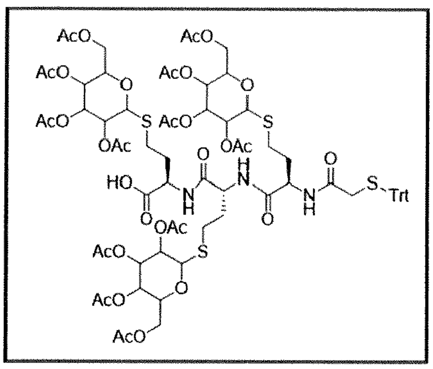

HO-(Hcy(Gluc(OAc)$_4$))$_3$-TMAA: HPLC (10 - 95% B in 15 min): $t_R$ = 12.97 min. Calculated monoisotopic mass ($C_{75}H_{93}N_3O_{32}S_4$): 1675.46, found: 1677.2[M+H]$^+$.

**HO-s(*t*Bu)-(Hcy(Gluc(OAC)$_4$))$_2$-TMAA**

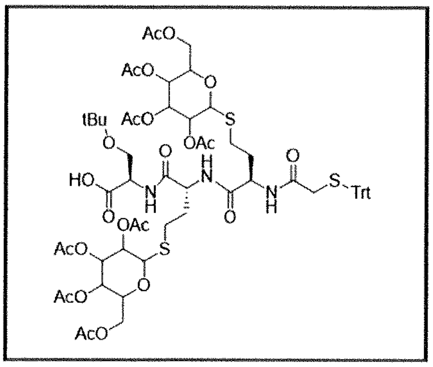

HO-s(*t*Bu)-(Hcy(Gluc(OAc)$_4$))$_2$-TMAA: HPLC (10 - 95% B in 15 min): $t_R$ = 13.0 min. Calculated monoisotopic mass ($C_{64}H_{81}N_3O_{24}S_3$): 1371.44, found: 1372.3[M+H]$^+$.

**HO-cit-Hcy(Lac(OAc)$_7$)-cit-TMAA**

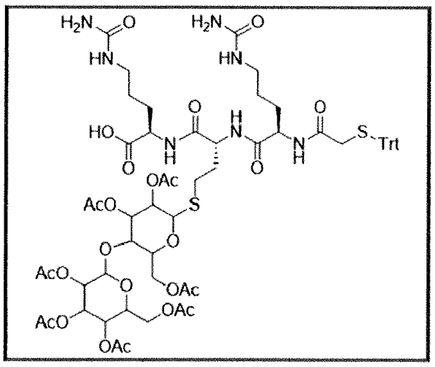

HO-cit-Hcy(Lac(OAc)$_7$)-cit-TMAA: HPLC (10 - 90% B in 15 min): $t_R$ = 7.97 min. Calculated monoisotopic mass ($C_{63}H_{81}N_7O_{24}S_2$): 1383.48, found: 1141.1[M-Trt+H]$^+$.

**HO-s(*t*Bu)-Hcy(Lac(OAc)$_7$)-s(*t*Bu)-TMAA**

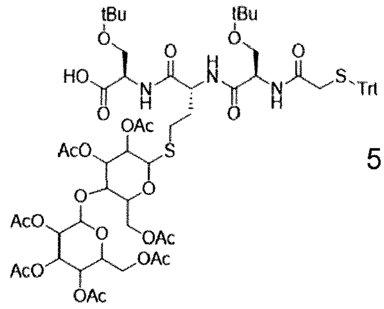

5

HO-s(*t*Bu)-Hcy(Lac(OAc)$_7$)-S(*t*Bu)-TMAA: HPLC (10 - 90% B in 15 min): $t_R$ = 8.47 min. Calculated monoisotopic mass ($C_{65}H_{85}N_3O_{24}S_2$): 1355.50, found: 1001.8[M-Trt, - 2tBu+H]$^+$.

**Deacetylation**

After cleavage off the resin, the chelators were dissolved in MeOH (2 mL/50 mg) and the pH was adjusted to 10-11 with KCN. After at least 4 h at ambient temperature, de-acetylated chelators were precipitated in $Et_2O$.

HO-(Hcy(Gluc(OAc)$_{12}$))$_3$-TMAA: HPLC (10 - 90% B in 15 min): $t_R$ = 12.97 min. Calculated monoisotopic mass ($C_{75}H_{93}N_3O_{32}S_4$): 1675.46, found: 1677.2[M+H]$^+$.

HO-(Hcy(Gluc))$_2$-TMAA: HPLC (10 - 95% B in 15 min): $t_R$ = 8.70 min. Calculated monoisotopic mass ($C_{48}H_{65}N_3$O16$S_3$): 1035.35, found: 1036.3[M+H]$^+$.

HO-cit-Hcy(Lac)-cit-TMAA: HPLC (10 - 95% B in 15 min): $t_R$ = 7.90 min. Calculated monoisotopic mass ($C_{49}H_{67}N_7O_{17}S_2$): 1089.40, found: 1089.9[M+H]$^+$.

HO-s(*t*Bu)-Hcy(Lac)-s(*t*Bu)-TMAA: HPLC (10 - 95% B in 15 min): $t_R$ = 11.59 min. Calculated monoisotopic mass ($C_{51}H_{71}N_3O_{17}S_2$): 10 61.42, found: 1061.8 [M+H]$^+$.

**HO-a-(s(*t*Bu)$_3$-TMAA**

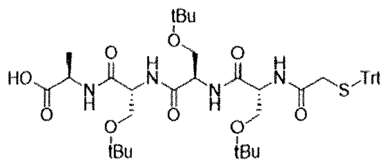

HO-a-(s(tBu))$_3$-TMAA: RP-HPLC (40 - 100% B in 15 min): $t_R$ = 16.54 min. Calculated monoisotopic mass ($C_{45}H_{62}N_4O_9S$): 834.42, found: 835.2[M+H]$^+$.

**HO-dap(Boc)-(s(*t*Bu))$_3$-TMAA**

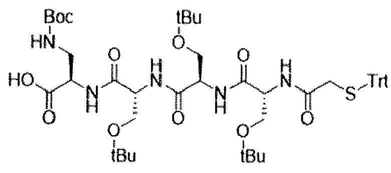

HO-dap(Boc)-(s(tBu))$_3$-TMAA: RP-HPLC (40 - 100% B in 15 min): $t_R$ = 17.60 min. Calculated monoisotopic mass ($C_{50}H_{71}N_5O_{11}S$): 949.49, found: 950.6[M+H]$^+$.

**2.3.8.2 LINKER-CHELATOR-CONSTRUCTS**

Linker-chelator-constructs were prepared by linear on-resin synthesis employing **GP1, GP2, GP3, GP9.**

**HO-Abz-a-r(Pbf)-r(Pbf)-s(*t*Bu))$_3$-TMAA**

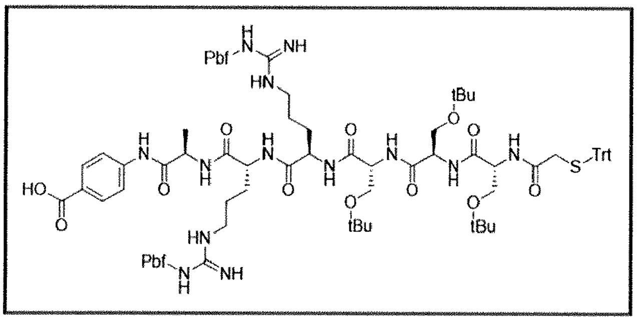

HO-Abz-a-r(Pbf)-r(Pbf)-(s(*t*Bu))$_3$-TMAA: RP-HPLC (10 - 90% B in 15 min): $t_R$ = 18.41 min. Calculated monoisotopic mass ($C_{90}H_{123}N_{13}O_{18}S_3$): 1769.83, found: 1771.2[M+H]$^+$ .

**HO-Abz-a-r(Pbf)-f-(s*t*Bu))$_3$-TMAA**

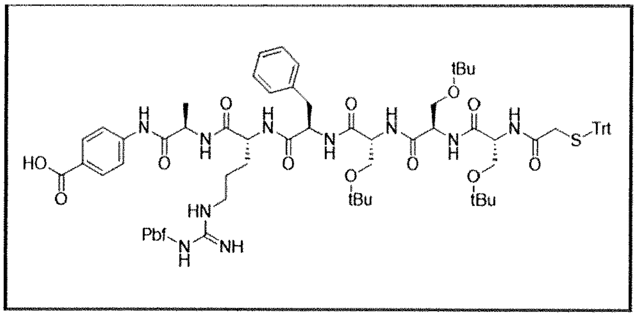

HO-Abz-a-r(Pbf)-f-(s(fBu))$_3$-TMAA: RP-HPLC (10 - 90% B in 15 min): $t_R$ = 17.44 min. Calculated monoisotopic mass ($C_{80}H_{104}N_{10}O_{15}S_2$): 1508.71, found: 1509.8[M+H]$^+$ .

**HO-Abz-a-r(Pbf)-h(Trt)-(s(*t*Bu))$_3$-TMAA**

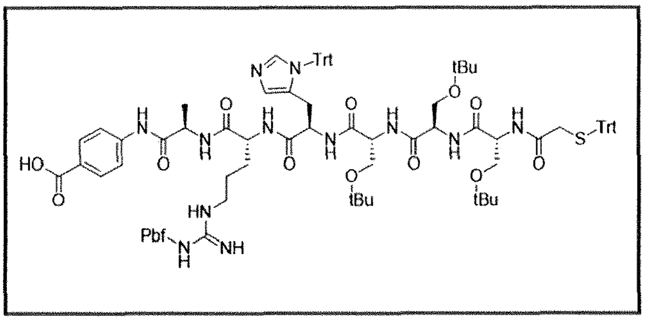

HO-Abz-a-r(Pbf)-h(Trt)-(s(*t*Bu))$_3$-TMAA: RP-HPLC (10 - 90% B in 15 min): $t_R$ = 17.80 min. Calculated monoisotopic mass ($C_{96}H_{116}N_{12}O_{15}S_2$): 1740.81, found: 1742.3[M+H]$^+$.

**(*t*Bu)e(HO-Ahx-dap(N4(Boc)$_4$)ue(*t*Bu)$_2$**

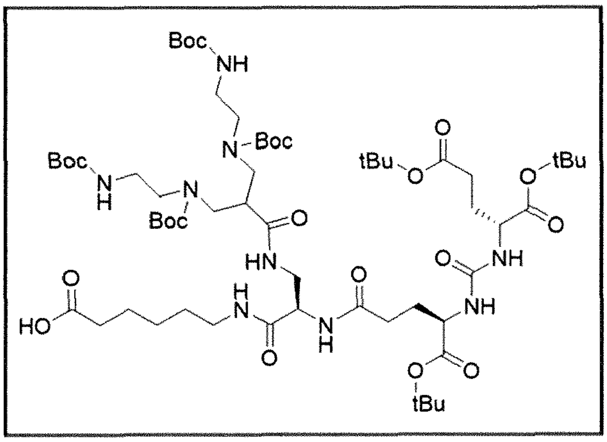

(*t*Bu)e(HO-Ahx-dap(N4(Boc)$_4$)ue(*t*Bu)$_2$: RP-HPLC (10 - 95% B in 15 min): $t_R$ = 16.57 min. Calculated monoisotopic mass ($C_{60}H_{107}N_9O_{20}$): 1273.76, found: 1274.6 [M+H]$^+$ .

**(*t*Bu)e(HO-Ahx-dap(R-DOTAGA(*t*Bu)$_4$-dap(SiFa-BA)ue(*t*Bu)$_2$**

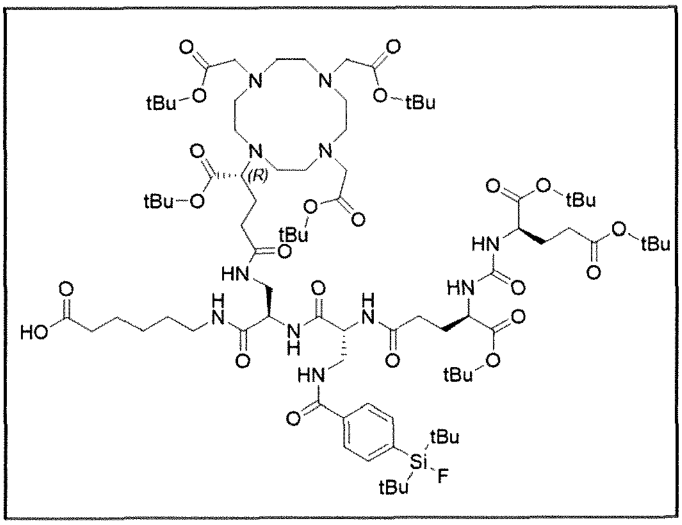

($t$Bu)e(HO-Ahx-dap(R-DOTAGA($t$Bu)$_4$-dap(SiFa-BA)ue($t$Bu)$_2$: RP-HPLC (10 - 95% B in 15 min): $t_R$ = 15.62 min. Calculated monoisotopic mass ($C_{85}H_{146}FN_{11}O_{22}Si$): 1720.04, found: 1721.3 $[M+H]^+$, 861.4 $[M+2H]^{2+}$.

**HO-Ahx-dap(R-DOTAGA(*t*Bu)$_4$)-SiFa*lin***

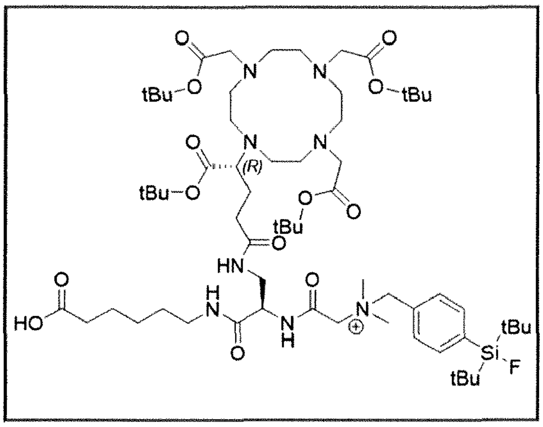

HO-Ahx-dap(R-DOTAGA($t$Bu)$_4$)-SiFalin: RP-HPLC (10 - 95% B in 15 min): $t_R$ = 11.64 min. Calculated monoisotopic mass ($C_{63}H_{112}FN_8O_{12}Si^+$): 1235.81, found: 1235.9 $[M+H]^+$.

**HO-Ahx-dap(DOTA(*t*Bu)$_3$)-D-HCy(Lactosyl)-SiFa*lin***

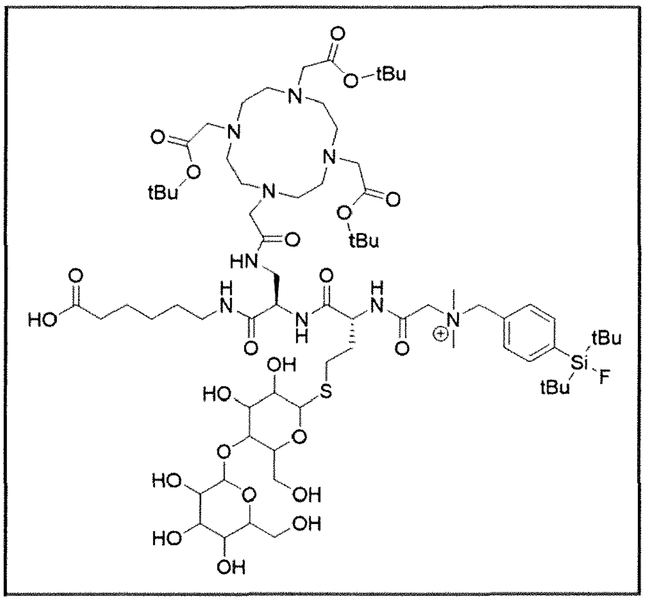

HO-Ahx-dap(DOTA(*t*Bu)$_3$)-D-HCy(Lactosyl)-SiFa*lin*: RP-HPLC (10 - 95% B in 15 min): $t_R$ = 9.55 min. Calculated monoisotopic mass ($C_{72}H_{127}FN_9O_{22}SSi^+$): 1548.86, found: 1550.3 [M+H]$^+$, 775.3 [M+2H]$^{2+}$.

**HO-Ahx-dap(R-DOTAGA(*t*B$_u$)$_4$)-D-HCy(Trt)-Ac**

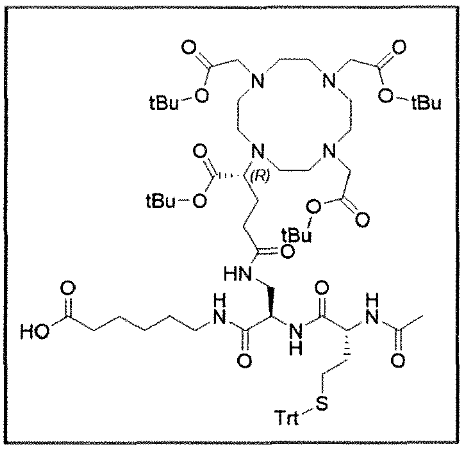

HO-Ahx-dap(R-DOTAGA(*t*Bu)$_4$)-D-HCy(Trt)-Ac: RP-HPLC (10 - 95% B in 15 min): $t_R$ = 12.49 min. Calculated monoisotopic mass ($C_{69}H_{104}N_8O_{14}S$): 1300.74, found: 1301.5 [M+H]$^+$.

**HO-Ahx-dap(DOTA(*t*Bu)$_3$)-Fmoc**

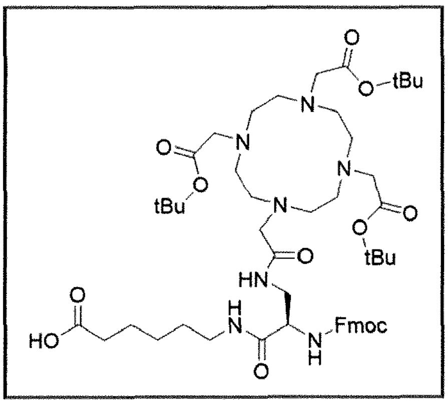

HO-Ahx-dap(DOTA(*t*Bu)$_4$)-Fmoc: RP-HPLC (10 - 95% B in 15 min): $t_R$ = 16.66 min. Calculated monoisotopic mass ($C_{52}H_{79}N_7O_{12}S$): 993.58, found: 938.7 [M-tBu+H]$^+$.

**2.3.8.3 HO-HYNIC(BOC)**

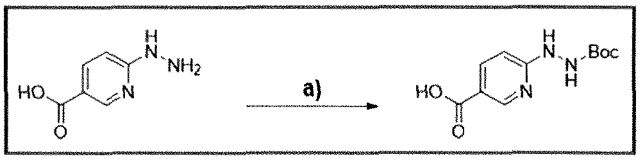


*Scheme 9: Boc protection of nicotinic acid:* ***a)*** *$Boc_2O$ (1.00 eq.), $Net_3$ (1.30 eq.).*

The synthesis of HO-HYNIC(Boc) was realized following the published procedure (45,46). In short, hydrazinonicotinic acid was reacted with $Boc_2O$ (1.00 eq.) and Triethylamine (1.30 eq.) in DMF over night. The solvent was evaporated under reduced pressure and the crude product was subjected to silica flash-chromatography employing EtOAc followed by EtOAc + 1vol% AcOH. The desired product was obtained after removal of the solvent as a white powder.

ESI-MS: Calculated monoisotopic mass ($C_{11}H_{15}N_3O_4$): 253.11, found: 254.4 [M+H]$^+$.

**2.3.8.4 3-((TERT-BUTOXYCARBONYL)(2-((TERT-BUTOXYCARBONYL)AMINO)ETHYL)AMINO)-2-(((TERT-BUTOXYCARBONYL)(2-((TERT-BUTOXYCARBONYL)AMINO)ETHYL)AMINO)METHYL)PROPANOIC ACID (= N4 CHELATOR [11])**

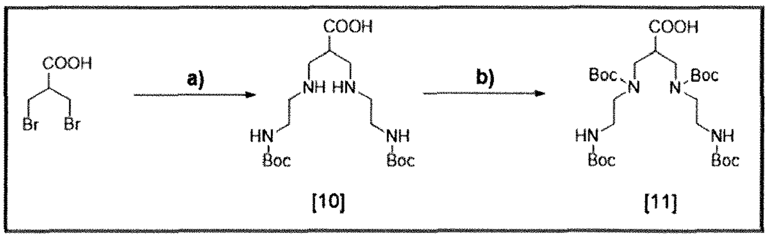

[10] [11]

*Scheme 10: Synthesis of the protected N4 chelator: a) Tert-butyl-(2-aminoethyl)-carbamate (4.00 eq.); b) $Net_3$ (3.00 eq.), $Boc_2O$ (4.00 eq.).*

*Tert*-butyl-(2-aminoethyl)-carbamate (4.00 eq.) was slowly added to 3-bromo-2-(bromomethyl)propanoic acid (1.00 eq.) in THF (25 mL/mmol) under vigorous

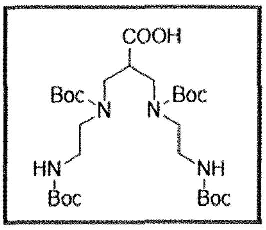

stirring. The mixture was stirred for 4 h at ambient temperature before removing the solvent under reduced pressure at room temperature. The crude product [10] was dissolved in acetone:$H_2O$ (1:1, 25 mL/mmol), cooled to 0 °C and $NEt_3$ (3.00 eq.) was added. After 5 min preactivation, $Boc_2O$ (4.00 eq.) was added. The mixture was stirred for 15 h (0°C to r.t.), the solvent removed under reduced pressure and the raw product purified via flash chromatography (35-95 % MeCN in $H_2O$, 15 min).

Rf(EtOAc+0.5%AcOH) = 0.65.

MS (ESI, positive): calculated monoisotopic mass for $C_{28}H_{52}N_4O_{10}$: 604.37; found by ESI-MS: m/z = 605.0 $[M+H]^+$.

$^1$H-NMR (400 MHz, DMSO-$d_6$) $\delta$ = 7.17-6.19 (m, 2H, NH), 3.28-3.17 (m, 6H, C$H_2$), 3.10-2.95 (m, 6H, C$H_2$), 2.94- 2.90 (m, 1H, CH), 1.38 (s, 18H, C$H_3$), 1.36 (s, 18H, C$H_3$).

### 3. SYNTHESIZED PEPTIDES

The vast majority of peptides within this study were synthesized via fragment condensation. The CPCR4 binding motif was separately synthesized and purified (see 2.3.1), as were the linker units in the most cases (see 2.3.6). These fragments were condensed according to **GP8,** Fmoc deprotected by dissolution in 20% piperidine/DMF (v/v) and purified via RP-HPLC. The resulting CPCR4-linker constructs were used as starting material for derivatization with the corresponding chelators or functional groups such as labeling moieties. The obtained peptides are listed in the following with their respective analytical data.

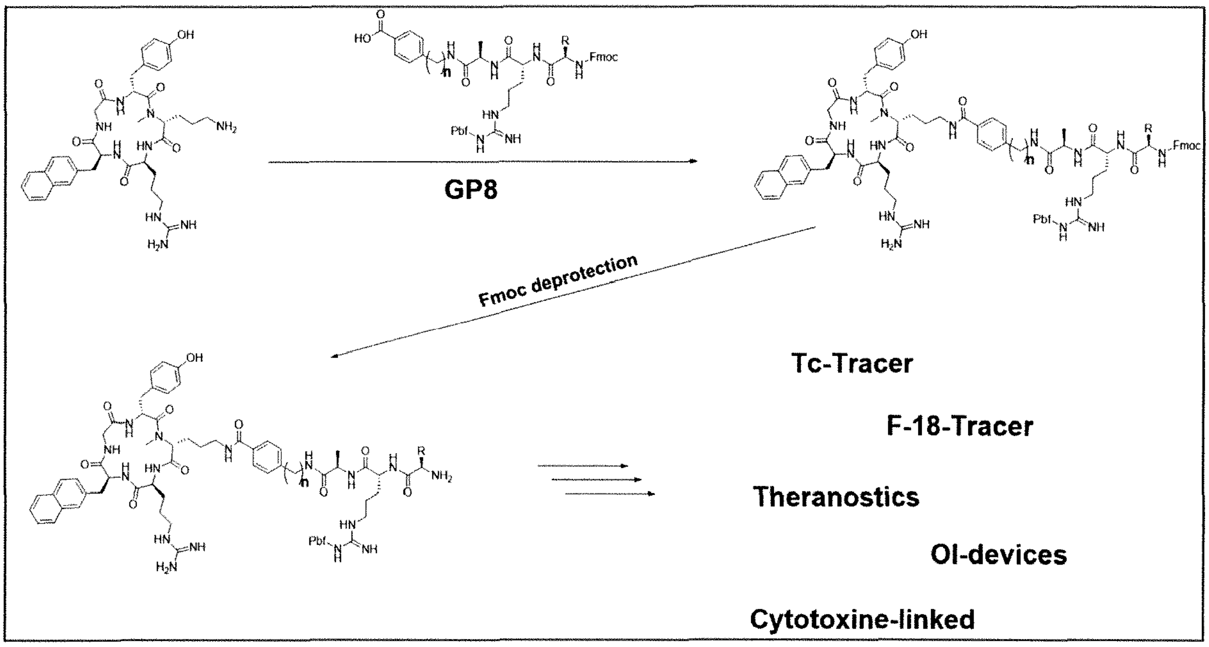


*Scheme 11: Fragment condensation to the CPCR-linker construct as a starting material for further derivatization.*

### 3.1 TECHNETIUM-99M SPECT TRACER

### 3.1.1 $MAS_3$-CONJUGATED PEPTIDES

**Tc-CXCR4-1**

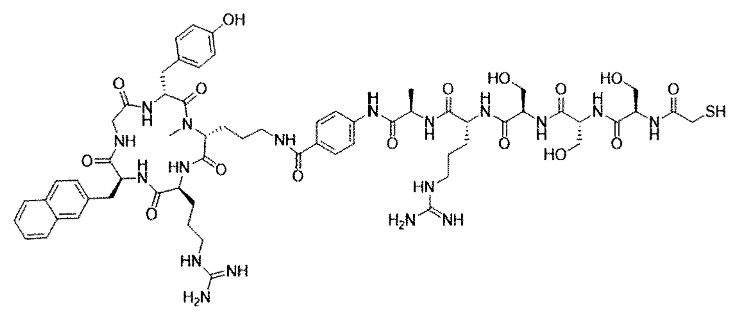

The synthesis of Tc-CXCR4-1 was facilitated by fragment condensation according to **GP8**. CPCR4 (see 2.3.1) and the linker HO-Abz-a-r(Pbf)-Fmoc (see 2.3.6) were condensed and the resulting peptide Fmoc deprotected before further coupling with HO-(s(*t*Bu))$_3$-TMAA (see 2.3.8.1). The crude product was deprotected (see **GP5**) und subjected to semi-preparative RP-HPLC purification.

Tc-CXCR4-1: RP-HPLC (10 - 90% B in 15 min): $t_R$ = 9.82 min. Calculated monoisotopic mass ($C_{63}H_{86}N_{18}O_{16}S$): 1382.62, found: 692.2[M+2H]$^{2+}$.

**Tc-CXCR4-2**

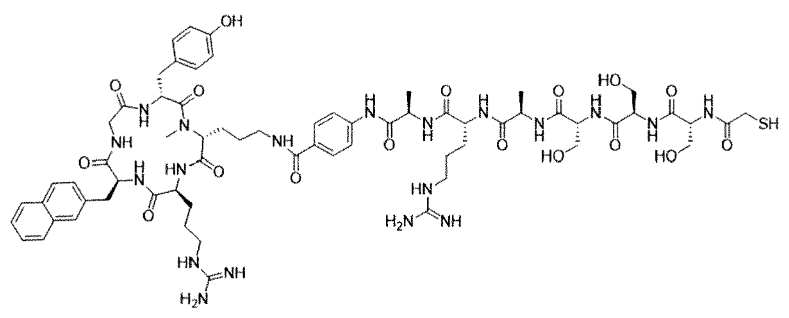

The synthesis of Tc-CXCR4-2 was conducted in analogy to Tc-CXCR4-1. CPCR4 (see 2.3.1) and the linker HO-Abz-a-r(Pbf)-Fmoc (see 2.3.6) were condensed and the resulting peptide Fmoc deprotected before further coupling with HO-a-(s(*t*Bu))$_3$-TMAA (see 2.3.8.1). The crude product was deprotected (see **GP5**) und subjected to semi-preparative RP-HPLC purification.

Tc-CXCR4-2: RP-HPLC (15 - 45% B in 15 min): $t_R$ = 9.10 min. Calculated monoisotopic mass ($C_{66}H_{91}N_{19}O_{17}S$): 1453.66, found: 728.6[M+2H]$^{2+}$.

**Tc-CXCR4-3**

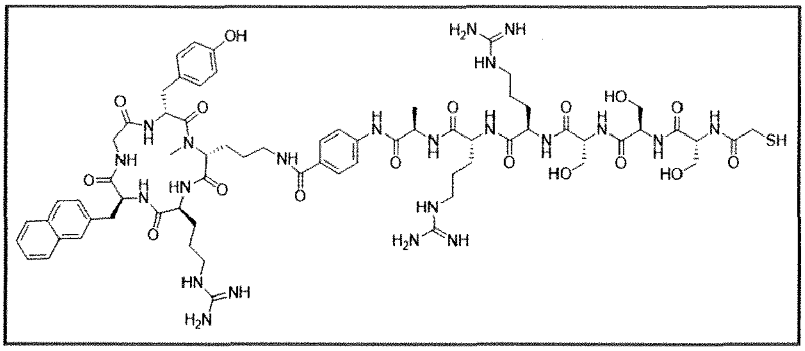

Tc-CXCR4-3 was synthesized by coupling CPCR4 (see 2.3.1) and the linker-chelator construct HO-Abz-a-r(Pbf)-r(Pbf)-(s(*t*Bu))$_3$-TMAA (see 2.3.8.2). The crude product was deprotected (see **GP5**) und subjected to semi-preparative RP-HPLC purification.

Tc-CXCR4-3: RP-HPLC (10 - 90% B in 15 min): $t_R$ = 9.00 min. Calculated monoisotopic mass ($C_{69}H_{98}N_{22}O_{17}S$): 1538.72, found: 770.4[M+2H]$^{2+}$.

## Tc-CXCR4-4

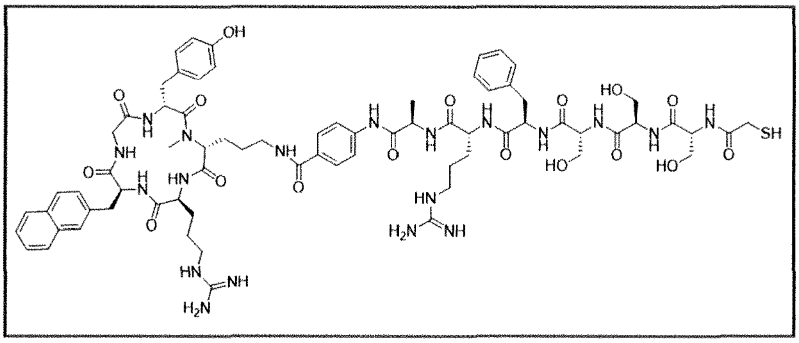

Tc-CXCR4-4 was synthesized in analogy to Tc-CXCR4-3 by coupling CPCR4 (see 2.3.1) and the linker-chelator construct HO-Abz-a-r(Pbf)-f-(s(*t*Bu))$_3$-TMAA (see 2.3.8.2). The crude product was deprotected (see **GP5**) und subjected to semi-preparative RP-HPLC purification.

Tc-CXCR4-4: RP-HPLC (10 - 90% B in 15 min): $t_R$ = 10.20 min. Calculated monoisotopic mass ($C_{72}H_{95}N_{19}O_{17}S$): 1529.69, found: 766.1 [M+2H]$^{2+}$.

## Tc-CXCR4-5

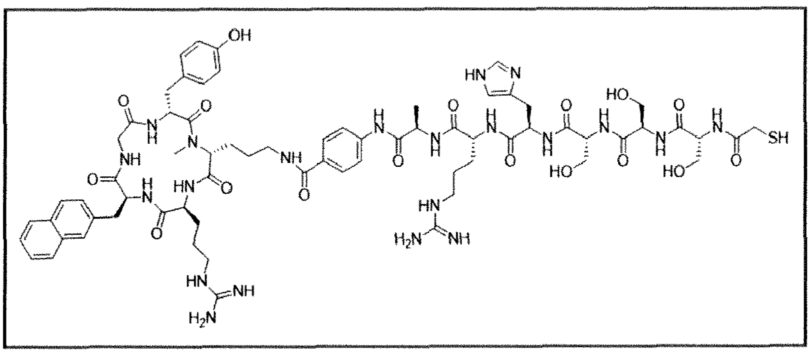

Tc-CXCR4-5 was synthesized in analogy to Tc-CXCR4-3 and -4 by coupling CPCR4 (see 2.3.1) and the linker-chelator construct HO-Abz-a-r(Pbf)-h(Trt)-(s(*t*Bu))$_3$-TMAA (see 2.3.8.2). The crude product was deprotected (see **GP5**) und subjected to semi-preparative RP-HPLC purification.

Tc-CXCR4-5: RP-HPLC (10 - 90% B in 15 min): $t_R$ = 9.53 min. Calculated monoisotopic mass ($C_{69}H_{93}N_{21}O_{17}S$): 1519.68, found: 761.2[M+2H]$^{2+}$.

**Tc-CXCR4-6**

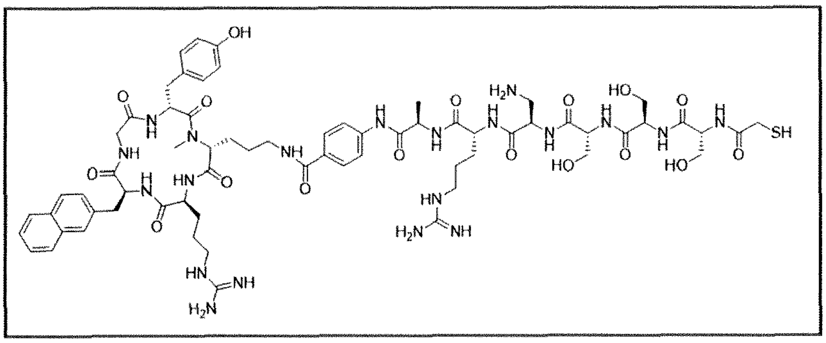

The synthesis of Tc-CXCR4-6 was achieved in analogy to Tc-CXCR4-1. CPCR4 (see 2.3.1) and the linker HO-Abz-a-r(Pbf)-Fmoc (see 2.3.6) were condensed and the resulting peptide Fmoc deprotected before further coupling with HO-dap(Boc)-(s(*t*Bu))$_3$-TMAA (see 2.3.8.1). The crude product was deprotected (see **GP5**) und subjected to semi-preparative RP-HPLC purification.

Tc-CXCR4-6: RP-HPLC (35 - 65% B in 15 min): $t_R$ = 8.85 min. Calculated monoisotopic mass ($C_{66}H_{92}N_{20}O_{17}S$): 1468.67, found: 735.1 [M+2H]$^{2+}$.

**Tc-CXCR4-7**

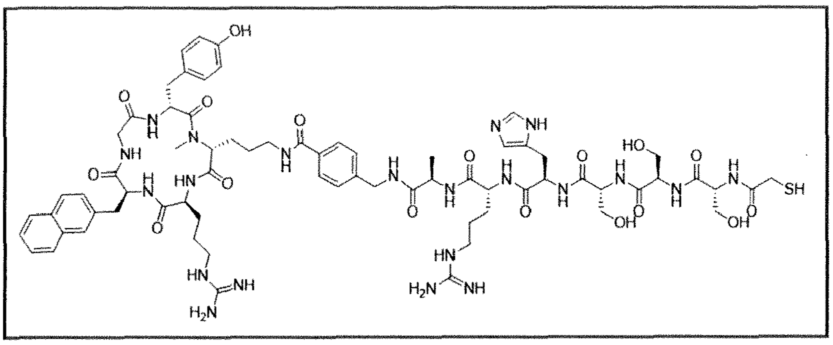

The synthesis was achieved by fragment condensation (**GP8**) of the CPCR4-linker-construct CPCR4-Ambz-a-r(Pbf)-his(Trt)-$NH_2$ (see 2.3.7) and the chelator HO-(s(*t*Bu))$_3$-TMAA (see 2.3.8.1). Adjacent deprotection of the peptide (**GP5**) and purification by semi-preparative RP-HPLC afforded the desired product.

**Tc-CXCR4-8**

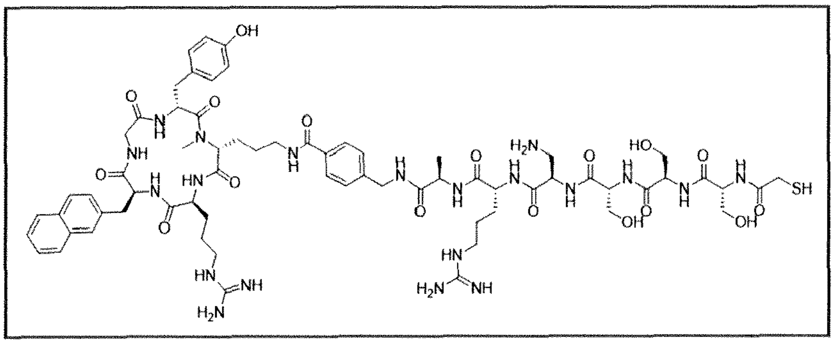

The synthesis was achieved by fragment condensation (**GP8**) of the CPCR4-linker -construct CPCR4-Ambz-a-r(Pbf)-$NH_2$ (see 2.3.7) and the modified chelator HO-a-(s(*t*Bu))$_3$-TMAA (see 2.3.8.1). Adjacent deprotection of the peptide (**GP5**) and purification by semi-preparative RP-HPLC afforded the desired product.

Tc-CXCR4-8: RP-HPLC (20 - 60% B in 15 min): $t_R$ = 7.26 min. Calculated monoisotopic mass ($C_{67}H_{94}N_{20}O_{17}S$): 1482.68, found: 742.4[M+2H]$^{2+}$.

### 3.1.2 MODIFIED MAS$_3$-CONJUGATED PEPTIDES

**Tc-CXCR4-9**

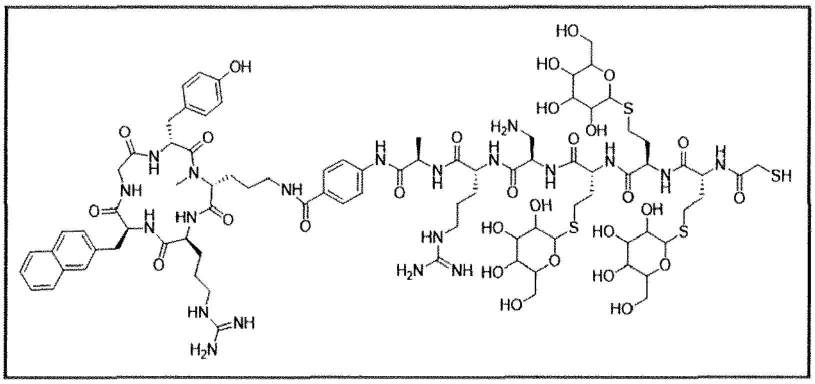

Tc-CXCR4-9 was synthesized via fragment condensation (see **GP8**). The CPCR4-linker construct CPCR4-Abz-a-r(Pbf)-dap(Boc)-$NH_2$ (see 2.3.7) was reacted with the protected chelator HO-(Hcy(Gluc(OAc)$_4$))$_3$-TMAA (see 2.3.8.1). Deacetylation as described and adjacent removal of acid-lablile protecting groups (see **GP5**) yielded the desired crude product, that was purified via semi-preparative RP-HPLC.

Tc-CXCR4-9: RP-HPLC (5 - 55% B in 15 min): $t_R$ = 9.51 min. Calculated monoisotopic mass ($C_{87}H_{128}N_{20}O_{29}S_4$): 2044.80, found: 1023.7[M+2H]$^{2+}$.

**Tc-CXCR4-10**

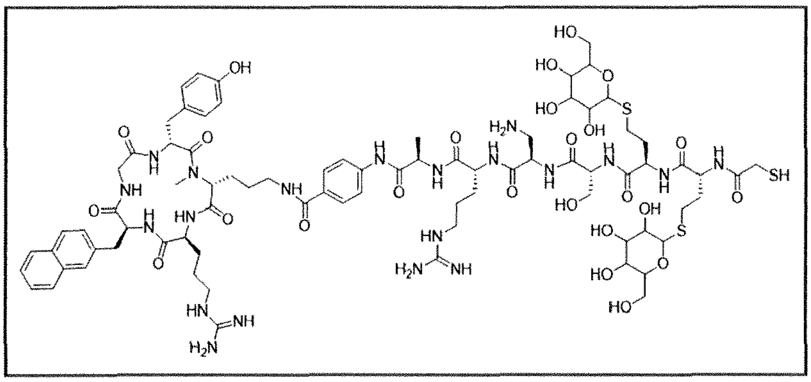

Tc-CXCR4-10 was synthesized in analogy to Tc-CXCR4-9 with the difference being that the chelator HO-s(*t*Bu)-(Hcy(Gluc))$_2$-TMAA (see 2.3.8.1) was de-acetylated before coupling with the CPCR4-linker construct. The crude product was fully deprotected (see **GP5**) and purified via semi-preparative RP-HPLC.

Tc-CXCR4-10: RP-HPLC (10 - 60% B in 15 min): $t_R$ = 9.00 min. Calculated monoisotopic mass ($C_{80}H_{116}N_{20}O_{25}S_3$): 1852.76, found: 927.4[M+2H]$^{2+}$, 618.7 [M+3H]$^{3+}$.

## Tc-CXCR4-11

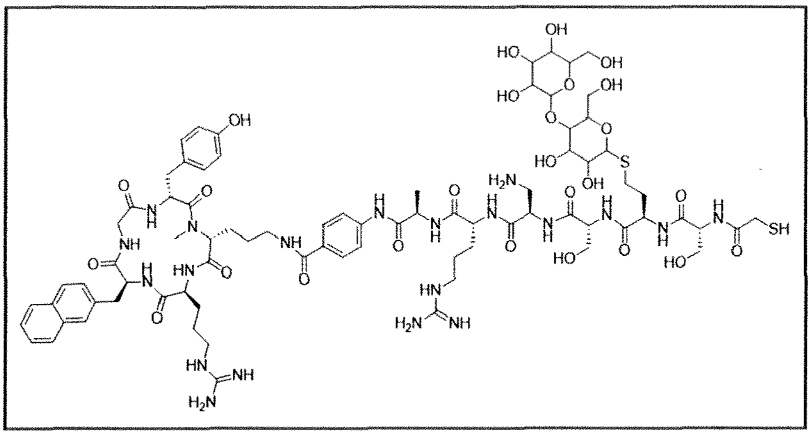

Tc-CXCR4-11 was synthesized in analogy to Tc-CXCR4-10 with the de-acetylated chelator HO-s(*t*Bu)-Hcy(Lac)-s(*t*Bu)-TMAA (see 2.3.8.1). The crude product was fully deprotected (see **GP5**) and purified via semi-preparative RP-HPLC.

Tc-CXCR4-11: RP-HPLC (10 - 60% B in 15 min): $t_R$ = 7.90 min. Calculated monoisotopic mass ($C_{79}H_{114}N_{20}O_{26}S_2$): 1822.77, found: 920.4[M+$H_2O$+2H]$^{2+}$, 614.1 [M+$H_2O$+3H]$^{3+}$.

## Tc-CXCR4-12

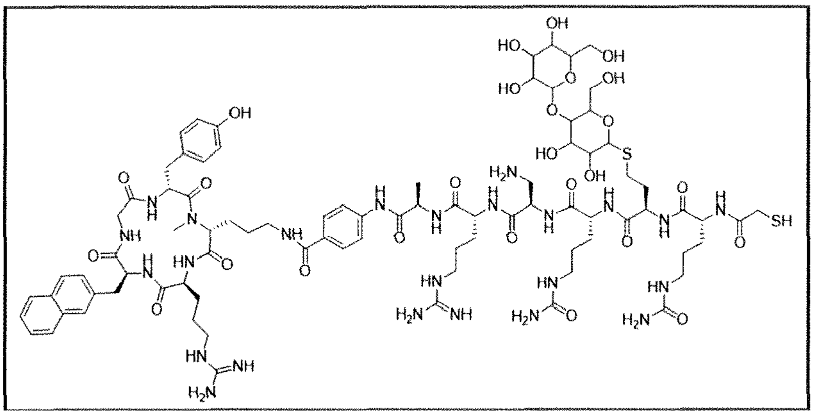

Tc-CXCR4-12 was synthesized in analogy to Tc-CXCR4-10 and -11 with the de-acetylated chelator HO-cit-Hcy(Lac)-cit-TMAA (see 2.3.8.1). The crude product was fully deprotected (see **GP5**) and purified via semi-preparative RP-HPLC.

Tc-CXCR4-12: RP-HPLC (10 - 60% B in 15 min): $t_R$ = 8.00 min. Calculated monoisotopic mass ($C_{85}H_{126}N_{24}O_{26}S_2$): 1962.87, found: 982.6[M+2H]$^{2+}$, 655.5 [M+3H]$^{3+}$.

### 3.1.3 FIXED-CHELATOR-BASED PEPTIDES

**Tc-CXCR4-13**

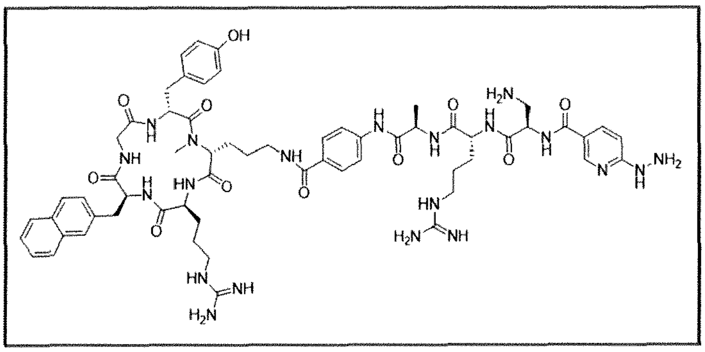

The synthesis of Tc-CXCR4-13 was achieved by coupling of HO-HYNIC(Boc) (see 2.3.8.3) to the CPCR4-linker construct CPCR4-Abz-a-r(Pbf)-dap(Boc)-$NH_2$ (see 2.3.7) according to **GP8** before deprotection via **GP5** and purification by semi-preparative RP-HPLC.

Tc-CXCR4-13: RP-HPLC (5 - 55% B in 15 min): $t_R$ = 7.88 min. Calculated monoisotopic mass ($C_{61}H_{80}N_{20}O_{11}$): 1268.63, found: 635.6[M+2H]$^{2+}$.

**Tc-CXCR4-14**

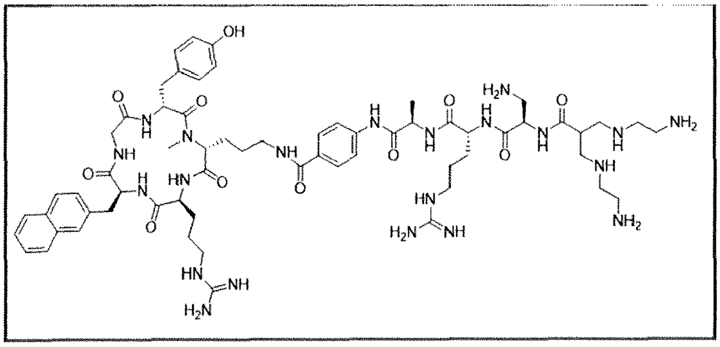

Tc-CXCR4-14 was synthesized in analogy to Tc-CXCR4-13. HO-N4(Boc)$_4$ (see 2.3.8.4) was coupled to the CPCR4-linker construct CPCR4-Abz-a-r(Pbf)-dap(Boc)-NH$_2$ (see 2.3.7) according to **GP8** before deprotection via **GP5** and purification by semi-preparative RP-HPLC.

Tc-CXCR4-14: RP-HPLC (10 - 60% B in 15 min): $t_R$ = 8.03 min. Calculated monoisotopic mass ($C_{63}H_{93}N_{21}O_{11}$): 1319.74, found: 660.7[M+2H]$^{2+}$, 441.0[M+3H]$^{3+}$.

## Tc-CXCR4-15

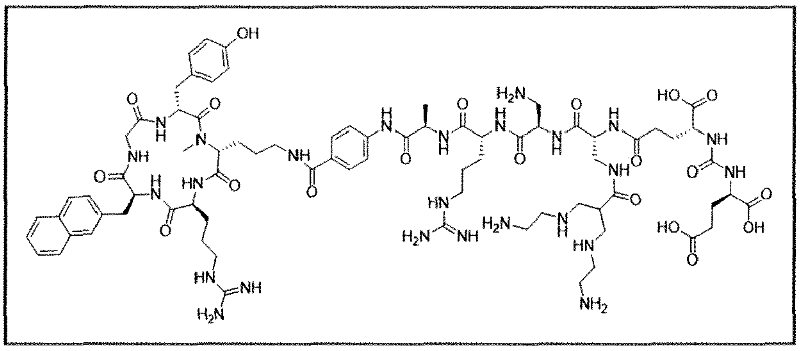

Tc-CXCR4-15 was synthesized in analogy to Tc-CXCR4-13 and -14. The chelator-bearing scaffold (*t*Bu)e(HO-dap(N4(Boc)$_4$))ue(*t*Bu)$_2$ was coupled to the CPCR4-linker construct CPCR4-Abz-a-r(Pbf)-dap(Boc)-NH$_2$ (see 2.3.7) according to **GP8** before deprotection via **GP5** and purification by semi-preparative RP-HPLC.

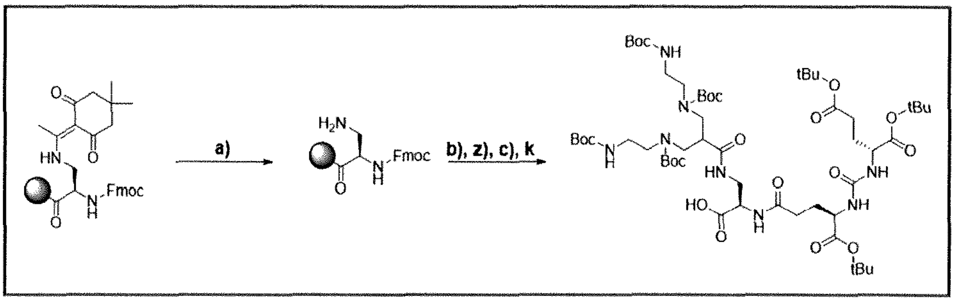


*Scheme 12: Synthesis of the N4-chelator-bearing moiety: a) Imidazole (0.46 g), [NH$_3$OH]Cl (0.63 g); b) HO-N4(Boc)$_4$ (1.50 eq.), HOAt (1.50 eq.), TBTU (1.50 eq.) 2,4,6,-Collidine (3.00 eq.); z) 20% Piperidine in DMF (v/v); c) (tBu)e(OH)ue(tBu)$_2$ (1.50 eq.), HOAT (1.50 eq.), TBTU (1.50 eq.), DIPEA (3.00 eq.); k) 20% HFIP in DCM (v/v).*

(*t*Bu)e(HO-dap(N4(Boc)$_4$))ue(*t*Bu)$_2$: RP-HPLC (10 - 95% B in 15 min): $t_R$ = 16.75 min. Calculated monoisotopic

mass ($C_{54}H_{96}N_8O_{19}$): 1160.68, found: 1162.2[M+H]$^+$, 531[M-Boc+2H]$^{2+}$.

Tc-CXCR4-15: RP-HPLC (10 - 60% B in 15 min): $t_R$ = 8.03min, Calculated monoisotopic mass ($C_{77}H_{113}N_{25}O_{20}$): 1707.86, found: 855.5[M+2H]$^{2+}$, 570.6[M+3H]$^{3+}$, 427.1 [M+4H]$^{4+}$.

## Tc-CXCR4-16

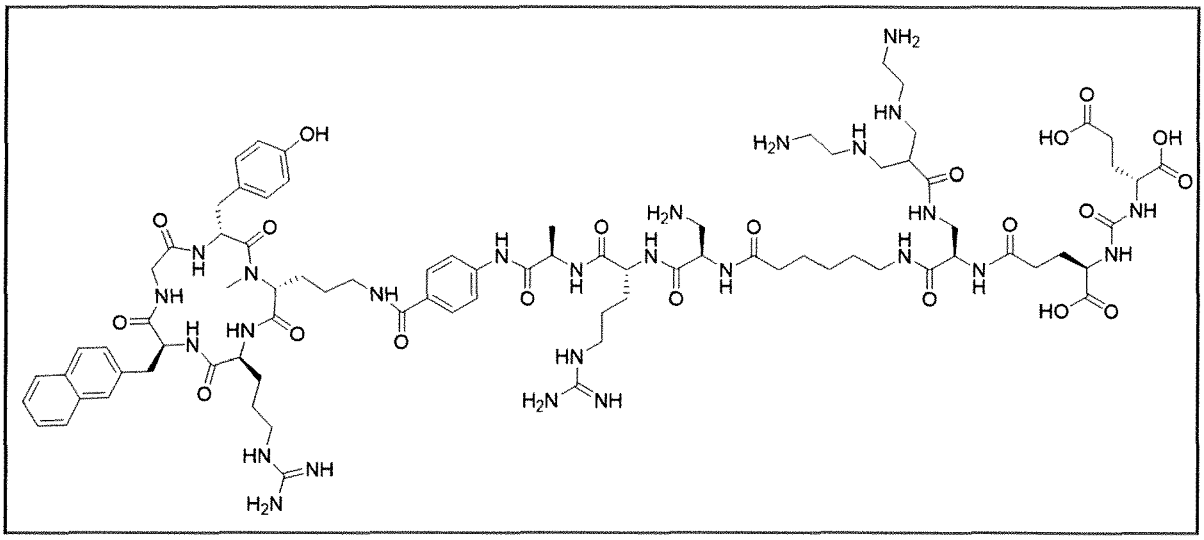

Tc-CXCR4-16 was synthesized in analogy to Tc-CXCR4-15. The chelator-bearing Ahx-based scaffold (*t*Bu)e(HO-Ahx-dap(N4(Boc)$_4$))ue(*t*Bu)$_2$ (see 0) was coupled to the CPCR4-linker construct CPCR4-Abz-a-r(Pbf)-dap(Boc)-NHz (see 2.3.7) according to **GP8** before deprotection via **GP5** and purification by semi-preparative RP-HPLC.

Tc-CXCR4-16: RP-HPLC (10 - 60% B in 15 min): $t_R$ = 7.83 min. Calculated monoisotopic mass ($C_{83}H_{124}N_{26}O_{21}$): 1820.94, found: 1821.5 [M+H]$^+$, 910.8 [M+2H]$^{2+}$, 607.4 [M+3H]$^{3+}$.

## 3.2 DOTA CONJUGATED PEPTIDES

Conjugation of DOTA to the respective Ambz- or Abz- bearing scaffold (see 2.3.7) was carried out according to a recently published procedure (*47*). In short, DOTA (4.00 eq.) was pre-activated with NHS (5.00 eq.), EDCI (5.00 eq.) and 2,4,6-Collidine (6.00 eq.) for 30 min in $H_2O$. The Fmoc deprotected peptide, dissolved in DMF (30 μL/mg) was given to the mixture and stirred for 2-4 h at r.t.. The solvent was evaporated *in vacuo* and the crude product treated with TFA for 1-2 h (see **GP5**). After removal of TFA in $N_2$ stream, the product was purified via semi preparative RP-HPLC.

Iodination of the tyr-bearing binding motif was achieved according to the general procedure **GP7,** based on the purified and fully deprotected peptide.

## CXCR4-DOTA-1

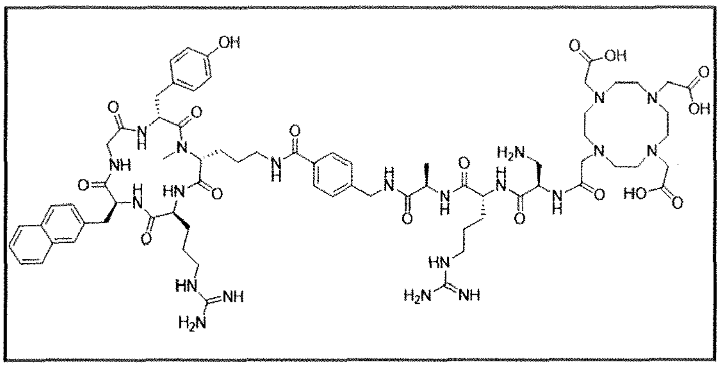

Synthesis of CXCR4-DOTA-1 was achieved, applying the general synthetic procedures **GP1, GP2**, **GP3** to yield CPCR4-Ambz-a-r(Pbf)-dap(Boc)-$NH_2$ (see 2.3.7). DOTA was coupled as described above and the peptide deprotected according to **GP5**.

CXCR4-DOTA-1: RP-HPLC (10 - 60% B in 15 min): $t_R$ = 7.97 min, . Calculated monoisotopic mass ($C_{72}H_{103}N_{21}O17$): 1533.78, found: 768.6 $[M+2H]^{2+}$, 512.6 $[M+3H]^{3+}$.

[natGa]CXCR4-DOTA-1: RP-HPLC (10 - 60% B in 15 min): $t_R$ = 7.97 min. Calculated monoisotopic mass ($C_{72}H_{101}GaN_{21}O_{17}$): 1600.69, found: 801.0 $[M+2H]^{2+}$, 534.8 $[M+3H]^{3+}$.

[natLu]CXCR4-DOTA-1: RP-HPLC (10 - 60% B in 15 min): $t_R$ = 7.99 min. Calculated monoisotopic mass ($C_{72}H_{100}LuN_{21}O_{17}$): 1705.70, found: 570.1 $[M+3H]^{3+}$.

## CXCR4-DOTA-2

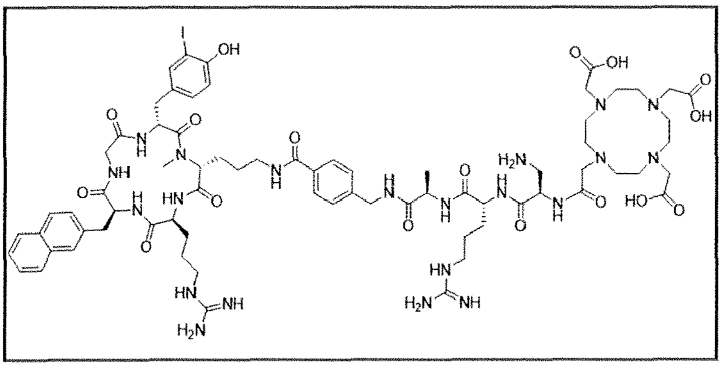

Synthesis of CXCR4-DOTA-2 was realized on the basis of the fully deprotected and purified CXCR4-DOTA-1 by iodination via **GP7**. The desired product was isolated by semi-preparative RP-HPLC.

CXCR4-DOTA-2: RP-HPLC (10 - 60% B in 15 min): $t_R$ = 8.85 min. Calculated monoisotopic mass ($C_{72}H_{102}IN_{21}O_{17}$): 1659.68, found: 1660.6 $[M+H]^{+}$, 830.7$[M+2H]^{2+}$, 553.9$[M+3H]^{3+}$.

[natGa]CXCR4-DOTA-2: RP-HPLC (10 - 60% B in 15 min): $t_R$ = 8.73 min. Calculated monoisotopic mass ($C_{72}H_{100}GaIN_{21}O_{17}$): 1726.59, found: 863.9 $[M+2H]^{2+}$, 576.6 $[M+3H]^{3+}$.

[natLu]CXCR4-DOTA-2: RP-HPLC (10 - 60% B in 15 min): $t_R$ = 8.80 min. Calculated monoisotopic mass ($C_{72}H_{99}ILuN_{21}O_{17}$): 1831.60, found: 916.8 $[M+2H]^{2+}$, 611.6 $[M+3H]^{3+}$.

## CXCR4-DOTA-3

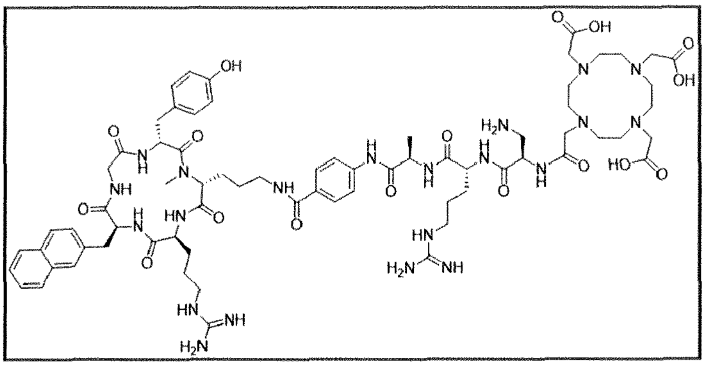

CXCR4-DOTA-3 was synthesized in analogy to CXCR4-DOTA-1 with the difference being the linker used. In this case HO-Abz-a-r-(Pbf)-dap(Boc)-Fmoc (see 2.3.6) was coupled to CPCR4 before Fmoc deprotection, purification and DOTA coupling.

CXCR4-DOTA-3: RP-HPLC (20 - 75% B in 15 min): $t_R$ = 5.36 min. Calculated monoisotopic mass ($C_{71}H_{101}N_{21}O_{17}$): 1519.77, found: 1521.0 $[M+H]^+$, 760.8 $[M+2H]^{2+}$, 507.9 $[M+3H]^{3+}$.

[natGa]CXCR4-DOTA-3: RP-HPLC (20 - 75% B in 15 min): $t_R$ = 6.65 min. Calculated monoisotopic mass ($C_{71}H_{99}GaN_{21}O_{17}$): 1586.68, found: 1569.5 $[M+H]^+$, 795.0 $[M+2H]^{2+}$, 529.6 $[M+3H]^{3+}$.

[natLu]CXCR4-DOTA-3: RP-HPLC (20 - 75% B in 15 min): $t_R$ = 6.62 min. Calculated monoisotopic mass ($C_{71}H_{98}LuN_{21}O_{17}$): 1691.69, found: 1691.9 $[M+H]^+$, 846.8 $[M+2H]^{2+}$, 565.1 $[M+3H]^{3+}$.

**CXCR4-DOTA-4**

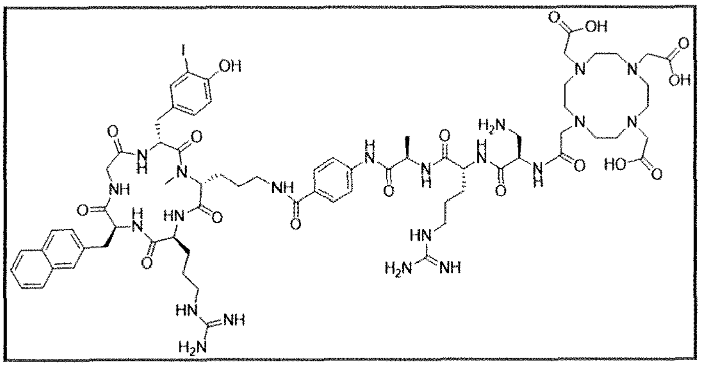

Synthesis of CXCR4-DOTA-4 was accomplished by iodination of the fully deprotected and purified CXCR4-DOTA-3 according to **GP7**. The desired product was isolated by semi-preparative RP-HPLC.

CXCR4-DOTA-4: RP-HPLC (20 - 80% B in 15 min): $t_R$ = 5.72 min. Calculated monoisotopic mass ($C_{71}H_{100}IN_{21}O_{17}$): 1645.67, found: 1647.2 $[M+H]^+$, 824.4 $[M+2H]^{2+}$, 550.0 $[M+3H]^{3+}$.

[natGa]CXCR4-DOTA-4: RP-HPLC (20 - 80% B in 15 min): $t_R$ = 5.52 min. Calculated monoisotopic mass ($C_{71}H_{98}GaIN_{21}O_{17}$): 1712.58, found: 857.1 $[M+2H]^{2+}$, 572.2 $[M+3H]^{3+}$.

[natLu]CXCR4-DOTA-4: RP-HPLC (20 - 80% B in 15 min): $t_R$ = 5.55 min. Calculated monoisotopic mass ($C_{71}H_{97}ILuN_{21}O_{17}$): 1817.58, found: 910.2 $[M+2H]^{2+}$, 607.1 $[M+3H]^{3+}$.

**CXCR4-DOTA-5**

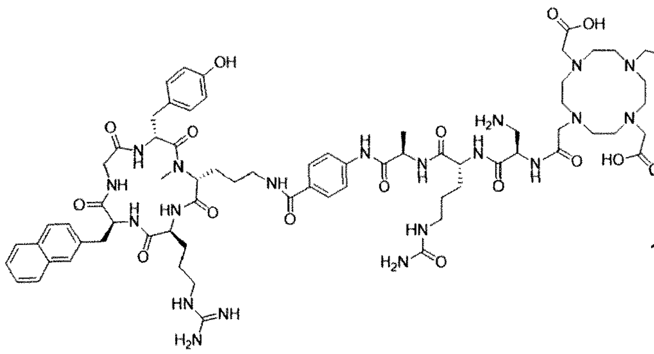

Synthesis of CXCR4-DOTA-5 was achieved in analogy to CXCR4-DOTA-3 with the difference being the CPCR4-linker construct CPCR4-Abz-a-cit-dap(Boc)-$NH_2$ (see 2.3.7). After Fmoc deprotection, DOTA was coupled as described above and the resulting peptide deprotected (**GP5**) before purification via semi-preparative RP-HPLC.

CXCR4-DOTA-5: RP-HPLC (10 - 60% B in 15 min): $t_R$ = 8.40 min. Calculated monoisotopic mass ($C_{71}H_{100}N_{20}O_{18}$): 1520.75, found: 1522.0 $[M+H]^+$, 761.6 $[M+2H]^{2+}$.

[$^{nat}$Ga]CXCR4-DOTA-5: RP-HPLC (10 - 60% B in 15 min): $t_R$ = 8.33 min. Calculated monoisotopic mass ($C_{71}H_{98}N_{20}GaO_{18}$): 1587.66, found: 794.6 $[M+2H]^{2+}$, 529.8 $[M+3H]^{3+}$.

[$^{nat}$Lu]CXCR4-DOTA-5: RP-HPLC (10 - 60% B in 15 min): $t_R$ = 8.42 min. Calculated monoisotopic mass ($C_{71}H_{97}LUN_{20}O_{18}$): 1692.67, found: 847.2 $[M+2H]^{2+}$, 565.1 $[M+3H]^{3+}$.

## Pentixafor

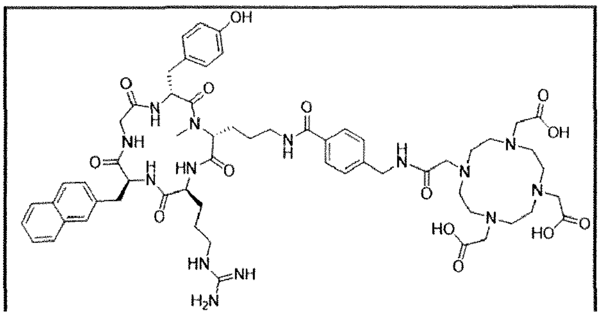

Synthesis of Pentixafor was carried out according to the published literature (*48*). In short, HO-Ambz-Fmoc was coupled to CPCR4 (**GP8**) and the product Fmoc-deprotected. After purification via semi-preparative RP-HPLC, DOTA was coupled according to the literature and as described above (*47*). The resulting peptide was deprotected (**GP5**) and purified by semi-preparative RP-HPLC.

Pentixafor: RP-HPLC (15 - 45% B in 15 min): $t_R$ = 14.96 min. Calculated monoisotopic mass ($C_{60}H_{80}N_{14}O_{14}$): 1220.60, found: 1220.8 $[M+H]^+$.

[$^{nat}$Ga]Pentixafor: RP-HPLC (10 - 60% B in 15 min): $t_R$ = 8.73 min. Calculated monoisotopic mass ($C_{60}H_{78}GaN_{14}O_{14}$): 1287.51, found: 863.9 $[M+2H]^{2+}$, 576.6 $[M+3H]^{3+}$.

## Pentixather

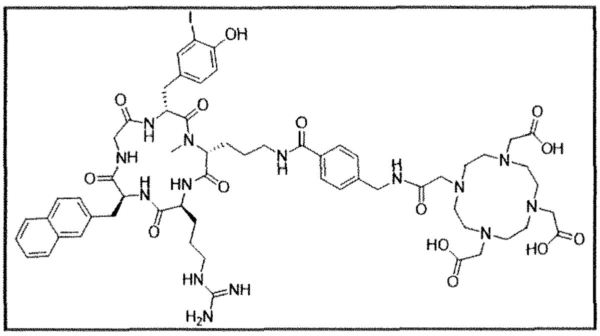

Pentixather was obtained by iodination of Pentixafor according to the literature (*39*) and **GP7,** based on the fully deprotected and purified peptide. The resulting mixture was subjected to semi-preparative RP-HPLC.

Pentixather: RP-HPLC (15 - 45% B in 15 min): $t_R$ = 10.60 min. Calculated monoisotopic mass ($C_{60}H_{79}IN_{14}O_{14}$): 1346.59, found: 1347.7 $[M+H]^+$, 676.2$[M+2H]^{2+}$.

[$^{nat}$Lu]Pentixather: RP-HPLC (10 - 60% B in 15 min): $t_R$ = 9.34 min. Calculated monoisotopic mass ($C_{60}H_{79}ILuN_{14}O_{14}$): 1518.41, found: 1519.2 $[M+H]^+$, 760.4 $[M+2H]^{2+}$.

**3.3 FLUORINE-18 PET TRACER/RADIOHYBRIDS**

**CXCR4-SiFA-1**

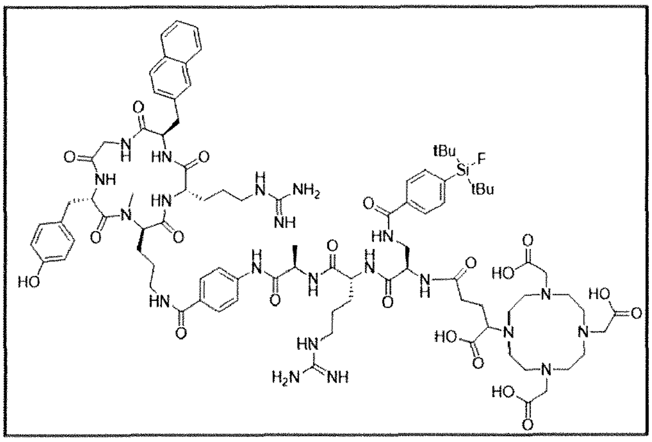

CXCR4-SiFA-1 was synthesized via fragment condensation (see **GP8**) of CPCR4 and the SiFA-bearing linker HO-Abz-a-r(Pbf)-dap(SiFA-BA)-Fmoc.

Adjacent Fmoc deprotection and coupling with DOTA-GA anhydride, followed by deprotection and purification via semi-preparative RP-HPLC yielded the desired product.

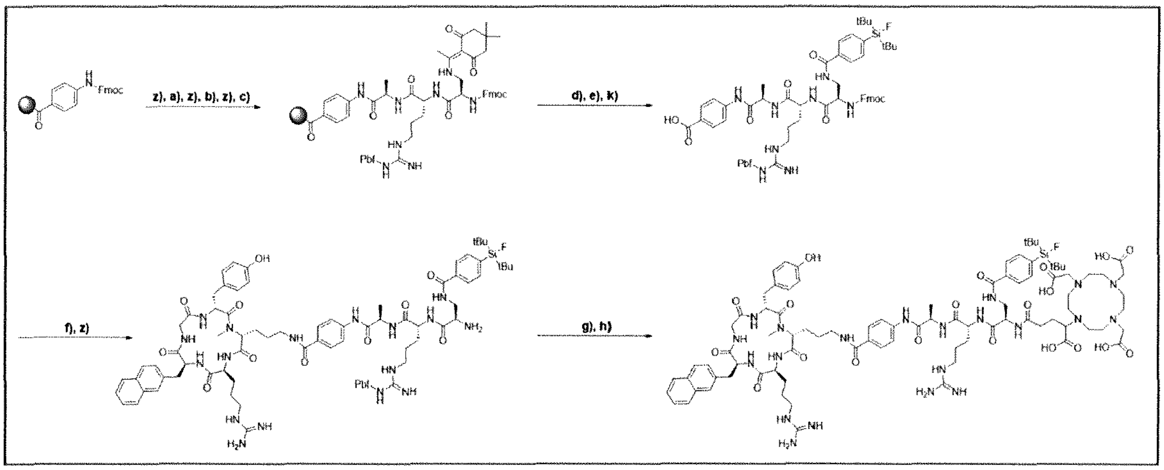


*Scheme 13: Synthesis of CXCR4-SiFA-1 via fragment condensation: z) 20% Piperidine in DMF (v/v); **a)** Fmoc-D-Ala-OH (2.00 eq.), HOAt (2.00 eq.), HATU (2.00 eq.), DIPEA (5.00 eq.); **b)** Fmoc-D-Arg(Pbf)-OH (1.50 eq.), HOAt (1.50 eq.), TBTU (1.50 eq.), DIPEA (3.00 eq.); .); **c)** Fmoc-dap(Dde)-OH (1.50 eq.), HOAt (1.50 eq.), TBTU (1.50 eq.), 2,4,6,-Collidine (3.00 eq.); **d)** Imidazole (0.46 g), [NH$_2$OH]Cl (0.63 g); **e)** SiFA-BA-OH (1.50 eq.), HOAt (1.50 eq.), TBTU (1.50 eq.), DIPEA (3.00 eq.); **k)** 20% HFIP in DCM (v/v); **f)** CPCR4 (1.00 eq.), HOAt (1.50 eq.), HATU (1.50 eq.), DIPEA (3.00 eq.); **g)** DOTA-GA anhydride (1.10 eq.), DIPEA (3.00 eq.); **h)** TFA.*

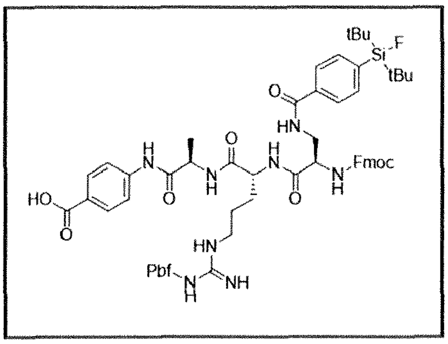

HO-Abz-a-r-(Pbf)-dap(SiFA-BA)-Fmoc: RP-HPLC (50 - 95% B in 15 min): $t_R$ = 17.70 min. Calculated monoisotopic mass ($C_{62}H_{77}FN_8O_{11}SSi$): 1188.52, found: 1189.3 $[M+H]^+$.

[$^{nat}$Ga]CXCR4-SiFA-1: RP-HPLC (10 - 90% B in 15 min): $t_R$ = 8.98 min. Calculated monoisotopic mass ($C_{89}H_{124}FGaN_{21}O_{20}Si$): 1922.83, found: 1924.0 $[M+H]^+$, 962.9 $[M+2H]^{2+}$.

[$^{nat}$Lu]CXCR4-SiFA-1: RP-HPLC (10 - 90% B in 15 min): $t_R$ = 9.77 min. Calculated monoisotopic mass ($C_{89}H_{124}FLuN_{21}O_{20}Si$): 2028.85, found: 1015.1 $[M+2H]^{2+}$.

**CXCR4-SiFA-2**

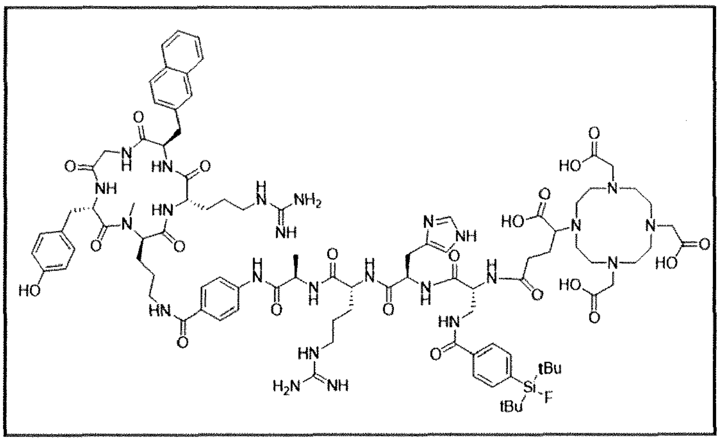

CXCR4-SiFA-2 was synthesized in analogy to CXCR4-SiFA-1. The Abz-based linker unit was elongated with Fmoc-h(Trt)-OH prior to Fmoc-dap(Dde)-OH. Adjacent Dde deprotection, SiFa-BA coupling and resin cleavage yielded the SiFa-bearing linker. Coupling with CPCR4, Fmoc deprotection, DOTA-GA connection and global deprotection was carried out alike.

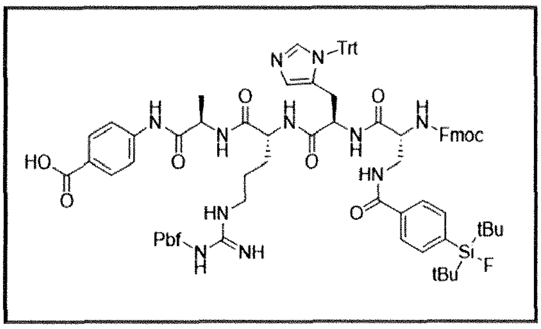

HO-Abz-a-r-(Pbf)-h(Trt)-dap(SiFA-BA)-Fmoc: RP-HPLC (70 - 95% B in 15 min): $t_R$ = 19.04 min. Calculated monoisotopic mass ($C_{87}H_{98}FN_{11}O_{12}SSi$): 1567.69, found: 1568.9 $[M+H]^+$.

CXCR4-SiFA-2: RP-HPLC (10 - 90% B in 15 min): $t_R$ = 8.43 min. Calculated monoisotopic mass ($C_{95}H_{133}FN_{24}O_{21}Si$): 1992.98, found: 997.2 $[M+2H]^{2+}$, 665.7 $[M+3H]^{3+}$.

[$^{nat}$Ga]CXCR4-SiFA-2: RP-HPLC (10 - 90% B in 15 min): $t_R$ = 5.40 min. Calculated monoisotopic mass ($C_{95}H_{131}FGaN_{24}O_{21}Si$): 2059.89, found: 1031.2 $[M+2H]^{2+}$, 688.8 $[M+3H]^{3+}$.

[$^{nat}$Lu]CXCR4-SiFA-2: RP-HPLC (10 - 90% B in 15 min): $t_R$ = 5.89 min. Calculated monoisotopic mass ($C_{95}H_{130}FLuN_{24}O_{21}Si$): 2164.90, found: 1084.8 $[M+2H]^{2+}$, 723.1 $[M+3H]^{3+}$ .

## CXCR4-SiFA-3

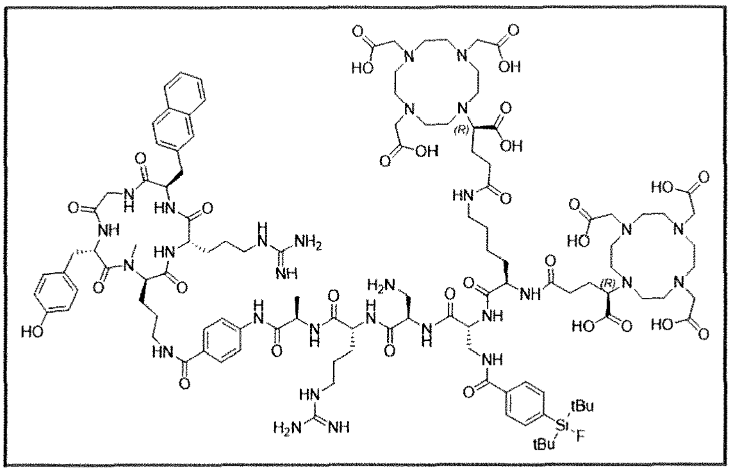

Synthesis of CXCR4-SiFA-3 was achieved by condensation of the SiFA-bearing fragment HO-dap(SiFA-BA)-k((*R*)-DOTA-GA(*t*Bu)$_4$)-(R)-DOTA-GA(*t*Bu)$_4$ and the CPCR4-linker construct CPCR4-Abz-a-r(Pbf)-dap(Boc)-$NH_2$ (see 2.3.7, **GP8**). The resulting peptide was deprotected for 12 h (**GP5**) and purified via semi-preparative RP-HPLC.

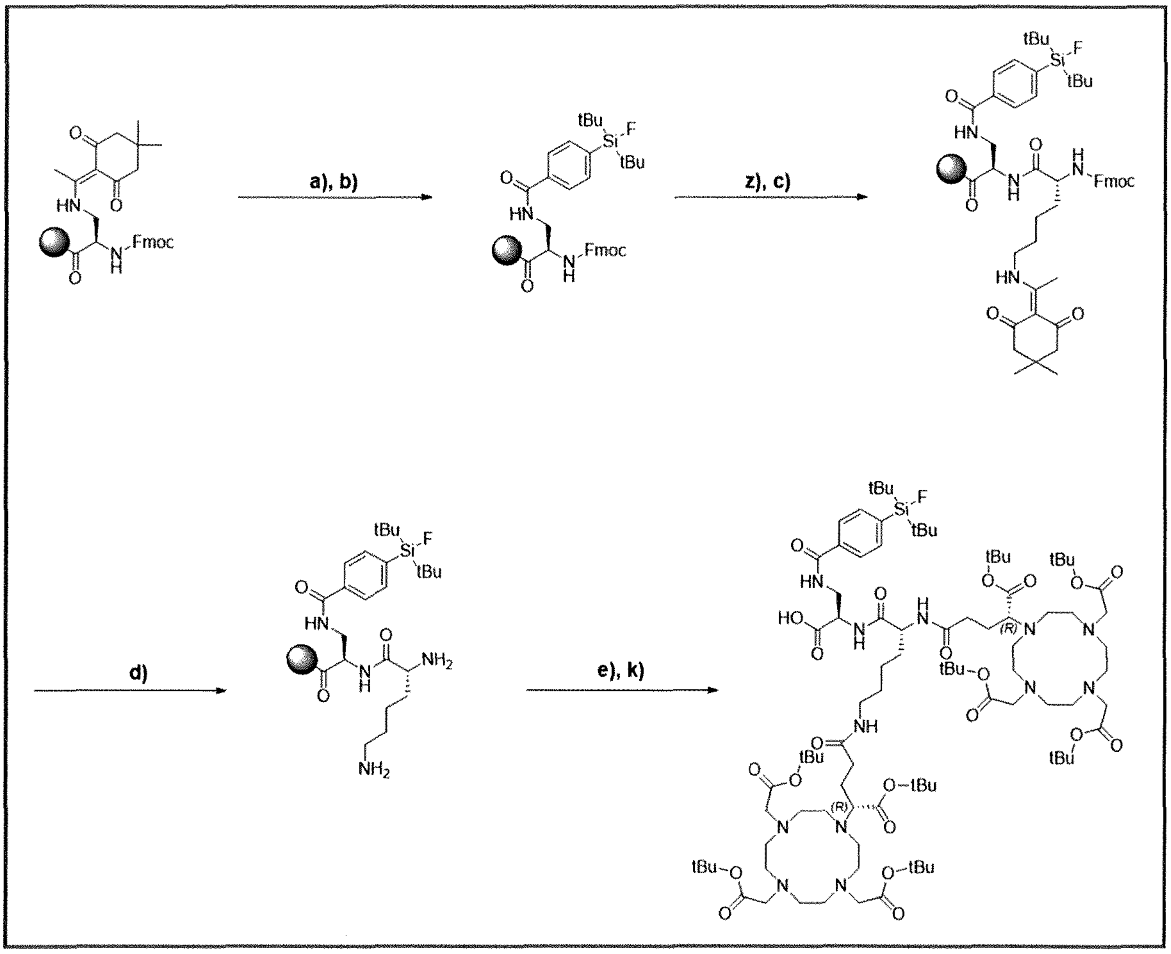


*Scheme 14: Synthesis of the SiFA-bearing fragment HO-dap(SiFA-BA)-k((R)-DOTA-GA(tBu)$_4$)-(R)-DOTA-GA(tBu)$_4$: **a)** imidazole (0.43 g), [$NH_2OH$]Cl (0.63 g); **b)** SiFA-BA-OH (1.50 eq.), HOAt (1.50 eq.), TBTU (1.50 eq.), DIPEA (3.00 eq.); **z)** 20% Piperidine in DMF (v/v); **c)** Fmoc-k(Dde)-OH (1.50 eq.), HOAt (1.50 eq.), TBTU (1.50 eq.), DIPEA (3.00 eq.); **d)** 2% hydrazine in DMF (v/v); **e)** (R)-DOTA-GA(tBu)$_4$-OH (3.00 eq.), HOAt (3.00 eq.), HATU (3.00 eq.), DIPEA (6.00 eq.); **k)** 20% HFIP in DCM (v/v).*

HO-dap(SiFA-BA)-k((R)-DOTA-GA(*t*Bu)$_4$)-(R)-DOTA-GA(*t*Bu)$_4$: RP-HPLC (25 - 95% B in 15 min): $t_R$ = 12.32 min. Calculated monoisotopic mass ($C_{94}H_{165}FN_{12}O_{22}Si$): 1861.19, found: 931.2[M+2H]$^{2+}$ .

CXCR4-SiFA-3: RP-HPLC (20 - 60% B in 15 min): $t_R$ = 8.77 min. Calculated monoisotopic mass ($C_{117}H_{174}FN_{29}O_{31}Si$): 2528.27, found: 844.5 [M+3H]$^{3+}$. 633.4 [M+4H]$^{4+}$.

[$^{nat}$Ga]CXCR4-SiFA-3: RP-HPLC (20 - 60% B in 15 min): $t_R$ = 8.82 min. Calculated monoisotopic mass ($C_{117}H_{170}FGa_2N_{29}O_{31}Si$): 2662.09, found: 1333.1 [M+2H]$^{2+}$, 889.5 [M+3H]$^{3+}$ .

[$^{nat}$Lu]CXCR4-SiFA-3: RP-HPLC (20 - 60% B in 15 min): $t_R$ = 9.71 min. Calculated monoisotopic mass ($C_{117}H_{168}FLuN_{29}O_{31}Si$): 2872.10, found: 1438.5 [M+2H]$^{2+}$, 958.7 [M+3H]$^{3+}$.

**CXCR4-SiFA-4**

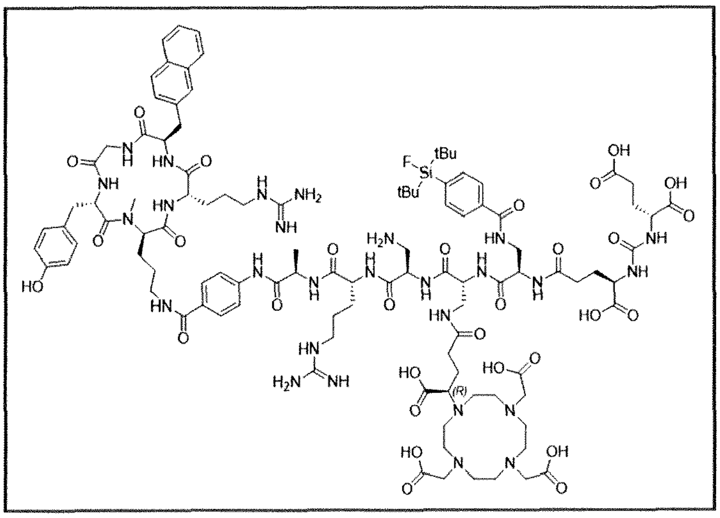

CXCR4-SiFA-4 was synthesized according to GP8, linking the SiFA-bearing moiety (*t*Bu)e(HO-dap((*R*)-DOTAGA(*t*Bu)$_4$)-dap(SiFA-BA))ue(*t*Bu)$_2$ with CPCR4-Abz-a-r(Pbf)-dap(Boc)-NH$_2$ (see 2.3.7). Adjacent deprotection (GP5) for 12 h and semi-preparative RP-HPLC yielded the desired product.

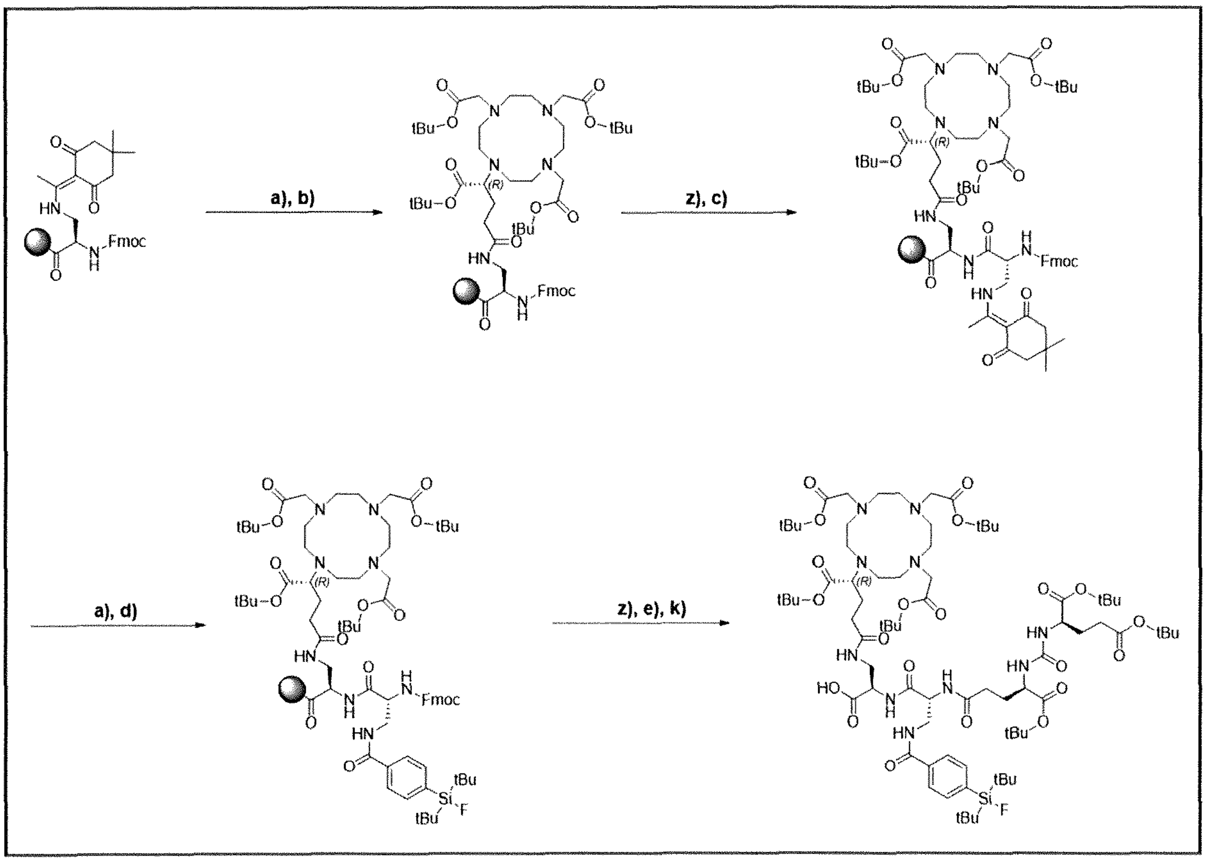


*Scheme 15: Synthesis of the SiFA-bearing fragment (tBu)e(HO-dap((R)-DOTAGA(tBu)$_4$)-dap(SiFA-BA))ue(tBu)$_2$:* ***a)*** *imidazole (0.43 g), [NH$_2$OH]Cl (0.63 g);* ***b)*** *(R)-DOTA-GA(tBu)$_4$-*

*OH (1.50 eq.), HOAt (1.50 eq.), TBTU (1.50 eq.), DIPEA (3.00 eq.);* ***z)*** *20% Piperidine in DMF (v/v);* ***c)*** *Fmoc-dap(Dde)-OH (1.50 eq.), HOAt (1.50 eq.), TBTU (1.50 eq.), 2,4,6-Collidine (3.00 eq.);* ***d)*** *SiFA-BA-OH (1.50 eq.), HOAt (1.50 eq.), TBTU (1.50 eq.), DIPEA (3.00 eq.);* ***e)*** *(tBu)e(OH)ue(tBu)$_2$, HOAt (1.50 eq.), TBTU (1.50 eq.), DIPEA (3.00 eq.);* ***k)*** *20% HFIP in DCM (v/v).*

(*t*Bu)e(HO-dap((*R*)-DOTAGA(*t*Bu)$_4$)-dap(SiFA-BA))ue(*t*Bu)$_2$: RP-HPLC (10 - 95% B in 15 min): $t_R$ = 15.97 min. Calculated monoisotopic mass ($C_{79}H_{135}FN_{10}O_{21}Si$): 1606.96, found: 1609.1[M+H]$^+$, 805.4[M+2H]$^{2+}$.

CXCR4-SiFA-4: RP-HPLC (10 - 60% B in 15 min): $t_R$ = 11.70 min. Calculated monoisotopic mass ($C_{106}H_{152}FN_{27}O_{30}Si$): 2330.10, found: 1167.2 [M+2H]$^{2+}$, 778.6 [M+3H]$^{3+}$. 584.3 [M+4H]$^{4+}$ .

[$^{nat}$Ga]CXCR4-SiFA-4: RP-HPLC (10 - 60% B in 15 min): $t_R$ = 12.32 min. Calculated monoisotopic mass ($C_{106}H_{150}FGaN_{27}O_{30}Si$): 2397.01, found: 1200.7 [M+2H]$^{2+}$, 801.0 [M+3H]$^{3+}$ .

[$^{nat}$Lu]CXCR4-SiFA-4: RP-HPLC (20 - 60% B in 15 min): $t_R$ = 11.72 min. Calculated monoisotopic mass ($C_{106}H_{149}FLuN_{27}O_{30}Si$): 2502.01, found: 835.7 [M+3H]$^{3+}$.

## CXCR4-SiFA-5

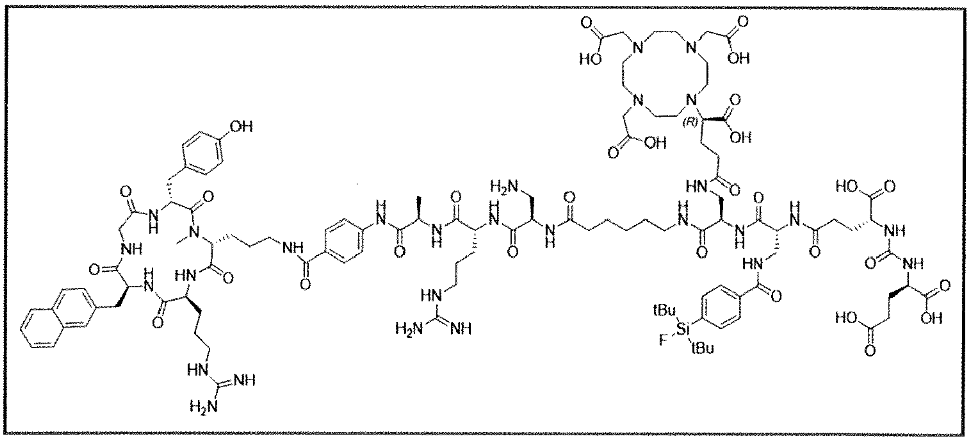

The synthesis of CXCR4-SiFA-5 was conducted in analogy to CXCR4-SiFA-4 with the difference being the SiFA-bearing moiety (*t*Bu)e(HO-Ahx-dap((*R*)-DOTAGA(*t*Bu)$_4$)-dap(SiFA-BA))ue(*t*Bu)$_2$ (see 0) that was coupled with CPCR4-Abz-a-r(Pbf)-dap(Boc)-NH$_2$ (see 2.3.7). Subsequent deprotection (**GP5**) for 12 h and semi-preparative RP-HPLC yielded the desired product.

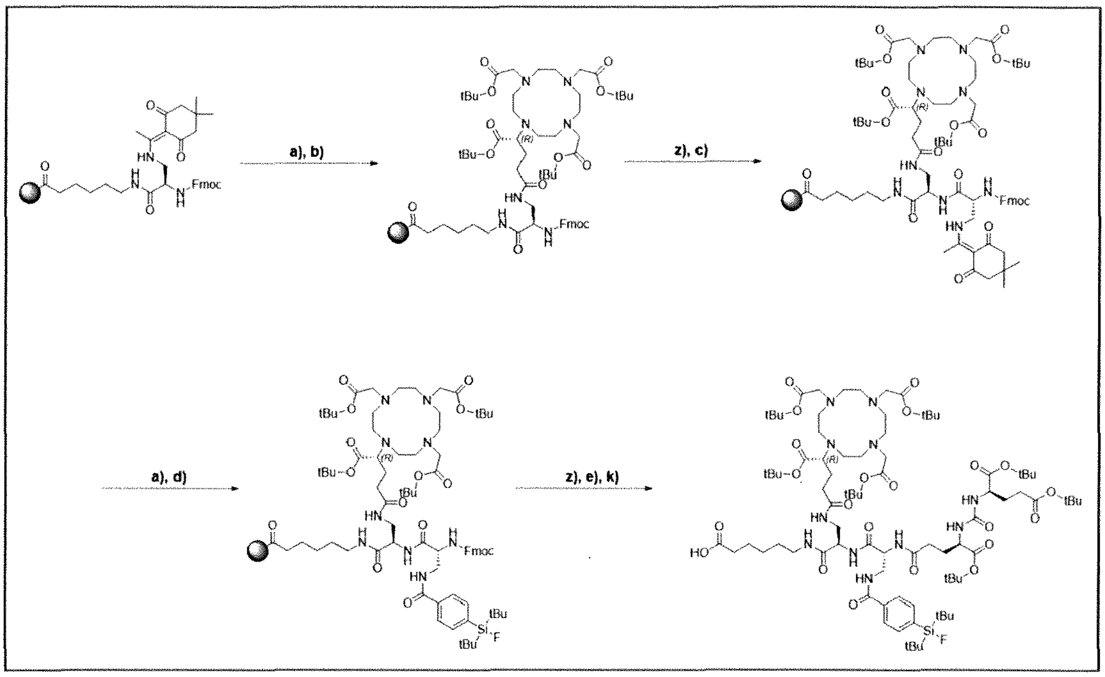


*Scheme 16: Synthesis of the SiFA-bearing fragment (tBu)e(HO-Ahx-dap((R)-DOTAGA(tBu)$_4$)-dap(SiFA-BA))ue(tBu)$_2$:* ***a)*** *imidazole (0.43 g), [$NH_2OH$]Cl (0.63 g);* ***b)*** *(R)-DOTA-GA(tBu)$_4$-OH (1.50 eq.), HOAt (1.50 eq.), TBTU (1.50 eq.), DIPEA (3.00 eq.);* ***z)*** *20% Piperidine in DMF (v/v);* ***c)*** *Fmoc-dap(Dde)-OH (1.50 eq.), HOAt (1.50 eq.), TBTU (1.50 eq.), 2,4,6-Collidine (3.00 eq.);* ***d)*** *SiFA-BA-OH (1.50 eq.), HOAt (1.50 eq.), TBTU (1.50 eq.), DIPEA (3.00 eq.);* ***e)*** *(tBu)e(OH)ue(tBu)$_2$, HOAt (1.50 eq.), TBTU (1.50 eq.), DIPEA (3.00 eq.);* ***k)*** *20% HFIP in DCM (v/v).*

(*t*Bu)e(HO-Ahx-dap((*R*)-DOTAGA(tBu)$_4$)-dap(SiFA-BA))ue(*t*Bu)$_2$: RP-HPLC (10 - 95% B in 15 min): $t_R$ = 15.62 min. Calculated monoisotopic mass ($C_{85}H_{146}FN_{11}O_{22}Si$): 1720.04, found: 1721.3 [M+H]$^+$, 861,6[M+2H]$^{2+}$.

CXCR4-SiFA-5: RP-HPLC (10 - 60% B in 15 min): $t_R$ = 11.70 min. Calculated monoisotopic mass ($C_{112}H_{163}FN_{28}O_{31}Si$): 2443.18, found: 1221.9 [M+2H]$^{2+}$, 814.8 [M+3H]$^{3+}$. 611.3 [M+4H]$^{4+}$ .

[$^{nat}$Ga]CXCR4-SiFA-5: RP-HPLC (10 - 60% B in 15 min): $t_R$ = 11.82 min. Calculated monoisotopic mass ($C_{112}H_{160}FGaN_{28}O_{31}Si$): 2509.08, found: 1255.3 [M+2H]$^{2+}$, 837.0 [M+3H]$^{3+}$ .

[$^{nat}$Lu]CXCR4-SiFA-5 RP-HPLC (10 - 60% B in 15 min): $t_R$ = 12.72 min. Calculated monoisotopic mass ($C_{112}H_{159}FLuN_{28}O_{31}Si$): 2614.09, found: 1307.8 [M+2H]$^{2+}$, 872.1 [M+3H]$^{3+}$ .

**CXCR4-SiFA-6**

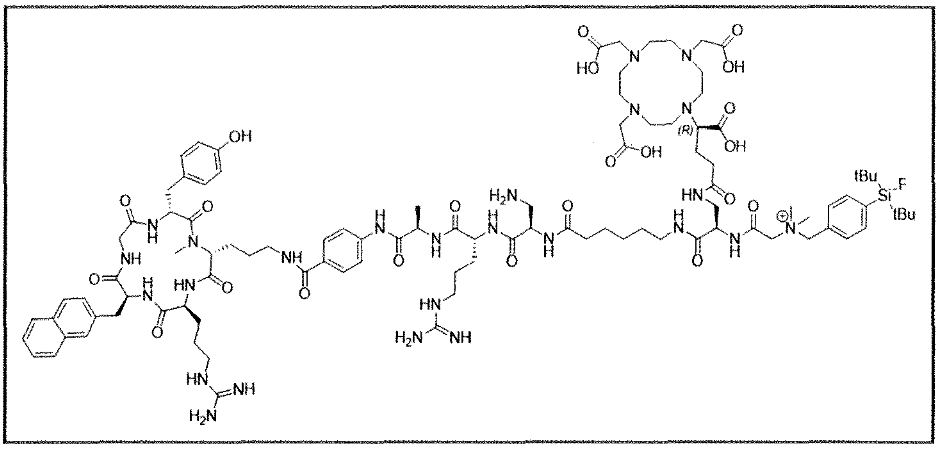

CXCR4-SiFA-6 was synthesized via fragment condensation of CPCR4-Abz-a-r(Pbf)-dap(Boc)-$NH_2$ (see 2.3.7) with the SiFA-bearing HO-Ahx-dap((*R*)-DOTAGA(*t*Bu)$_4$)-SiFA*lin* (see 0) **(GP8).** SiFA-benzylbromide was obtained during the synthesis of the SiFA-BA synthon (see 2.3.4) and coupled to the DMG *N*-terminus in DCM over night (3.00 eq. SiFA-benzylbromide, 3.00 eq. DIPEA). Final deprotection of the peptide **(GP5)** and semi-preparative RP-HPLC purification afforded the desired product.

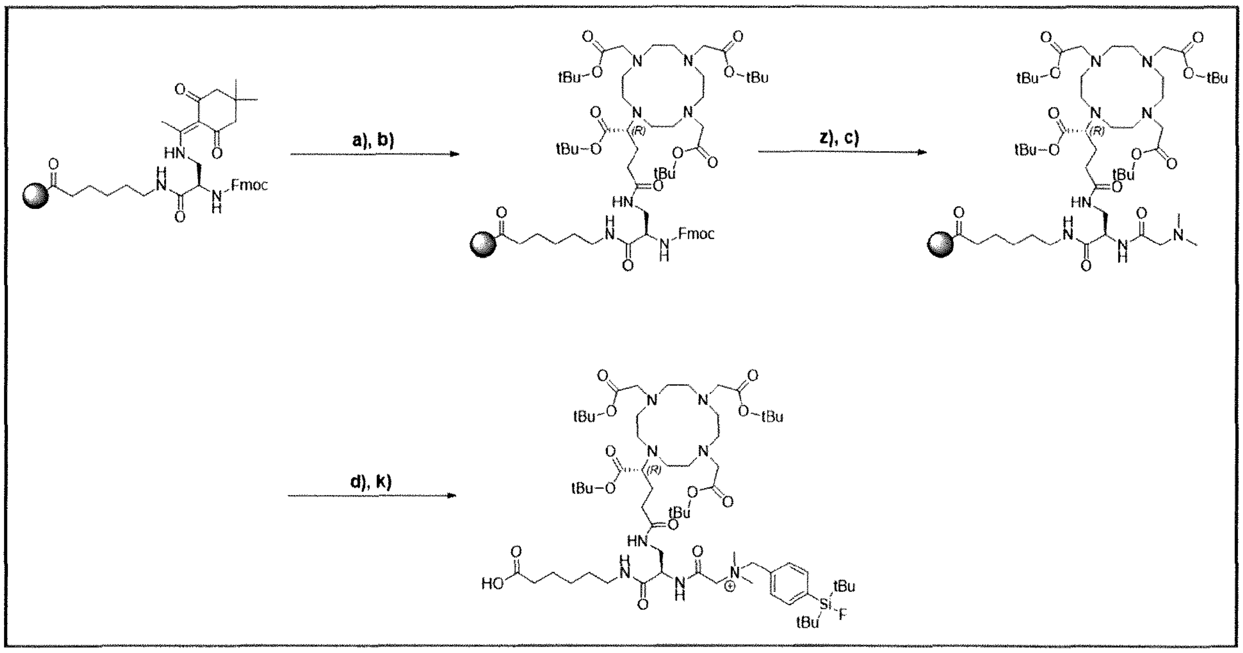


*Scheme 17: Synthesis of the SiFA-bearing fragment HO-Ahx-dap((R)-DOTAGA(tBu)$_4$)-SiFAlin: **a)** imidazole (0.43 g), [$NH_2OH$]Cl (0.63 g); **b)** (R)-DOTA-GA(tBu)$_4$-OH (1.50 eq.), HOAt (1.50 eq.), TBTU (1.50 eq.), DIPEA (3.00 eq.); **z)** 20% Piperidine in DMF (v/v); **c)** DMG-OH (1.50 eq.), HOAt (1.50 eq.), TBTU (1.50 eq.), 2,4,6-Collidine (3.00 eq.); **d)** SiFA-benzylbromide (3.00 eq.), DIPEA (3.00 eq.); **k)** 20% HFIP in DCM (v/v).*

HO-Ahx-dap((*R*)-DOTAGA(*t*Bu)$_4$)-SiFA*lin*: RP-HPLC (10 - 95% B in 15 min): $t_R$ = 11.64 min. Calculated monoisotopic mass ($C_{63}H_{112}FN_8O_{13}Si^+$): 1235.81, found: 1235.9 $[M+H]^+$.

CXCR4-SiFA-6: RP-HPLC (10 - 60% B in 15 min): $t_R$ = 11.70 min. Calculated monoisotopic mass ($C_{102}H_{153}FN_{25}O_{22}Si^+$): 2127.14, found: 1063.4 $[M+2H]^{2+}$, 709.2 $[M+3H]^{3+}$.

[$^{nat}$Ga]CXCR4-SiFA-6: RP-HPLC (10 - 60% B in 15 min): $t_R$ = 11.73 min. Calculated monoisotopic mass ($C_{102}H_{150}FGaN_{25}O_{22}Si^+$): 2193.04, found: 1096.7 $[M+2H]^{2+}$, 731.3 $[M+3H]^{3+}$, 548.7 $[M+4H]^{4+}$.

[$^{nat}$Lu]CXCR4-SiFA-6: RP-HPLC (10 - 60% B in 15 min): $t_R$ = 12.71 min. Calculated monoisotopic mass

($C_{102}H_{149}FLuN_{25}O_{22}Si^{+}$): 2298.05, found: 1149.3 $[M+2H]^{2+}$, 766.4 $[M+3H]^{3+}$.

**CXCR4-SiFA-7**

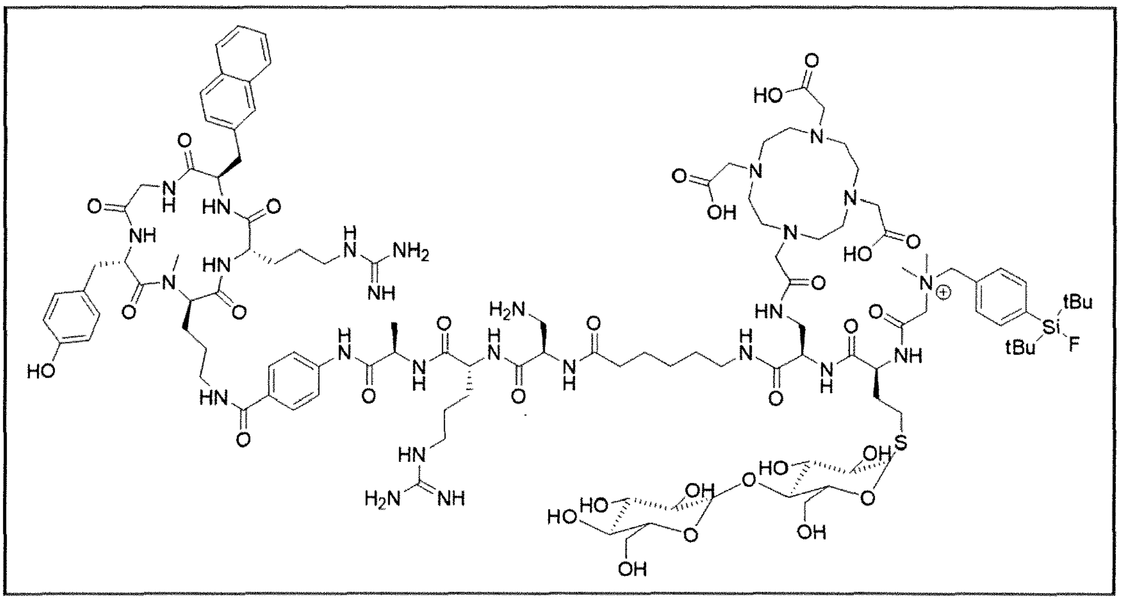

CXCR4-SiFA-7 was synthesized in analogy to CXCR4-SiFA-6, employing the SiFA-bearing HO-Ahx-dap(DOTA(*t*Bu)$_3$)-D-HCy(Lactosyl)-SiFA*lin* (see 0) **(GP8).** Final deprotection of the peptide **(GP5)** and semi-preparative RP-HPLC purification afforded the desired product.

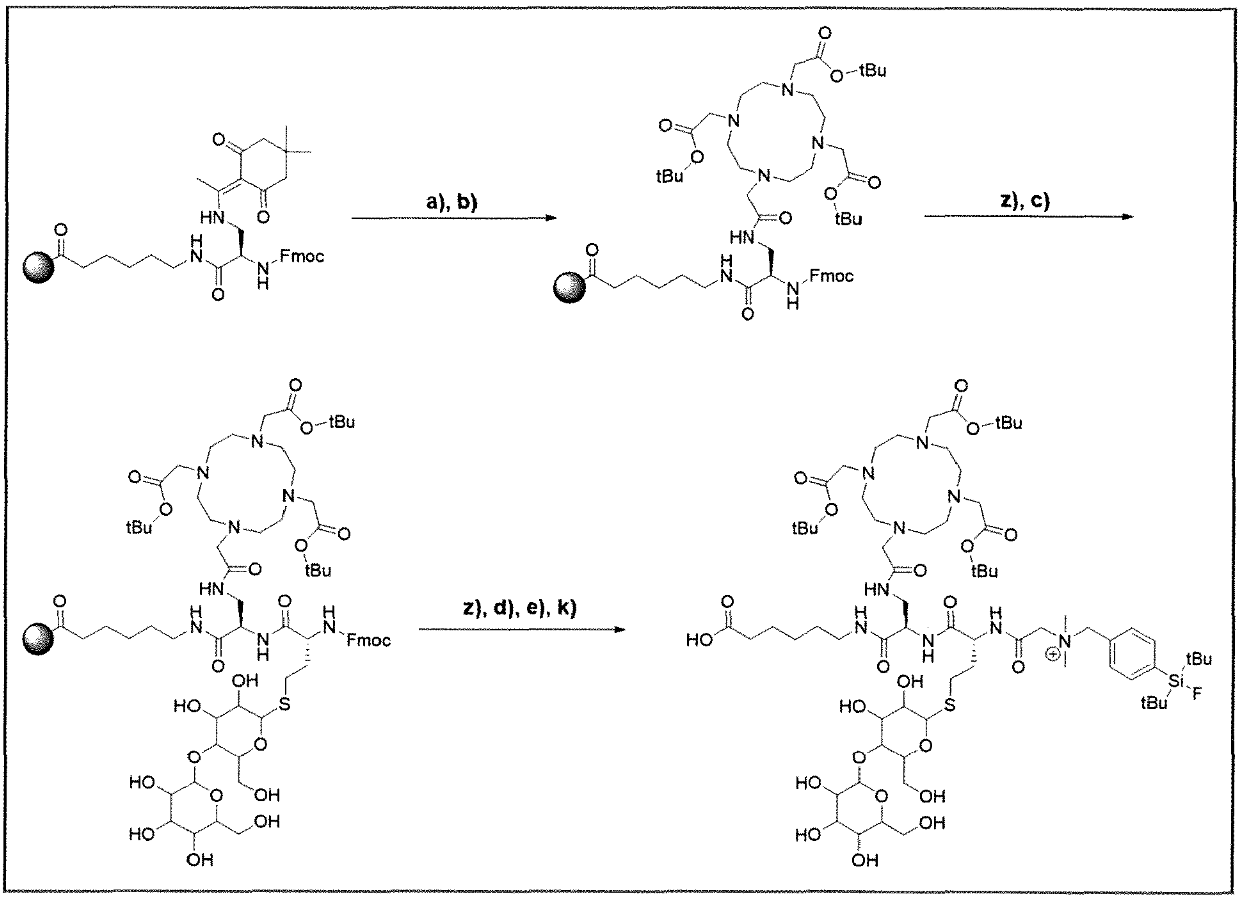


*Scheme 18: Synthesis of the SiFA-bearing fragment HO-Ahx-dap(DOTA(tBu)$_3$)-D-HCy(Lactosyl)-SiFAlin:* ***a)*** *imidazole (0.43 g), [$NH_2OH$]Cl (0.63 g);* ***b)*** *DOTA(tBu)$_3$-OH (1.50 eq.), HOAt (1.50 eq.), TBTU (1.50 eq.), DIPEA (3.00 eq.);* ***z)*** *20% Piperidine in DMF (v/v);* ***c)*** *Fmoc-D-HCy(Lactosyl)-OH (1.50 eq.), HOAt (1.50 eq.), HATU (1.50 eq.), DIPEA (3.00 eq.); d) DMG-OH (1.50 eq.), HOAt (1.50 eq.), TBTU (1.50 eq.), 2,4,6-Collidine (3.00 eq.);* ***e)*** *SiFA-benzylbromide (3.00 eq.), DIPEA (3.00 eq.);* ***k)*** *20% HFIP in DCM (v/v).*

HO-Ahx-dap(DOTA(*t*Bu)$_3$)-D-HCy(Lactosyl)-SiFa*lin*: RP-HPLC (10 - 95% B in 15 min): $t_R$ = 9.55 min. Calculated monoisotopic mass ($C_{72}H_{127}FN_9O_{22}SSi^+$): 1548.86, found: 1550.3 $[M+H]^+$, 775.3 $[M+2H]^{2+}$.

CXCR4-SiFA-7: RP-HPLC (10 - 95% B in 15 min): $t_R$ = 7.61 min. Calculated monoisotopic mass ($C_{102}H_{153}FN_{25}O_{22}Si^+$): 2496.25, found: 838.8 $[M+H_2O+3H]^{3+}$.

[$^{nat}$Ga]CXCR4-SiFA-7: RP-HPLC (10 - 60% B in 15 min): $t_R$ = 11.23 min. Calculated monoisotopic mass ($C_{115}H_{174}FGaN_{26}O_{31}SSi^+$): 2563.16, found: 861.5 $[M+H_2O+3H]^{3+}$.

[$^{nat}$Lu]CXCR4-SiFA-7: RP-HPLC (10 - 60% B in 15 min): $t_R$ = 11.47 min. Calculated monoisotopic mass ($C_{115}H_{173}FLuN_{26}O_{31}SSi^+$): 2668.16, found: 896.3 $[M+H_2O+3H]^{3+}$.

**3.4 CYTOTOXINE-LINKED PEPTIDES**

**CXCR4-MMAE-1**

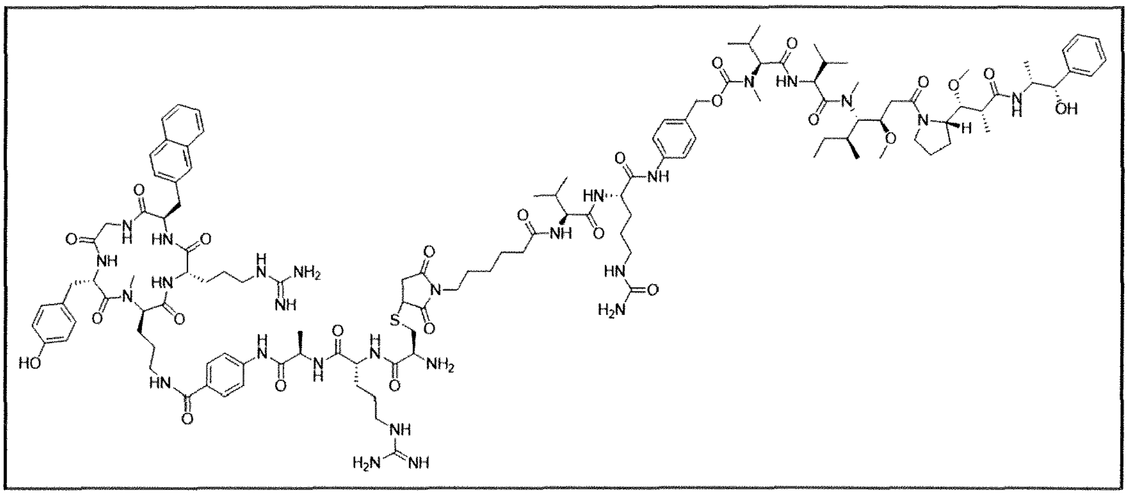

The synthesis of the cysteine-bearing binding scaffold HO-Abz-a-r(Pbf)-c(Trt)-Fmoc (see 2.3.6) was achieved as depicted. Condensation of the CPCR4 scaffold (see 2.3.1) with the linker moiety with adjacent Fmoc deprotection and deprotection of acid-labile protecting groups was attained using **GP5, GP8.** Sulfhydryl-maleimido coupling was conducted as described above (see **GP10**) and the resulting peptide purified via semi-preparative RP-HPLC.

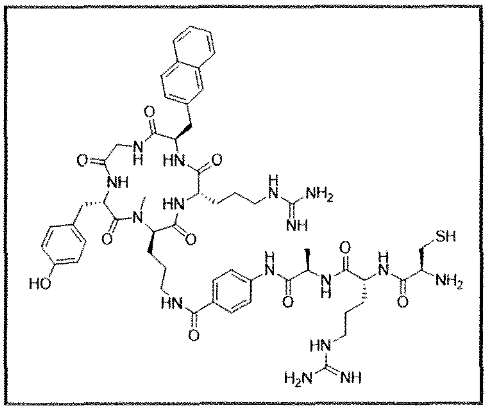

CPCR4-Abz-a-r-c-$NH_2$: RP-HPLC: (10 -90% B in 15 min): $t_R$ = 9.80 min. Calculated monoisotopic mass ($C_{55}H_{74}N_{16}O_{10}S$): 1150.55, found: 1151.3 $[M+H]^+$, 576.4 $[M+2H]^{2+}$.

CXCR4-MMAE-1: RP-HPLC: (25 -70% B in 15 min): $t_R$ = 9.31 min. Calculated monoisotopic mass ($C_{123}H_{179}N_{27}O_{25}S$): 2466.33, found: 1234.0 $[M+2H]^{2+}$, 823.2 $[M+3H]^{3+}$.

**CXCR4-MMAE-2**

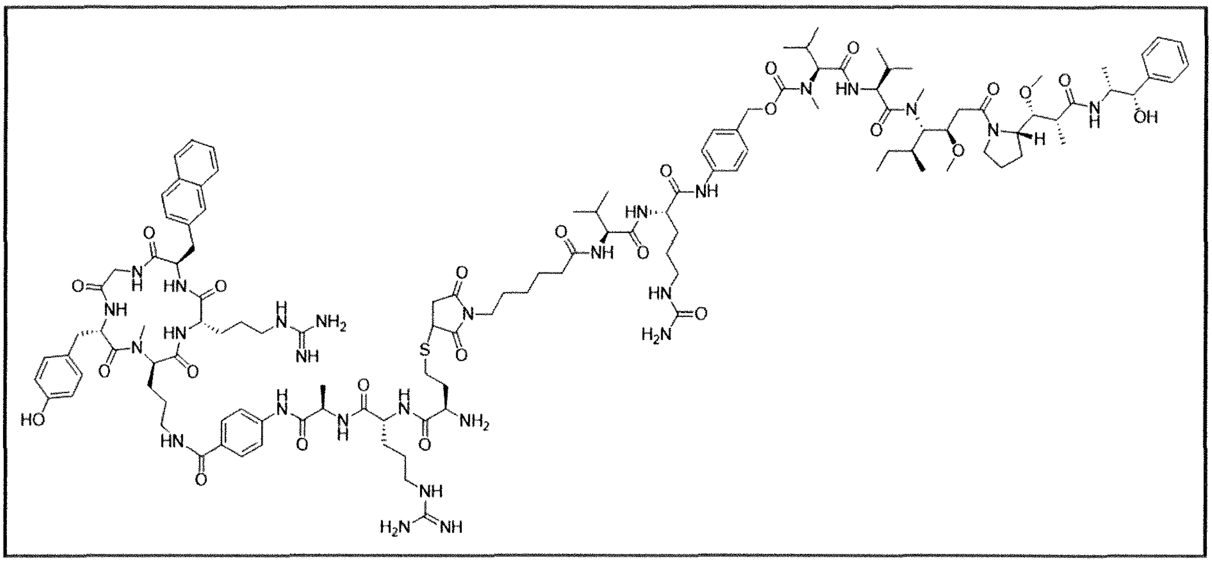

CXCR4-MMAE-2 was synthesized in analogy to CXCR4-MMAE-1 with the difference being *N*-terminal D-Hcy(Trt)-$NH_2$ instead of c(Trt)-$NH_2$ (see 2.3.6). The deprotected intermediate product CPCR4-Abz-a-r-Hcy(SH)-$NH_2$ was coupled to VcMMAE alike (see **GP10)** and purified by semi-preparative RP-HPLC.

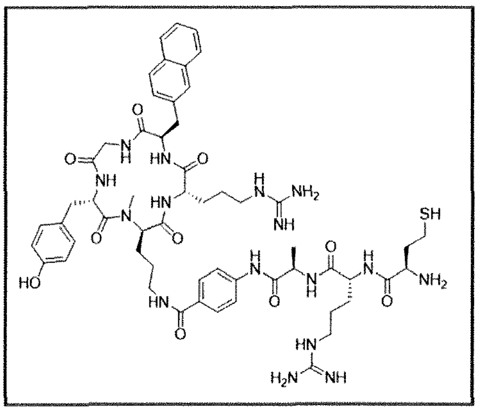

CXCR4-MMAE-2: RP-HPLC: (25 -70% B in 15 min): $t_R$ = 9.39 min. Calculated monoisotopic mass ($C_{124}H_{181}N_{27}O_{25}S$): 2480.34, found: 1241.0 $[M+2H]^{2+}$, 827.8 $[M+3H]^{3+}$.

$^{125}I$-CXCR4-MMAE-2: HPLC (0% B for 2 min, 0 - 35% B in 1 min, 35 - 50% B in 15 min): $t_R$ = 14.19 min.

**CXCR4-MMAE-3**

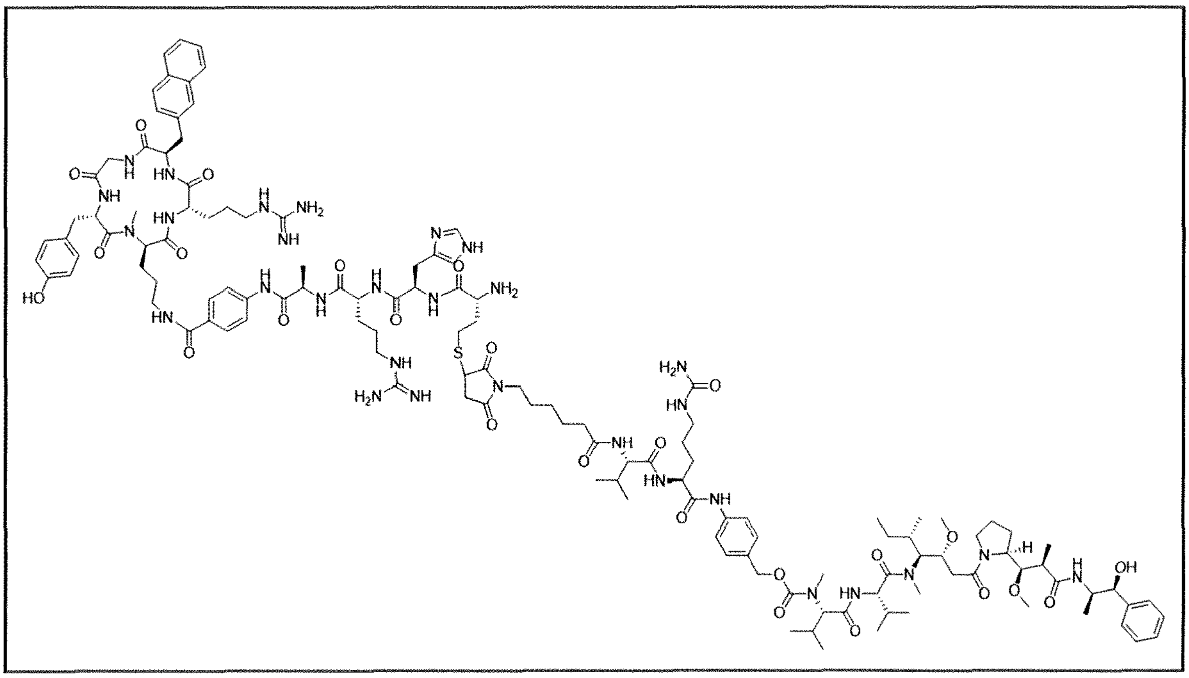

CXCR4-MMAE-3 was synthesized in analogy to CXCR4-MMAE-1 and -2. The linker HO-Abz-a-r(Pbf)-h(Trt)-Fmoc (see 2.3.6) was coupled to CPCR4 **(GP8),** Fmoc deprotected and the peptide coupled with Fmoc-D-Hcy(Trt)-OH. After complete deprotection, sulfhydryl-maleimido coupling was achieved as described (see **GP10**) and the peptide purified via semi-preparative RP-HPLC.

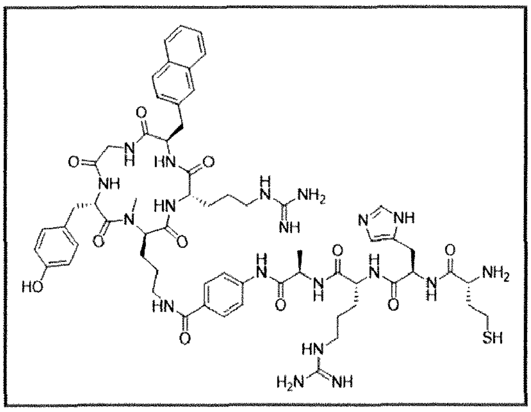

CXCR4-MMAE-3: RP-HPLC (30 - 70% B in 15 min): $t_R$ = 5.93 min. Calculated monoisotopic mass ($C_{130}H_{188}N_{30}O_{26}S$): 2617.40, found: 1310.1 $[M+2H]^{2+}$, 873.8 $[M+3H]^{3+}$.

$^{125}$I-CXCR4-MMAE-3: HPLC (0% B for 2 min, 0 - 35% B in 1 min, 35 - 50% B in 15 min): $t_R$ = 15.18 min.

**CXCR4-MMAE-4**

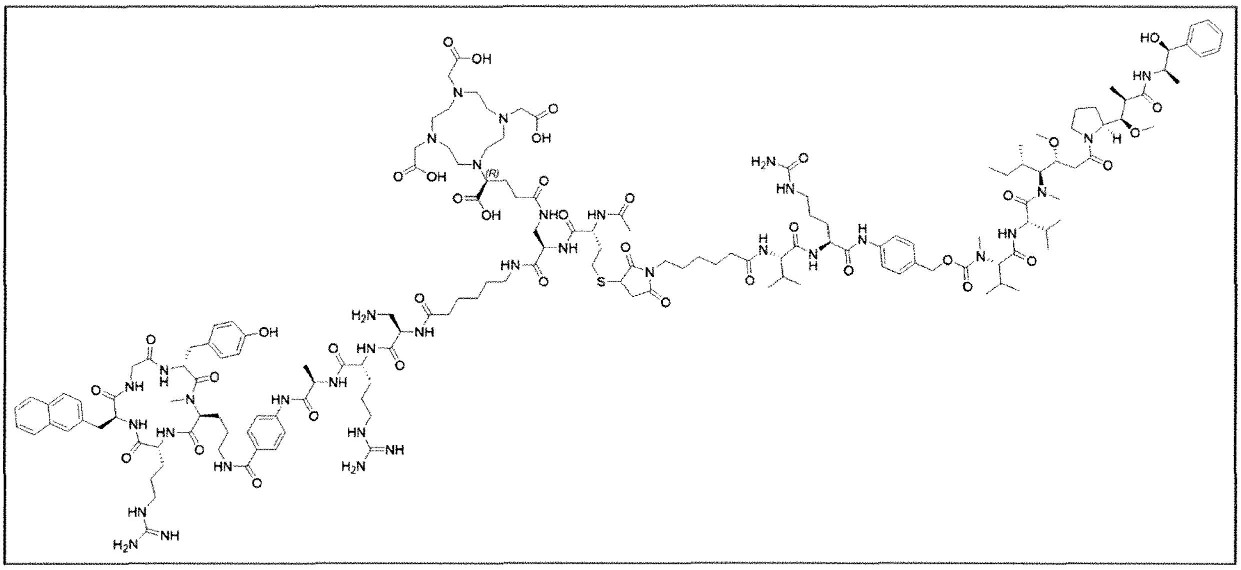

CXCR4-MMAE-4 was synthesized via fragment condensation of the CPCR4-linker construct CPCR4-Abz-a-r(Pbf)-dap(Boc)-$NH_2$ (see 2.3.7) and the Ahx-based chelator-bearing moiety HO-Ahx-dap(R-DOTAGA($t$Bu)$_4$)-D-HCy(Trt)-Ac (see 0). The peptide was globally deprotected and the toxine coupled according to <u>**GP10**</u> and the peptide purified via semi-preparative RP-HPLC.

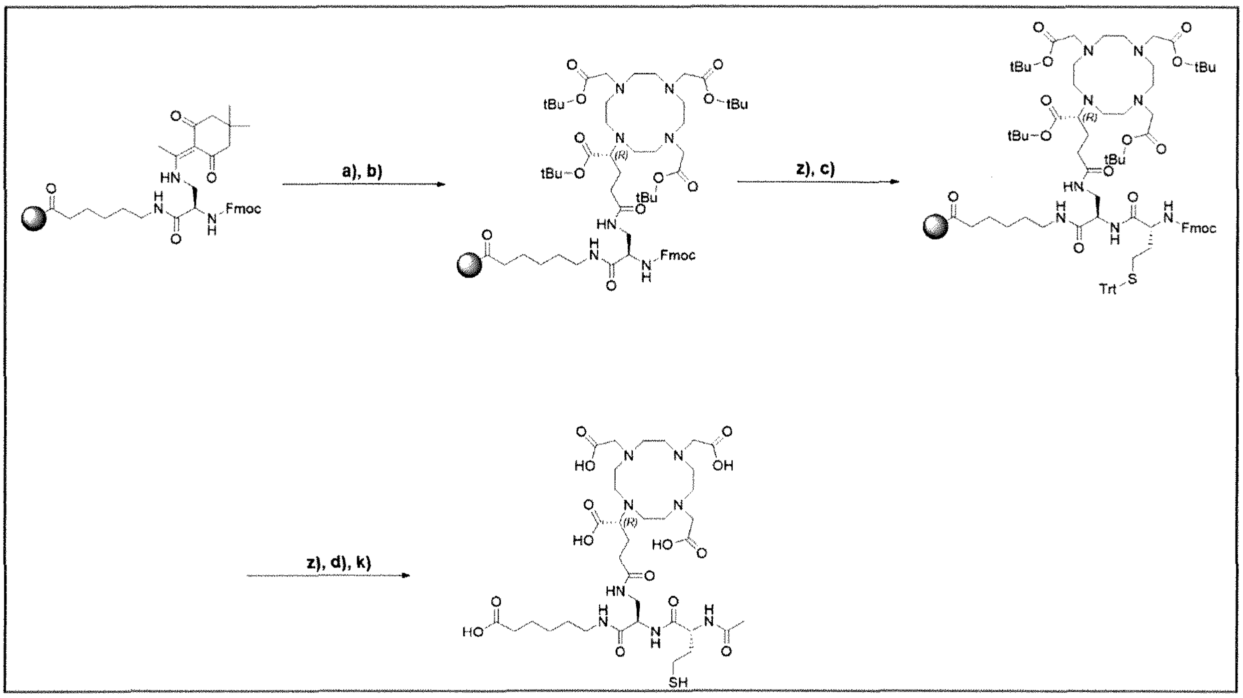


*Scheme 19: Synthesis of the (R)-DOTA-GA(tBu)$_4$-bearing fragment HO-Ahx-dap(R-DOTA-GA(tBu)$_4$)-D-HCy(H)-Ac: **a)** imidazole (0.43 g), [$NH_2OH$]Cl (0.63 g); **b) R-**DOTA-GA(tBu)$_4$-OH (1.50 eq.), HOAt (1.50 eq.), TBTU (1.50 eq.), DIPEA (3.00 eq.); **z)** 20% Piperidine in DMF (v/v); **c)** Fmoc-D-HCy(Trt)-OH (1.50 eq.), HOAt (1.50 eq.), HATU (1.50 eq.), DIPEA (3.00 eq.); **d)** DIPEA (5.0 eq.), $Ac_2O$ (5.00 eq.) **k)** TFA/TIPS/$H_2O$ (95/2.5/2.5; v/v/v).*

HO-Ahx-dap((R)-DOTA-GA($t$Bu)$_4$)-D-HCy(Trt)-Ac: RP-HPLC (10 - 95% B in 15 min): $t_R$ = 12.64 min. Calculated

monoisotopic mass ($C_{69}H_{104}N_8O_{14}S$): 1300.74, found: 1060.2 [M-Trt+H]$^+$, 651.6 [M+2H]$^{2+}$.

CPCR4-Abz-a-r-dap-Ahx-dap((R)-DOTA-GA)-D-HCy(VcMMAE)-Ac: RP-HPLC (10 - 95% B in 15 min): $t_R$ = 6.22 min. Calculated monoisotopic mass ($C_{89}H_{131}N_{25}O_{23}S$): 1949.96, found: 652.3 [M+3H]$^{3+}$.

CXCR4-MMAE-4: RP-HPLC (20 - 70% B in 15 min): $t_R$ = 9.63 min. Calculated monoisotopic mass ($C_{157}H_{236}N_{36}O_{38}S$): 3265.74, found: 1634.4 [M+2H]$^{2+}$, 1089.8 [M+3H]$^{3+}$, 817.7 [M+4H]$^{4+}$.

[$^{nat}$Lu]CXCR4-MMAE-4: RP-HPLC (20 - 70% B in 15 min): $t_R$ = 10.46 min. Calculated monoisotopic mass ($C_{157}H_{233}N_{36}O_{38}S$): 3437.65, found: 1720.1 [M+2H]$^{2+}$, 1146.9 [M+3H]$^{3+}$,

**3.5 OPTICAL IMAGING COMPOUNDS**

**CXCR4-OI-1**

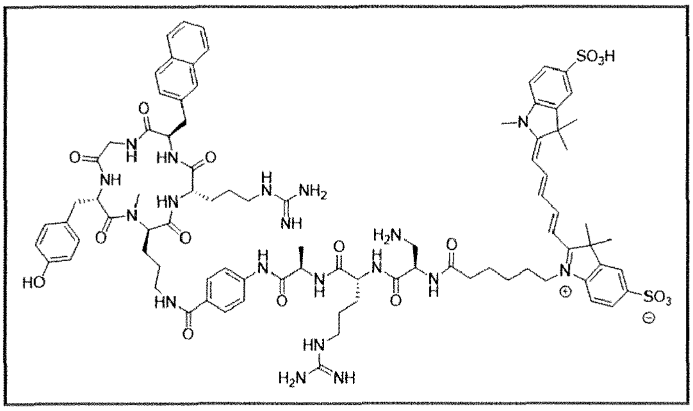

Synthesis of CXCR4-OI-1 was based on fragment condensation of the CPCR4-linker construct CPCR4-Abz-a-r(Pbf)-dap(Boc)-$NH_2$ (see 2.3.7) and the Sulfo-Cy5-carboxylic acid **(GP8).** The resulting peptide was treated with TFA **(GP5)** and purified via semi-preparative RP-HPLC.

CXCR4-OI-1: RP-HPLC (5 - 55% B in 15 min): $t_R$ = 8.73 min. Calculated monoisotopic mass ($C_87H_{111}N_{19}O_{17}S_2$): 1757.78, found: 880.5 [M+2H]$^{2+}$, 587.3 [M+3H]$^{3+}$.

**CXCR4-OI-2**

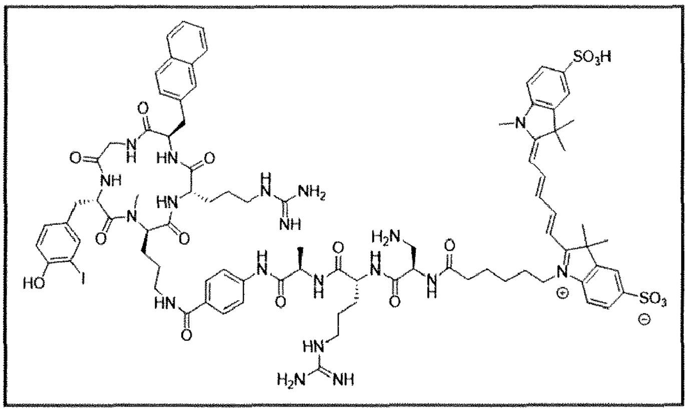

CXCR4-OI-2 was synthesized on the basis of the fully deprotected and purified CXCR4-OI-1 by iodination according to the described method (see **GP7**). The mixture was subjected to semi-preparative RP-HPLC to afford the desired product.

CXCR4-OI-2: RP-HPLC (5 - 55% B in 15 min): *tR* = 11.00 min. Calculated monoisotopic mass

$(C_87H_{110}IN_{19}O_{17}S_2)$: 1883.68, found: 942.7 $[M+2H]^{2+}$, 630.4 $[M+3H]^{3+}$.

**CXCR4-OI-3**

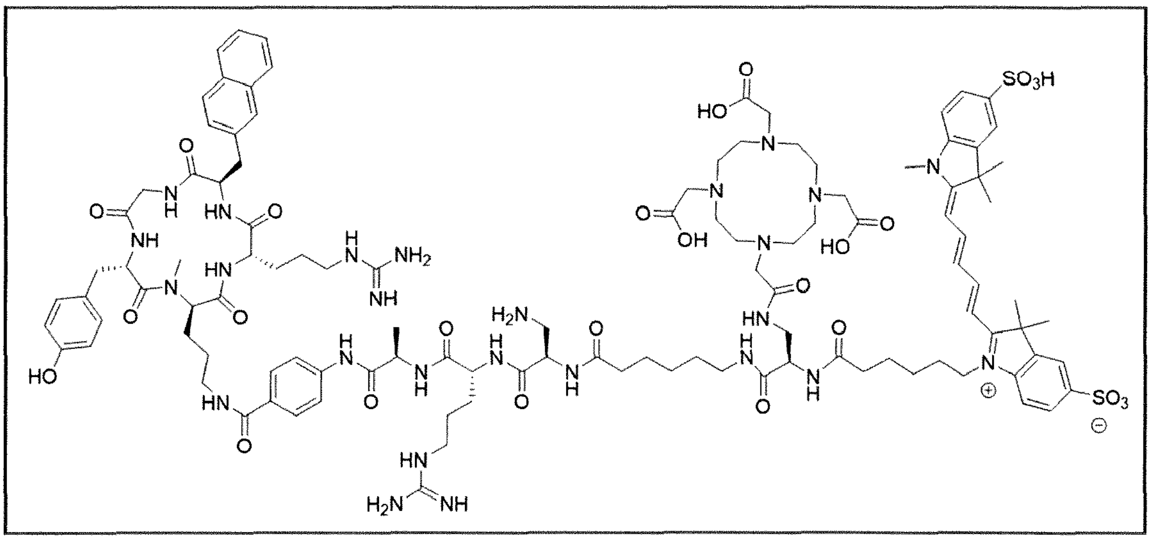

CXCR4-OI-3 was synthesized by fragment condensation of CPCR4-Abz-a-r(Pbf)-dap(Boc)-$NH_2$ and HO-Ahx-dap(DOTA(*t*Bu)$_3$)-Fmoc (see 0). Adjacent Fmoc deprotection and standard coupling of Cy5.5 according to **GP3, GP5** and **GP8** afforded the desired product.

HO-Ahx-dap(DOTA(*t*Bu)$_4$)-Fmoc: RP-HPLC (10 - 95% B in 15 min): $t_R$ = 16.66 min. Calculated monoisotopic mass $(C_{52}H_{79}N_7O_{12}S)$: 993.58, found: 938.7 [M-*t*Bu+H]$^+$.

CPCR4-Abz-a-r(Pbf)-dap(Boc)-Ahx-dap(DOTA(*t*Bu)$_3$)-NH2: RP-HPLC (10 - 95% B in 15 min): $t_R$ = 9.14 min. Calculated monoisotopic mass $(C_{110}H_{166}N_{24}O_{24}S)$: 2239.22, found: 1120.3 $[M+2H]^{2+}$.

CXCR4-OI-3: RP-HPLC (10 - 60% B in 15 min): $t_R$ = 8.86 min. Calculated monoisotopic mass $(C_{112}H_{154}N_{26}O_{26}S_2)$: 2343.10, found: 1173.4 $[M+2H]^{2+}$, 782.7 $[M+3H]^{3+}$.

[$^{nat}$Lu]CXCR4-OI-3: RP-HPLC (10 - 60% B in 15 min): $t_R$ = 8.78 min. Calculated monoisotopic mass $(C_{112}H_{151}N_{26}O_{26}S_2)$: 2515.01, found: 1258.4 $[M+2H]^{2+}$, 839.3 $[M+3H]^{3+}$.

## 4. RADIOLABELING

### 4.1 $^{125}$I-LABELING/$^{125}$I-Fc-131

Approximately 50 - 150 μg of unlabeled precursor were dissolved in 20 μL DMSO and 280 μL TRIS buffer (25 mM TRIS-HCl, 0.40 mM NaCl, pH = 7.5) were added. After addition of 5 μL [$^{125}$I]NaI solution (15 - 20 MBq, see 1.2), the mixture was transferred into a reaction tube, coated with 150 μg Iodogen®. After incubation for 15 min at r.t., the mixture was removed from the oxidant and subjected to RP-HPLC purification.

**$^{121}$I-Fc-131**

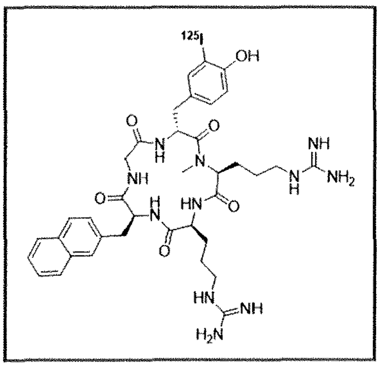

$^{125}$I-Fc-131 was prepared as described above and purified according to the RP-HPLC procedure: RP-HPLC (20 - 55% B in 15 min): $t_R$ = 9.35 min.

### 4.2 $^{99M}$TC-LABELING

Labeling of peptides with $^{99m}TcO_4^-$ was carried out dependent on the chelator used. The peptides and respective admixture, either freshly taken from aqueous solutions or used as pre-formulated and freeze-dried kits, were reacted with freshly eluted $^{99m}$Tc-Pertechnetate (see 1.2).

#### 4.2.1 $MAS_3$-DERIVED CHELATORS

Labeling mixtures, either in solution or with a freeze-dried formulation contained the same ingredients (49):

**Stock 1:** 1.78 g Sodium-Phosphate-dibasic dihydrate were dissolved in 50.0 mL $H_2O$ (= solution 1). 1.38 g Sodium-Phosphate-Monobasic Monohydrate were dissolved in 50.0 mL $H_2O$ (= solution 2). 47.35 mL of solution 1 and 2.65 mL of solution 2 were mixed to yield stock 1.

**Stock 2:** 5.00 mL of stock 1 were diluted with 5.00 mL of $H_2O$ to yield stock 2.

**Stock 3:** The respective peptide was dissolved in DMSO or $H_2O$ or a mixture thereof to a concentration of usually 1.00 mM.

**Stock 4:** 2.50 g Disodiumtartrate dehydrate were dissolved in stock 1 to yield stock 4.

**Stock 5:** 30.0 mg ascorbic acid were mixed with 10.0 mL aqueous HCl (10.0 mM).

**Stock 6:** 4.00 mg Tin(II)chloride dihydrate were dissolved in 1.0 mL stock 5. This solution was freshly prepared for every labeling experiment.

A labeling mixture or kit was prepared by mixing the stock solutions according to the following indication:

Stock 1: 6.76 μL

Stock 2: 10.0 μL

Stock 3: 5.00 μL

Stock 4: 8.00 μL

Stock 6: 2.00 μL

Freshly eluted $^{99m}$Tc-Pertechnetate (0.10 - 5.00 μL, 50.0 - 850 MBq) was added and the mixture heated for 30 min at 95°C. Quality control was performed by radio-TLC and radio-RP-HPLC directly from the reaction mixture.

**radio-RP-HPLC:** $R_t$ ($^{99m}TcO_4^-$) = $t_0$, $R_t$ ($^{99m}$Tc-colloid) = $t_0$, $R_t$ ($^{99m}$Tc-tartrate) = 2 - 3 min, $R_t$ ($^{99m}$Tc-peptide) = > 3min.

**radio-TLC** on Silica-coated 60 RP-18 $F_{254}$s strips employing different mobile phases: $NH_4OAc$/DMF (1/1, v/v): $R_f$ ($^{99m}TcO_4^-$) = 1, $R_f$ ($^{99m}$Tc-colloid) = 0, $R_f$ ($^{99m}$Tc-tartrate) = 0.5 - 0.8 min, $R_f$ ($^{99m}$Tc-peptide) = 0.8 - 1.

2-Butanone: $R_f$ ($^{99m}TcO_4^-$) = 1, $R_f$ ($^{99m}$Tc-colloid) = 0, $R_f$ ($^{99m}$Tc-tartrate) = 0 min, $R_f$ ($^{99m}$Tc-peptide) = 0.

NaCl (25 vol% in $H_2O$): $R_f$ ($^{99m}TcO_4^-$) = 0, $R_f$ ($^{99m}$Tc-colloid) = 1, $R_f$ ($^{99m}$Tc-tartrate) = 0.5 - 0.8 min, $R_f$ ($^{99m}$Tc-peptide) = 0.

### 4.2.2 HYNIC AS CHELATOR

Labeling mixtures, either in solution or with a freeze-dried formulation contained the same ingredients (50):

**Stock 1:** Ethylendiaminediacetic acid (EDDA) was dissolved in aqueous NaOH (0.10 M) to a concentration of 10.0 g/L.

**Stock 2:** Disodiumtartrate dihydrate was dissolved in $NaH_2PO_4$ buffer (40.0 g/L) to a concentration of 40.0 g/L.

**Stock 3:** Tin(II)chloride dihydrate was dissolved in aqueous sodium ascorbate (3.00 g/L in 0.01 M HCl) to a concentration of 1.50 g/L. This mixture was freshly prepared for every labeling experiment.

**Stock 4:** The respective peptide was dissolved in DMSO or $H_2O$ or a mixture thereof to a concentration of usually 1.00 mM.

A labeling mixture or kit was prepared by mixing the stock solutions according to the following indication:

Stock 1: 50.0 μL

Stock 2: 50.0 μL

Stock 3: 5.33 μL

Stock 4: 5.00 μL

Freshly eluted $^{99m}$Tc-Pertechnetate (0.10 - 5.00 μL, 50.0 - 850 MBq) was added and the mixture heated for 20 min at 95°C. Quality control was performed by radio-TLC and radio-RP-HPLC directly from the reaction mixture as stated above (see 4.2.1).

### 4.2.3 N4 AS CHELATOR

Labeling mixtures, either in solution or with a freeze-dried formulation contained the same ingredients:

**Stock 1:** $Na_2HPO_4$ was dissolved in $H_2O$ to yield a concentration of 0.05 M (pH = 11.5).

**Stock 2:** Disodiumcitrate sesquihydrate was dissolved in $H_2O$ to a concentration of 0.10 M.

**Stock 3:** Tin(II)chloride dihydrate was dissolved in aqueous sodium ascorbate (3.00 g/L in 0.01 M HCl) to a concentration of 1.00 g/L. This mixture was freshly prepared for every labeling experiment.

**Stock 4:** The respective peptide was dissolved in DMSO or $H_2O$ or a mixture thereof to a concentration of usually 1.00 mM.

A labeling mixture or kit was prepared by mixing the stock solutions according to the following indication:

Stock 1: 25.0 μL

Stock 2: 3.00 μL

Stock 3: 5.00 μL

Stock 4: 7.50 μL

Freshly eluted $^{99m}$Tc-Pertechnetate (0.10 - 5.00 $\mu$L, 50.0 - 850 MBq) was added and the mixture heated for 10 min at 90°C. Quality control was performed by radio-TLC and radio-RP-HPLC directly from the reaction mixture as stated above (see 4.2.1).

### 4.3 $^{177}$Lu-LABELING

For $^{177}$Lu-labeling of DOTA- or DOTA-GA-bearing peptides, 0.5 - 2 nmol of the respective peptide (directly from stock, DMSO or $H_2O$ or mixtures thereof) were mixed with 10 $\mu$L of an aqueous sodium acetate buffer (1.00 M, pH = 5.50). The desired activity [$^{177}$Lu]$LuCl_3$ (0.04 M in HCl), usually between 5 and 80 MBq, was added and the mixture diluted with HCl (0.04 M) to a total volume of 100 $\mu$L. After 30 min at 95°C, 10 $\mu$L sodium ascorbate (0.10 M) was added to prevent radiolysis and reaction control via radio-RP-HPLC and radio-TLC was performed.
Silica-coated 60 RP-18 $F_{254}$s with mobile phase $NH_4$OAc/DMF (1/1; v/v):
$R_f$ ($^{177}LuCl_3$) = 0, $R_f$ ($^{177}$Lu-colloid) = 0, $R_f$ ($^{177}$Lu-peptide) = 1.
Cellulose ITLC-SG paper with mobile phase trisodium citrate (0.10 M):
$R_f$ ($^{177}LuCl_3$) = 1, $R_f$ ($^{177}$Lu-colloid) = 0, $R_f$ ($^{177}$Lu-peptide) = 0.

### 4.4 $^{18}$F-LABELING

Labeling of SiFA-bearing peptides was achieved according to recently published literature (51). Briefly, a SAX cartridge (Sep-Pak Accell Plus QMA Carbonate light) was conditioned with 10 mL $H_2O$ prior to passage of aqueous $^{18}$F-fluoride. The cartridge was purged with 10 mL air, dried with 10 mL ACN, followed by purging with another 20 mL air to remove traces of water. $^{18}$F-fluoride was eluted with 100 $\mu$mol [$K^+\subset$2.2.2]$OH^-$ (in 500 $\mu$L ACN) and the pH adjusted by addition of 25 $\mu$L oxalic acid (1.00 M in ACN).
This mixture was used for either one or several labeling experiments. The desired activity (usually 30 - 500 MBq) was mixed with 10 - 25 $\mu$mol of the respective SiFA-bearing peptide (directly from stock in DMSO) and the mixture allowed to incubate at r.t. for 5 min. The reaction mixture was then diluted with 9 mL HEPES buffer (0.10 M, pH = 3). The product was isolated from unreacted $^{18}$F-fluoride by passage of the mixture through a Sep-Pak C18 light cartridge. After purging the cartridge with 10 mL $H_2O$, the peptide was eluted with 500 $\mu$L of a EtOH/PBS mixture (1/1; v/v). Radiochemical purity was determined using radio-RP-HPLC and radio-TLC.
Silica-coated 60 RP-18 $F_{254}$s with mobile phase ACN/PBS (1/1; v/v; + 10 vol% NaOAc (2.00 M in $H_2O$); + 1 vol% TFA): $R_f$ ($^{18}$F-fluoride) = 0, $R_f$ ($^{18}$F-peptide) = 0.8 - 1.

### 4.5 $^{68}$GA-LABELING

$^{68}$Ga-labeling of DOTA and DOTA-GA-bearing peptides was achieved in accordance to the literature (34). An automated GallElut$^+$ sytem from *Scintomics* was used. Briefly, the $^{68}$Ge/$^{68}$Ga-generator from *IThemba LABS* was eluted with aqueous HCl (1.00 M) and a fraction (usually 1.25 mL, 500 - 700 MBq) was transferred into the reaction vial (ALLTECH, 5 mL), loaded beforehand with 2 - 5 nmol of the respective peptide. The reaction mixture was heated 5 min at 95°C before passage through a Sep-Pak C8 light cartridge, preconditioned with 10 mL $H_2O$. The product was eluted with 2 mL EtOH/$H_2O$ (1/1; v/v), the cartridge purged with 1 mL PBS and 1 mL $H_2O$ before removal of EtOH in vacuo. Radiochemical purity assessed by radio-TLC.
Silica-coated 60 RP-18 $F_{254}$s with mobile phase $NH_4$OAc/DMF (1/1; v/v):
$R_f$ ($^{68}GaCl_3$) = 0, $R_f$ ($^{68}$Ga-colloid) = 0, $R_f$ ($^{68}$Ga-peptide) = 1.
Cellulose ITLC-SG paper with mobile phase trisodium citrate (0.10 M):
$R_f$ ($^{68}GaCl_3$) = 1, $R_f$ ($^{68}$Ga-colloid) = 0, $R_f$ ($^{68}$Ga-peptide) = 0.

### 4.6 $^{67}$GA-LABELING

Labeling of DOTA-bearing peptides with $^{67}$Ga was carried out in analogy to a method, described in literature (52). In short, [$^{67}$Ga]Ga-citrate was immobilized on a SEP-Pak Silica light cartridge, washed with $H_2O$ (10 mL) and eluted with the desired volume of HCl (0.1 M). A fraction of the resulting $^{67}GaCl_3$ solution was then diluted with HEPES to a total volume of 200 $\mu$L and given onto the peptide (5 nmol, $H_2O$). The mixture was then heated to 95°C for 30 min, diluted with PBS to a volume of at least 3 mL and passed through a SEP Pak C8 light cartridge to remove excess $^{17}GaCl_3$. The labelled peptide was eluted with 0.5 mL of a EtOH/PBS (1/1; v/v) mixture. Radiochemical yields and purities were assessed via radio-TLC.
Silica-coated 60 RP-18 $F_{254}$s with mobile phase $NH_4$OAc/DMF (1/1; v/v):
$R_f$ ($^{67}GaCl_3$) = 0, $R_f$ ($^{67}$Ga-colloid) = 0, $R_f$ ($^{67}$Ga-peptide) = 1.
Cellulose ITLC-SG paper with mobile phase trisodium citrate (0.10 M):

$R_f$ ($^{67}GaCl_3$) = 1, $R_f$ ($^{67}$Ga-colloid) = 0, $R_f$ ($^{67}$Ga-peptide) = 0.

## 5. *IN VITRO* EXPERIMENTS

### 5.1 DETERMINATION OF $IC_{50}$

CXCR4-positive Jurkat T lymphocyte cells were grown in *Gibco's* RPMI 1640 GlutaMAX medium supplemented with 10 vol% FBS and maintained in a 5% $CO_2$ atmosphere at 37°C.

For *in vitro* experiments, cells were counted in a hemocytometer using trypan blue as contrast agent. The cell suspension was centrifuged and the pellet resuspended in HBSS (+1 wt% BSA) to a concentration of 2 mio cells/mL. 8x3 polystyrene tubes were loaded with 25 μL of the standard ligand $^{125}$I-Fc-131 (see 4.1, 1.00 nM in HBSS) and 25 μL of the ligand to investigate in the respective concentration ($10^{-4}$ - $10^{-10}$ M, n = 3 for each concentration). 200 μL of cell suspension (400.000 cells per well) were added to obtain a final peptide concentration range of $10^{-5}$ - $10^{-11}$ M. The tubes were cooled for 2 h at 8°C to prevent internalization. The experiment was stopped by removal of the supernatant. 200 μL HBSS were added onto the cells, the suspension centrifuged and the supernatant pooled with the respective first fractions. This step was repeated once more before the tubes containing the supernatants and the ones containing the cell pellets were subjected to activity measurement in the γ-counter.

### 5.2 DETERMINATION OF $INVIC_{50}$

For the determination of inverse $IC_{50}$ ($invIC_{50}$) values the protocol for the determination of regular $IC_{50}$ values was resumed with minor changes. The peptide under investigation was radioactively labeled and a stock solution was prepared in HBSS (2.00 nM). A concentration gradient of the standard ligand Fc-131 was prepared in HBSS ($10^{-4}$ - $10^{-10}$ M). A cell tube was then containing 25 μL of the radioactive peptide solution, 25 μL of the respective Fc-131 solution and 200 μL cell suspension (400.000 cells). The experiment was conducted as described above.

### 5.3 DETERMINATION OF INTERNALIZATION

CXCR4-expressing Chem_1 cells were grown in DMEM-F12 medium supplemented with 10 vol% FBS, 1 vol% NEA, 1 vol% PenStrep and 1 vol% HEPES (1.00 M). Cells were maintained in a 5% $CO_2$ atmosphere at 37°C.

For *in vitro* experiments, cells were harvested by removal of the medium and incubation of the cells with trypsine/EDTA (0.05%/0.02%; w/v) for 30 in at 37°C. The cells were counted and seeded in 24-well plates (100.000 cell per well) 24 ± 2 h prior to the experiment.

The medium was removed and the cells incubated in 200 μL DMEM-F12 (+5 wt% BSA) for 15 min at 37°C. Each well (n = 3 for every time point) was either treated with 25 μL DMEM-F12 (+5 wt% BSA) or 25 μL of an AMD3100 stock (1 mM in $H_2O$) for blockage of the receptors. As the experiment was carried out as an dual-tracer approach, a radio-tracer solution was prepared, containing the standard ligand $^{12S}$I-Fc-131 and the radioactively labeled peptide under investigation each in a concentration of 2.00 nM. 25 μL of this stock was added to the wells (final $^{125}$Fc-131 concentration: 0.20 nM; final peptide concentration under investigation: 0.20 nM) and the cells incubated at 37°C for the respective period.

The experiment was stopped by placing the well-plate on ice and removing the supernatant. The cells were washed with 250 μL cold HBSS and the both fractions for each well combined, comprising the amount of unbound radioligand.

250 μL of cold acid wash (0.02 M NaOAc in aqueous acetic acid, pH = 5) were added and the cells incubated for 15 min on ice. The supernatant was removed, the cells washed with cold HBSS and the respective fractions combined to obtain the amount of surface-bound radioligand.

The cells were then incubated with 300 μL NaOH (1.00 M in $H_2O$) for at least 30 at r.t. before the supernatant was removed. The well was washed with another 300 μL NaOH and the fractions combined that contain the amount of internalized radioligand.

The three different fractions were subjected to activity measurement in the γ-counter, measuring the activity of the radionuclide used for the labeling of the peptide under investigation first. The same fractions were measured again for the activity of $^{125}$I after an adequate time, in dependence on the half-life of the first radionuclide. Data was corrected for non-specific internalization and referred to the specific internalization of the standard ligand $^{121}$I-Fc-131.

### 5.4 DETERMINATION OF THE OCTANOL/PBS PARTITION COEFFICIENT

The ligand under investigation (usually 0.50 - 3.00 MBq, depending on the radioisotope) was diluted in PBS (pH = 7.4) to a total volume of 1.00 mL and mixed with 1.00 mL n-octanol in a low-bind Eppendorf tube (n = 8). The

tubes were vortexed at maximum speed for 3 min to ensure equilibrium before centrifugation at 15.000xg for 5 min on a Biofuge 15 from *Heraeus Holding GmbH* (Osterode, Germany). An aliquot of 100 $\mu$L of each fraction was measured in the $\gamma$-counter and the $logD_{7.4}$ calculated as follows:

$$\log D_{7.4} = average(log_{10}(\frac{activity\ in\ octanol}{activity\ in\ PBS}))$$

### 6. *IN VIVO* EXPERIMENTS

#### 6.1 MOUSE MODEL AND TUMOR MODEL

All animal experiments were conducted in accordance with general animal welfare regulations in Germany and the institutional guidelines for the care and use of animals. To establish tumor xenografts, Jurkat cells (2 - 3x$10^7$ cells) were suspended in a mixture of *Gibco's* RPMI 1640 medium and Matrigel (1/1; v/v) from *BD Biosciences* (Heidelberg, Germany), and inoculated subcutaneously onto the right shoulder of 6-10 weeks old CB17-SCID mice from either *Charles River GmbH* (Sulzfeld, Germany) or the in-house mouse breeding facility. Mice were used when tumors had grown to a diameter of 5-8 mm (4 - 10 weeks after inoculation).

#### 6.2 $\mu$SPECT/$\mu$PET/CT IMAGING

Imaging experiments were conducted on a VECTor$^4$ small-animal SPECT/PET/OI/CT from *MILabs BV.* (Utrecht, The Netherlands). The resulting data were analyzed with the associated PMOD (version 4.0) software. Mice were anaesthetized with *iso*flurane and the radioactively labeled compounds were injected via the tail vein. Mice were euthanized after the respective distance and blood samples for later biodistribution studies were taken by cardiac puncture before image acquisition. Static images were acquired with 45 min acquisition time using the HE-GP-RM collimator and a step-wise multi-planar bed movement for SPECT isotopes and the HE-UHR-M collimator and a step-wise spiral bed movement for PET isotopes. All images were reconstructed using the MILabs-Rec software (version 10.02) and a pixel-based Similarity-Regulated Ordered Subsets Expectation Maximization (SROSEM) algorithm with a window-based scatter correction (20% below and 20% above the photopeak, respectively). (Voxel size CT: 80 $\mu$m, voxel size SPECT/PET: 0.8 mm, 1.6 mm (FWHM) Gaussian blurring post processing filter, with calibration factor in kBq/mL and decay correction, no attenuation correction)

#### 6.3 BIODISTRIBUTION STUDIES

Approximately 0.5 - 20 MBq (0.02 - 0.20 nmol) of the $^{125}$I-, $^{177}$Lu-, $^{99m}$Tc-,$^{18}$F- or $^{68}$Ga-labeled ligand were injected into the tail vein of Jurkat tumor-bearing CB-17 SCID mice and the animals sacrificed after the respective biodistribution time. Selected organs were removed, weighted and measured in a $\gamma$-counter.

## II. Results

### 1. TECHNETIUM-99M SPECT TRACER

#### 1.1 $MAS_3$-CONJUGATED PEPTIDES

##### *1.1.1 Chemical Structures*

Chemical Structures of $mas_3$-conjugated compounds Tc-CXCR4-1 to -8 are illustrated in the following.

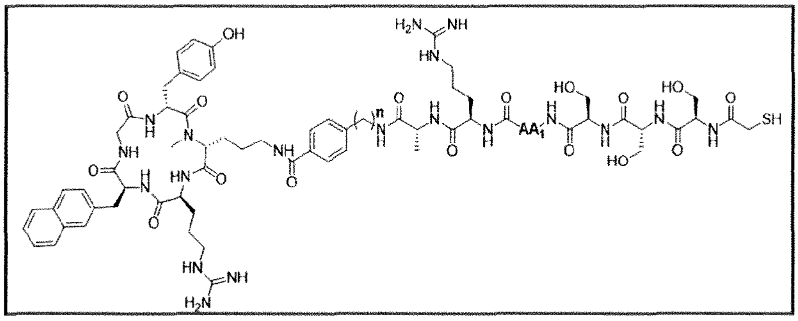

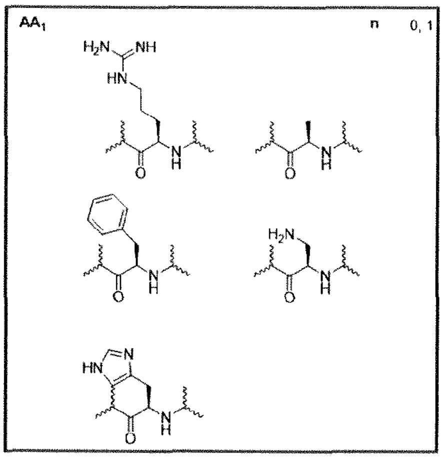


Table 1: *Summary of structural modifications and assigned abbreviations of mas$_3$-conjugated peptides.*

| A$_1$ | n | Tc-CXCR4 |
|---|---|---|
| / | 0 | -1* |
| D-Ala | 0 | -2* |
| D-Arg | 0 | -3* |
| D-Phe | 0 | -4* |
| D-His | 0 | -5 |
| D-Dap | 0 | -6 |
| D-His | 1 | -7 |
| D-Dap | 1 | -8 |

**compound provided for comparative purposes*

***1.1.2 In-Vitro Data***

*Table 2: IC$_{50}$ values of unlabeled compounds Tc-CXCR4-1 to -8.*

| Tc-CXCR4 | ICso [nM] (n = x) |
|---|---|
| -1 | 20.6 ± 7.52 (3) |
| -2 | 32.4 ± 9.90 (3) |
| -3 | 184 ± 25.3 (3) |
| -4 | 3490 ± 502 (3) |
| -5 | 11.0 ± 1.30 (3) |
| -6 | 4.97 ± 1.34 (5) |
| -7 | n.d. |
| -8 | n.d. |

***1.1.3 Biodistribution Studies***

*Table 3: Biodistribution of $^{99m}$Tc-CXCR4-1 (comparative compound) in 5 Daudi-tumor-bearing mice, 1h p.i.*

| **$^{99m}$Tc-CXCR4-1, 1h p.i. CB-17 SCID female mice, Daudi xenograft (n = 5)** | | |
|---|---|---|
| **Organ** | **%iD/g** | **Std.** |
| Blood | 0.83 | 0.17 |
| Heart | 0.54 | 0.12 |
| Lung | 1.79 | 0.40 |
| Liver | 3.54 | 0.54 |
| Pancreas | 0.22 | 0.05 |
| Spleen | 1.09 | 0.22 |
| Stomach | 0.86 | 0.28 |
| Intestine | 1.12 | 0.72 |
| Kidney | 4.00 | 0.86 |
| Muscle | 0.16 | 0.04 |
| Tumor | 1.41 | 0.56 |

*Table 4: Biodistribution of $^{99m}$Tc-CXCR4-5 in 4 Jurkat tumor-bearing mice, 1h p.i.*

| **$^{99m}$Tc-CXCR4-5, 1h p.i. CB-17 SCID female mice, Jurkat xenograft (n = 4)** | | |
|---|---|---|
| **Organ** | **%iD/g** | **Std.** |
| Blood | 0.65 | 0.10 |
| Heart | 0.48 | 0.02 |
| Lung | 1.57 | 0.25 |
| Liver | 2.42 | 0.53 |
| Pancreas | 0.40 | 0.18 |
| Spleen | 8.47 | 0.23 |
| Stomach | 0.70 | 0.19 |
| Intestine | 0.76 | 0.06 |
| Adrenal | 0.42 | 0.45 |
| Kidney | 2.89 | 0.27 |
| Muscle | 0.15 | 0.01 |
| Bone | 1.55 | 0.28 |
| Tumor | 1.48 | 0.28 |

*Table 5: Biodistribution of $^{99m}$Tc-CXCR4-6 in 5 Jurkat tumor-bearing mice, 1h p.i.*

| **$^{99m}$Tc-CXCR4-6, 1h p.i. CB-17 SCID female mice, Jurkat xenograft (n = 5)** | | |
|---|---|---|
| **Organ** | **%iD/g** | **Std.** |
| Blood | 2.31 | 0.53 |
| Heart | 1.76 | 0.38 |
| Lung | 6.04 | 1.05 |
| Liver | 8.41 | 1.47 |
| Pancreas | 0.78 | 0.19 |
| Spleen | 3.84 | 0.40 |
| Stomach | 2.35 | 0.42 |
| Intestine | 1.58 | 0.21 |
| Kidney | 22.8 | 5.56 |
| Muscle | 0.51 | 0.14 |
| Bone | 1.60 | 0.63 |

(continued)

| $^{99m}$Tc-CXCR4-6, 1h p.i. CB-17 SCID female mice, Jurkat xenograft (n = 5) | | |
|---|---|---|
| **Organ** | **%iD/g** | **Std.** |
| Tumor | 6.63 | 1.02 |

*Table 6: Biodistribution of $^{99m}$Tc-CXCR4-6 co-injected with 100 nmol AMD 3100 in female Jurkat tumor-bearing mice, 1h p.i.*

| $^{99m}$Tc-CXCR4-6, 1h p.i. CB-17 SCID female mice, Jurkat xenograft +100 nmol AMD 3100 (n = 1) | |
|---|---|
| **Organ** | **%iD/g** |
| Blood | 1.93 |
| Heart | 1.32 |
| Lung | 4.52 |
| Liver | 4.56 |
| Pancreas | 0.73 |
| Spleen | 5.55 |
| Stomach | 1.51 |
| Intestine | 1.53 |
| Kidney | 18.4 |
| Muscle | 0.43 |
| Bone | 1.11 |
| Tumor | 2.69 |

### *1.1.4 Mouse Imaging Studies*

Figure 1 shows the MIP of both CT and SPECT Scans of $^{99m}$Tc-CXCR4-6 1h p.i. in female Jurkat tumor-bearing mice without (left) and with competitor (right, 100 nmol AMD 3100) from 1 - 10%iD/mL; white arrows indicate organs of interest: solid = tumor, points = kidney, dashed = liver.

### *1.1.5 Internalization Studies*

*Table 7: Cell-bound and internalized ligand in correlation to total cellular activity and internalized activity in comparison to $^{125}$I-Fc-131 of $^{99m}$Tc-CXCR4-6 and -8.*

| Internalization of labeled compounds With internal standard $^{125}$I-Fc-131 | | | |
|---|---|---|---|
| | | **$^{99m}$Tc-CXCR4-6** | |
| **Time [min]** | **Cell-bound [%]** | **Internalized [%]** | **Internalized in relation to $^{125}$I-Fc-131 [%]** |
| 30 | 6.56 ± 0.31 | 17.4 ± 0.40 | 470 ± 17.4 |
| 90 | 6.90 ± 0.29 | 27.1 ± 0.50 | 526 ± 29.0 |
| | | **$^{99m}$Tc-CXCR4-8** | |
| **Time [min]** | **Cell-bound [%]** | **Internalized [%]** | **Internalized in relation to $^{125}$I-Fc-131 [%]** |
| 30 | 9.78 ± 0.61 | 6.85 ± 0.77 | 112 ± 17.8 |
| 90 | 7.63 ± 0.20 | 10.7 ± 0.09 | 160 ± 8.28 |

## 1.2 MODIFIED $MAS_3$-CONJUGATED PEPTIDES

### *1.2.1 Chemical Structures*

Chemical Structures of modified $mas_3$-conjugated compounds Tc-CXCR4-9 to -12 are illustrated in the following.

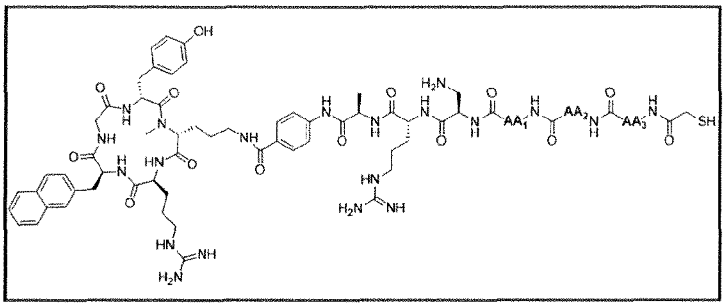

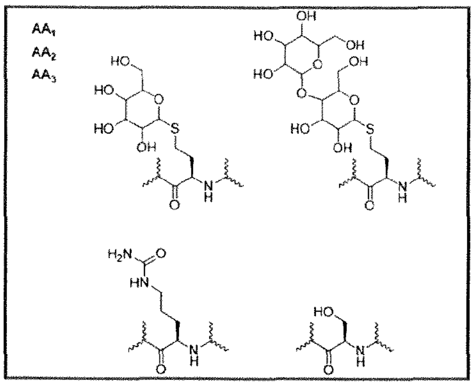

*Table 8: Summary of structural modifications and assigned abbreviations for modified $mas_3$-conjugated peptides.*

| $AA_1$ | $AA_2$ | $AA_3$ | Tc-CXCR4 |
|---|---|---|---|
| D-hcys(Glucosyl) | D-hcys(Glucosyl) | D-hcys(Glucosyl) | -9 |
| D-ser | D-hcys(Glucosyl) | D-hcys(Glucosyl) | -10 |
| D-ser | D-hcys(Lactosyl) | D-ser | -11 |
| D-cit | D-hcys(Lactosyl) | D-cit | -12 |

### *1.2.2 In Vitro Data*

*Table 9: inverse $IC_{50}$ values, $logD_{7.4}$ and Internalization values of Tc-labeled compounds Tc-CXCR4-6, -8 and -9 to -12.*

| [$^{99m}$Tc]Tc-CXCR4 | invICso [nM] (n = x) | $logD_{7.4}$ (n = x) | Internalization [%Fc-131] |
|---|---|---|---|
| -6 | 3.67 ± 0.77 (3) | - 1.54 ± 0.02 (6) | 525 (90 min) |
| -8 | 3.65 ± 1.01 (4) | - 1.36 ± 0.02 (8) | 160 (90 min) |
| -9 | 3.50 ± 0.65 (3) | - 2.56 ± 0.03 (6) | 103 (90 min) |
| -10 | 5.13 ± 1.81 (3) | - 2.32 ± 0.02 (7) | 267 (90 min) |
| -11 | 2.05 ± 0.95 (3) | - 2.94 ± 0.02 (8) | 221 (120 min) |
| -12 | 4.82 ± 1.36 (3) | - 2.92 ± 0.06 (8) | 473 (120 min) |

### *1.2.3 Biodistribution Studies*

*Table 10: Biodistribution of $^{99m}$Tc-CXCR4-9, -11 and -12 in female Jurkat tumor-bearing mice, 1h p.i.*

| 1h p.i. CB-17 SCID female mice, Jurkat xenograft (n = 5) | | | | | | |
|---|---|---|---|---|---|---|
| | $^{99m}$Tc-CXCR4-9 | | $^{99m}$Tc-CXCR4-11 | | $^{99m}$Tc-CXCR4-12 | |
| Organ | %iD/g | Std. | %iD/g | Std. | %iD/g | Std. |
| Blood | 1.42 | 0.38 | 1.36 | 0.13 | 1.74 | 0.26 |
| Heart | 0.87 | 0.13 | 0.95 | 0.07 | 1.14 | 0.10 |

(continued)

| 1h p.i. CB-17 SCID female mice, Jurkat xenograft (n = 5) | | | | | | |
|---|---|---|---|---|---|---|
| | $^{99m}$Tc-CXCR4-9 | | $^{99m}$Tc-CXCR4-11 | | $^{99m}$Tc-CXCR4-12 | |
| **Organ** | **%iD/g** | **Std.** | **%iD/g** | **Std.** | **%iD/g** | **Std.** |
| Lung | 2.40 | 0.49 | 2.85 | 0.49 | 3.96 | 0.36 |
| Liver | 3.97 | 0.67 | 5.94 | 0.31 | 11.0 | 0.36 |
| Pancreas | 0.50 | 0.08 | 0.48 | 0.08 | 0.80 | 0.37 |
| Spleen | 1.24 | 0.45 | 2.31 | 0.31 | 4.72 | 0.22 |
| Stomach | 3.51 | 1.42 | 1.16 | 0.21 | 2.03 | 0.28 |
| Intestine | 1.50 | 0.63 | 0.72 | 0.09 | 1.25 | 0.12 |
| Kidney | 14.8 | 0.94 | 29.0 | 2.83 | 35.0 | 2.77 |
| Muscle | 0.24 | 0.08 | 0.28 | 0.04 | 0.34 | 0.04 |
| Bone | 0.47 | 0.11 | 0.71 | 0.11 | 0.95 | 0.14 |
| Tumor | 5.92 | 0.11 | 6.71 | 1.55 | 7.39 | 0.64 |

*Table 11: Biodistribution of $^{99m}$Tc-CXCR4-9, -11 and -12 co-injected with 100 nmol AMD 3100 in female Jurkat tumor-bearing mice, 1h p.i.*

| 1h p.i. CB-17 SCID female mice, Jurkat xenograft (n = 1) | | | |
|---|---|---|---|
| **+100 nmol AMD 3100** | **$^{99m}$Tc-CXCR4-9** | **$^{99m}$Tc-CXCR4-11** | **$^{99m}$Tc-CXCR4-12** |
| **Organ** | **%iD/g** | | |
| Blood | 0.69 | 1.75 | 2.16 |
| Heart | 0.42 | 1.08 | 1.15 |
| Lung | 1.08 | 3.42 | 4.31 |
| Liver | 1.08 | 4.39 | 5.34 |
| Pancreas | 0.28 | 0.68 | 0.82 |
| Spleen | 0.58 | 3.09 | 17.7 |
| Stomach | 1.29 | 1.28 | 1.30 |
| Intestine | 0.48 | 1.02 | 1.36 |
| Kidney | 8.17 | 34.9 | 37.2 |
| Muscle | 0.14 | 0.34 | 0.38 |
| Bone | 0.41 | 0.83 | 0.64 |
| Tumor | 0.65 | 1.62 | 3.03 |

### *1.2.4 Mouse Imaging Studies*

Figure 2 shows the MIP of both CT and SPECT Scans of $^{99m}$Tc-CXCR4-9 1h p.i. in female Jurkat tumor-bearing mice without (left) and with competitor (right, 100 nmol AMD 3100) from 1 - 10%iD/mL; white arrows indicate organs of interest: solid = tumor, points = kidney, dashed = liver.

Figure 1 shows the MIP of both CT and SPECT Scans of $^{99m}$Tc-CXCR4-11 1h p.i. in female Jurkat tumor-bearing mice without (left) and with competitor (right, 100 nmol AMD 3100) from 1 - 10%iD/mL; white arrows indicate organs of interest: solid = tumor, points = kidney, dashed = liver.

Figure 2 shows the MIP of both CT and SPECT Scans of $^{99m}$Tc-CXCR4-12 1h p.i. in female Jurkat tumor-bearing mice without (left) and with competitor (right, 100 nmol AMD 3100) from 1 - 10%iD/mL; white arrows indicate organs of interest: solid = tumor, points = kidney, dashed = liver.

### *1.2.5 Internalization Studies*

*Table 12: Cell-bound and internalized ligand in correlation to total cellular activity and internalized activity in comparison to $^{125}$I-Fc-131 of $^{99m}$Tc-CXCR4-9 and -10.*

| Internalization of labeled compounds With internal standard $^{125}$I-Fc-131 | | | |
|---|---|---|---|
| $^{99m}$Tc-CXCR4-9 | | | |
| Time | Cell-bound [%] | Internalized [%] | Internalized in relation to |
| [min] | | | $^{125}$I-Fc-131 [%] |
| 30 | 3.69 ± 0.56 | 7.00 ± 0.74 | 86.4 ± 9.62 |
| 90 | 3.69 ± 0.49 | 12.5 ± 0.67 | 103 ± 6.56 |
| $^{99m}$Tc-CXCR4-10 | | | |
| Time [min] | Cell-bound [%] | Internalized [%] | Internalized in relation to $^{125}$I-Fc-131 [%] |
| 30 | 6.75 ± 0.76 | 18.8 ± 0.66 | 226 ± 10.2 |
| 90 | 6.06 ± 0.34 | 31.7 ± 0.52 | 267 ± 8.94 |

*Table 13: Cell-bound and internalized ligand in correlation to total cellular activity and internalized activity in comparison to $^{125}$I-Fc-131 of $^{99m}$Tc-GXGR4-11 and -12.*

| Internalization of labeled compounds With internal standard $^{125}$I-Fc-131 | | | |
|---|---|---|---|
| $^{99m}$Tc-CXCR4-11 | | | |
| Time [min] | Cell-bound [%] | Internalized [%] | Internalized in relation to $^{125}$I-Fc-131 [%] |
| 15 | 1. 79 ± 0.36 | 3.16 ± 0.46 | 71.9 ± 11.4 |
| 30 | 3.74 ± 0.33 | 6.98 ± 0.78 | 178 ± 26.2 |
| 60 | 7.02 ± 0.06 | 7.10 ± 0.84 | 145 ± 19.8 |
| 120 | 4.25 ± 0.67 | 10.8 ± 1.13 | 221 ± 23.4 |
| $^{99m}$Tc-CXCR4-12 | | | |
| Time [min] | Cell-bound [%] | Internalized [%] | Internalized in relation to $^{125}$I-Fc-131 [%] |
| 15 | 3.77 ± 0.83 | 3.79 ± 0.69 | 192 ± 38.1 |
| 30 | 8.56 ± 0.66 | 9.64 ± 1.31 | 284 ± 92.3 |
| 60 | 14.1 ± 0.71 | 11.6 ± 2.01 | 395 ± 72.2 |
| 120 | 7.42 ± 2.12 | 18.1 ± 0.85 | 473 ± 41.9 |

## 1.3 FIXED-CHELATOR-BASED PEPTIDES

### *1.3.1 Chemical Structures*

Chemical Structures of peptides with fixed-chelators comprising HYNIC and N4, Tc-CXCR4-13 to -16 are illustrated in the following

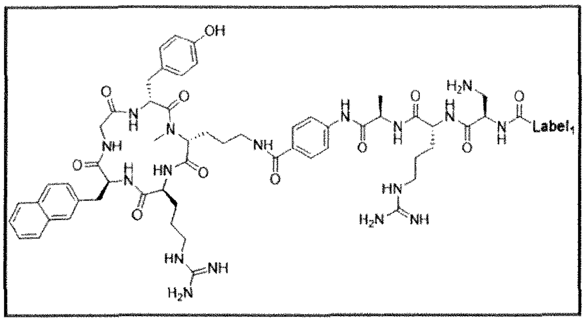

Label$_1$

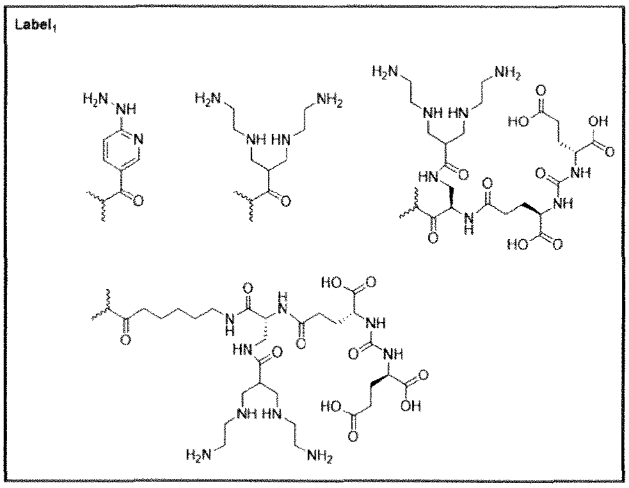

*Table 14: Summary of structural modifications and assigned abbreviations for peptides with fixed chelators Tc-CXCR4-13 to -16.*

| Label$_1$ | Tc-CXCR4 |
|---|---|
| HYNIC (EDDA) | -13 |
| N4 | -14 |
| d-dap(N4)-eue | -15 |
| Ahx-dap(N4)-eue | -16 |

***1.3.2 In Vitro Data***

*Table 15: Inverse $IC_{50}$ values, $logD_{7.4}$ and internalization of Tc-labeled compounds Tc-CXCR4-13 to -16.*

| [$^{99m}$Tc]Tc-CXCR4 | invIC$_{50}$ [nM] (n = x) | logD$_{7.4}$ (n = x) | Internalization [%Fc-131] |
|---|---|---|---|
| -13 | 4.16 ± 1.50 (3) | - 2.74 ± 0.03 (5) | 503 (90 min) |
| -14 | 10.2 ± 2.35 (3) | - 1.75 ± 0.08 (8) | 666 (120 min) |
| -15 | 3.69 ± 1.32 (3) | - 3.60 ± 0.02 (8) | 232 (90 min) |
| -16 | 6.08 ± 1.03 (3) | - 2.70 ± 0.04 (8) | 284 (90 min) |
| CPCR4-HYNIC* | No displacement | - 1.84 ± 0.01 (8) | 29 (90 min) |
| [$^{177}$Lu]-Pentixather | 1.91 ± 1.11 (3) | - 1.76 ± 0.03 (8) | 283 (120 min) |

*CPCR4-HYNIC: In Literature: $^{99m}$Tc-CXCR4-L

***1.3.3 Biodistribution Studies***

*Table 16: Biodistribution of $^{99m}$Tc-CXCR4-13 and -14 in female Jurkat tumor-bearing mice, 1h p.i.*

| 1h p.i. CB-17 SCID female mice, Jurkat xenograft (n = 5) | | | | |
|---|---|---|---|---|
| | $^{99m}$Tc-CXCR4-13 | | $^{99m}$Tc-CXCR4-14 | |
| **Organ** | **%iD/g** | **Std.** | **%iD/g** | **Std.** |
| Blood | 1.56 | 0.58 | 1.62 | 0.19 |
| Heart | 0.78 | 0.28 | 1.21 | 0.10 |

(continued)

| 1h p.i. CB-17 SCID female mice, Jurkat xenograft (n = 5) | | | | |
|---|---|---|---|---|
| | $^{99m}$Tc-CXCR4-13 | | $^{99m}$Tc-CXCR4-14 | |
| **Organ** | **%iD/g** | **Std.** | **%iD/g** | **Std.** |
| Lung | 1.94 | 0.44 | 3.99 | 0.56 |
| Liver | 2.62 | 0.23 | 7.67 | 0.70 |
| Pancreas | 0.36 | 0.30 | 0.56 | 0.10 |
| Spleen | 2.43 | 1.11 | 5.60 | 0.82 |
| Stomach | 1.98 | 0.67 | 1.97 | 0.39 |
| Intestine | 0.57 | 0.12 | 0.92 | 0.11 |
| Kidney | 29.5 | 4.38 | 37.4 | 2.74 |
| Muscle | 1.52 | 2.61 | 0.30 | 0.05 |
| Bone | 0.81 | 0.41 | 1.31 | 0.28 |
| Tumor | 8.02 | 2.21 | 8.63 | 1.26 |

*Table 17: Biodistribution of $^{99m}$Tc-CXCR4-13 and -14 co-injected with 100 nmol AMD 3100 in female Jurkat tumor-bearing mice, 1h p.i.*

| 1h p.i. CB-17 SCID female mice, Jurkat xenograft (n = 1) | | |
|---|---|---|
| **+100 nmol AMD 3100** | **$^{99m}$Tc-CXCR4-13** | **$^{99m}$Tc-CXCR4-14** |
| **Organ** | **%iD/g** | |
| Blood | 0.94 | 1.30 |
| Heart | 0.49 | 0.94 |
| Lung | 1.10 | 2.60 |
| Liver | 0.79 | 4.82 |
| Pancreas | 0.13 | 0.55 |
| Spleen | 0.49 | 1.83 |
| Stomach | 1.16 | 1.78 |
| Intestine | 0.31 | 0.80 |
| Kidney | 13.1 | 32.0 |
| Muscle | 0.10 | 0.25 |
| Bone | 0.36 | 0.76 |
| Tumor | 0.97 | 2.89 |

### *1.3.4 Mouse Imaging Studies*

Figure 5 shows the MIP of both CT and SPECT Scans of $^{99m}$Tc-CXCR4-13 1h p.i. in female Jurkat tumor-bearing mice without competitor from 1 - 10%iD/mL; white arrows indicate organs of interest: solid = tumor, points = kidney, dashed = liver.

Figure 6 shows the MIP of both CT and SPECT Scans of $^{99m}$Tc-CXCR4-14 1h p.i. in female Jurkat tumor-bearing mice without (left) and with competitor (right, 100 nmol AMD 3100) from 1 - 10%iD/mL; white arrows indicate organs of interest: solid = tumor, points = kidney, dashed = liver.

Figure 7 shows the MIP of both CT and SPECT Scans of $^{99m}$Tc-CXCR4-14 2h p.i. in female Jurkat tumor-bearing mice without competitor from 1 - 10%iD/mL; white arrows indicate organs of interest: solid = tumor, points = kidney, dashed = liver.

### *1.3.5 Internalization Studies*

*Table 18: Cell-bound and internalized ligand in correlation to total cellular activity and internalized activity of $^{99m}$Tc-CXCR4-13 in comparison to $^{125}$I-Fc-131.*

| **Internalization of labeled compounds With internal standard $^{125}$I-Fc-131** | | | |
|---|---|---|---|
| **$^{99m}$Tc-CXCR4-13** | | | |
| **Time [min]** | **Cell-bound [%]** | **Internalized [%]** | **Internalized in relation to $^{125}$I-Fc-131 [%]** |
| 30 | 7.56 ± 0.38 | 18.0 ± 0.27 | 434 ± 18.8 |
| 90 | 7.65 ± 0.47 | 25.5 ± 0.57 | 503 ± 16.2 |

*Table 19: Cell-bound and internalized ligand in correlation to total cellular activity and internalized activity of $^{99m}$Tc-CXCR4-14, -15 and -16 in comparison to $^{125}$I-Fc-131.*

| **Internalization of labeled compounds With internal standard $^{125}$I-Fc-131** | | | |
|---|---|---|---|
| **$^{99m}$Tc-CXCR4-14** | | | |
| **Time [min]** | **Cell-bound [%]** | **Internalized [%]** | **Internalized in relation to $^{125}$I-Fc-131 [%]** |
| 10 | 0.67 ± 0.14 | 17.9 ± 0.59 | 486 ± 18.9 |
| 30 | 1.56 ± 0.41 | 30.2 ± 0.74 | 683 ± 51.3 |
| 60 | 0.91 ± 0.14 | 36.4 ± 0.20 | 742 ± 45.2 |
| 120 | 1.11 ± 0.48 | 37.9 ± 0.56 | 694 ± 42.5 |
| **$^{99m}$Tc-CXCR4-15** | | | |
| **Time [min]** | **Cell-bound [%]** | **Internalized [%]** | **Internalized in relation to $^{125}$I-Fc-131 [%]** |
| **30** | 4.35 ± 0.16 | 12.6 ± 0.34 | 214 ± 11.7 |
| 90 | 3.42 ± 0.27 | 17.9 ± 1.06 | 232 ± 15.3 |
| **$^{99m}$Tc-CXCR4-16** | | | |
| **Time [min]** | **Cell-bound [%]** | **Internalized [%]** | **Internalized in relation to $^{125}$I-Fc-131 [%]** |
| 30 | 3.36 ± 0.45 | 15.0 ± 0.31 | 230 ± 10.7 |
| 90 | 3.53 ± 0.21 | 19.8 ± 0.15 | 284 ± 10.4 |

## 2. LUTETIUM-177/GALLIUM-68 THERANOSTICS

### *2.1 Chemical Structures*

Chemical Structures of DOTA-bearing peptides CXCR4-DOTA-1 to -5 are illustrated in the following.

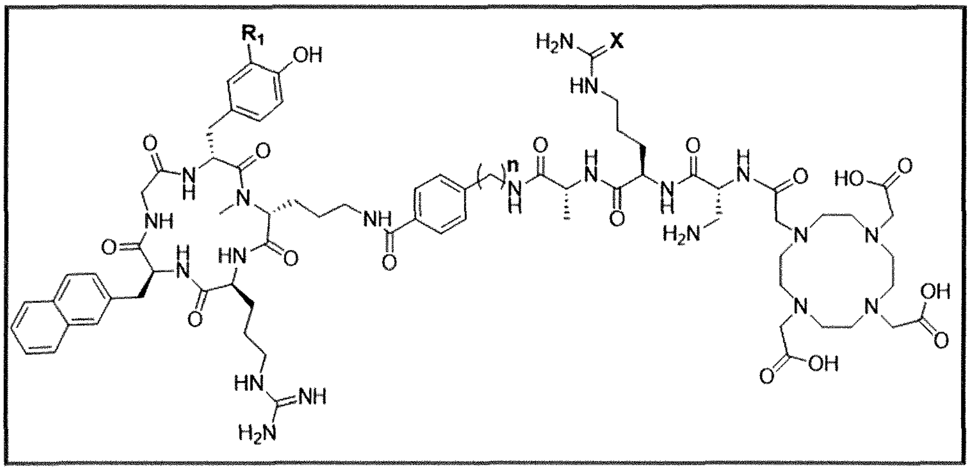

| | |
|---|---|
| $R_1$ | H, I |
| n | 0, 1 |
| X | NH, O |

*Table 20: Summary of structural modifications and assigned abbreviations for DOTA-bearing peptides CXCR4-DOTA-1 to -5.*

| n | $R_1$ | X | CXCR4-DOTA |
|---|---|---|---|
| 1 | H | NH | -1 |
| 1 | I | NH | -2 |
| 0 | H | NH | -3 |
| 0 | I | NH | -4 |
| 0 | H | O | -5 |

***2.2 In Vitro Data***

*Table 21: $IC_{50}$ values and $logD_{7.4}$ of $^{177}Lu$- and $^{67}Ga$-labeled compounds CXCR4-DOTA-1 to - 5 and Pentixather.*

| CXCR4-DOTA | Chelate | $IC_{50}$ [nM] (n = x) | $logD_{7.4}$ (n = x) | Internalization [%Fc-131]$_{2h}$ |
|---|---|---|---|---|
| -1 | / [Ga] [Lu] | 12.1 ± 2.42 (3) 2.95 ± 0.59 (3) 5.05 ± 1.75 (4) | / n.d. - 3.41 ± 0.05 (6) | / n.d. 133 |
| -2 | / | 7.03 ± 0.59 (3) | / | / |
| | [Ga] | 3.62 ± 1.57 (4) | n.d. | n.d. |
| | [Lu] | 6.47 ± 1.75 (4) | - 2.20 ± 0.06 (8) | 456 |
| -3 | / | 3.16 ± 0.63 (3) | / | / |
| | [Ga] | 1.64 ± 0.13 (3) | - 3.35 ± 0.08 (5) | n.d. |
| | [Lu] | 1.80 ± 0.11 (3) | - 3.35 ± 0.19 (6) | 366 |
| -4 | / | 4.64 ± 0.96 (3) | / | / |
| | [Ga] | 2.73 ± 1.45 (3) | - 2.65 ± 0.22 (6) | n.d. |
| | [Lu] | 3.67 ± 1.31 (3) | - 2.89 ± 0.10 (6) | 675 |

(continued)

| CXCR4-DOTA | Chelate | $IC_{50}$ [nM] (n = x) | $logD_{7.4}$ (n = x) | Internalization [%Fc-131]$_{2h}$ |
|---|---|---|---|---|
| -5 | / | 21.3 ± 6.64 (3) | / | / |
| | [Ga] | 12.9 ± 3.23 (3) | n.d. | n.d. |
| | [Lu] | 10.7 ± 3.59 (3) | - 3.58 ± 0.08 (8) | 190 |
| Pentixather | [Lu] | 19.5 ± 2.80 (3) | - 1.76 ± 0.03 (8) | 283 |

***2.3 Biodistribution Studies***

*Table 22: Biodistribution of $^{177}$Lu-labeled compounds Pentixather and CXCR4-DOTA-1 to -4 and $^{68}$Ga-labeled Pentixafor in female Jurkat tumor-bearing mice, 1h p.i.*

**1h p.i.**
**CB-17 SCID female mice, Jurkat xenograft (n = 5)**

| | $^{68}$Ga-Pentixafor | | $^{177}$Lu-Pentixather | | $^{177}$Lu-CXCR4-DOTA-1 | | $^{177}$Lu-CXCR4-DOTA-2 | | $^{177}$Lu-CXCR4-DOTA-3 | | $^{177}$Lu-CXCR4-DOTA-4 | |
|---|---|---|---|---|---|---|---|---|---|---|---|---|
| **Organ** | **%iD/ g** | **Std.** | **%iD/ g** | **Std.** | **%iD/ g** | **Std.** | **%iD/ g** | **Std.** | **%iD/ g** | **Std.** | **%iD/ g** | **Std.** |
| Blood | 1.29 | 0.41 | 1.43 | 0.43 | 1.24 | 0.20 | 1.67 | 0.33 | 0.91 | 0.26 | 1.72 | 0.34 |
| Heart | 0.56 | 0.23 | 0.86 | 0.27 | 0.73 | 0.13 | 1.04 | 0.20 | 0.53 | 0.15 | 1.13 | 0.17 |
| Lung | 1.57 | 0.57 | 2.19 | 0.59 | 2.16 | 0.51 | 2.94 | 0.58 | 1.67 | 0.42 | 3.69 | 0.80 |
| Liver | 1.59 | 0.64 | 8.02 | 1.83 | 11.0 | 1.42 | 17.7 | 2.52 | 8.48 | 1.69 | 19.2 | 1.89 |
| Pancreas | 0.14 | 0.05 | 0.40 | 0.10 | 0.39 | 0.08 | 0.53 | 0.13 | 0.28 | 0.08 | 0.52 | 0.10 |
| Spleen | 1.48 | 0.81 | 1.63 | 0.23 | 3.19 | 0.60 | 3.62 | 0.75 | 1.55 | 0.39 | 4.57 | 0.39 |
| Stomach | 0.51 | 0.23 | 1.31 | 0.28 | 1.06 | 0.44 | 1.41 | 0.41 | 0.94 | 0.41 | 1.35 | 0.24 |
| Intestine | 0.43 | 0.19 | 0.83 | 0.06 | 0.67 | 0.14 | 0.90 | 0.15 | 0.44 | 0.09 | 0.87 | 0.05 |
| Kidney | 2.75 | 1.10 | 3.87 | 0.61 | 18.8 | 1.92 | 15.7 | 2.77 | 14.1 | 0.54 | 14.4 | 1.85 |
| Muscle | 0.19 | 0.09 | 0.28 | 0.08 | 0.22 | 0.05 | 0.30 | 0.06 | 0.18 | 0.05 | 0.30 | 0.05 |
| Bone | 0.48 | 0.21 | 0.59 | 0.10 | 0.72 | 0.09 | 0.90 | 0.18 | 0.55 | 0.17 | 1.07 | 0.27 |
| Tumor | 7.58 | 2.97 | 5.87 | 1.88 | 5.98 | 0.81 | 5.27 | 0.83 | 8.42 | 1.12 | 6.19 | 0.52 |

*Table 23: Biodistribution of $^{177}$Lu-labeled compounds CXCR4-DOTA-1, -3 and -4 co-injected with 100 nmol AMD 3100 in female Jurkat tumor-bearing mice, 1h p.i.*

| **1h p.i. CB-17 SCID female mice, Jurkat xenograft (n = 1)** | | | |
|---|---|---|---|
| **+100 nmol AMD 3100** | **$^{177}$Lu-CXCR4-DOTA-1** | **$^{177}$Lu-CXCR4-DOTA-3** | **$^{177}$Lu-CXCR4-DOTA-4** |
| **Organ** | **%iD/g** | | |
| Blood | 1.39 | 1.04 | 2.21 |
| Heart | 0.80 | 0.55 | 1.38 |
| Lung | 2.21 | 1.71 | 4.26 |
| Liver | 4.20 | 2.37 | 7.19 |
| Pancreas | 0.46 | 0.31 | 0.78 |
| Spleen | 2.15 | 0.81 | 1.74 |
| Stomach | 1.22 | 1.22 | 2.38 |
| Intestine | 0.64 | 0.50 | 1.13 |
| Kidney | 21.0 | 15.5 | 17.7 |
| Muscle | 0.26 | 0.22 | 0.59 |
| Bone | 0.76 | 0.61 | 1.43 |
| Tumor | 1.72 | 2.11 | 3.44 |

*Table 24: Biodistribution of $^{177}$Lu-labeled compounds Pentixather, CXCR4-DOTA-3 and -4 in female Jurkat tumor-bearing mice, 6h p.i.*

| **6h p.i. CB-17 SCID female mice. Jurkat xenograft (n = 5)** | | | | | | |
|---|---|---|---|---|---|---|
| | **$^{177}$Lu-Pentixather** | | **$^{177}$Lu-CXCR4-DOTA-3** | | **$^{177}$Lu-CXCR4-DOTA-4** | |
| **Organ** | **%iD/g** | **Std.** | **%iD/g** | **Std.** | **%iD/g** | **Std.** |
| Blood | 0.05 | 0.02 | 0.03 | 0.01 | 0.05 | 0.01 |
| Heart | 0.15 | 0.02 | 0.09 | 0.01 | 0.22 | 0.02 |
| Lung | 0.36 | 0.07 | 0.25 | 0.04 | 0.60 | 0.09 |
| Liver | 10.3 | 1.25 | 9.43 | 1.24 | 20.5 | 1.86 |
| Pancreas | 0.11 | 0.01 | 0.09 | 0.01 | 0.16 | 0.04 |
| Spleen | 0.95 | 0.13 | 1.38 | 0.37 | 2.68 | 0.56 |
| Stomach | 0.74 | 0.52 | 0.25 | 0.06 | 0.41 | 0.16 |
| Intestine | 1.29 | 1.14 | 0.44 | 0.18 | 0.82 | 0.44 |
| Kidney | 1.75 | 0.11 | 13.4 | 2.68 | 11.3 | 2.06 |
| Muscle | 0.05 | 0.01 | 0.03 | 0.01 | 0.07 | 0.01 |
| Bone | 0.20 | 0.05 | 0.28 | 0.06 | 0.57 | 0.12 |
| Tumor | 5.53 | 1.00 | 6.88 | 1.16 | 7.78 | 1.32 |

*Table 25: Biodistribution of $^{177}$Lu-labeled compounds Pentixather, CXCR4-DOTA-3 and -4 in female Jurkat tumor-bearing mice, 48h p.i.*

| **48h p.i. CB-17 SCID female mice. Jurkat xenograft (n = 5)** | | | | | | |
|---|---|---|---|---|---|---|
| | **$^{177}$Lu-Pentixather** | | **$^{177}$Lu-CXCR4-DOTA-3** | | **$^{177}$Lu-CXCR4-DOTA-4** | |
| **Organ** | **%iD/g** | **Std.** | **%iD/g** | **Std.** | **%iD/g** | **Std.** |
| Blood | 0.01 | 0.00 | 0.00 | 0.00 | 0.02 | 0.00 |

(continued)

| 48h p.i. CB-17 SCID female mice. Jurkat xenograft (n = 5) | | | | | | |
|---|---|---|---|---|---|---|
| | $^{177}$Lu-Pentixather | | $^{177}$Lu-CXCR4-DOTA-3 | | $^{177}$Lu-CXCR4-DOTA-4 | |
| **Organ** | **%iD/g** | **Std.** | **%iD/g** | **Std.** | **%iD/g** | **Std.** |
| Heart | 0.11 | 0.01 | 0.06 | 0.01 | 0.21 | 0.02 |
| Lung | 0.20 | 0.04 | 0.14 | 0.02 | 0.47 | 0.06 |
| Liver | 8.12 | 0.93 | 8.84 | 0.76 | 21.7 | 3.03 |
| Pancreas | 0.09 | 0.02 | 0.05 | 0.01 | 0.24 | 0.08 |
| Spleen | 1.05 | 0.19 | 1.32 | 0.27 | 3.63 | 0.85 |
| Stomach | 0.12 | 0.02 | 0.07 | 0.01 | 0.23 | 0.05 |
| Intestine | 0.22 | 0.06 | 0.11 | 0.04 | 0.28 | 0.06 |
| Kidney | 0.96 | 0.09 | 4.47 | 0.44 | 5.31 | 1.02 |
| Muscle | 0.04 | 0.01 | 0.01 | 0.00 | 0.05 | 0.01 |
| Bone | 0.28 | 0.10 | 0.18 | 0.05 | 1.47 | 0.40 |
| Tumor | 3.46 | 0.11 | 5.44 | 0.42 | 7.23 | 0.71 |

*Table 26: Tumor uptake of $^{177}$Lu-labeled compounds CXCR4-DOTA-3 and -4 and $^{177}$Lu-labeled comparative compounds with a shorter linker*

| Cpd. | Tumor activity 1h p.i. | Tumor activity 6h p.i. | Tumor activity 48h p.i. |
|---|---|---|---|
| CXCR4-DOTA-3[1)] | 8.42 | 6.88 | 5.44 |
| DOTA-r-a-ABA-CPCR4[2)] | 18.3 | 13.6 | 8.81 |
| CXCR4-DOTA-4[1)] | 6.19 | 7.78 | 7.23 |
| DOTA-r-a-ABA-iodoCPCR4[3)] | 17.2 | 12.5 | 8.11 |

1) acitivty values reported in tables 23 to 25
2) comparative ligand compound[$^{177}$Lu]DOTA-r-a-ABA-CPCR4; data taken from (38), Supplementary Material, determined in Daudi xenograft bearing CB-17 SCID mice
3) comparative ligand compound [$^{177}$Lu]DOTA-r-a-ABA-iodoCPCR4; data taken from (38), Supplementary Material, determined in Daudi xenograft bearing CB-17 SCID mice,

*Table 27: Calculated tumor retention of $^{177}$Lu-labeled compounds CXCR4-DOTA-3 and -4 in %, and of $^{177}$Lu-labeled comparative compounds with a shorter linker, compared to the 1h-value (based on activity values in Table 26)*

| Cpd. | 6h | 48h |
|---|---|---|
| CXCR4-DOTA-3 | 82 | 65 |
| DOTA-r-a-ABA-CPCR4 | 74 | 48 |
| CXCR4-DOTA-4 | 126 | 117 |
| DOTA-r-a-ABA-iodoCPCR4 | 73 | 47 |

*Table 28: Tumor/Blood ratio (T/B) of $^{177}$Lu-labeled compounds CXCR4-DOTA-3 and -4, and of $^{177}$Lu-labeled comparative compounds with a shorter linker*

| Cpd. | T/B 1h p.i. | T/B | 48h T/B |
|---|---|---|---|
| CXCR4-DOTA-3[1)] | 9.25 | 229 | 1360 |
| DOTA-r-a-ABA-CPCR4[2)] | 12.2 | 272 | 881 |

(continued)

| Cpd. | T/B 1h p.i. | T/B | 48h T/B |
|---|---|---|---|
| CXCR4-DOTA-4[1)] | 3.6 | 156 | 362 |
| DOT A-r-a-ABA-iodoCPCR4[3)] | 5.93 | 73.5 | 270 |

[1)] calculated from activity values reported in tables 23 to 25
[2)] comparative ligand compound[$^{177}$Lu]DOTA-r-a-ABA-CPCR4; data taken from (38), Supplementary Material, determined in Daudi xenograft bearing CB-17 SCID mice
[3)] comparative ligand compound [$^{177}$Lu]DOTA-r-a-ABA-iodoCPCR4; data taken from (38), Supplementary Material, determined in Daudi xenograft bearing CB-17 SCID mice,

### *2.4 Mouse Imaging Studies*

Figure 8 shows the MIP of both CT and SPECT Scans of $^{177}$Lu-CXCR4-DOTA-1 1h p.i. in female Jurkat tumor-bearing mice without (left) and with competitor (right, 100 nmol AMD 3100) from 2 - 10%iD/mL; white arrows indicate organs of interest: solid = tumor, points = kidney, dashed = liver.
Figure 9 shows the MIP of both CT and SPECT Scans of $^{177}$Lu-CXCR4-DOTA-2 1h p.i. in female Jurkat tumor-bearing mice without competitor from 2 - 10%iD/mL; white arrows indicate organs of interest: solid = tumor, points = kidney, dashed = liver.
Figure 10 shows the MIP of both CT and SPECT Scans of $^{177}$Lu-CXCR4-DOTA-3 1h p.i. in female Jurkat tumor-bearing mice without (left) and with competitor (right, 100 nmol AMD 3100) from 2 - 10%iD/mL; white arrows indicate organs of interest: solid = tumor, points = kidney, dashed = liver.
Figure 11 shows the MIP of both CT and SPECT Scans of $^{177}$Lu-CXCR4-DOTA-4 1h p.i. in female Jurkat tumor-bearing mice without (left) and with competitor (right, 100 nmol AMD 3100) from 2 - 10%iD/mL; white arrows indicate organs of interest: solid = tumor, points = kidney, dashed = liver.
Figure 12 shows the MIP of both CT and SPECT Scans of $^{177}$Lu-CXCR4-DOTA-4 6h p.i. in female Jurkat tumor-bearing mice without competitor from 2 - 10%iD/mL; white arrows indicate organs of interest: solid = tumor, points = kidney, dashed = liver.

### *2.5 Internalization Studies*

*Table 29: Cell-bound and internalized ligand in correlation to total cellular activity and internalized activity of $^{177}$Lu-Pentixather and $^{177}$Lu-CXCR4-DOTA -1 to -5 in comparison to $^{125}$I-Fc-131.*

| Internalization of labeled compounds With internal standard $^{125}$I-Fc-131 | | | |
|---|---|---|---|
| **$^{177}$Lu-Pentixather** | | | |
| **Time [min]** | **Cell-bound [%]** | **Internalized [%]** | **Internalized in relation to $^{125}$I-Fc-131 [%]** |
| 15 | 8.27 ± 0.42 | 3.11 ± 0.15 | 142 ± 10.8 |
| 30 | 12.1 ± 1.27 | 5.75 ± 0.61 | 186 ± 21.0 |
| 60 | 12.2 ± 1.26 | 7.28 ± 1.39 | 197 ± 39.4 |
| 120 | 8.83 ± 0.44 | 11.4 ± 0.74 | 283 ± 21.1 |
| **$^{177}$Lu-CXCR4-DOTA-1** | | | |
| **Time [min]** | **Cell-bound [%]** | **Internalized [%]** | **Internalized in relation to $^{125}$I-Fc-131 [%]** |
| 15 | 4.27 ± 0.38 | 2.61 ± 0.20 | 78.3 ± 6.93 |
| 30 | 4.43 ± 0.08 | 3.28 ± 0.27 | 95.0 ± 9.39 |
| 60 | 6.02 ± 0.67 | 5.04 ± 0.92 | 109 ± 24.6 |
| 120 | 4.97 ± 0.31 | 7.08 ± 0.22 | 133 ± 7.31 |

(continued)

| Time [min] | Cell-bound [%] | Internalized [%] | Internalized in relation to $^{125}$I-Fc-131 [%] |
|---|---|---|---|
| **$^{177}$Lu-CXCR4-DOTA-2** | | | |
| **Time [min]** | **Cell-bound [%]** | **Internalized [%]** | **Internalized in relation to $^{125}$I-Fc-131 [%]** |
| 15 | 8.31 ± 1.20 | 11.2 ± 1.93 | 247 ± 26.2 |
| 30 | 13.5 ± 1.38 | 17.8 ± 2.02 | 347 ± 36.1 |
| 60 | 16.4 ± 1.31 | 21.8 ± 1.10 | 368 ± 36.9 |
| 120 | 22.2 ± 2.68 | 27.3 ± 3.62 | 456 ± 76.3 |
| **$^{177}$Lu-CXCR4-DOTA-3** | | | |
| **Time [min]** | **Cell-bound [%]** | **Internalized [%]** | **Internalized in relation to $^{125}$I-Fc-131 [%]** |
| 15 | 6.46 ± 1.07 | 15.2 ± 0.99 | 180 ± 32.6 |
| 30 | 9.88 ± 0.54 | 22.2 ± 0.78 | 259 ± 23.0 |
| 60 | 13.4 ± 0.84 | 33.4 ± 0.87 | 289 ± 22.4 |
| 120 | 17.1 ± 2.69 | 38.4 ± 1.78 | 366 ± 69.5 |
| **$^{177}$Lu-CXCR4-DOTA-4** | | | |
| **Time [min]** | **Cell-bound [%]** | **Internalized [%]** | **Internalized in relation to $^{125}$I-Fc-131 [%]** |
| 15 | 16.0 ± 0.58 | 20.7 ± 0.59 | 381 ± 16.2 |
| 30 | 24.6 ± 1.38 | 30.6 ± 1.56 | 588 ± 34.1 |
| 60 | 32.4 ± 0.60 | 39.3 ± 0.52 | 685 ± 50.1 |
| 120 | 37.2 ± 2.16 | 53.23 ± 1.06 | 675 ± 47.4 |
| **$^{177}$Lu-CXCR4-DOTA-5** | | | |
| **Time [min]** | **Cell-bound [%]** | **Internalized [%]** | **Internalized in relation to $^{125}$I-Fc-131 [%]** |
| 15 | 3.47 ± 0.46 | 3.52 ± 0.31 | 97.3 ± 8.78 |
| 30 | 4.66 ± 0.45 | 5.80 ± 0.14 | 151 ± 4.51 |
| 60 | 5.21 ± 0.48 | 6.94 ± 0.59 | 151 ± 15.9 |
| 120 | 3.94 ± 0.45 | 9.65 ± 0.16 | 190 ± 13.0 |

## 3. FLUORINE-18 PET TRACER/RADIOHYBRIDS

### *3.1 Chemical Structures*

Chemical Structures of SiFA-bearing peptides and radiohybrids are illustrated below

Type A

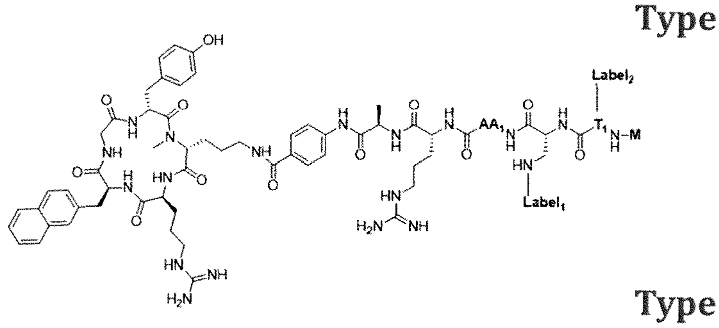

Type B

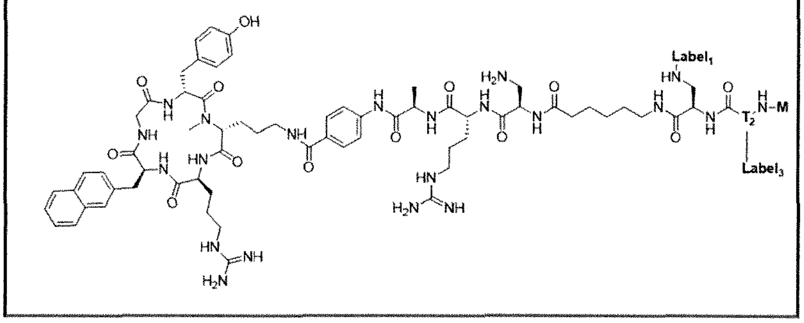

$AA_1$

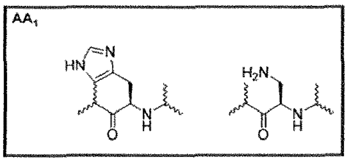

$T_1$

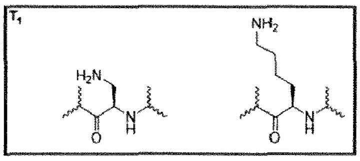

$Label_1$

$Label_2$

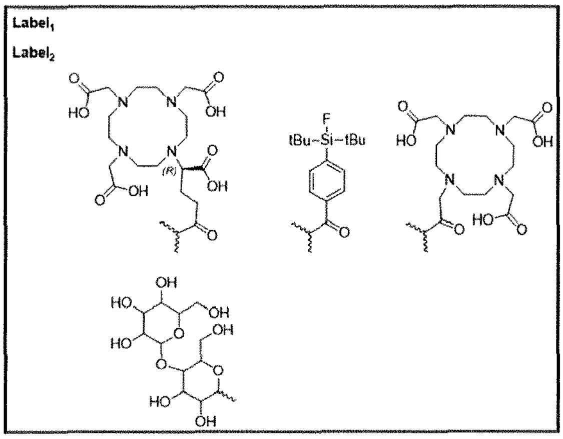

$T_2$

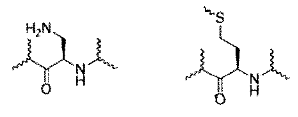

Label3

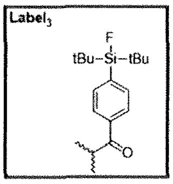

M

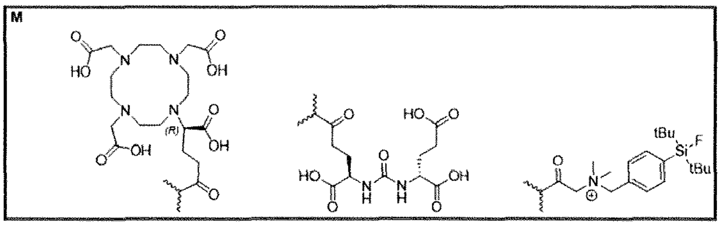

*Table 30: Summary of structural modifications and assigned abbreviations for SiFA-bearing peptides CXCR4-SiFA-1 to -7.*

| Type | $AA_1$ | $Label_1$ | $T_1$ | $Label_2$ | $T_2$ | $Label_3$ | M | CXCR4-SiFA |
|---|---|---|---|---|---|---|---|---|
| A | / | SiFA-BA | / | / | / | / | R-DOTAGA | -1* |
| A | d-his | SiFA-BA | / | / | / | / | R-DOTAGA | -2 |
| A | d-dap | SiFA-BA | d-lys | R-DOTAGA | / | / | R-DOTAGA | -3 |
| A | d-dap | R-DOTAGA | d-dap | SiFA-BA | / | / | eue | -4 |
| B | / | R-DOTAGA | / | / | d-dap | SiFA-BA | eue | -5 |
| B | / | R-DOTAGA | / | / | / | / | SiFA-lin | -6 |
| B | / | DOTA | / | / | D-HCy | β-D-Lactosyl | SiFA-lin | -7 |
| *compound provided for comparative purposes | | | | | | | | |

### *3.2 In Vitro Data*

*Table 31: $IC_{50}$ values of cold complexed compounds and $logD_{7.4}$ and internalization values of hot compounds CXCR4-SiFA-1 to -7.*

| CXCR4-SiFA | Chelate | $IC_{50}$ [nM] (n = x) | $logD_{7.4}$ (n = x) | Internalization [%Fc-131]$_{90min}$ |
|---|---|---|---|---|
| -1 | [Ga] | 180 ± 27.4 (4) | 0.66 ± 0.01 (8) | n.d. |
| | [Lu] | 206 ± 14.4 (3) | 1.41 ± 0.04 (8) | n.d. |
| -2 | [Ga] | 28.5 ± 1.96 (3) | 0.39 ± 0.07 (8) | 562 |
| | [Lu] | 39.1 ± 5.14 (4) | 0.80 ± 0.04 (8) | n.d. |
| -3 | [Ga] | 23.7 ± 2.09 (3) | - 1.65 ± 0.09 (8) | n.d. |
| | [Lu] | 50.2 ± 5.68 (3) | n.d. | n.d. |
| -4 | [Ga] | 102 ± 30.8 (3) | - 1.92 ± 0.06 (8) | 58 |
| | [Lu] | 93.4 ± 10.2 (3) | - 1.71 ± 0.09 (8) | n.d. |
| -5 | [Ga] | 36.5 ± 4.51 (3) | - 1.85 ± 0.07 (7) | 209 |
| | [Lu] | 45.8 ± 17.4 (3) | - 1.73 ± 0.05 (6) | n.d. |
| -6 | [Ga] | 5.52 ± 0.71 (3) | - 1.05 ± 0.06 (8) | 980 |
| | [Lu] | 8.29 ± 3.11 (3) | - 0.85 ± 0.04 (6) | n.d. |
| -7 | [Ga] | 6.23 ± 1.57 (3) | - 1.95 ± 0.05 (8) | 1029 |
| | [Lu] | 6.05 ± 1.17 (4) | - 2.23 ± 0.11 (7) | n.d. |

### *3.3 Biodistribution Studies*

*Table 32: Biodistribution of $^{18}$F-labeled compound CXCR4-SiFa-7 in 5 female Jurkat tumor-bearing mice, 48h p.i.*

| $^{18}$F-CXCR4-SiFa-7, 1h p.i. CB-17 SCID female mice, Jurkat xenograft (n = 5) | | |
|---|---|---|
| **Organ** | **%iD/g** | **Std.** |
| Blood | 0.85 | 0.32 |
| Heart | 1.13 | 0.12 |
| Lung | 2.13 | 0.63 |
| Liver | 53.5 | 13.0 |
| Pancreas | 0.51 | 0.07 |
| Spleen | 6.96 | 2.21 |
| Stomach | 0.63 | 0.08 |
| Intestine | 0.81 | 0.12 |
| Kidney | 11.1 | 1.16 |
| Muscle | 0.26 | 0.04 |
| Bone | 2.89 | 0.87 |
| Tumor | 0.67 | 0.32 |

***3.4 Internalization Studies***

*Table 33: Cell-bound and internalized ligand in correlation to total cellular activity and internalized activity of $^{18}$F-GXGR4-SiFa-2 [$^{nat}$Ga] in comparison to $^{125}$I-Fc-131.*

| Internalization of labeled compounds With internal standard $^{125}$I-Fc-131 | | | |
|---|---|---|---|
| $^{18}$F-CXCR4-SiFa-2 [$^{nat}$Ga] | | | |
| **Time [min]** | **Cell-bound [%]** | **Internalized [%]** | **Internalized in relation to $^{125}$I-Fc-131 [%]** |
| 30 | 0.08 ± 0.23 | 12.5 ± 0.46 | 500 ± 23.1 |
| 90 | 0.43 ± 0.26 | 16.5 ± 0.94 | 562 ± 33.0 |

*Table 34: Cell-bound and internalized ligand in correlation to total cellular activity and internalized activity of $^{18}$F-CXCR4-SiFa-4 and -5 [$^{mat}$Ga] in comparison to $^{125}$I-Fc-131.*

| Internalization of labeled compounds With internal standard $^{125}$I-Fc-131 | | | |
|---|---|---|---|
| $^{18}$F-CXCR4-SiFa-4 [$^{nat}$Ga] | | | |
| **Time [min]** | **Cell-bound [%]** | **Internalized [%]** | **Internalized in relation to $^{125}$I-Fc-131 [%]** |
| 30 | 0.10 ± 0.15 | 1.36 ± 0.18 | 56.6 ± 8.36 |
| 90 | 0.37 ± 0.20 | 1.80 ± 0.15 | 57.7 ± 5.43 |
| $^{18}$F-CXCR4-SiFa-5 [$^{nat}$Ga] | | | |
| **Time [min]** | **Cell-bound [%]** | **Internalized [%]** | **Internalized in relation to $^{125}$I-Fc-131 [%]** |
| 30 | 0.66 ± 0.22 | 3.46 ± 0.57 | 233 ± 44.1 |
| 90 | 0.72 ± 0.34 | 3.70 ± 0.41 | 209 ± 27.2 |

*Table 35: Cell-bound and internalized ligand in correlation to total cellular activity and internalized activity of $^{18}$F-CXCR4-SiFa-6 and -7 [$^{nat}$Ga] in comparison to $^{125}$I-Fc-131.*

| Internalization of labeled compounds With internal standard $^{12S}$I-Fc-131 | | | |
|---|---|---|---|
| $^{18}$F-CXCR4-SiFa-6 [$^{nat}$Ga] | | | |
| **Time [min]** | **Cell-bound [%]** | **Internalized [%]** | **Internalized in relation to $^{125}$I-Fc-131 [%]** |
| 30 | 0 ± 0.29 | 37.0 ± 1.06 | 829 ± 48.1 |
| 90 | 0.81 ± 0.14 | 48.1 ± 1.26 | 980 ± 85.6 |
| $^{18}$F-CXCR4-SiFa-7 [$^{nat}$Ga] | | | |
| **Time [min]** | **Cell-bound [%]** | **Internalized [%]** | **Internalized in relation to $^{125}$I-Fc-131 [%]** |
| 30 | 0.11 ± 0.29 | 38.9 ± 1.09 | 837 ± 51.4 |
| 90 | 0 ± 0.41 | 50.0 ± 1.12 | 1029 ± 66.7 |

## 4. CYTOTOXINE-LINKED PEPTIDES

### *4.1 Chemical Structures*

Chemical Structures of cytotoxine-linked compounds CXCR4-MMAE-1 to -4 are illustrated in the following.

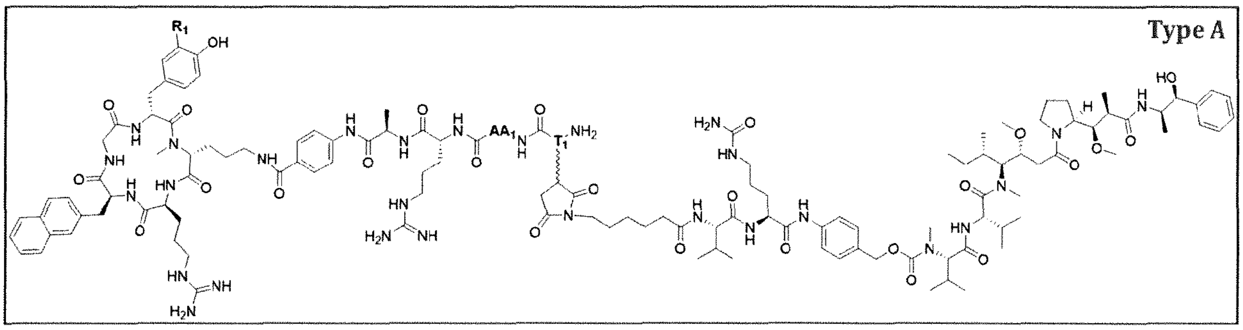

Type A

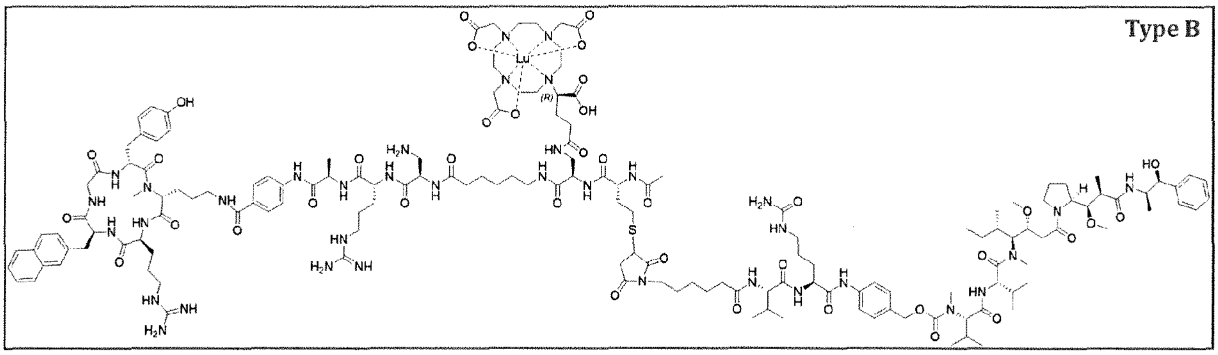

Type B

| | |
|---|---|
| $R_1$ | —H |
| | —I |
| $T_1$ | 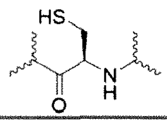 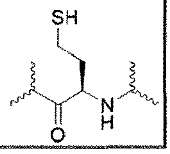 |

*Table 36: Summary of structural modifications and assigned abbreviations for cytotoxine-linked peptides CXCR4-MMAE-1 to -4.*

| Type | $AA_1$ | $T_1$ | $Label_1$ | CXCR4-MMAE |
|---|---|---|---|---|
| A | / | d-cys | mc-vc-PAB-MMAE | -1 |
| A | / | d-Hcy | mc-vc-PAB-MMAE | -2 |
| A | d-his | d-Hcy | mc-vc-PAB-MMAE | -3 |
| B | / | / | / | -4 |

### *4.2 In Vitro Data*

*Table 37: $IC_{50}$ values of unlabeled compounds and $logD_{7.4}$ and internalization of $^{125}I$- and $^{177}Lu$-labeled compounds CXCR4-MMAE-1 to -4.*

| CXCR4-MMAE | $IC_{50}$ [nM] (n = x) | $logD_{7.4}$ $^{125}I/^{177}Lu$-labeled (n = x) | $Internalization_{2h}$ [% of bound peptide] |
|---|---|---|---|
| -1 | 99.8 ± 7.55 (3) | n.d. | n.d. |
| -2 | 42.6 ± 8.30 (3) | 1.11 ± 0.08 (5) | 27.9 ± 1.14 |
| -3 | 43.9 ± 6.18 (3) | 0.80 ± 0.12 (6) | 24.9 ± 0.85 |
| -4 | tbd | - 1.04 ± 0.07 (8) | tbd |

### *4.3 Biodistribution Studies*

*Table 38: Biodistribution of $^{125}I$-labeled compound CXCR4-MMAE-2 in 5 female Jurkat tumor-bearing mice, 1h p.i.*

| 1h p.i. CB-17 SCID female mice. Jurkat xenograft (n = 5) | | |
|---|---|---|
| | $^{125}I$-CXCR4-MMAE-2 | |
| Organ | %iD/g | Std |
| Blood | 2.28 | 0.07 |
| Heart | 1.31 | 0.13 |
| Lung | 3.67 | 0.48 |
| Liver | 27.8 | 2.50 |
| Pancreas | 0.80 | 0.09 |
| Spleen | 4.31 | 0.78 |
| Stomach | 1.85 | 0.32 |
| Intestine | 1.13 | 0.14 |
| Kidney | 18.3 | 1.55 |

(continued)

| 1h p.i. CB-17 SCID female mice. Jurkat xenograft (n = 5) | | |
|---|---|---|
| | $^{125}$I-CXCR4-MMAE-2 | |
| Organ | %iD/g | Std |
| Muscle | 0.33 | 0.04 |
| Bone | 0.80 | 0.14 |
| Tumor | 2.41 | 0.43 |

### *4.4 Internalization Studies*

*Table 39: Cell-bound and internalized ligand in correlation to total cellular activity of $^{125}$I-CXCR4-MMAE-2 and -3.*

| Internalization of labeled compound | | |
|---|---|---|
| $^{125}$I-CXCR4-MMAE-2 | | |
| Time [min] | Cell-bound [%] | Internalized [%] |
| 10 | 1.56 ± 0.78 | 2.55 ± 0.34 |
| 30 | 2.09 ± 0.64 | 7.78 ± 0.58 |
| 60 | 0.73 ± 0.54 | 14.6 ± 0.89 |
| 120 | 0.27 ± 0.35 | 27.9 ± 1.14 |
| $^{125}$I-CXCR4-MMAE-3 | | |
| Time [min] | Cell-bound [%] | Internalized [%] |
| 10 | 1.42 ± 0.56 | 3.39 ± 0.36 |
| 30 | 1.63 ± 0.36 | 8.12 ± 1.59 |
| 60 | 1.12 ± 0.57 | 15.3 ± 1.59 |
| 120 | 0.12 ± 0.53 | 24.9 ± 0.85 |

## 5. OPTICAL IMAGING COMPOUNDS

### *5.1 Chemical Structures*

Chemical Structures of optical imaging compounds CXCR4-OI-1 to -3 are illustrated in the following.

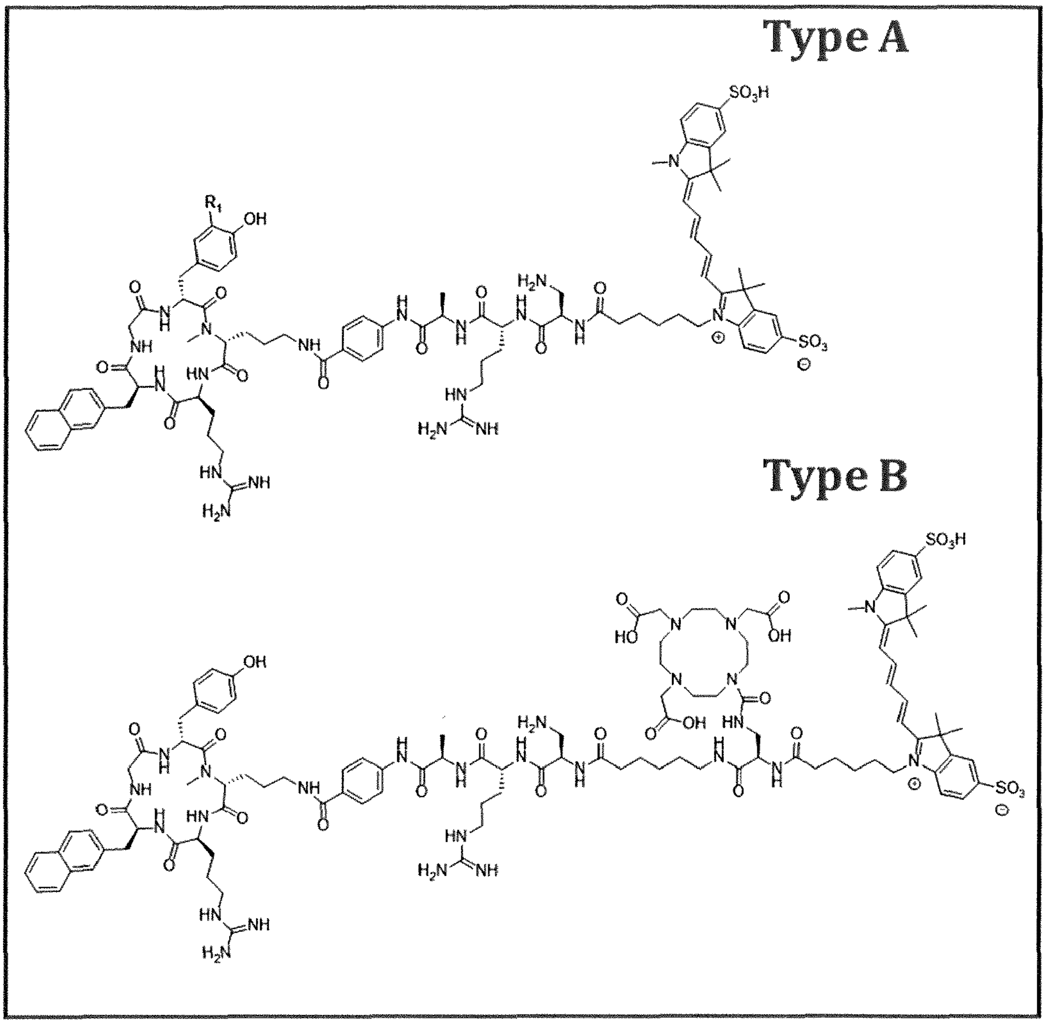

Type A

Type B

| $R_1$ | H, I |
|---|---|

*Table 40: Summary of structural modifications and assigned abbreviations for optical imaging compounds CXCR4-OI-1 to -3.*

| Type | $R_1$ | $Label_1$ | Dye | CXCR4-OI |
|---|---|---|---|---|
| A | H | / | Cy5.5 | -1 |
| A | I | / | Cy5.5 | -2 |
| B | / | / | Cy5.5 | -3 |

### *5.2 In Vitro Data*

*Table 41: $IC_{50}$ values of cold compounds and $logD_{7.4}$ values of $^{125}I$- and $^{177}Lu$-labeled compounds CXCR4-OI-1 to -3.*

| Compound | $IC_{50}$ [nM] | $logD_{7.4}$ | Internalization |
|---|---|---|---|
| | (n = x) | $^{125}I/^{177}Lu$-labeled (n = x) | [%Fc-131]$_{90min}$ |
| OI-1 | 4.43 ± 2.57 (4) | n.d. | n.d. |
| OI-2 | 10.2 ± 3.95 (3) | - 1.83 ± 0.02 (5) | n.d. |
| OI-3 | 7.34 ± 1.17 (3) [Lu] | - 3.38 ± 0.18 (6) | 540 [$^{177}Lu$] |

### *5.3 Biodistribution Studies*

*Table 42: Biodistribution of $^{125}$I-labeled compound CXCR4-OI-2 in 5 female Jurkat tumor-bearing mice, 1h p.i.*

| 1h p.i. CB-17 SCID female mice, Jurkat xenograft (n = 5) | | |
|---|---|---|
| | $^{125}$I-CXCR4-OI-2 | |
| **Organ** | **%iD/g** | **Std.** |
| Blood | 1.73 | 0.14 |
| Heart | 1.84 | 0.19 |
| Lung | 5.54 | 1.80 |
| Liver | 35.3 | 0.97 |
| Pancreas | 1.10 | 0.07 |
| Spleen | 16.1 | 2.32 |
| Stomach | 4.01 | 0.94 |
| Intestine | 1.88 | 0.11 |
| Kidney | 13.9 | 0.45 |
| Thyroid | 1.46 | 0.46 |
| Muscle | 0.41 | 0.10 |
| Bone | 1.20 | 0.10 |
| Tumor | 1.10 | 0.06 |

*Table 43: Biodistribution of $^{177}$Lu-labeled compound CXCR4-OI-3 in 3 female Jurkat tumor-bearing mice, 1h p.i.*

| 1h p.i. CB-17 SCID female mice, Jurkat xenograft (n = 3) | | |
|---|---|---|
| | $^{177}$Lu-CXCR4-OI-3 | |
| **Organ** | **%iD/g** | **Std.** |
| Blood | 1.90 | 1.05 |
| Heart | 1.39 | 0.18 |
| Lung | 4.28 | 1.34 |
| Liver | 36.3 | 7.00 |
| Pancreas | 0.44 | 0.16 |
| Spleen | 5.94 | 0.93 |
| Stomach | 1.43 | 0.23 |
| Intestine | 0.85 | 0.10 |
| Kidney | 35.0 | 1.83 |
| Muscle | 0.33 | 0.12 |
| Bone | 1.10 | 0.65 |
| Tumor | 2.13 | 0.74 |

### *5.4 Internalization Studies*

*Table 44: Cell-bound and internalized ligand in correlation to total cellular activity and internalized activity of $^{171}$Lu-CXCR4-OI-3 in comparison to $^{125}$I-Fc-131.*

| Internalization of labeled compounds With internal standard $^{125}$I-Fc-131 | | | |
|---|---|---|---|
| $^{177}$Lu-CXCR4-OI-3 | | | |
| **Time [min]** | **Cell-bound [%]** | **Internalized [%]** | **Internalized in relation to $^{125}$I-Fc-131 [%]** |
| 30 | 5.39 ± 0.41 | 16.5 ± 0.19 | 442 ± 12.1 |
| 90 | 5.36 ± 0.47 | 25.2 ± 1.08 | 540 ± 42.7 |

## Claims

**1.** A compound of formula (I) or a salt thereof:

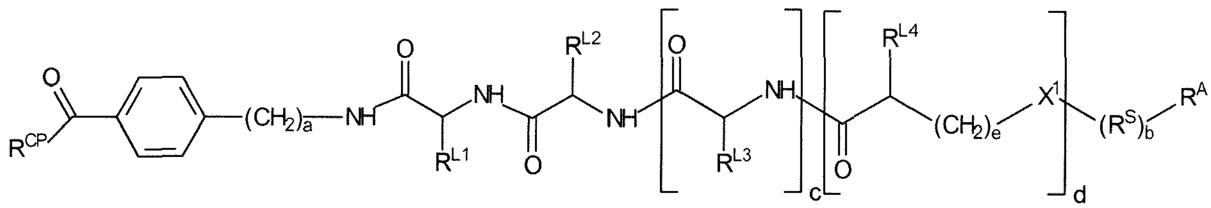

(I)

wherein:

a is 0 or 1;
b is 0 or 1;
c is 0 or 1, and
d is 0 or 1, with the proviso that at least one of c and d is 1;
e is an integer of 1 to 4;
$R^{CP}$ is a cyclopeptide group of formula (II):

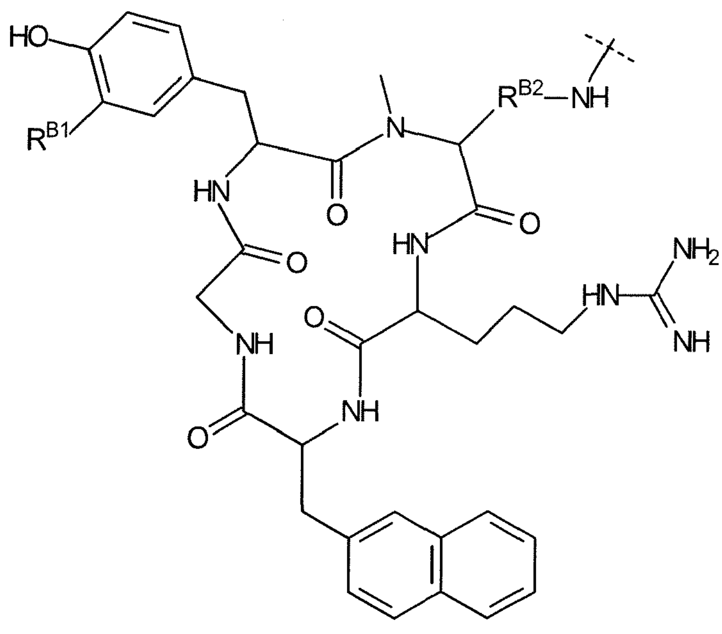

(II)

wherein, in formula (II)

$R^{B1}$ is H or I;
$R^{B2}$ is an alkanediyl chain;

and wherein the dashed line marks a bond which attaches the group $R^{CP}$ to the remainder of the compound of formula (I);
$R^{L1}$ is H or alkyl;
$R^{L2}$ is substituted alkyl, which substituted alkyl is substituted with at least one group selected from $-NH_2$ and $-NH-C(=X)-NH_2$ with X being selected from NH and O;
$R^{L3}$ is $-CH_2-NH_2$ or $-CH_2$-(1*H*-imidazol-4-yl);
$R^{L4}$ is $-NH_2$;
$X^1$ is a coupling group;
$R^S$ is a divalent spacer group; and
$R^A$ is a functional group comprising a moiety with diagnostic or therapeutic utility.

**2.** The compound or salt of claim 1, wherein the group $R^{L1}$ in formula (I) is H or C1-C6 alkyl, more preferably H or C1-3 alkyl.

**3.** The compound or salt of claim 1 or 2, wherein $R^{L2}$ in formula (I) is C1-C6 alkyl, more preferably C1-C4 alkyl, and still more preferably C2-C4 alkyl, carrying one substituent which is selected from $-NH_2$ and the group $-NH-C(=X)-NH_2$, wherein X is NH or O.

**4.** The compound or salt of any of claims 1 to 3, wherein $X^1$ in formula (I) is -S-.

**5.** The compound or salt of any of claims 1 to 4, wherein c in formula (I) is 1 and d in formula (I) is 0.

**6.** The compound or salt of any of claims 1 to 5, wherein $R^S$ in formula (I) is $-C(O)-(CH_2)_B-NH-$, wherein B is an integer of 3 to 10, preferably 4 to 6, and wherein the bond at the N-terminus is attached to $R^A$.

**7.** The compound of or salt any of claims 1 to 6, wherein the moiety with diagnostic or therapeutic utility comprised by $R^A$ in formula (I) is selected from:

(i) a chelating moiety;
(ii) a chelate formed by a chelating moiety (i) with a chelated radioactive or non-radioactive cation or anion, preferably a chelated radioactive or non-radioactive cation;
(iii) a silicon-fluoride acceptor (SiFA) moiety which comprises a silicon atom and a fluorine atom, wherein the fluorine atom is linked via a covalent bond directly to the silicon atom, and which SiFA moiety can be labeled with $^{18}F$ by isotopic exchange of $^{19}F$ by $^{18}F$ or which is labeled by $^{18}F$;
(iv) a cytotoxic moiety; and
(v) a fluorescent moiety.

**8.** The compound or salt of claim 7, wherein the chelating moiety referred to in (i) and (ii) is a chelating moiety which is suitable as a chelate ligand for a cation selected from the cations of $^{43}Sc$, $^{44}Sc$, $^{47}Sc$, $^{51}Cr$, $^{52m}Mn$, $^{58}Co$, $^{52}Fe$, $^{56}Ni$, $^{57}Ni$, $^{nat}Cu$, $^{62}Cu$, $^{64}Cu$, $^{67}Cu$, $^{66}Ga$, $^{nat}Ga$, $^{68}Ga$, $^{67}Ga$, $^{89}Zr$, $^{90}Y$, $^{86}Y$, $^{94m}Tc$, $^{99m}Tc$, $^{97}Ru$, $^{105}Rh$, $^{109}Pd$, $^{111}Ag$,$^{110m}In$, $^{111}In$ $^{113m}In$, $^{114m}In$, $^{117m}Sn$, $^{121}Sn$, $^{127}Te$, $^{142}Pr$, $^{143}Pr$, $^{147}Nd$, $^{149}Gd$, $^{149}Pm$, $^{151}Pm$, $^{149}Tb$, $^{152}Tb$, $^{155}Tb$, $^{153}Sm$, $^{156}Eu$, $^{157}Gd$, $^{161}Tb$, $^{164}Tb$, $^{161}Ho$, $^{166}Ho$, $^{157}Dy$, $^{165}Dy$, $^{166}Dy$, $^{160}Er$, $^{165}Er$, $^{169}Er$, $^{171}Er$, $^{166}Yb$, $^{169}Yb$, $^{175}Yb$, $^{167}Tm$, $^{172}Tm$, $^{nat}Lu$, $^{177}Lu$, $^{186}Re$, $^{188}Re$, $^{188}W$, $^{191}Pt$, $^{195m}Pt$, $^{194}Ir$, $^{197}Hg$, $^{198}Au$, $^{199}Au$, $^{nat}Pb$, $^{212}Pb$, $^{203}Pb$, $^{211}At$, $^{212}Bi$, $^{213}Bi$, $^{223}Ra$, $^{224}Ra$, $^{225}Ac$, and $^{227}Th$, and from a cationic molecule comprising $^{18}F$, such as $^{18}F-[AlF]^{2+}$.

**9.** The compound or salt of claim 7 or 8, wherein the chelating moiety referred to in (i) and (ii) is provided by a chelating agent selected from bis(carboxymethyl)-1,4,8,11-tetraazabicyclo[6.6.2]hexadecane (CBTE2a), cyclohexyl-1,2-diaminetetraacetic acid (CDTA), 4-(1,4,8,11-tetraazacyclotetradec-1-yl)-methylbenzoic acid (CPTA), N'-[5-[acetyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxymethyl)-1,4,8,11-tetraazabicycle[6.6.2]hexadecan (DO2A), 1,4,7,10-tetraazacyclododecan-N,N',N'',N'''-tetraacetic acid (DOTA), 2-[1,4,7,10-tetraazacyclododecane-4,7,10-triacetic acid]-pentanedioic acid (DOTAGA or DOTA-GA), N,N'-dipyridoxylethylendiamine-N,N'-diacetate-5,5'-bis(phosphat) (DPDP), diethylenetriaminepentaacetic acid (DTPA), ethylenediamine-N,N'-tetraacetic acid (EDTA), ethyleneglykol-O,O-bis(2-aminoethyl)-N,N,N',N'-tetraacetic acid (EGTA), N,N-bis(hydroxybenzyl)-ethylenediamine-N,N'-diacetic acid (HBED), hydroxyethyldiaminetriacetic acid (HEDTA), 1-(p-nitrobenzyl)-1,4,7,10-tetraazacyclodecan-4,7,10-triacetate (HP-DOA3), 1,4,7-triazacyclononan-1-succinic acid-4,7-diacetic acid (NODASA), 1-(1-carboxy-3-carboxypropyl)-4,7-(carboxy)-1,4,7-triazacyclononane (NODAGA), 1,4,7-triazacyclononanetriacetic acid (NOTA), 4,11-bis(carboxymethyl)-1,4,8,11-tetraazabicyclo[6.6.2]hexadecane (TE2A), 1,4,8,11-tetraazacyclododecane-1,4,8,11-tetraacetic acid (TETA), terpyridine-bis(methyleneamine) tetraacetic acid (TMT), 1,4,7,10-tetraazacyclotridecan-N,N',N'',N'''-tetraacetic acid (TRITA), and triethylenetetraaminehexaacetic acid (TTHA), *N,N'*-bis[(6-carboxy-2-pyridyl)methyl]-4,13-diaza-18-crown-6 ($H_2$macropa), 4-amino-4-{2-[(3-hydroxy-1,6-dimethyl-4-oxo-1,4-dihydro-pyridin-2-ylmethyl)-carbamoyl]-ethyl} heptanedioic acid bis-[(3-hydroxy-1,6-dimethyl-4-oxo-1,4-dihydro-pyridin-2-ylmethyl)-amide] (THP), 1,4,7-triazacyclononane-1,4,7-tris[methylene(2-carboxyethyl)phosphinic acid (TRAP), 2-(4,7,10-tris(2-amino-2-oxoethyl)-1,4,7,10-tetraazacyclododecan-1-yl)acetic acid (DO3AM), and 1,4,7,10-tetraazacyclododecane-1,4,7, 10-tetrakis[methylene(2-carboxyethylphosphinic acid)] (DOTPI), S-2-(4-isothiocyanatobenzyl)-1,4,7,10-tetraazacyclododecane tetraacetic acid, mercaptoacetyltriserine ($mas_3$), hydrazinonicotinic acid (HYNIC) and 3-(2-aminoethylamino)-2-[(2-aminoethylamino)methyl]propanoic acid (N4 chelator, 6-carboxy-1,4,8,11-tetraazaundecane), or by a modified mercaptoacetylserine chelating agent, wherein one or more of the serine residues are replaced by another amino acid containing a hydrophilic side chain.

**10.** The compound or salt of any of claims 7 to 9, wherein the SiFA moiety (iii) comprises a group of formula (S-1):

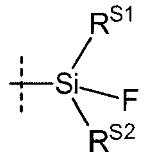

(S-1)

wherein
$R^{S1}$ and $R^{S2}$ are independently a linear or branched C3 to C10 alkyl group, preferably $R^{S1}$ and $R^{S2}$ are independently selected from isopropyl and tert-butyl, and more preferably $R^{S1}$ and $R^{S2}$ are both tert-butyl, and wherein the bond marked by the dashed line attaches the group to the remainder of the compound of formula (I).

**11.** The compound or salt of claim 10, wherein the SiFA moiety (iii) is selected from a group of formula (S-2) and a group of formula (S-3):

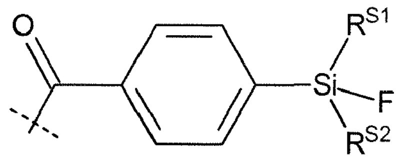

(S-2)

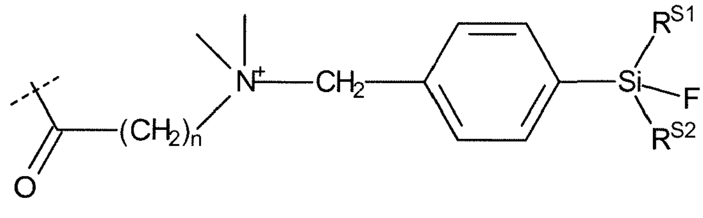

(S-3)

wherein
n is 1, 2, or 3 and is preferably 1, $R^{S1}$ and $R^{S2}$ are independently a linear or branched C3 to C10 alkyl group, preferably $R^{S1}$ and $R^{S2}$ are independently selected from isopropyl and *tert*-butyl, and more preferably $R^{S1}$ and $R^{S2}$ are both *tert*-butyl, and wherein the bond marked by the dashed line attaches the group to the remainder of the compound of formula (I).

**12.** The compound or salt of any of claims 7 to 11, wherein the cytotoxic moiety (iv) is provided by a residue of an auristatin analogue, preferably selected from monomethyl auristatin E (MMAE) and monomethyl auristatin F (MMAF), or by a residue of PF-06380101.

**13.** The compound or salt of any of claims 7 to 12, wherein the fluorescent moiety (v) is provided by a residue of a fluorescent dye, preferably a Cy5- or Cy7-based cyanine dye.

**14.** A pharmaceutical or diagnostic composition comprising or consisting of a compound or salt of any of claims 1 to 13.

**15.** A compound or salt of any of claims 1 to 13 for use in the treatment or prevention of cancer, a cardiovascular disorder or an inflammatory disorder, or for use in a method of diagnosis *in vivo* of cancer, a cardiovascular disorder or an inflammatory disorder.

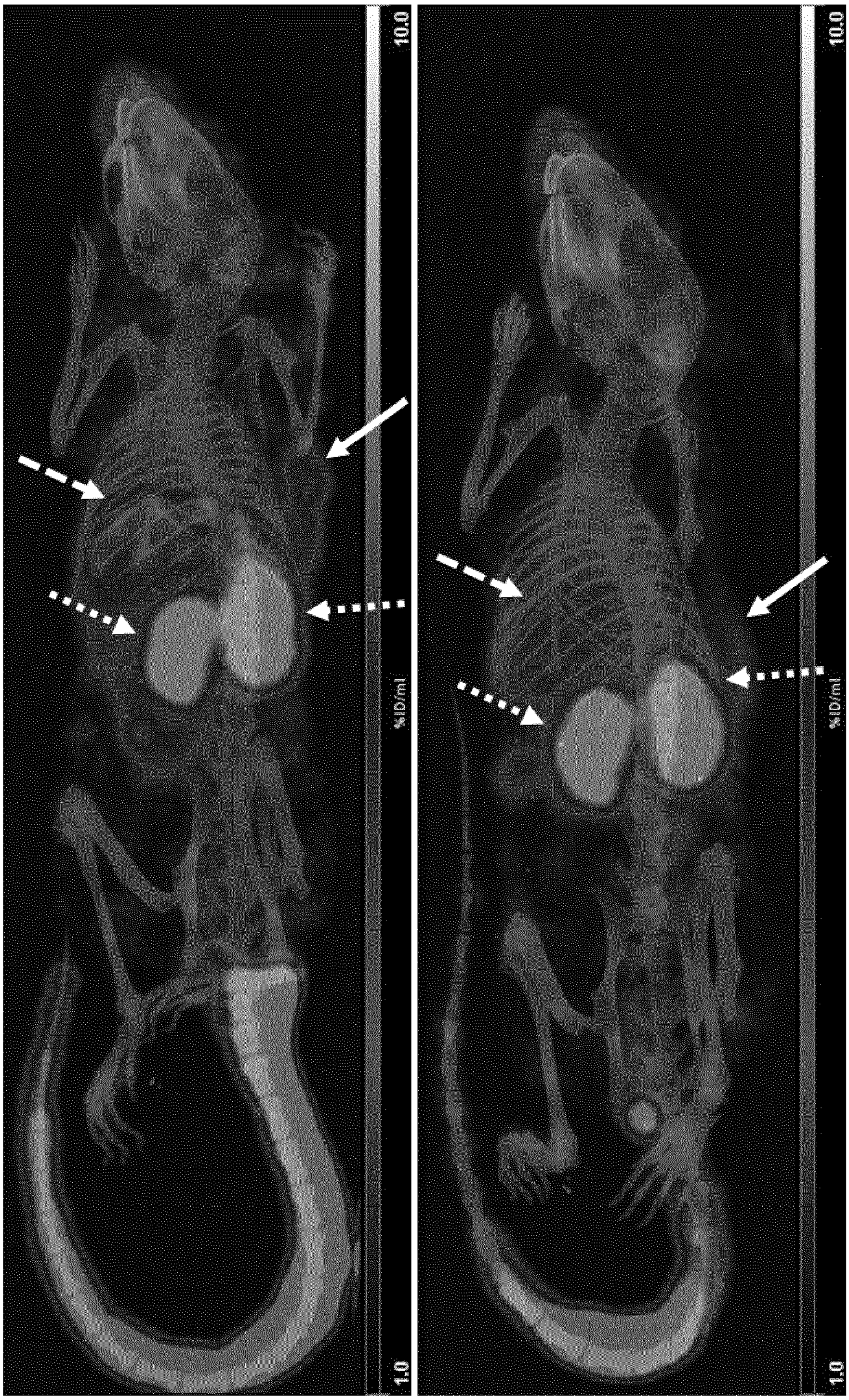


Figure 1

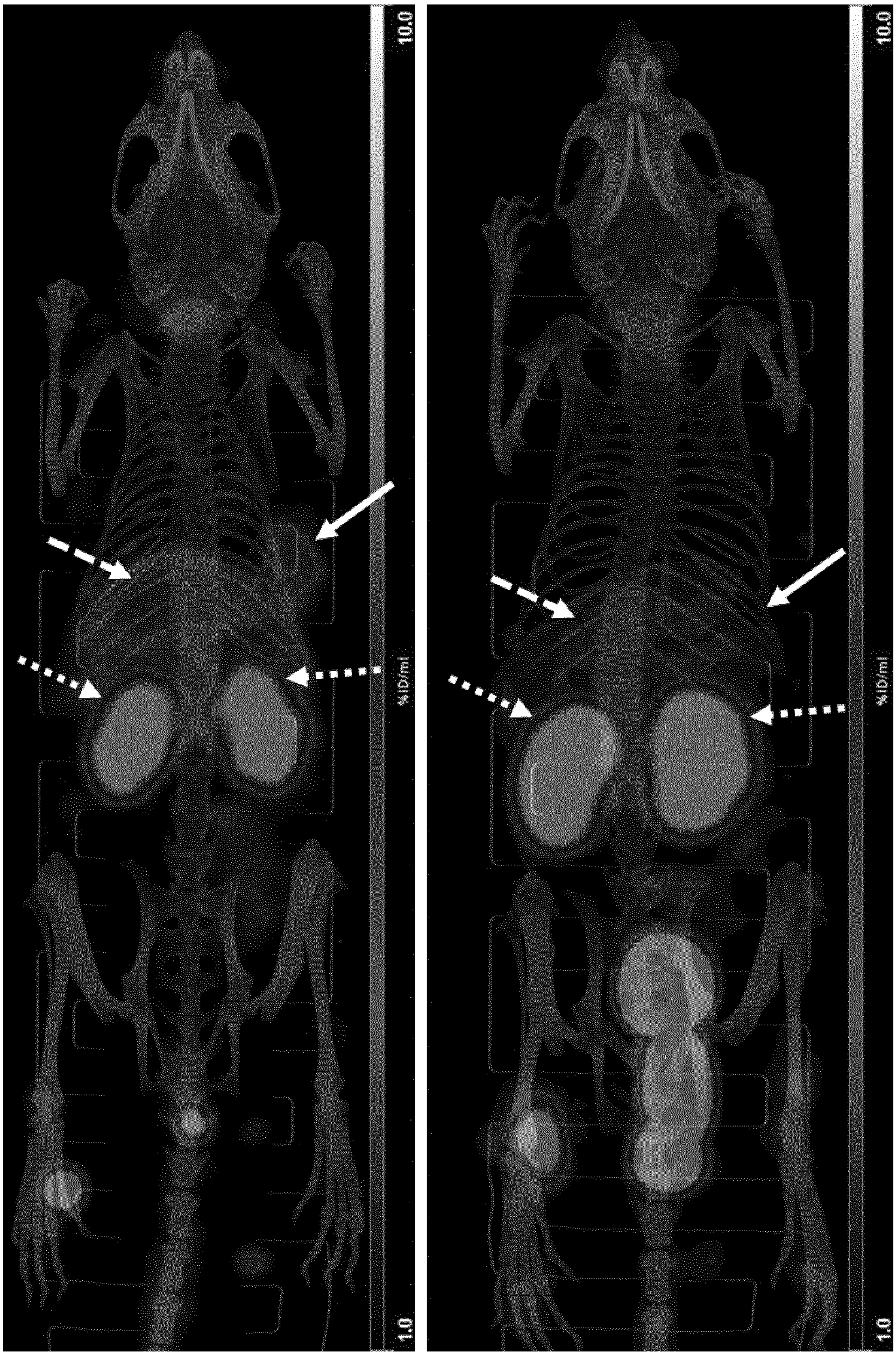


Figure 2

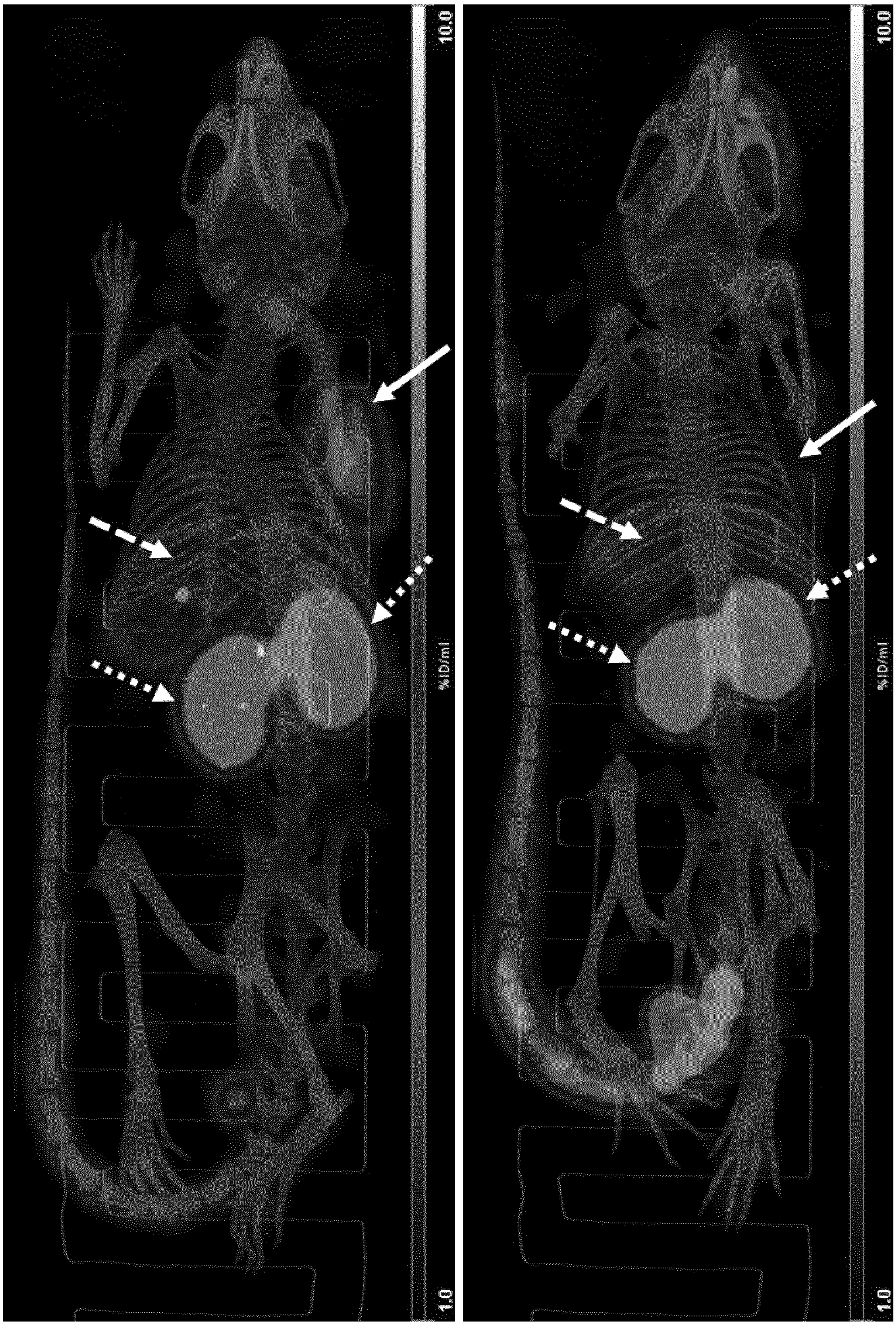


Figure 3

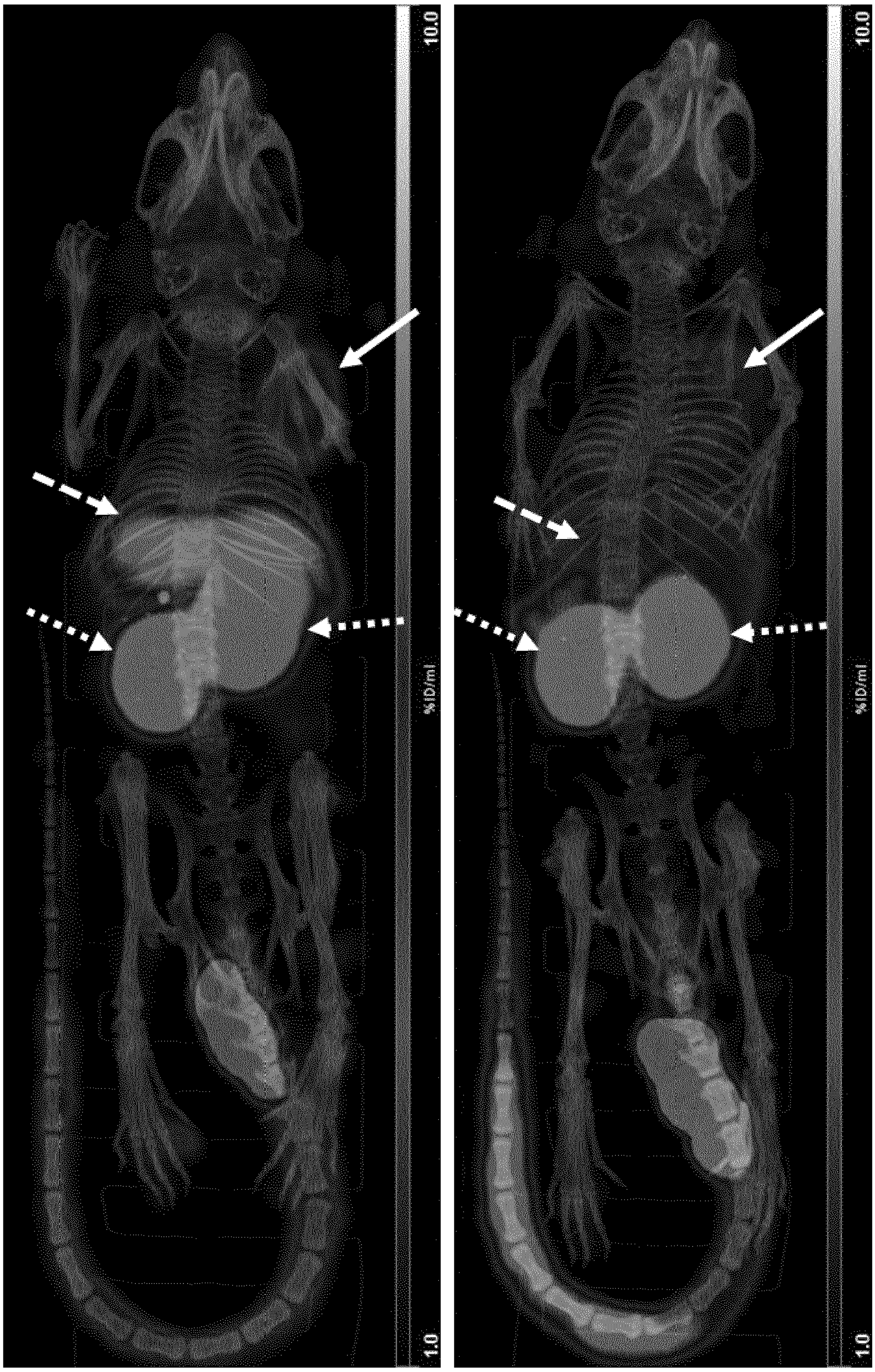


Figure 4

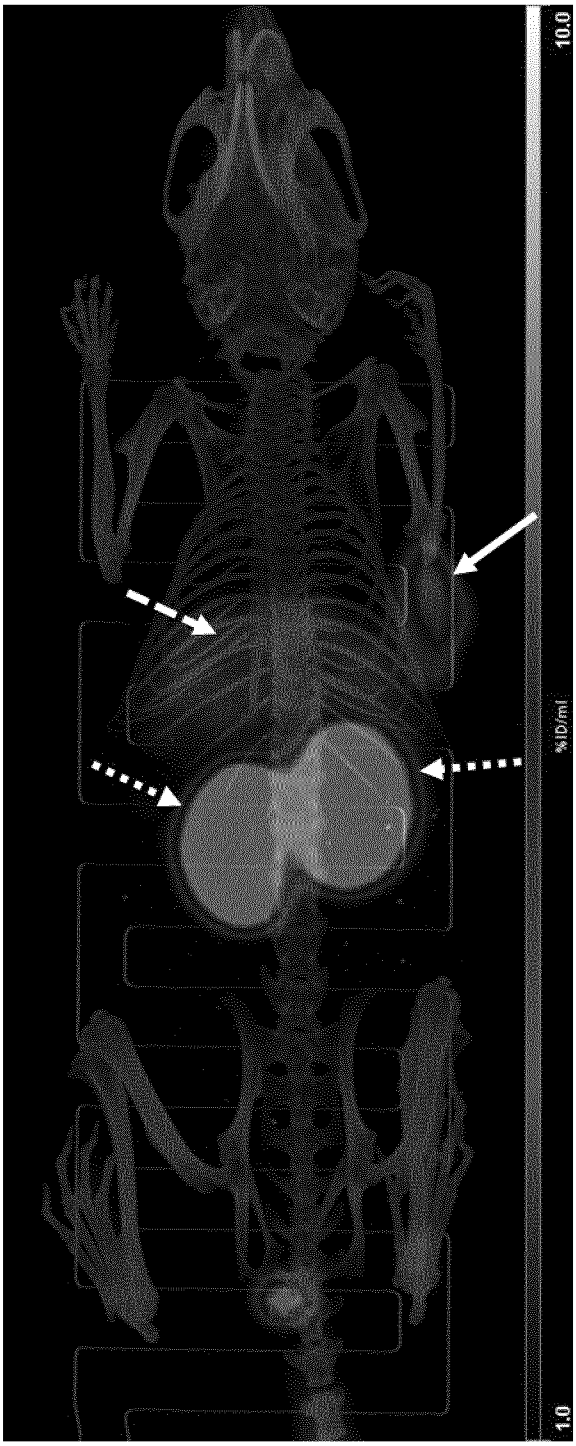


Figure 5

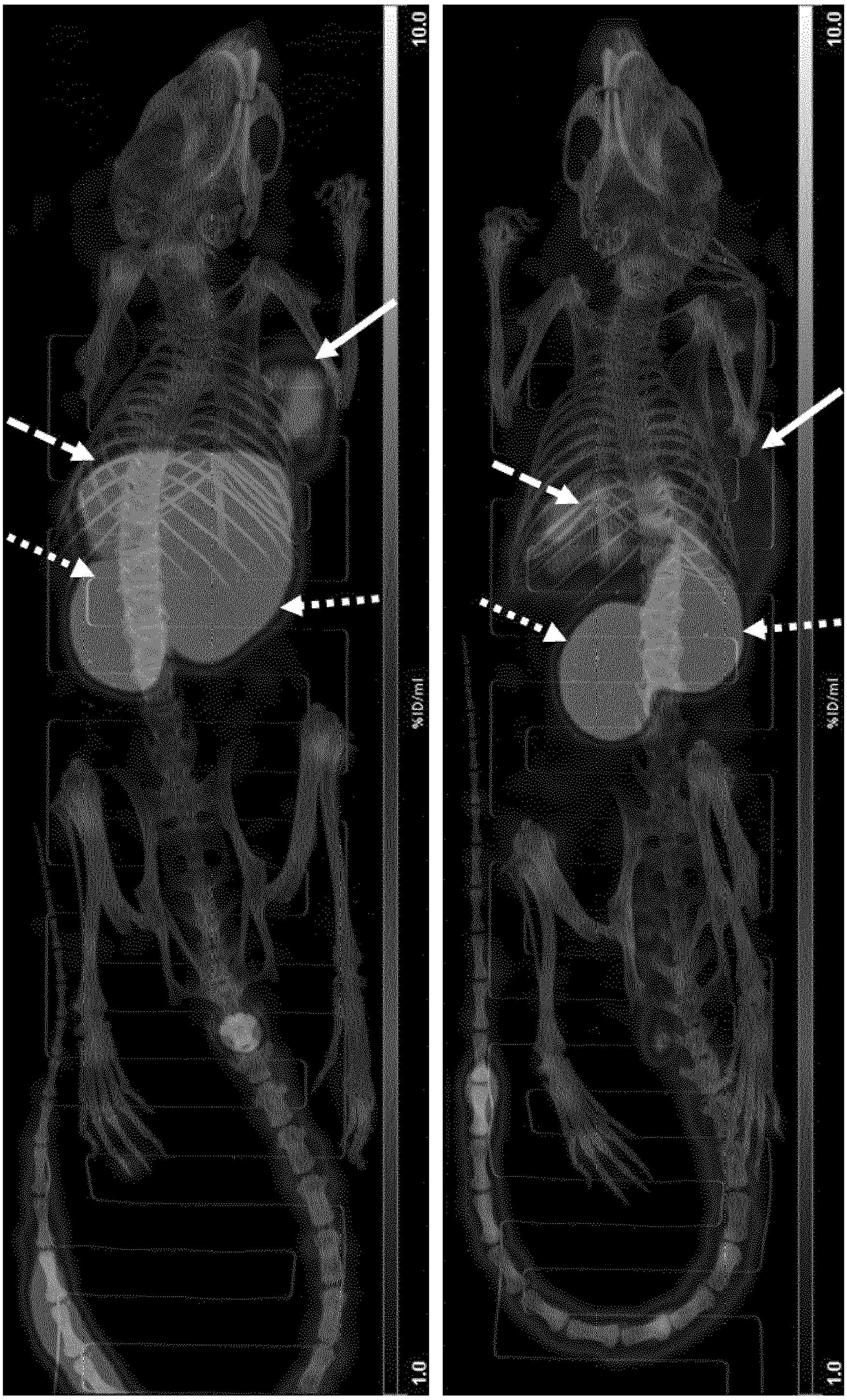


Figure 6

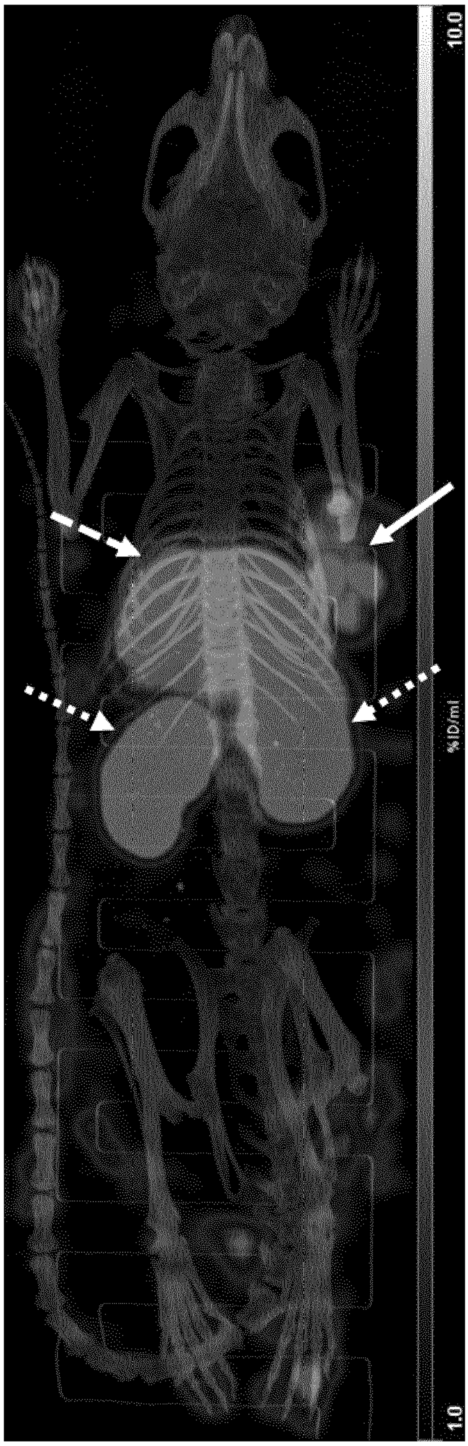


Figure 7

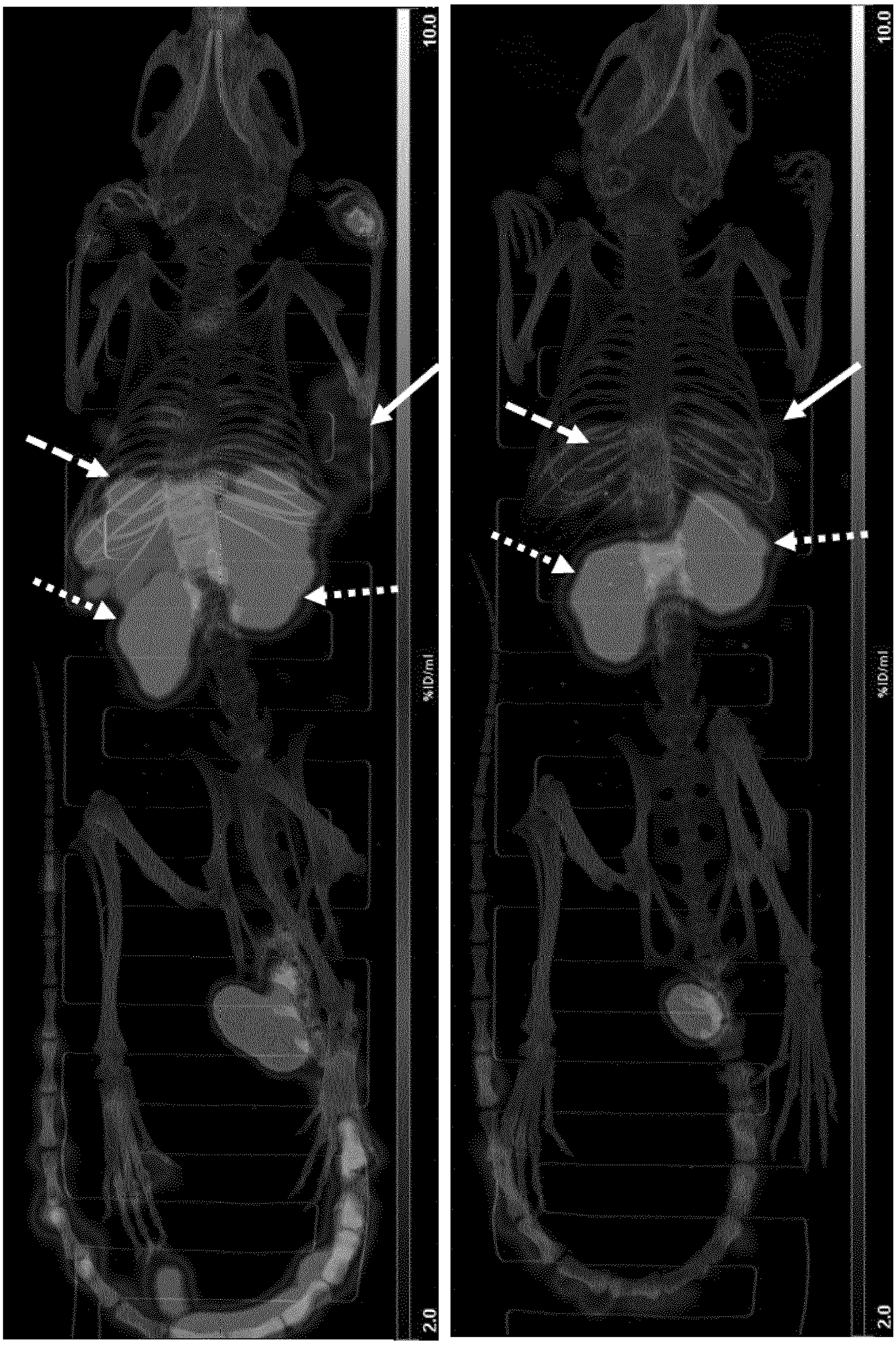


Figure 8

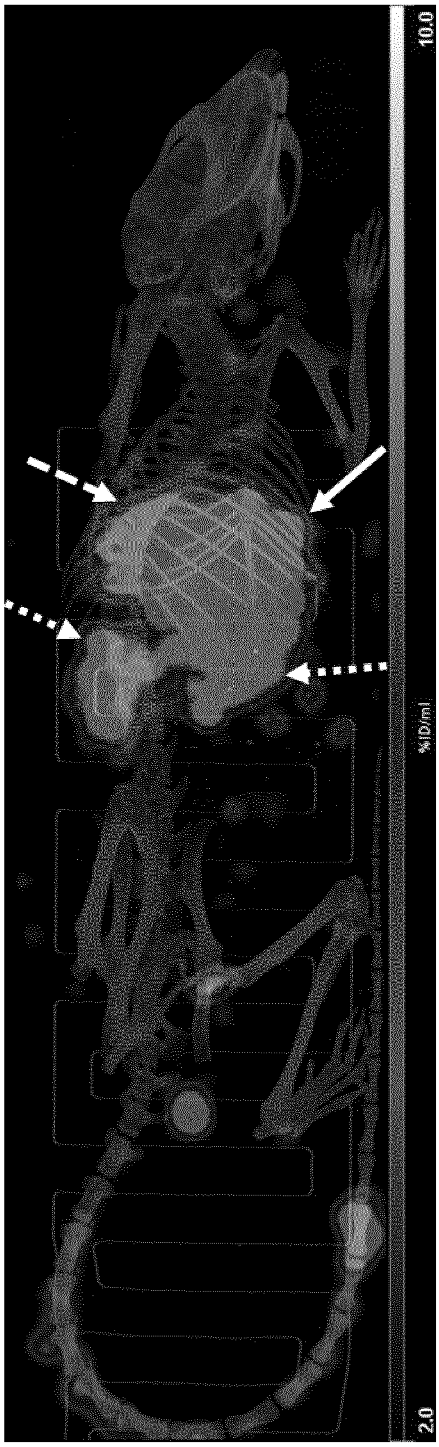


Figure 9

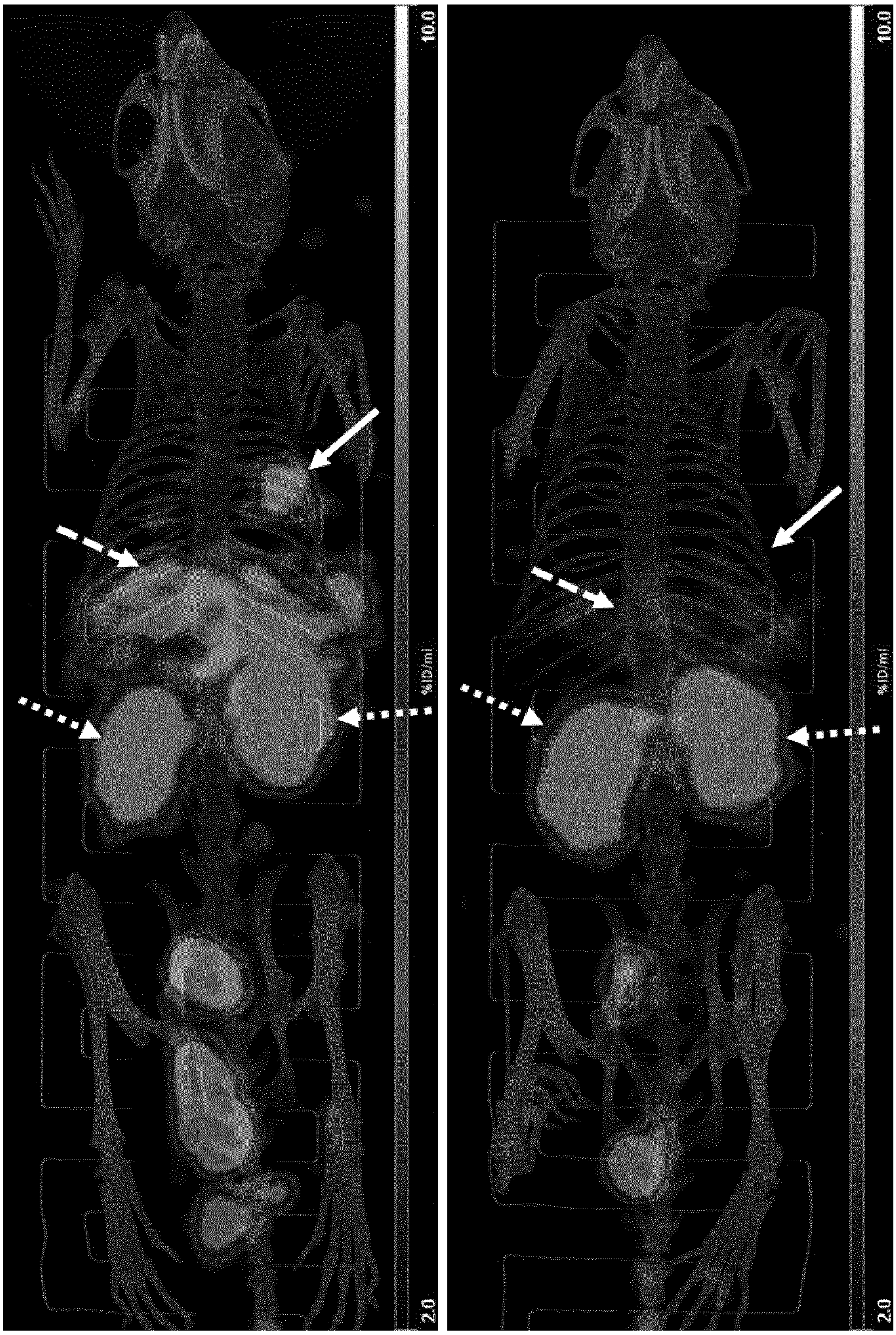


Figure 10

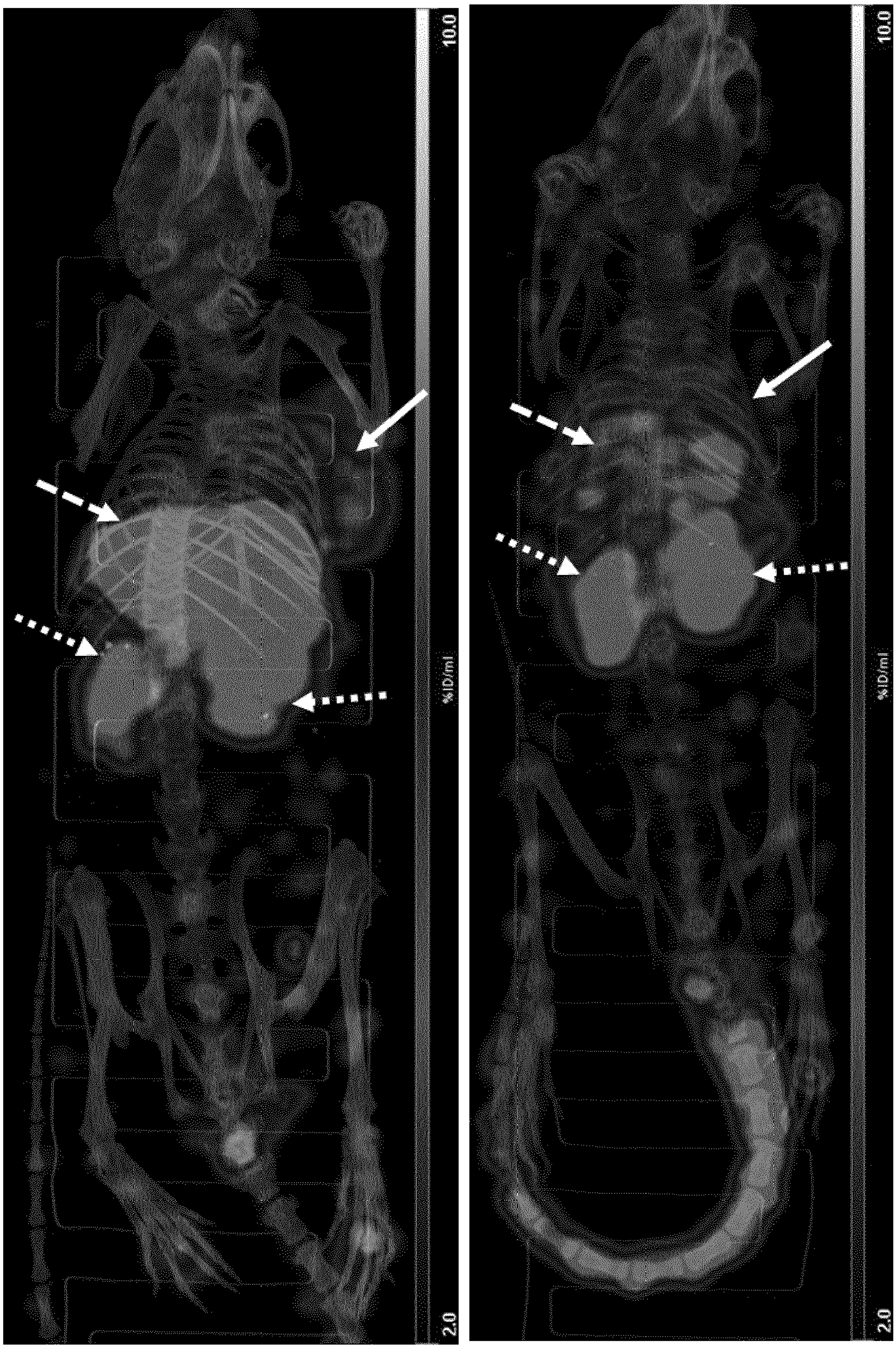


Figure 11

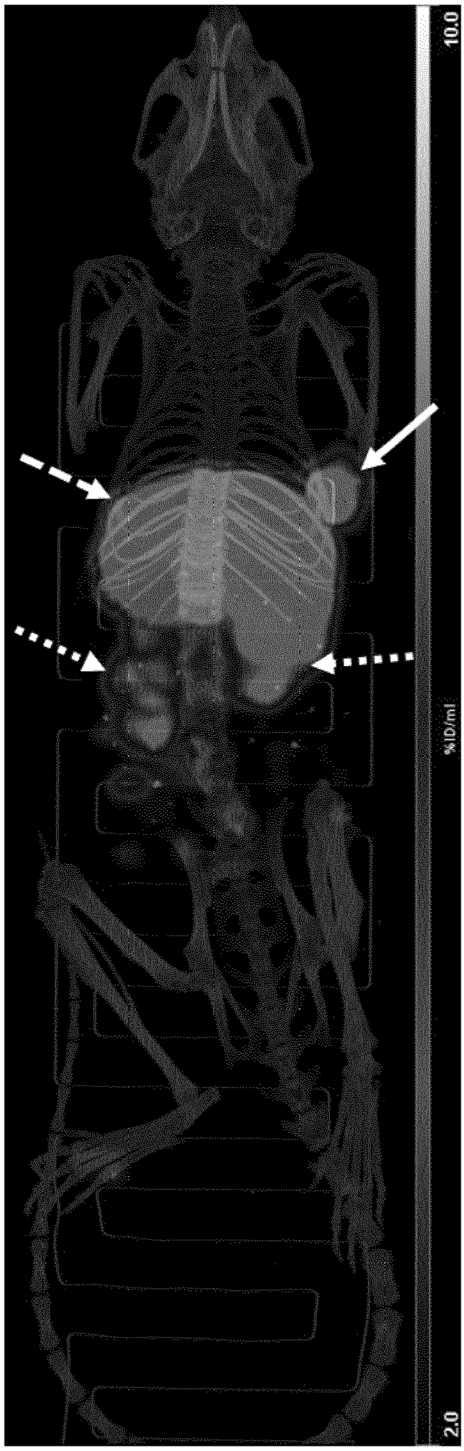


Figure 12

Europäisches Patentamt
European Patent Office
Office européen des brevets

# EUROPEAN SEARCH REPORT

Application Number
EP 21 15 7225

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>Y | WO 2020/053255 A1 (UNIV MUENCHEN TECH [DE]) 19 March 2020 (2020-03-19)<br>* claims 1, 9, 10, 12,13, formula (I)-(III); page 13, line 9 to page 15, line 2; page 59, lines 2ff *<br>----- | 1-15<br>6 | INV.<br>A61K51/08<br>A61P35/00<br>A61K101/02<br>A61K103/00<br>A61K103/30 |
| A | Theresa Osl: "Development of cyclic pentapeptide ligands for chemokine receptor targeting",<br>,<br>1 January 2017 (2017-01-01), XP055626839, Retrieved from the Internet: URL:http://mediatum.ub.tum.de/doc/1342124/1342124.pdf<br>* the whole document * | 1-15 | |
| A | -& Osl ET AL: "Elektronische Prüfungsarbeiten",<br>,<br>15 March 2018 (2018-03-15), XP055626918, Retrieved from the Internet: URL:http://mediatum.ub.tum.de/print/1342124.pdf<br>[retrieved on 2019-09-27]<br>* the whole document *<br>----- | 1-15 | TECHNICAL FIELDS SEARCHED (IPC)<br>A61K |

-/--

1 The present search report has been drawn up for all claims

| Place of search | Date of completion of the search | Examiner |
|---|---|---|
| The Hague | 29 July 2021 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 15 7225

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | OSL THERESA ET AL: "A new class of PentixaFor- and PentixaTher-based theranostic agents with enhanced CXCR4-targeting efficiency", THERANOSTICS, vol. 10, no. 18, 1 January 2020 (2020-01-01), pages 8264-8280, XP055827484, AU ISSN: 1838-7640, DOI: 10.7150/thno.45537 Retrieved from the Internet: URL:https://www.thno.org/v10p8264.pdf> * figure 1; paragraph bridging the columns of page 8271 * ----- | 6 | |
| A | CN 102 626 522 B (HAN YANJIANG) 10 September 2014 (2014-09-10) * the whole document * ----- | 1-15 | |
| A,D | WO 2015/185162 A1 (TECH UNIVERSITÄT MÜNCHEN [DE]) 10 December 2015 (2015-12-10) * the whole document * ----- | 1-15 | TECHNICAL FIELDS SEARCHED (IPC) |
| A,D | WO 2011/131735 A1 (UNIV MUENCHEN TECH [DE]; DEMMER OLIVER [DE] ET AL.) 27 October 2011 (2011-10-27) * the whole document * ----- | 1-15 | |

The present search report has been drawn up for all claims

1

| Place of search | Date of completion of the search | Examiner |
|---|---|---|
| The Hague | 29 July 2021 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 15 7225

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2021

| Patent document cited in search report | Publication date | Patent family member(s) | Publication date |
|---|---|---|---|
| WO 2020053255 A1 | 19-03-2020 | EA 202190755 A1 | 11-06-2021 |
| | | WO 2020053255 A1 | 19-03-2020 |
| CN 102626522 B | 10-09-2014 | NONE | |
| WO 2015185162 A1 | 10-12-2015 | AU 2014396468 A1 | 01-12-2016 |
| | | CN 106573959 A | 19-04-2017 |
| | | EP 3152226 A1 | 12-04-2017 |
| | | HU E044571 T2 | 28-11-2019 |
| | | PL 3152226 T3 | 31-10-2019 |
| | | US 2018037608 A1 | 08-02-2018 |
| | | WO 2015185162 A1 | 10-12-2015 |
| WO 2011131735 A1 | 27-10-2011 | EP 2380596 A1 | 26-10-2011 |
| | | EP 2560690 A1 | 27-02-2013 |
| | | US 2013129622 A1 | 23-05-2013 |
| | | WO 2011131735 A1 | 27-10-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8410059 B2
- US 8765683 B2
- WO 2008150689 A1
- WO 2012118124 A1
- WO 2007096662 A
- WO 2009027706 A
- WO 2011131735 A
- WO 2015185162 A
- WO 2020053255 A

### Non-patent literature cited in the description


- **ZLOTNIK A ; YOSHIE O.** Chemokines. *Immunity,* 2000, vol. 12, 121-127
- **DOMANSKA UM ; KRUIZINGA RC ; NAGENGAST WB et al.** A review on CXCR4/CXCL12 axis in oncology: No place to hide. *Eur J Cancer.,* 2013, vol. 49, 219-230
- **FENG Y ; BRODER CC ; KENNEDY PE ; BERGER EA.** HIV-1 entry cofactor: Functional cDNA cloning of a seven-transmembrane, G protein-coupled receptor. *Science,* 1996, vol. 272, 872-877
- **NAGASAWA T ; HIROTA S ; TACHIBANA K et al.** Defects of B-cell lymphopoiesis and bone-marrow myelopoiesis in mice lacking the CXC chemokine PBSF/SDF-1. *Nature,* 1996, vol. 382, 635-638
- Chemokines and Their Receptors in Lymphocyte Traffic and HIV Infection. **LOETSCHER P ; MOSER B ; BAGGIOLINI M.** Advances in Immunology. Elsevier, 1999, vol. 74, 127-180
- **AIUTI A ; WEBB IJ ; BLEUL C ; SPRINGER T ; GUTIERREZ-RAMOS JC.** The chemokine SDF-1 is a chemoattractant for human CD34+ hematopoietic progenitor cells and provides a new mechanism to explain the mobilization of CD34+ progenitors to peripheral blood. *J Exp Med.,* 1997, vol. 185, 111-120
- **BURGER JA ; BURGER M ; KIPPS TJ.** Chronic lymphocytic leukemia B cells express functional CXCR4 chemokine receptors that mediate spontaneous migration beneath bone marrow stromal cells. *Blood,* 1999, vol. 94, 3658-3667
- **MÜLLER A ; HOMEY B ; SOTO H et al.** Involvement of chemokine receptors in breast cancer metastasis. *Nature,* 2001, vol. 410, 50-56
- **BURGER JA ; KIPPS TJ.** CXCR4: A key receptor in the crosstalk between tumor cells and their microenvironment. *Blood,* 2006, vol. 107, 1761-1767
- **CHATTERJEE S ; BEHNAM AZAD B ; NIMMAGADDA S.** The intricate role of CXCR4 in cancer. *Adv Cancer Res.,* 2014, vol. 124, 31-82
- **GUO F ; WANG Y ; LIU J ; MOK SC ; XUE F ; ZHANG W.** CXCL12/CXCR4: A symbiotic bridge linking cancer cells and their stromal neighbors in oncogenic communication networks. *Oncogene,* 2016, vol. 35, 816-826
- **ORIMO A ; GUPTA PB ; SGROI DC et al.** Stromal fibroblasts present in invasive human breast carcinomas promote tumor growth and angiogenesis through elevated SDF-1/CXCL12 secretion. *Cell,* 2005, vol. 121, 335-348
- **TAMAMURA H ; XU Y ; HATTORI T et al.** A low-molecular-weight inhibitor against the chemokine receptor CXCR4: A strong anti-HIV peptide T140. *Biochem Biophys Res Commun,* 1998, vol. 253, 877-882
- **TAMAMURA H ; KURODA M ; MASUDA M et al.** A comparative study of the solution structures of tachyplesin I and a novel anti-HIV synthetic peptide, T22 ([Tyr5,12, Lys7]-polyphemusin II), determined by nuclear magnetic resonance. *Biochimica et Biophysica Acta (BBA) - Protein Structure and Molecular Enzymology,* 1993, vol. 1163, 209-216
- **TAMAMURA H ; WAKI M ; IMAI M et al.** Downsizing of an HIV-cell fusion inhibitor, T22 ([Tyr 5, 12 , Lys 7]-Polyphemusin II), with the maintenance of anti-HIV activity and solution structure 1. *Bioorganic & Medicinal Chemistry,* 1998, vol. 6, 473-479
- **TAMAMURA H ; HIRAMATSU K ; MIZUMOTO M et al.** Enhancement of the T140-based pharmacophores leads to the development of more potent and bio-stable CXCR4 antagonists. *Org Biomol Chem.,* 2003, vol. 1, 3663-3669
- **GEORGE GPC ; STEVENS E ; ABERG O et al.** Preclinical evaluation of a CXCR4-specific (68)Ga-labelled TN14003 derivative for cancer PET imaging. *Bioorganic & Medicinal Chemistry,* 2014, vol. 22, 796-803

- **JACOBSON O ; WEISS ID ; KIESEWETTER DO ; FARBER JM ; CHEN X.** PET of tumor CXCR4 expression with 4-18F-T140. *J Nucl Med.,* 2010, vol. 51, 1796-1804
- **YAN X ; NIU G ; WANG Z et al.** Al18FNOTA-T140 Peptide for Noninvasive Visualization of CXCR4 Expression. *Mol Imaging Biol.,* 2016, vol. 18, 135-142
- **PENG S-B ; ZHANG X ; PAUL D et al.** Inhibition of CXCR4 by LY2624587, a Fully Humanized Anti-CXCR4 Antibody Induces Apoptosis of Hematologic Malignancies. *PLoS ONE,* 2016, vol. 11, e0150585
- **PORTELLA L ; VITALE R ; LUCA S DE et al.** Preclinical development of a novel class of CXCR4 antagonist impairing solid tumors growth and metastases. *PLoS ONE,* 2013, vol. 8, e74548
- **TSUTSUMI H ; TANAKA T ; OHASHI N et al.** Therapeutic potential of the chemokine receptor CXCR4 antagonists as multifunctional agents. *Biopolymers,* 2007, vol. 88, 279-289
- **TAMAMURA H ; ARAKI T ; UEDA S et al.** Identification of novel low molecular weight CXCR4 antagonists by structural tuning of cyclic tetrapeptide scaffolds. *J Med Chem.,* 2005, vol. 48, 3280-3289
- **TAMAMURA H ; ESAKA A ; OGAWA T et al.** Structure-activity relationship studies on CXCR4 antagonists having cyclic pentapeptide scaffolds. *Org Biomol Chem.,* 2005, vol. 3, 4392-4394
- **TANAKA T ; NOMURA W ; NARUMI T et al.** Structure-activity relationship study on artificial CXCR4 ligands possessing the cyclic pentapeptide scaffold: The exploration of amino acid residues of pentapeptides by substitutions of several aromatic amino acids. *Org Biomol Chem.,* 2009, vol. 7, 3805-3809
- **NARUMI T ; HAYASHI R ; TOMITA K et al.** Synthesis and biological evaluation of selective CXCR4 antagonists containing alkene dipeptide isosteres. *Org Biomol Chem.,* 2010, vol. 8, 616-621
- **UEDA S ; OISHI S ; WANG Z-X et al.** Structure-activity relationships of cyclic peptide-based chemokine receptor CXCR4 antagonists: Disclosing the importance of side-chain and backbone functionalities. *J Med Chem.,* 2007, vol. 50, 192-198
- **WU B ; CHIEN EYT ; MOL CD et al.** Structures of the CXCR4 chemokine GPCR with small-molecule and cyclic peptide antagonists. *Science,* 2010, vol. 330, 1066-1071
- **DEMMER O ; DIJKGRAAF I ; SCHUMACHER U et al.** Design, synthesis, and functionalization of dimeric peptides targeting chemokine receptor CXCR4. *J Med Chem.,* 2011, vol. 54, 7648-7662
- **TANAKA T ; NOMURA W ; NARUMI T ; MASUDA A ; TAMAMURA H.** Bivalent ligands of CXCR4 with rigid linkers for elucidation of the dimerization state in cells. *J Am Chem Soc.,* 2010, vol. 132, 15899-15901
- **ÁVILA-SÁNCHEZ M ; FERRO-FLORES G ; JIMÉNEZ-MANCILLA N et al.** Synthesis and preclinical evaluation of the 99mTc-/177Lu-CXCR4-L theranostic pair for in vivo chemokine-4 receptor-specific targeting. *J Radioanal Nucl Chem.,* 2020, vol. 324, 21-32
- **VALLEJO-ARMENTA P ; SANTOS-CUEVAS C ; SOTO-ANDONAEGUI J et al.** 99mTc-CXCR4-L for Imaging of the Chemokine-4 Receptor Associated with Brain Tumor Invasiveness: Biokinetics, Radiation Dosimetry, and Proof of Concept in Humans. *Contrast Media Mol Imaging,* 2020, 2525037
- **DEMMER O ; DIJKGRAAF I ; SCHOTTELIUS M ; WESTER H-J ; KESSLER H.** Introduction of functional groups into peptides via N-alkylation. *Org Lett.,* 2008, vol. 10, 2015-2018
- **DEMMER O ; GOURNI E ; SCHUMACHER U ; KESSLER H ; WESTER H-J.** PET imaging of CXCR4 receptors in cancer by a new optimized ligand. *ChemMedChem,* 2011, vol. 6, 1789-1791
- **GOURNI E ; DEMMER O ; SCHOTTELIUS M et al.** PET of CXCR4 expression by a (68)Galabeled highly specific targeted contrast agent. *J Nucl Med.,* 2011, vol. 52, 1803-1810
- **POSCHENRIEDER A ; SCHOTTELIUS M ; SCHWAIGER M ; KESSLER H ; WESTER H-J.** The influence of different metal-chelate conjugates of pentixafor on the CXCR4 affinity. *EJNMMI Res.,* 2016, vol. 6, 36
- **HERRMANN K ; SCHOTTELIUS M ; LAPA C et al.** First-in-Human Experience of CXCR4-Directed Endoradiotherapy with 177Lu- and 90Y-Labeled Pentixather in Advanced-Stage Multiple Myeloma with Extensive Intra- and Extramedullary Disease. *J Nucl Med.,* 2016, vol. 57, 248-251
- **OSL T ; SCHMIDT A ; SCHWAIGER M ; SCHOTTELIUS M ; WESTER H-J.** A new class of PentixaFor- and PentixaTher-based theranostic agents with enhanced CXCR4-targeting efficiency. *Theranostics,* 2020, vol. 10, 8264-8280
- **SCHOTTELIUS M ; KONRAD M ; OSL T ; POSCHENRIEDER A ; WESTER H-J.** An optimized strategy for the mild and efficient solution phase iodination of tyrosine residues in bioactive peptides. *Tetrahedron Letters,* 2015, vol. 56, 6602-6605
- **DEMMER O ; FRANK AO ; HAGN F et al.** Erhöhte CXCR4-Affinität und Anti-HIV-Aktivität eines Peptoids durch Konformationsfixierung. *Angew. Chem.,* 2012, vol. 124, 8234-8237
- **CHATTERJEE J ; GILON C ; HOFFMAN A ; KESSLER H.** N-methylation of peptides: A new perspective in medicinal chemistry. *Acc Chem Res.,* 2008, vol. 41, 1331-1342

- **WEINEISEN M ; SIMECEK J ; SCHOTTELIUS M ; SCHWAIGER M ; WESTER H-J.** Synthesis and preclinical evaluation of DOTAGA-conjugated PSMA ligands for functional imaging and endoradiotherapy of prostate cancer. *EJNMMI Res.,* 2014, vol. 4, 63
- **LOVKOVA L ; WÄNGLER B ; SCHIRRMACHER E et al.** para-Functionalized aryl-di-tert-butylfluorosilanes as potential labeling synthons for (18)F radiopharmaceuticals. *Chemistry,* 2009, vol. 15, 2140-2147
- **KOSTIKOV AP ; LOVKOVA L ; CHIN J et al.** N-(4-(di-tert-butyl[18F]fluorosilyl)benzyl)-2-hydroxy-N,N-dimethylethylammonium bromide ([18F]SiFAN+Br-): A novel lead compound for the development of hydrophilic SiFA-based prosthetic groups for 18F-labeling. *Journal of Fluorine Chemistry.,* 2011, vol. 132, 27-34
- **ABRAMS MJ ; JUWEID M ; TENKATE CL et al.** Technetium-99m-human polyclonal IgG radiolabeled via the hydrazino nicotinamide derivative for imaging focal sites of infection in rats. *J Nucl Med.,* 1990, vol. 31, 2022-2028
- **JOYARD Y ; BISCHOFF L ; LEVACHER V ; PAPAMICAEL C ; VERA P ; BOHN P.** Synthesis and Stability Evaluation of New HYNIC Derivatives as Ligands for Technetium-99m. *LOC,* 2014, vol. 11, 208-214
- **SCHOTTELIUS M ; SCHWAIGER M ; WESTER H-J.** Rapid and high-yield solution-phase synthesis of DOTA-Tyr3-octreotide and DOTA-Tyr3-octreotate using unprotected DOTA. *Tetrahedron Letters,* 2003, vol. 44, 2393-2396
- **SCHOTTELIUS M ; OSL T ; POSCHENRIEDER A et al.** 177Lupentixather: Comprehensive Preclinical Characterization of a First CXCR4-directed Endoradiotherapeutic Agent. *Theranostics,* 2017, vol. 7, 2350-2362
- **ROBU S ; SCHOTTELIUS M ; EIBER M et al.** Preclinical Evaluation and First Patient Application of 99mTc-PSMA-I&S for SPECT Imaging and Radioguided Surgery in Prostate Cancer. *J Nucl Med.,* 2017, vol. 58, 235-242
- **KUZMANOVSKA S ; VASKOVA O ; ZDRAVESKA KOCOVSKA M.** In-house'' preparation of 99mTc-EDDA/HYNIC-TOC, a specific targeting agent for somatostatin receptor scintigraphy. *Maced. Pharm. Bull.,* 2011, vol. 57, 65-70
- **WÄNGLER C ; NIEDERMOSER S ; CHIN J et al.** One-step (18)F-labeling of peptides for positron emission tomography imaging using the SiFA methodology. *Nat Protoc.,* 2012, vol. 7, 1946-1955
- **OTHMAN MFB ; MITRY NR ; LEWINGTON VJ ; BLOWER PJ ; TERRY SYA.** Re-assessing gallium-67 as a therapeutic radionuclide. *Nucl Med Biol.,* 2017, vol. 46, 12-18
- **YAMAZAKI K ; KANAOKA M.** Computational prediction of the plasma protein-binding percent of diverse pharmaceutical compounds. *J Pharm Sci.,* 2004, vol. 93, 1480-1494